# EUROPEAN PATENT APPLICATION

(11) **EP 3 613 738 A1**
(43) Date of publication of application: **26.02.2020**
(21) Application number: 18190603.3
(22) Date of filing: 23.08.2018
(51) Int. Cl.: C07D 471/04

(54) **4-SUBSTITUTED PYRROLO[2,3-B]PYRIDINE AS ERBB MODULATORS USEFUL FOR TREATING CANCER**

(71) Applicant: Lead Discovery Center GmbH, 44227 Dortmund (DE)
(72) Inventor: SCHULTZ-FADEMRECHT, Carsten, 44309 Dortmund (DE); KLEBL, Bert, 44227 Dortmund (DE); NUSSBAUMER, Peter, 44227 Dortmund (DE); DEGENHART, Carsten, 44225 Dortmund (DE); BAUMANN, Matthias, 44267 Dortmund (DE)
(74) Representative: Arth, Hans-Lothar

(57) **Abstract**

The present invention relates to certain 4-substituted 1*H*-pyrrolo[2,3-b]pyridine compounds of the formula (I) and pharmaceutically acceptable salts thereof. These compounds is useful in the treatment or prevention of a disease or medical condition mediated through certain mutated forms of ErbB receptor, especially of Exon20 Her2 and EGFR mutations.

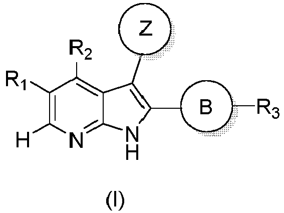

## Description

The present invention relates to certain 4-substituted 1*H*-pyrrolo[2,3-b]pyridine compounds of the formula (I) and pharmaceutically acceptable salts thereof. These compounds is useful in the treatment or prevention of a disease or medical condition mediated through certain mutated forms of ErbB receptor, especially of Exon20 Her2 and EGFR mutations.

### Field of the Invention

The present invention relates to certain 4-substituted 1*H*-pyrrolo[2,3-b]pyridine compounds of the formula (I) and pharmaceutically acceptable salts thereof. These compounds is useful in the treatment or prevention of a disease or medical condition mediated through certain mutated forms of ErbB receptor, especially of Exon20 Her2 and EGFR mutations.

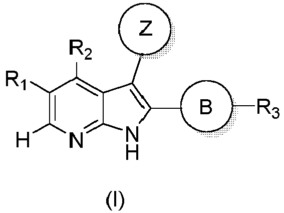

### Background of the Invention

Receptor tyrosine kinases (RTKs) are cell surface receptors that transmit signals from the extracellular environment to control growth, differentiation and survival of cells. Deregulated expression of protein kinases by gene deletion, - mutation or -amplification has been found to be important for tumor initiation and -progression, involving cancer cell proliferation, -survival, -motility and - invasivity as well tumor angiogenesis and chemotherapy resistance. Because of the advanced understanding of their critical role, protein kinases are important targets for novel therapies, especially for cancer (Hananhan et al. Cell 2000, 7, 100(1): 57-70; Blume-Jensen et al., Nature 2001, 17, 411(6835): 355-65).

In humans the receptor tyrosine kinase family ErbB comprises four members: EGFR (Her1), ErbB2 (Her2), ErbB3 (Her3) and ErbB4 (Her4). The binding of a ligand induces conformational change in receptors to form homo- and heterodimerization. The extracellular domain of Her2 is already fixed in a conformation without ligand binding that resembles the other ligand-activated ErbB members and hereby acts as a preferred dimerization partner for other ligand-bound ErbBs. The dimerization of receptors activates the intrinsic kinase activity and hereby yielding to the phosphorylation of its substrates, resulting in activation of multiple downstream pathways within the cell, including the anti-apoptotic/survival PI3K-AKT-mTOR and the mitogenic RAS-RAF-MEK-ERK-MAPK pathways (Chong et al. Nature Med. 2013; 19 (11):1389-1400).

There is strong precedent for the involvement of ErbB kinase family in human cancer as overexpression and/or mutations of ErbB is commonly found in cancers (for example breast, lung, head, neck and bladder). First generation small molecule EGFR inhibitors like Tarceva (Erlotinib) and Iressa (Gefitinib), both binding reversibly to EGFR, are currently first-line therapy for non-small cell lung cancer patients with tumors harbouring EGFR mutations in exon 19 and 21 (like L858R and delE746-A750). Second and third generation small molecule EGFR inhibitors have been designed as irreversible EGFR inhibitors. These compounds (for example Afatinib, HKI-272, CI-1033, EKB-569, WZ-4002, AZ9291, CO-1686) bind irreversibly to EGFR, preferably to cysteine 773.

Approximately 10% of patients with NSCLC in the United States are reported to have tumor-associated EGFR mutations (Lynch et al. N Engl J Med. 2004, 350 (21): 2129-39). The EGFR mutations mostly occur within EGFR Exon 18-21 (Yasuda et al. Sci Transl Med. 2013, 18, 5(216): 216ra177; Leduc et al. Annals of Oncology 2017, 28, 11: 2715-2724; Arcila et al. Mol Cancer Ther. 2013, 12 (2): 220-229). These mutations increase the kinase activity of EGFR, leading to hyperactivation of downstream pro-survival signalling pathways (Pao et al. Nat Rev Cancer 2010, 10: 760-774). EGFR Exon 20 insertions reportedly comprise approximately 4-9.2 % of all EGFR mutant lung tumors (Arcila et al. Mol Cancer Ther. 2013, 12 (2): 220-9; Oxnard et al. J Thorac Oncol. 2013, 8(2): 179-184; Yasuda et al. Lancet Oncol. 2012, 13 (1): e23-31). Most EGFR Exon 20 insertions occur in the region encoding amino acids 767 through 774 of exon 20 within the loop that follows the C-helix of the kinase donmain of EGFR. Analysis of patients with tumors harbouring EGFR Exon 20 insertion mutations mostly displayed progressive disease in the course of treatment with Gefitinib, Erlotinib or Afatinib (Yasuda et al. Lancet Oncol. 2012, 13 (1): e23-31; Yasuda et al. Sci Transl Med. 2013, 18, 5(216): 216ra177).

Her2 mutations are reportedly present in ∼2-4% of NSCLC (Buttitta et al. Int J Cancer. 2006, 119: 2586-2591). The most common mutation is an in-frame insertion within Exon 20. In 83% of patients having Her2 associated NSCLC, a four amino acid YVMA insertion mutation occurs at codon 775 in Exon 20 of Her2 (Arcila et al. Clin Cancer Res 2012, 18: 4910-4918). The Her2 Exon 20 insertion results in increased kinase activity and enhanced signalling through downstream pathways, resulting in increased survival, invasiveness, and tumorgenicity (Wang et al. Cancer Cell 2006; 10: 25-38). Tumors harbouring the Her2 YVMA mutation are largely resistant to known EGFR inhibitors (Arcila et al. Clin Cancer Res 2012, 18: 4910-4918).

It is object of the present invention to provide compounds as mutant-selective ErbB inhibitors, especially for the Exon 20 EGFR/Her2 mutations, and/or pharmaceutically acceptable salts thereof which can be used as pharmaceutically active agents, and use thereof especially for prophylaxis and/ or treatment of cell proliferative disease, especially cancer, a combination comprising the inventive compound and at least one anticancer drug; as well as compositions comprising at least one of those compounds and/or pharmaceutically acceptable salts thereof as pharmaceutically active ingredients

The present invention provides novel compound(s) being mutant-selective ErbB inhibitors, especially for the Exon 20 EGFR/Her2 mutations, and additionally being inhibitors for other mutants like EGFR T790ML858R mutation.

The objective of the present invention is solved by the teaching of the independent claims. Further advantageous features, aspects and details of the invention are evident from the dependent claims, the description, the figures, and the examples of the present application.

### Summary of this invention

The present invention provides a compound that exhibits inhibition of HER2 Exon20 A775_G776insYVMA (and similar mutations) and/or EGFR Exon20 H773_V774insNPH (and similar mutations). The present invention provides compound(s) being mutant-selective ErbB inhibitors, especially for the Exon20 EGFR/Her2 mutations, and additionally being inhibitors for other mutants like EGFR T790ML858R mutation. In certain embodiments the current invention is directed towards a compound that is mutant-specific for Exon20 of EGFR and Her2. In one aspect, the invention provides a compound comprising an irreversible kinase inhibitor. The compound covalently modifies cysteine 773/797 in EGFR/Her2.

The compound of the present invention and/or pharmaceutically acceptable salts thereof as pharmaceutical active ingredient can be used for treatment and/or prophylxis of a cell proliferative disease.

The cell proliferative disease is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, osteosarcoma, melanoma, glioblastoma and neuroblastoma. In an especially preferred embodiment, the disorders are selected from breast cancer, glioblastoma, renal cancer, non-small cell lung cancer (NSCLC), and melanoma. The compounds are also suitable for the prevention and/or treatment of other hyperproliferative disorders, particulary benign hyperproliferative disorders such as benign prostate hyperplasia.

Thus, the present invention is directed to a compound of the formula (I) 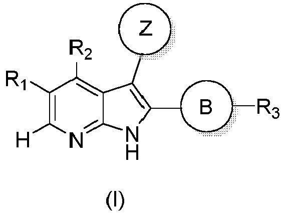 wherein
Z represents 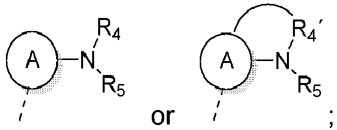
A represents 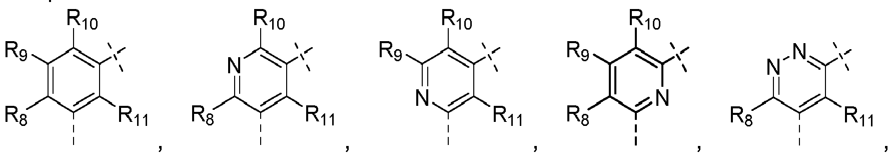 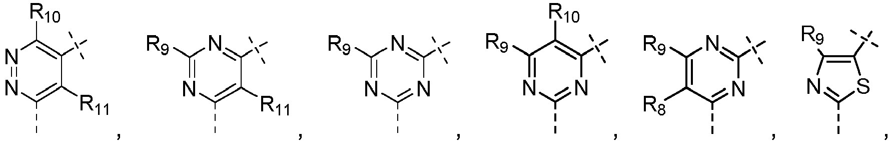 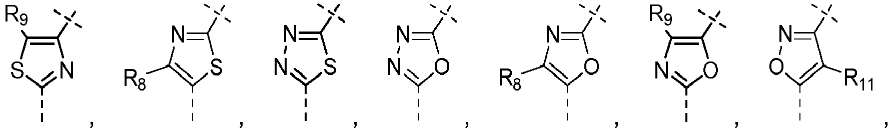 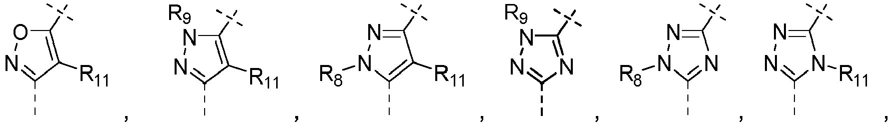 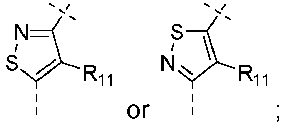 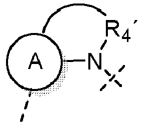 represents 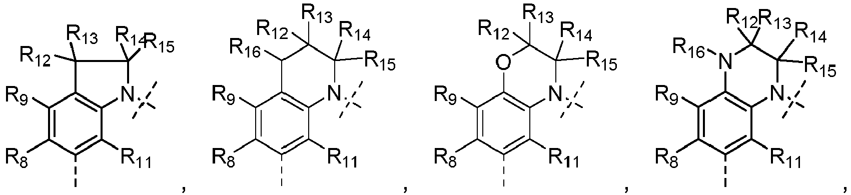 or 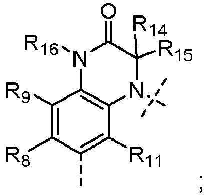
B represents 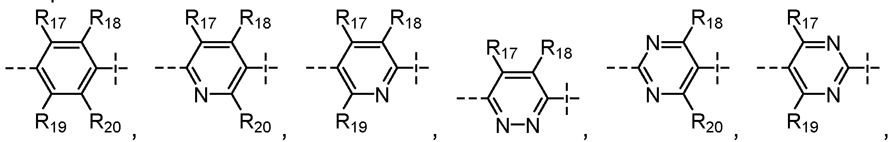 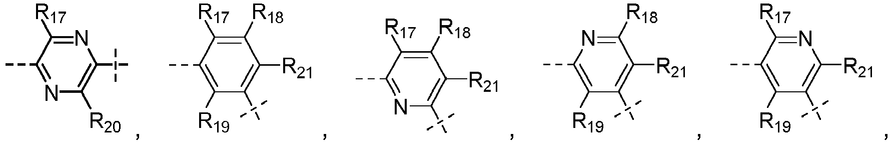 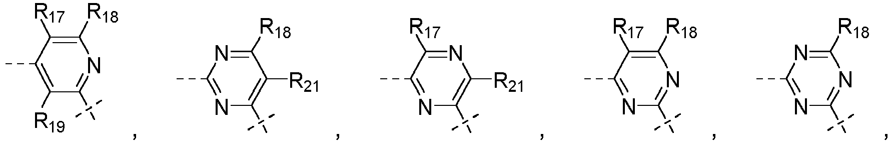 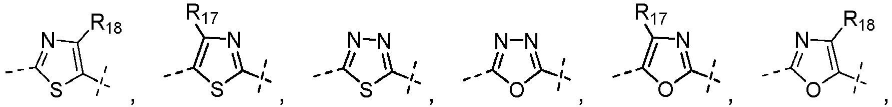 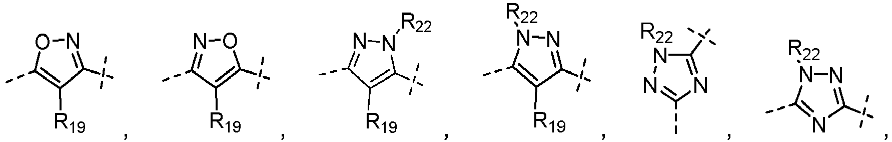 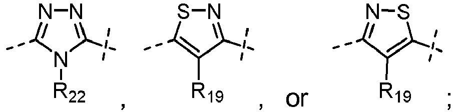
**R₁** represents -H, **-R*,** -CH₂-**R***, -CHR^{a}-**R***, -CR^{a}R^{b}-**R***, -CH₂CH₂-**R***, -CR^{a}R^{b}-CR^{c}R^{d}-**R***, -CH=CH-**R***, -CR^{a}=CR^{b}-**R***, -CH₂CH₂CH₂-**R*** or -CR^{a}R^{b}-CR^{c}R^{d}-CR^{e}R^{f}-**R***;
**R**^{a} - **R**^{f} represent independently of each other -H, -F, -CI, -CH₃, -OCH₃, -CH₂CH₃, -CH₂CH₂CH₃, -CH(CH₃)₂, -CH₂CH(CH₃)₂, or -cyclo-C₃H₅;
**R*** represents -H, -F, -CI, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHSO₂CH₃, -NHSO₂C₂H₅, -NHSO₂C₃H₇, -NHSO₂CH(CH₃)₂, -NHSO₂-cyclo-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclo-C₃H₅, -SO(NH)CH₃, -SO(NH)C₂H₅, -SO(NH)C₃H₇, -SO(NH)CH(CH₃)₂, -SO(NH)-cyclo-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclo-C₃H₅, 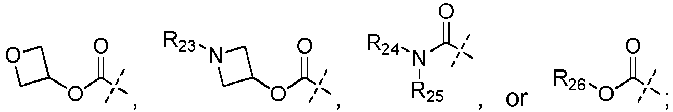
**R₂** represents -H, -F, -CI, -Br, -I, -OH, -CN, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅, or -O-C₂H₄-cyclo-C₃H₅;
**R₃** represents -H, -CH₃, -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇R₂₈, -OCH₂CH₂NR₂₇R₂₈, -OCH₂CH₂CH₂NR₂₇R₂₈, -NR₂₉CH₂NR₂₇R₂₈, -N R₂₉CH₂CH₂N R₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 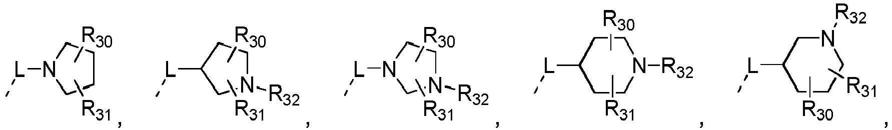 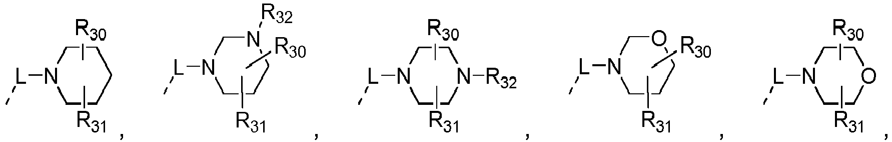 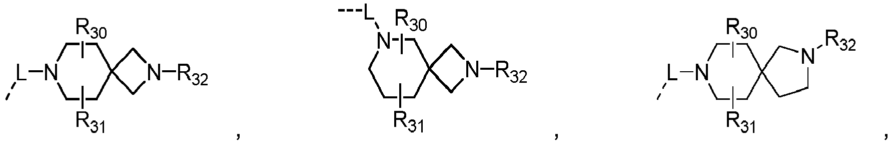 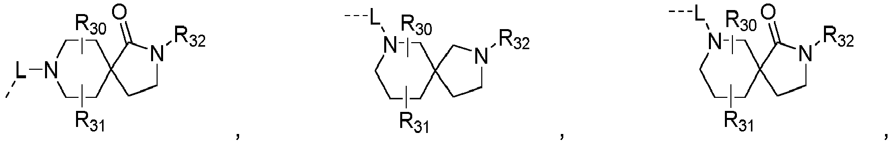 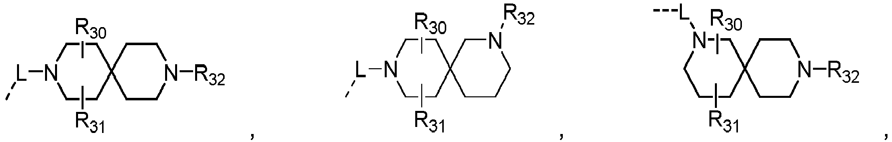 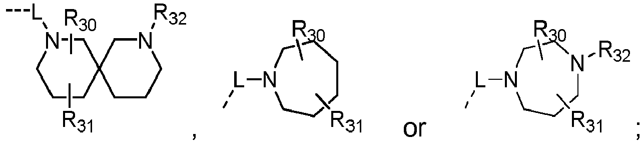
L is a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -CH₂OCO- or -CH₂CH₂OCO-;
**R₄** represents -H, -CH₃, or -C₂H₅;
**R₅** represents 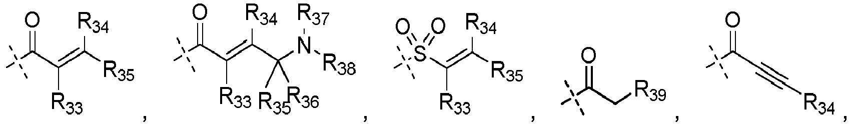 or 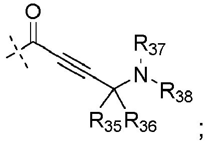
**R₆** and **R₇** represent independently of each other -H, -F, -CH₃, -C₂H₅, -CN, -CF₃, -NO₂, -CHO, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -CH₂NHCH₃, -CH₂NHC₂H₅, -CH₂NHC₃H₇, -CH₂NHCH(CH₃)₂, -CH₂N(CH₃)₂, -CH₂N(C₂H₅)₂, -CH₂N(C₃H₇)₂, -CH₂CH₂NHCH₃, -CH₂CH₂NHC₂H₅, -CH₂CH₂NHC₃H₇, -CH₂CH₂NHCH(CH₃)₂, -CH₂CH₂N(CH₃)₂, - CH₂CH₂N(C₂H₅)₂, or -CH₂CH₂N(C₃H₇)₂;
**R₈** - **R₁₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇,
or **R₈** and **R₉** or **R₉** and **R₁₀** form together -CH₂-, -CH₂CH₂-, -CH₂CH₂CH₂-, -CH₂CH₂CH₂CH₂-, -CH₂OCH₂-, -CH₂OCH₂CH₂-, -OCH₂O-, or -OCH₂CH₂O-;
**R₁₂** - **R₁₆** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₁₇** - **R₂₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₂₂** represents -H, -CH₃, -CF₃, -C₂H₅, -C₃H₇, -COCH₃, -COC2H₅, -SO₂CH₃, or -SO₂C₂H₅;
**R₂₃** represents -H, -CH₃, -CF₃, -C₂H₅, -C₃H₇, -CH₂CH₂OCH₃, or -C₄H₉;
**R₂₄** and **R₂₅** represent independently of each other -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 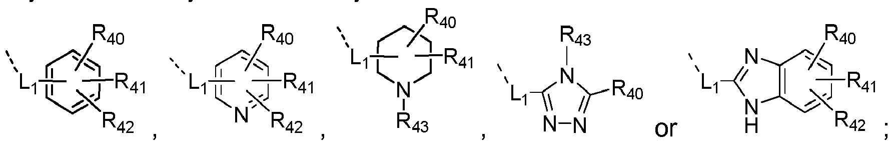
or **R²⁴** and **R²⁵** form together -CH₂-, -CH₂CH₂-, -CH₂CH₂CH₂-, -CH₂CH₂CH₂CH₂-, -CH₂OCH₂-, or -CH₂OCH₂CH₂-;
**L₁** represents a bond, -CH₂-, -CH₂CH₂-, or -CH₂CH₂CH₂-;
**R₂₆** represents -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph, or -CH₂Ph;
**R₂₇** - **R₃₁** represent independently of each other -H, -F, -CI, -OH, -CN, -NH₂, -OCH₃, -OC₂H₅, -OC₃H₇, -OCH(CH₃)₂, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, or -cyclo-C₆H₁₁;
**R₃₂, R₃₇, R₃₈,** and **R₄₃** represent independently of each other -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃, -SO₂C₂H₅, -SO₂CF₃, or -SO₂Ph;
**R₃₃, R₃₄, R₃₅,** and **R₃₆** represent independently of each other -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, or -COOC(CH₃)₃;
**R₃₉** represents -F, -Br, -CI, or -I;
**R₄₀, R₄₁,** and **R₄₂** represent independently of each other -H, -F, -CI, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂, -SO₂CH₃, -SO₂C₂H₅, -SO₂CF₃, -SO₂Ph, -SO₂NH₂, -SO₂NHCH₃, 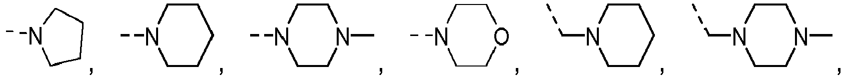 or 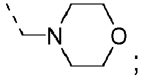
or an enantiomer, a mixture of enantiomers, a diastereomer, a mixture of diastereomers, a tautomer, a prodrug, a hydrate, a solvate, or pharmaceutically acceptable salts thereof.

In the definition of **A** ring, a bond " 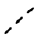 " of **A** ring is connected to C-3 of pyrrolo[2,3-b]pyridine backbone and a bond " 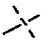 " of **A** ring is connected to nitrogen atom of the group NR₄R₅. Similary, in the definition of the moiety 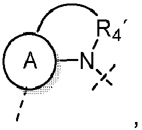 a bond " 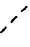 " is connected to C-3 of pyrrolo[2,3-b]pyridine backbone and a bond " 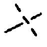 " is connected to the group **R₅.**

**R₄'** represents

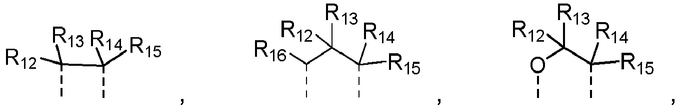

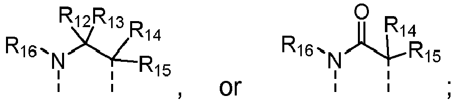

In the definition of **B** ring, a bond " 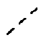 " of **B** ring is connected to C-2 of pyrrolo[2,3-b]pyridine backbone and a bond " 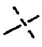 " of **A** ring is connected to the group **R₃.**

Preferred is the compound of the formula (I), wherein
A represents 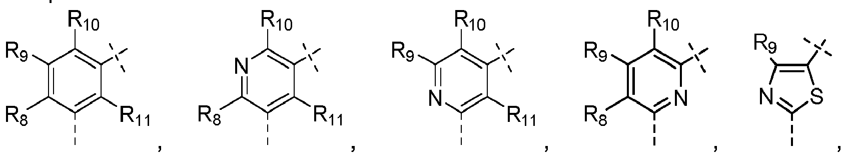 or 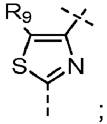 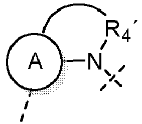 represents 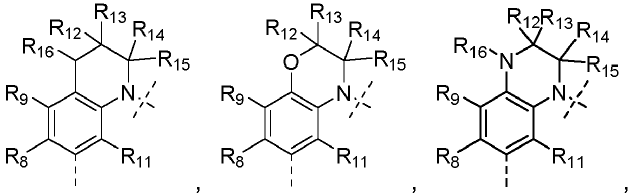 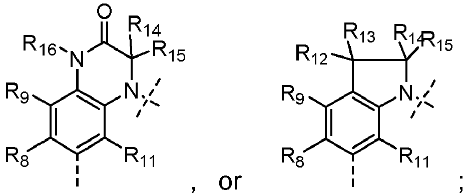
B represents 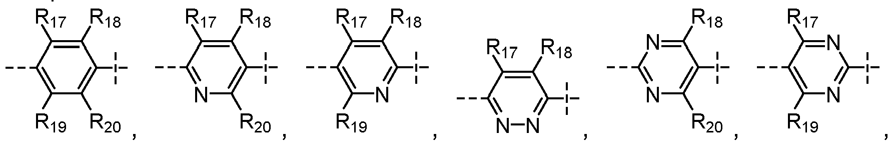 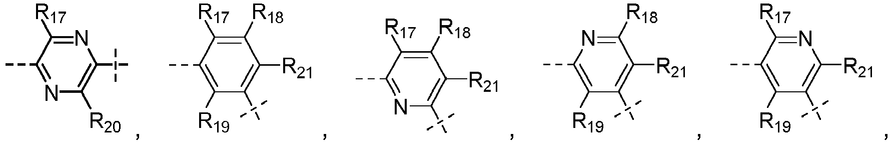 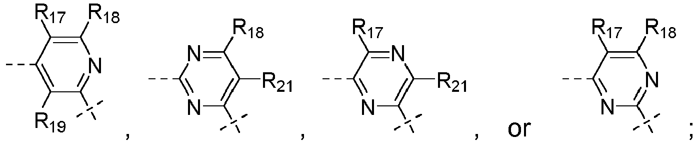
and **R₈** - **R₂₁** have the same meanings as defined herein.

Also preferred is the compound of the formula (I), wherein
**A** represents 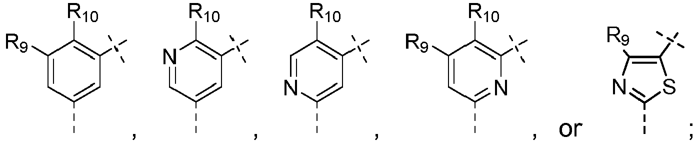 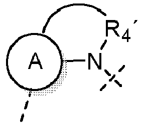 represents 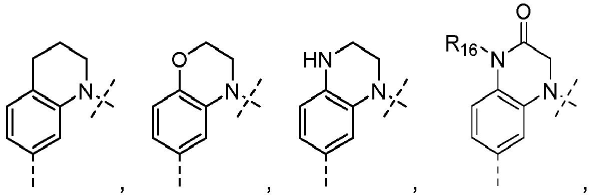 or 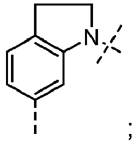
**B** represents 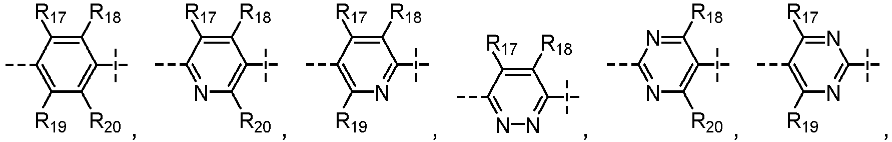 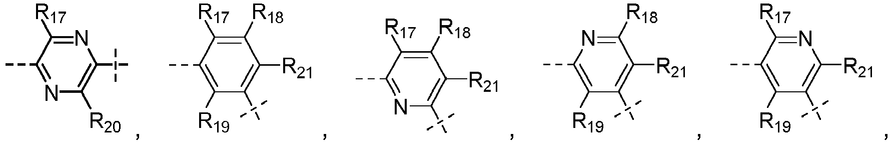 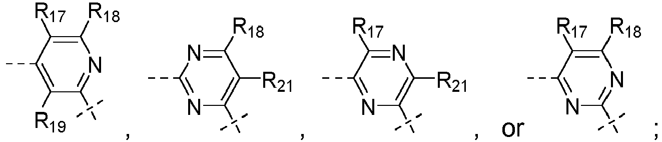
and **R₉ - R₁₀,** and **R₁₆- R₂₁** have the meanings as defined herein.

More preferred is the compound of the formula (I), wherein
**A** represents 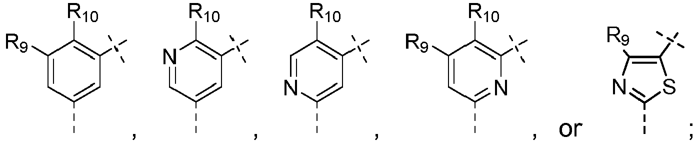 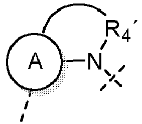 represents 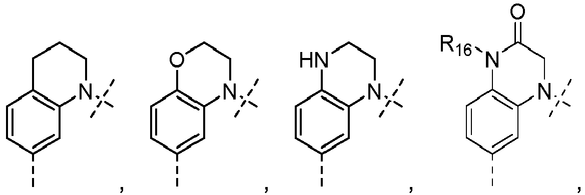 or 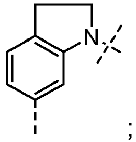
**B** represents 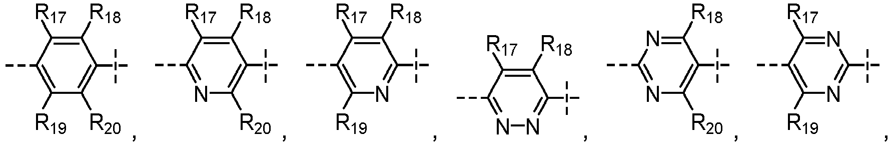 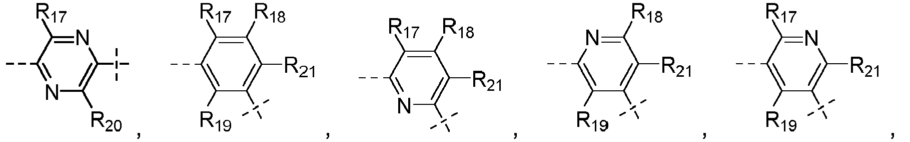 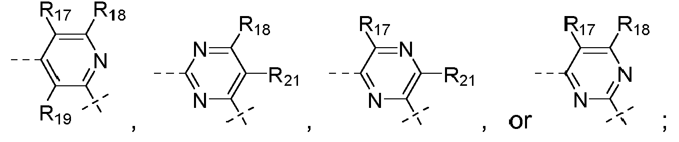
and **R₉** - **R₁₀,** and **R₁₆ - R₂₁** have the meanings as defined herein.

More preferred is the compound of the formula (I), wherein
**A** represents 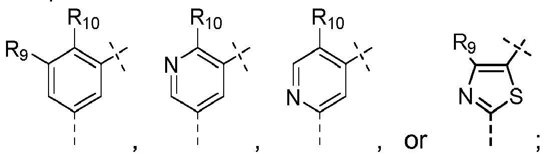 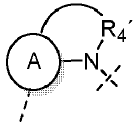 represents 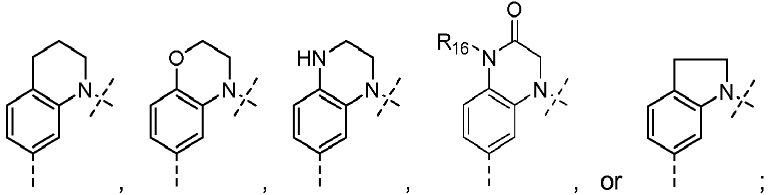
**B** represents 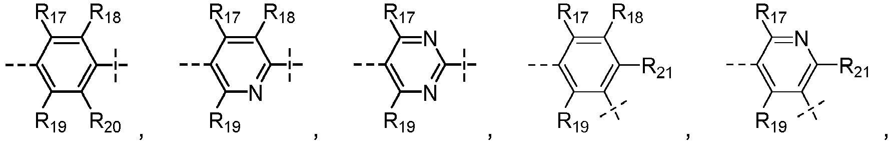 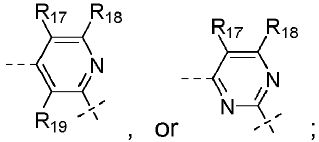
**R₉** - **R₁₀** and **R₁₆ - R₂₁** have the meanings as defined herein.

Still more preferred is the compound of the formula (I), wherein the compound has the formula **(Ia)** or **(Ib):** 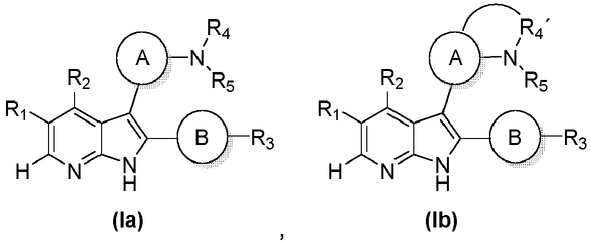 wherein
**A** represents 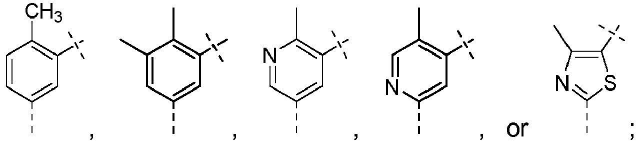 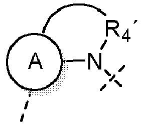 represents 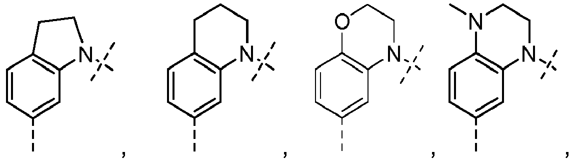 or 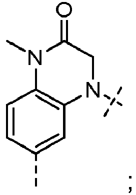
**B** represents 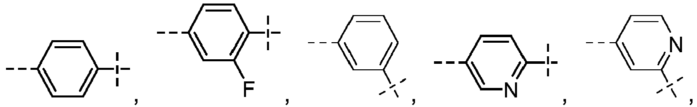 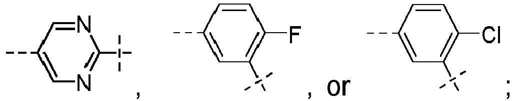
and **R₁, R₂, R₃, R₄,** and **R₅** have the same meanings as defined herein.

Still more preferred is the compound of any one of the formulae **(I), (Ia)** and **(Ib)**, wherein
**Z** represents 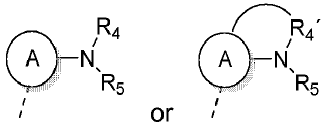 and is selected from 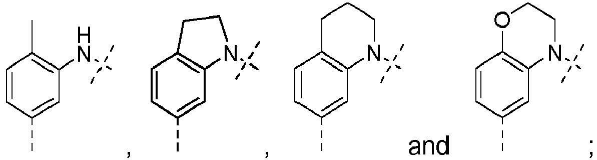
**R₁** represents -H, -CN, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, 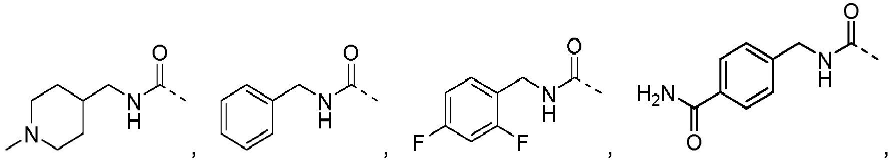 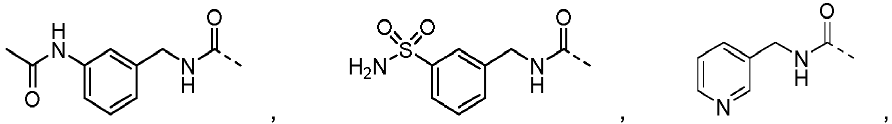 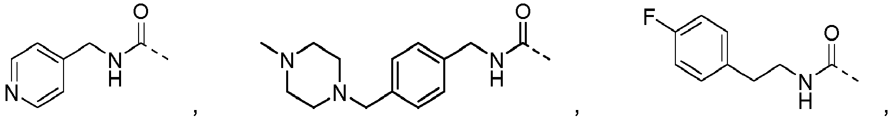 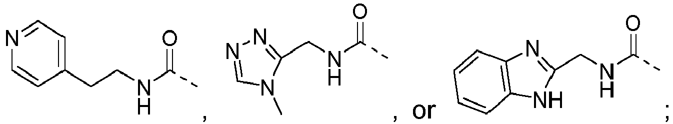
**R₂** represents -H, -CI, -OCH₃, -OC₂H₅, or -OCH(CH₃)₂;
**B** represents 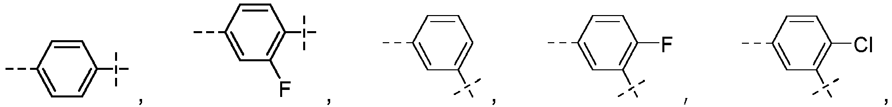 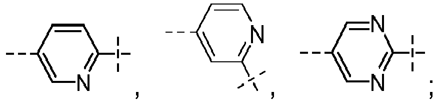
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, 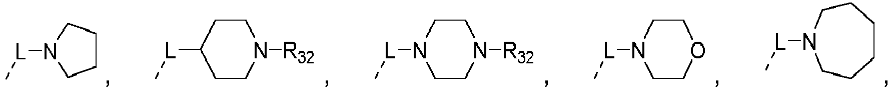 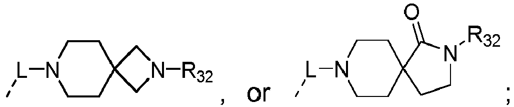
**R₅** represents 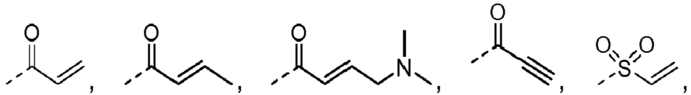 or 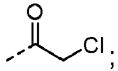
**L** is a bond, -CH₂-, -OCH₂CH₂-, -CO-, -CONH-, or -CONHCH₂CH₂CH₂-; and
**R₃₂** represents -H, -CH₃, -C₂H₅, -CH(CH₃)₂, -CH₂Ph, -COCH₃, or -SO₂C₂H₅.

In certain aspects, the compound described above is a compound of formula **(II)** and (**III**): 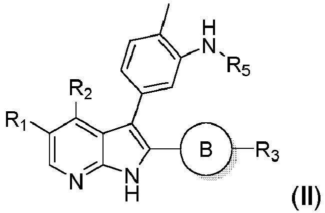 wherein
**B, R₁, R₂, R₃** and **R₅** have the same meanings as defined herein.

Preferred is the compound of the formula **(II),** wherein
**B** represents 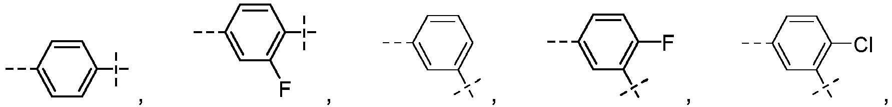 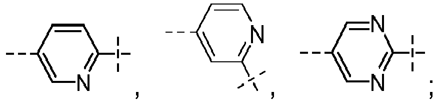
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, 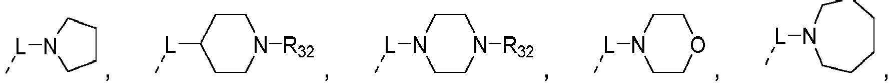 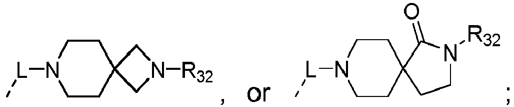
**R₅** represents 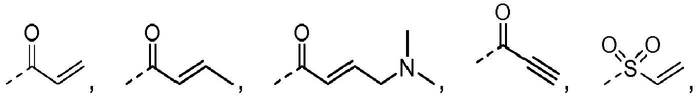 or 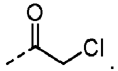 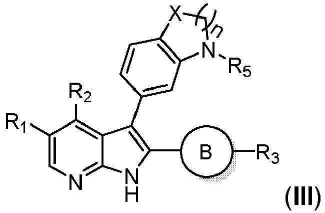 wherein
**R₁, R₂, R₃** and **R₅** have the same meanings as defined above;
**X** represents CH₂, when n is 1, or 2; or
**X** represents O, when n is 2;

Preferred is the compound of the formula **(III),** wherein
**B** represents 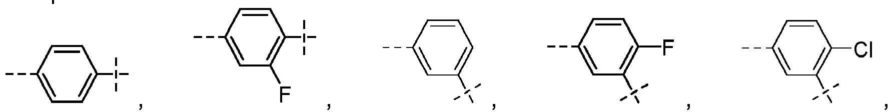 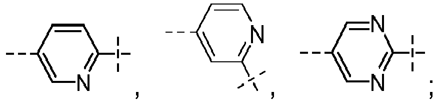
**R₅** represents 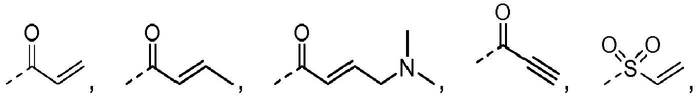 or

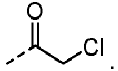

Also preferred is the compound of any one of the formulae **(IIIa), (IIIb)** and **(IIIc**): 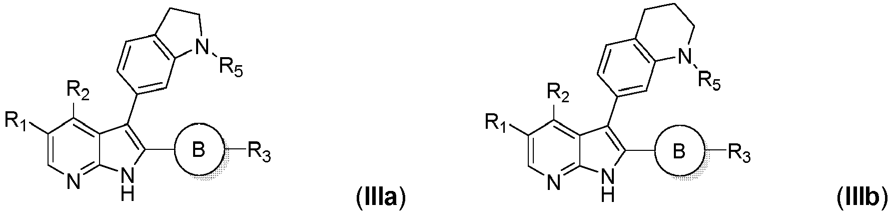 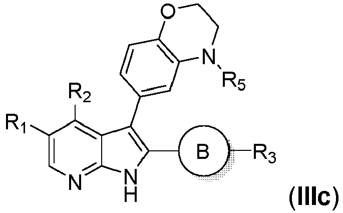 wherein, B, **R₁, R₂, R₃,** and **R₅,** have the same meanings as defined in the formula **(I).**

Still more preferred are the compounds of any one of the formulae **(II-1)** - **(II-5), (IIIa-1)** - **(IIIa-5), (IIIb-1)** - **(IIIb-5)** and **(IIIc-1)** - **(IIIc-5).** 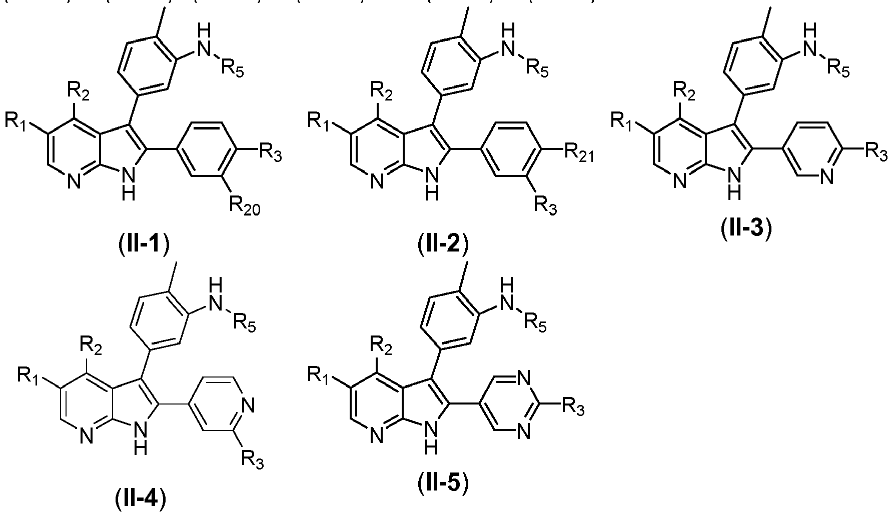 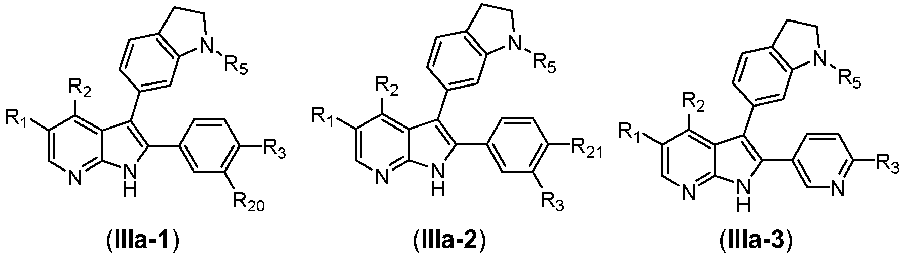 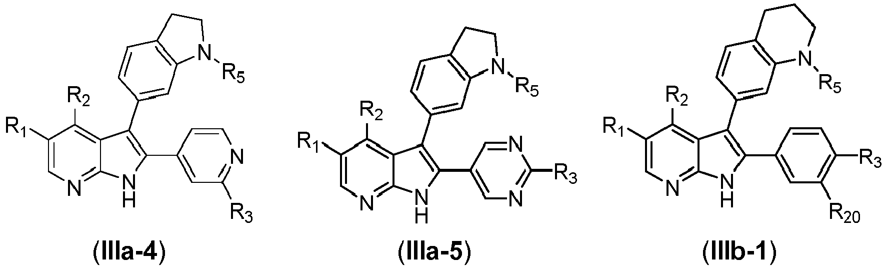 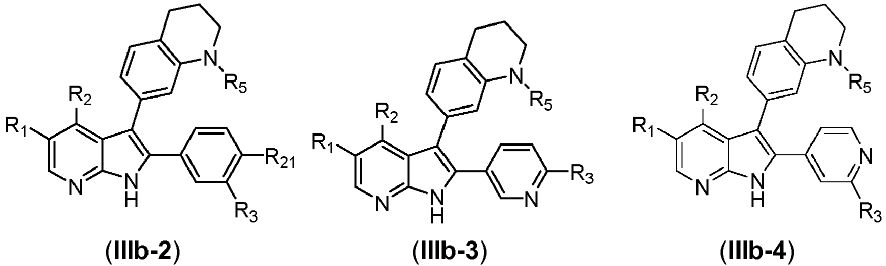 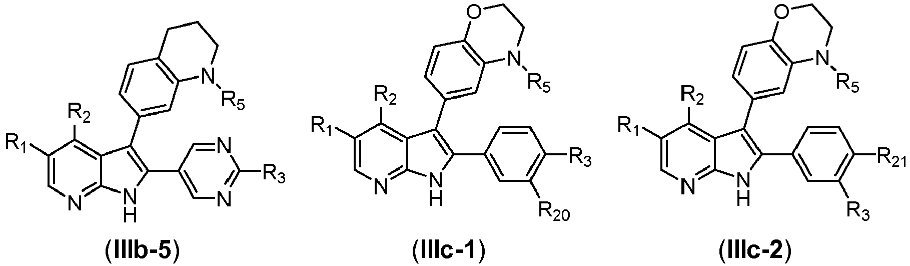 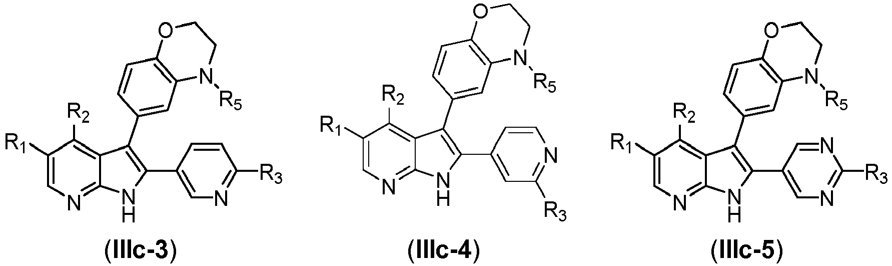 wherein, **R₁, R₂, R₃, R₅, R₂₀** and **R₂₁** have the same meanings as defined in the formula **(I).**

In any one of the formulae **(II-1)** - **(II-5), (IIIa-1)** - **(IIIa-5), (IIIb-1)** - **(IIIb-5), (IIIc-1)** - **(IIIc-5),** preferred, **R₁** represents -H, -CN, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, 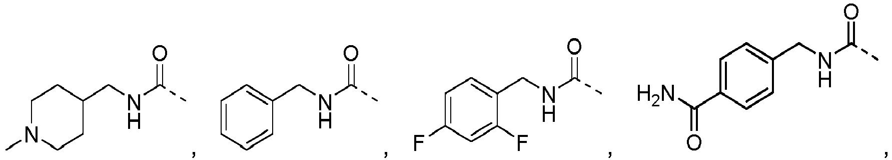 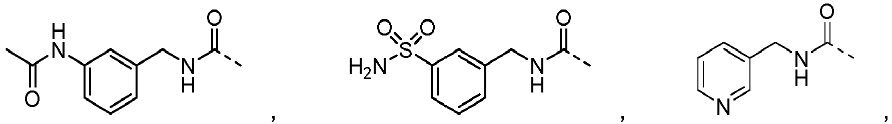 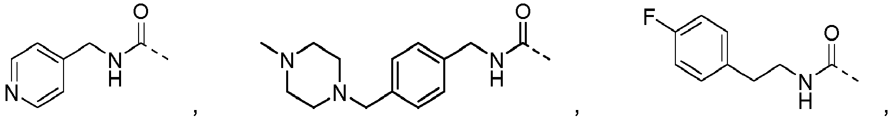 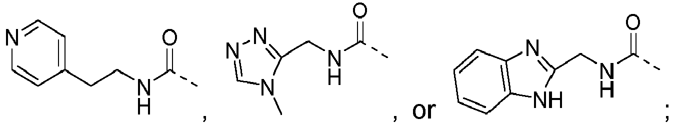
**R₂** represents -H, -CI, -OCH₃, -OC₂H₅, or -OCH(CH₃)₂;
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, 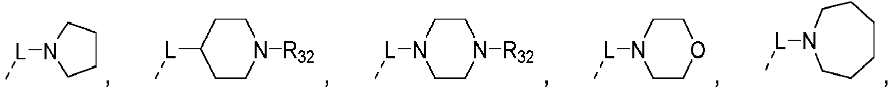 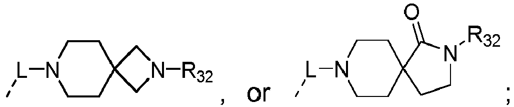
**R₅** represents 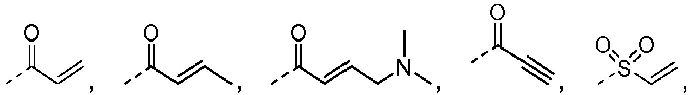 or 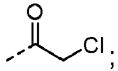
**L** is a bond, -CH₂-, -OCH₂CH₂-, -CO-, -CONH-, or -CONHCH₂CH₂CH₂-; and
**R₃₂** represents -H, -CH₃, -C₂H₅, -CH(CH₃)₂, -CH₂Ph, -COCH₃, or -SO₂C₂H₅.

Especially preferred compounds according to the present invention include compounds presented by Table 1.

**Table 1**

| **Example** | **Structure** | **Nomenclature** |
|---|---|---|
| **1** | 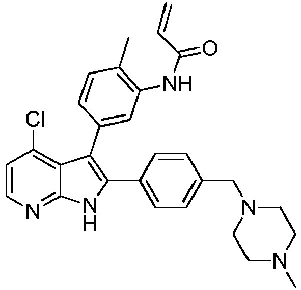 | N-(5-(4-chloro-2-(4-((4-methylpiperazin-1-yl)methyl)phenyl)-1H-pyrrolo[2,3-b]pyridin-3-yl)-2-methylphenyl)acrylamide |
| **2** | 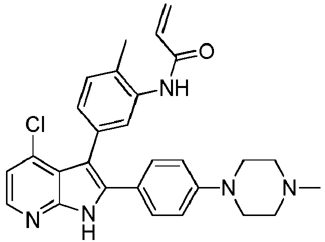 | N-(5-(4-chloro-2-(4-(4-methylpiperazin-1-yl)phenyl)-1H-pyrrolo[2,3-b]pyridin-3-yl)-2-methylphenyl)acrylamide |
| **3** | 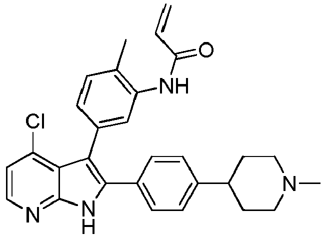 | N-(5-(4-chloro-2-(4-(1-methylpiperidin-4-yl)phenyl)-1H-pyrrolo[2,3-b]pyridin-3-yl)-2-methylphenyl)acrylamide |
| **4** | 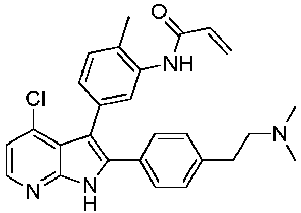 | N-(5-(4-chloro-2-(4-(2-(dimethylamino)ethyl)phenyl)-1H-pyrrolo[2,3-b]pyridin-3-yl)-2-methylphenyl)acrylamide |
| **5** | 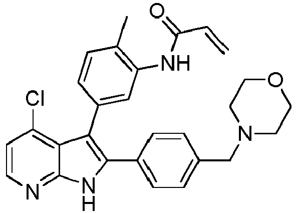 | N-(5-(4-chloro-2-(4-(morpholinomethyl)phenyl)-1H-pyrrolo[2,3-b]pyridin-3-yl)-2-methylphenyl)acrylamide |
| **6** | 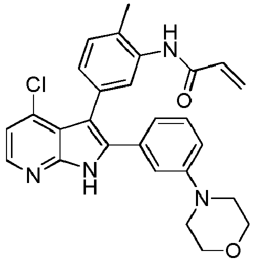 | N-(5-(4-chloro-2-(3-morpholinophenyl)-1H-pyrrolo[2,3-b]pyridin-3-yl)-2-methylphenyl)acrylamide |
| **7** | 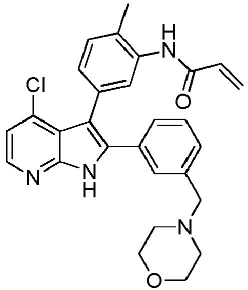 | N-(5-(4-chloro-2-(3-(morpholinomethyl)phenyl)-1H-pyrrolo[2,3-b]pyridin-3-yl)-2-methylphenyl)acrylamide |
| **8** | 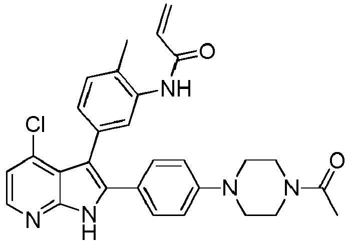 | N-(5-(2-(4-(4-acetylpiperazin-1-yl)phenyl)-4-chloro-1H-pyrrolo[2,3-b]pyridin-3-yl)-2-methylphenyl)acrylamide |
| **9** | 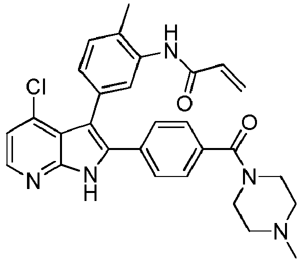 | N-(5-(4-chloro-2-(4-(4-methylpiperazine-1-carbonyl)phenyl)-1H-pyrrolo[2,3-b]pyridin-3-yl)-2-methylphenyl)acrylamide |
| **10** | 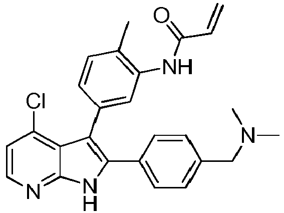 | N-(5-(4-chloro-2-(4-((dimethylamino)methyl)phenyl )-1H-pyrrolo[2,3-b]pyridin-3-yl)-2-methylphenyl)acrylamide |
| **11** | 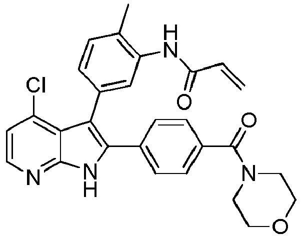 | N-(5-(4-chloro-2-(4-(morpholine-4-carbonyl)phenyl)-1H-pyrrolo[2,3-b]pyridin-3-yl)-2-methylphenyl)acrylamide |
| **12** | 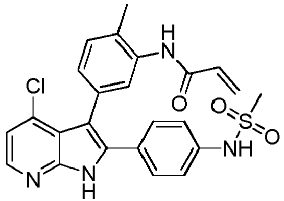 | N-(5-(4-chloro-2-(4-(methylsulfonamido)phenyl)-1H-pyrrolo[2,3-b]pyridin-3-yl)-2-methylphenyl)acrylamide |
| **13** | 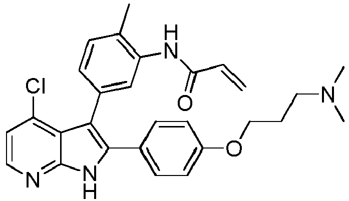 | N-(5-(4-chloro-2-(4-(3-(dimethylamino)propoxy)pheny l)-1H-pyrrolo[2,3-b]pyridin-3-yl)-2-methylphenyl)acrylamide |
| **14** | 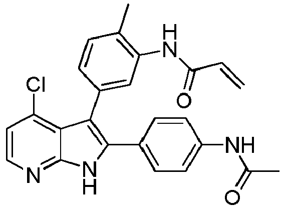 | N-(5-(2-(4-acetamidophenyl)-4-chloro-1H-pyrrolo[2,3-b]pyridin-3-yl)-2-methylphenyl)acrylamide |
| **15** | 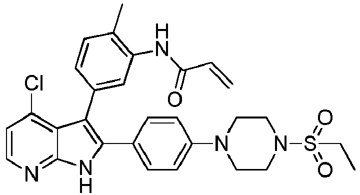 | N-(5-(4-chloro-2-(4-(4-(ethylsulfonyl)piperazin-1-yl)phenyl)-1H-pyrrolo[2,3-b]pyridin-3-yl)-2-methylphenyl)acrylamide |
| **16** | 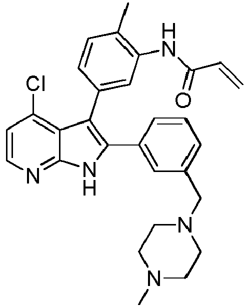 | N-(5-(4-chloro-2-(3-((4-methylpiperazin-1-yl)methyl)phenyl)-1H-pyrrolo[2,3-b]pyridin-3-yl)-2-methylphenyl)acrylamide |
| **17** | 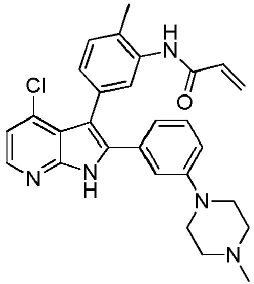 | N-(5-(4-chloro-2-(3-(4-methylpiperazin-1-yl)phenyl)-1H-pyrrolo[2,3-b]pyridin-3-yl)-2-methylphenyl)acrylamide |
| **18** | 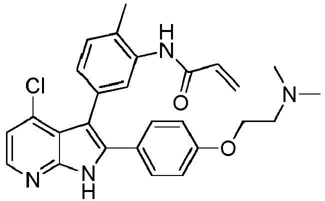 | N-(5-(4-chloro-2-(4-(2-(dimethylamino)ethoxy)phenyl) -1H-pyrrolo[2,3-b]pyridin-3-yl)-2-methylphenyl)acrylamide |
| **19** | 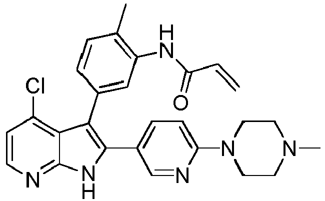 | N-(5-(4-chloro-2-(6-(4-methylpiperazin-1-yl)pyridin-3-yl)-1H-pyrrolo[2,3-b]pyridin-3-yl)-2-methylphenyl)acrylamide |
| **20** | 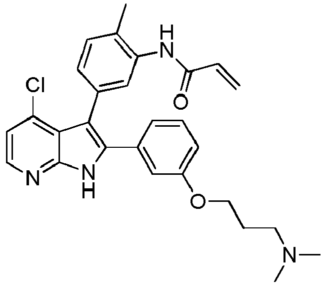 | N-(5-(4-chloro-2-(3-(3-(dimethylamino)propoxy)pheny l)-1H-pyrrolo[2,3-b]pyridin-3-yl)-2-methylphenyl)acrylamide |
| **21** | 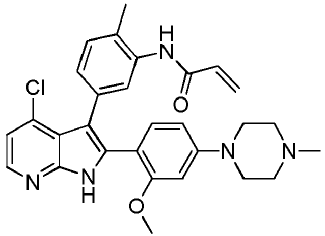 | N-(5-(4-chloro-2-(2-methoxy-4-(4-methylpiperazin-1-yl)phenyl)-1H-pyrrolo[2,3-b]pyridin-3-yl)-2-methylphenyl)acrylamide |
| **22** | 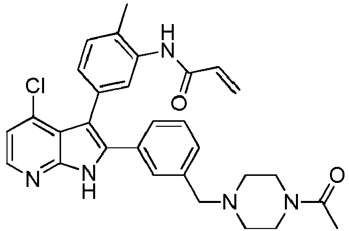 | N-(5-(2-(3-((4-acetylpiperazin-1-yl)methyl)phenyl)-4-chloro-1H-pyrrolo[2,3-b]pyridin-3-yl)-2-methylphenyl)acrylamide |
| **23** | 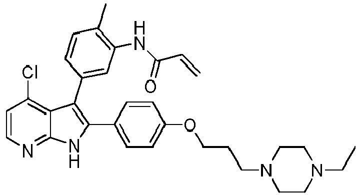 | N-(5-(4-chloro-2-(4-(3-(4-ethylpiperazin-1-yl)propoxy)phenyl)-1H-pyrrolo[2,3-b]pyridin-3-yl)-2-methyl phenyl )acrylamide |
| **24** | 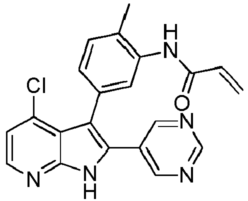 | N-(5-(4-chl loro-2-(pyrimidin-5-yl)-1H-pyrrolo[2,3-b]pyridin-3-yl)-2-methylphenyl)acrylamide |
| **25** | 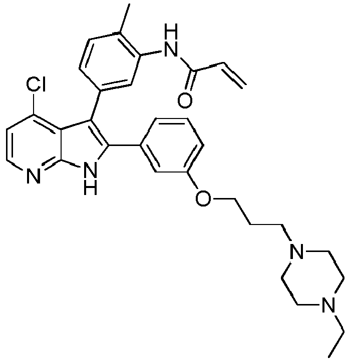 | N-(5-(4-chloro-2-(3-(3-(4-ethylpiperazin-1-yl)propoxy)phenyl)-1H-pyrrolo[2,3-b]pyridin-3-yl)-2-methylphenyl)acrylamide |
| **26** | 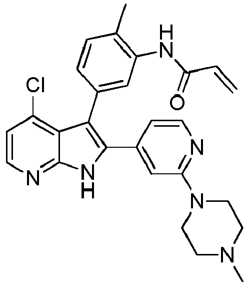 | N-(5-(4-chloro-2-(2-(4-methylpiperazin-1-yl)pyridin-4-yl)-1H-pyrrolo[2,3-b]pyridin-3-yl)-2-methylphenyl)acrylamide |
| **27** | 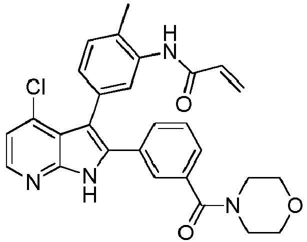 | N-(5-(4-chloro-2-(3-(morpholine-4-carbonyl)phenyl)-1H-pyrrolo[2,3-b]pyridin-3-yl)-2-methylphenyl)acrylamide |
| **28** | 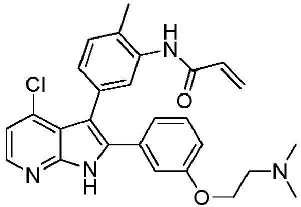 | N-(5-(4-chloro-2-(3-(2-(dimethylamino)ethoxy)phenyl) -1H-pyrrolo[2,3-b]pyridin-3-yl)-2-methylphenyl)acrylamide |
| **29** | 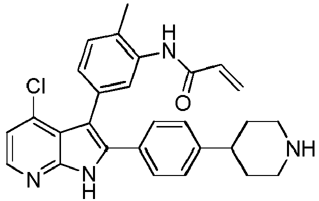 | N-(5-(4-chloro-2-(4-(piperidin-4-yl)phenyl)-1H-pyrrolo[2,3-b]pyridin-3-yl)-2-methylphenyl)acrylamide |
| **30** | 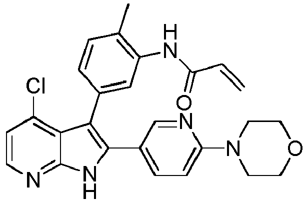 | N-(5-(4-chloro-2-(6-morpholinopyridin-3-yl)-1H-pyrrolo[2,3-b]pyridin-3-yl)-2-methylphenyl)acrylamide |
| **31** | 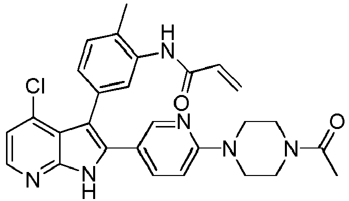 | N-(5-(2-(6-(4-acetylpiperazin-1-yl)pyridin-3-yl)-4-chloro-1H-pyrrolo[2,3-b]pyridin-3-yl)-2-methylphenyl)acrylamide |
| **32** | 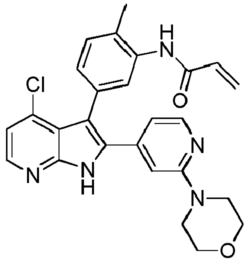 | N-(5-(4-chloro-2-(2-morpholinopyridin-4-yl)-1H-pyrrolo[2,3-b]pyridin-3-yl)-2-methylphenyl)acrylamide |
| **33** | 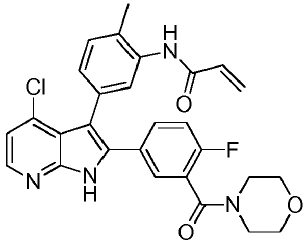 | N-(5-(4-chloro-2-(4-fluoro-3-(morpholine-4-carbonyl)phenyl)-1H-pyrrolo[2,3-b]pyridin-3-yl)-2-methylphenyl)acrylamide |
| **34** | 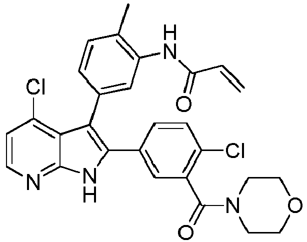 | N-(5-(4-chloro-2-(4-chloro-3-(morpholine-4-carbonyl)phenyl)-1H-pyrrolo[2,3-b]pyridin-3-yl)-2-methylphenyl)acrylamide |
| 35 | 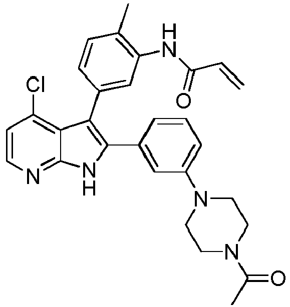 | N-(5-(2-(3-(4-acetylpiperazin-1-yl)phenyl)-4-chloro-1H-pyrrolo[2,3-b]pyridin-3-yl)-2-methylphenyl)acrylamide |
| **36** | 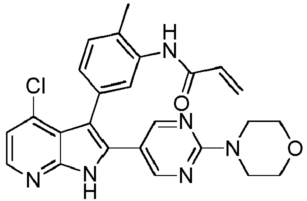 | N-(5-(4-chloro-2-(2-morpholinopyrimidin-5-yl)-1H-pyrrolo[2,3-b]pyridin-3-yl)-2-methylphenyl)acrylamide |
| **37** | 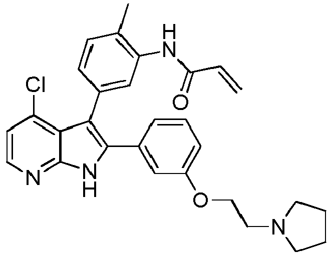 | N-(5-(4-chloro-2-(3-(2-(pyrrolidin-1-yl)ethoxy)phenyl)-1H-pyrrolo[2,3-b]pyridin-3-yl)-2-methylphenyl)acrylamide |
| **38** | 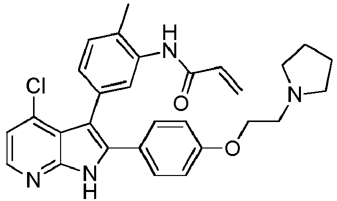 | N-(5-(4-chloro-2-(4-(2-(pyrrolidin-1-yl)ethoxy)phenyl)-1H-pyrrolo[2,3-b]pyridin-3-yl)-2-methylphenyl)acrylamide |
| **39** | 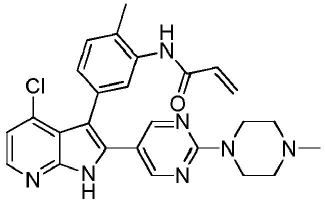 | N-(5-(4-chloro-2-(2-(4-methylpiperazin-1-yl)pyrimidin-5-yl)-1H-pyrrolo[2,3-b]pyridin-3-yl)-2-methylphenyl)acrylamide |
| **40** | 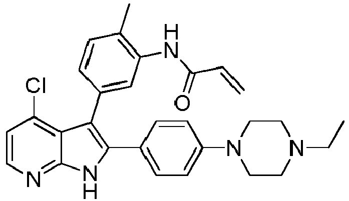 | N-(5-(4-chloro-2-(4-(4-ethylpiperazin-1-yl)phenyl)-1H-pyrrolo[2,3-b]pyridin-3-yl)-2-methylphenyl)acrylamide |
| **41** | 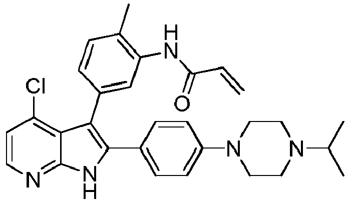 | N-(5-(4-chloro-2-(4-(4-isopropylpiperazin-1-yl)phenyl)-1H-pyrrolo[2,3-b]pyridin-3-yl)-2-methylphenyl)acrylamide |
| **42** | 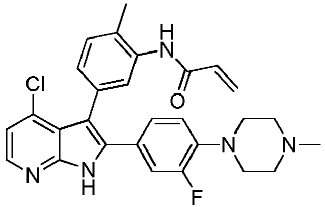 | N-(5-(4-chloro-2-(3-fluoro-4-(4-methylpiperazin-1-yl)phenyl)-1H-pyrrolo[2,3-b]pyridin-3-yl)-2-methylphenyl)acrylamide |
| **43** | 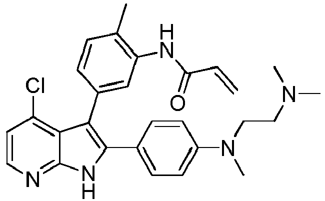 | N-(5-(4-chloro-2-(4-((2-(dimethylamino)ethyl)(methyl)a mino)phenyl)-1H-pyrrolo[2,3-b]pyridin-3-yl)-2-methylphenyl)acrylamide |
| **44** | 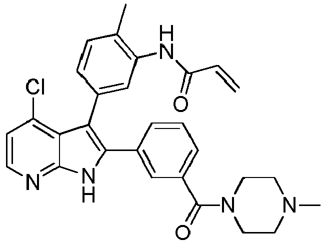 | N-(5-(4-chloro-2-(3-(4-methylpiperazine-1-carbonyl)phenyl)-1H-pyrrolo[2,3-b]pyridin-3-yl)-2-methylphenyl)acrylamide |
| **45** | 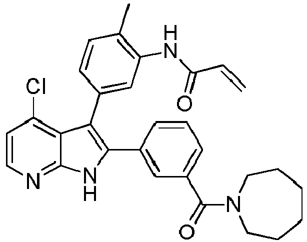 | N-(5-(2-(3-(azepane-1-carbonyl)phenyl)-4-chloro-1H-pyrrolo[2,3-b]pyridin-3-yl)-2-methylphenyl)acrylamide |
| **46** | 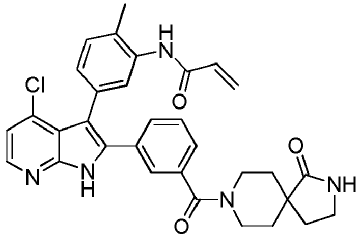 | N-(5-(4-chloro-2-(3-(1-oxo-2,8-diazaspiro[4.5]decane-8-carbonyl)phenyl)-1H-pyrrolo[2,3-b]pyridin-3-yl)-2-methylphenyl)acrylamide |
| **47** | 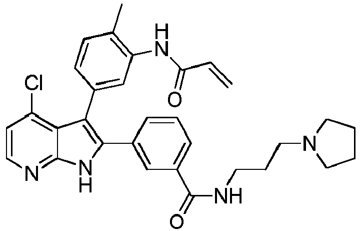 | 3-(3-(3-acrylamido-4-methylphenyl)-4-chloro-1H-pyrrolo[2,3-b]pyridin-2-yl)-N-(3-(pyrrolidin-1-yl)propyl)benzamide |
| **48** | 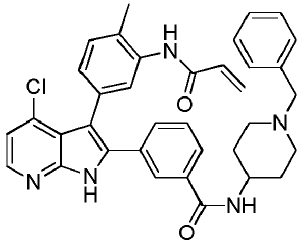 | 3-(3-(3-acrylamido-4-methylphenyl)-4-chloro-1H-pyrrolo[2,3-b]pyridin-2-yl)-N-(1-benzylpiperidin-4-yl)benzamide |
| **49** | 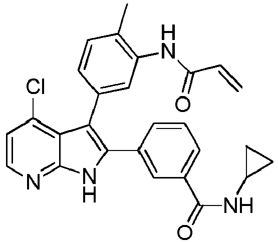 | 3-(3-(3-acrylamido-4-methylphenyl)-4-chloro-1H-pyrrolo[2,3-b]pyridin-2-yl)-N-cyclopropylbenzamide |
| **50** | 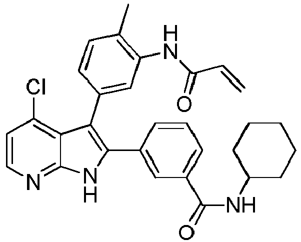 | 3-(3-(3-acrylamido-4-methylphenyl)-4-chloro-1H-pyrrolo[2,3-b]pyridin-2-yl)-N-cyclohexylbenzamide |
| **51** | 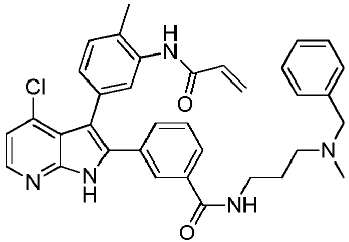 | 3-(3-(3-acrylamido-4-methylphenyl)-4-chloro-1H-pyrrolo[2,3-b]pyridin-2-yl)-N-(3-(benzyl(methyl)amino)propyl)b enzamide |
| **52** | 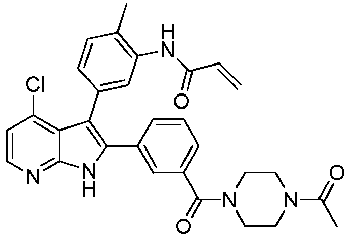 | N-(5-(2-(3-(4-acetylpiperazine-1-carbonyl)phenyl)-4-chloro-1H-pyrrolo[2,3-b]pyridin-3-yl)-2-methylphenyl)acrylamide |
| **53** | 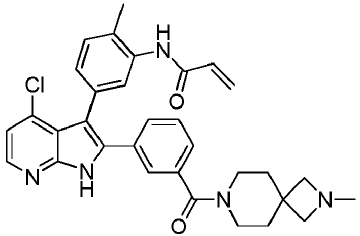 | N-(5-(4-chloro-2-(3-(2-methyl-2,7-diazaspiro[3.5]nonane-7-carbonyl)phenyl)-1H-pyrrolo[2,3-b]pyridin-3-yl)-2-methylphenyl)acrylamide |
| **54** | 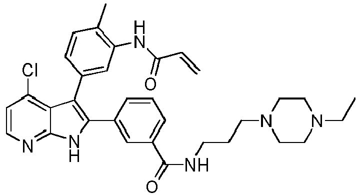 | 3-(3-(3-acrylamido-4-methylphenyl)-4-chloro-1H-pyrrolo[2,3-b]pyridin-2-yl)-N-(3-(4-ethylpiperazin-1-yl)propyl)benzamide |
| **55** | 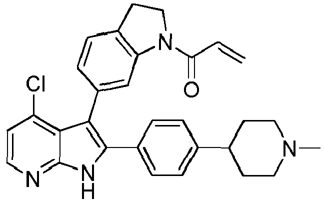 | 1-(6-(4-chloro-2-(4-(1-methylpiperidin-4-yl)phenyl)-1H-pyrrolo[2,3-b]pyridin-3-yl)indolin-1-yl)prop-2-en-1-one |
| **56** | 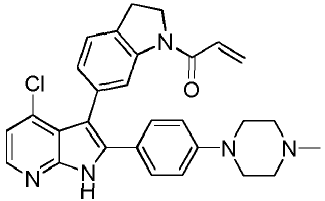 | 1-(6-(4-chloro-2-(4-(4-methylpiperazin-1-yl)phenyl)-1H-pyrrolo[2,3-b]pyridin-3-yl)indolin-1-yl)prop-2-en-1-one |
| **57** | 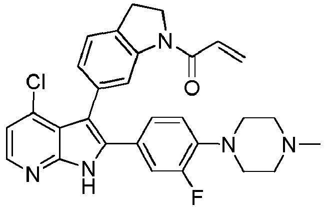 | 1-(6-(4-chloro-2-(3-fluoro-4-(4-methylpiperazin-1-yl)phenyl)-1H-pyrrolo[2,3-b]pyridin-3-yl)indolin-1-yl)prop-2-en-1-one |
| **58** | 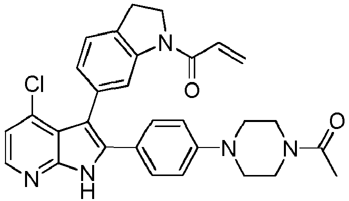 | 1-(6-(2-(4-(4-acetylpiperazin-1-yl)phenyl)-4-chloro-1H-pyrrolo[2,3-b]pyridin-3-yl)indolin-1-yl)prop-2-en-1-one |
| **59** | 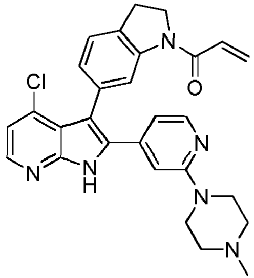 | 1-(6-(4-chloro-2-(2-(4-methylpiperazin-1-yl)pyridin-4-yl)-1H-pyrrolo[2,3-b]pyridin-3-yl)indolin-1-yl)prop-2-en-1-one |
| **60** | 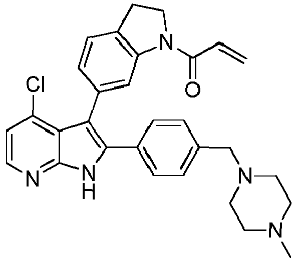 | 1-(6-(4-chloro-2-(4-((4-methylpiperazin-1-yl)methyl)phenyl)-1H-pyrrolo[2,3-b]pyridin-3-yl)indolin-1-yl)prop-2-en-1-one |
| **61** | 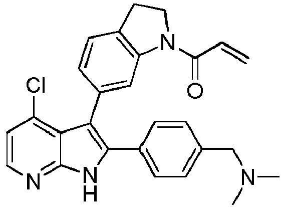 | 1-(6-(4-chloro-2-(4-((dimethylamino)methyl)phenyl )-1H-pyrrolo[2,3-b]pyridin-3-yl)indolin-1-yl)prop-2-en-1-one |
| **62** | 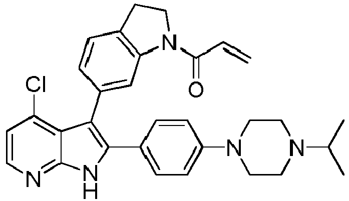 | 1-(6-(4-chloro-2-(4-(4-isopropylpiperazin-1-yl)phenyl)-1H-pyrrolo[2,3-b]pyridin-3-yl)indolin-1-yl)prop-2-en-1-one |
| **63** | 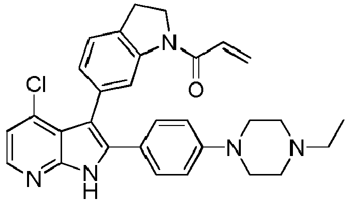 | 1-(6-(4-chloro-2-(4-(4-ethylpiperazin-1-yl)phenyl)-1H-pyrrolo[2,3-b]pyridin-3-yl)indolin-1-yl)prop-2-en-1-one |
| **64** | 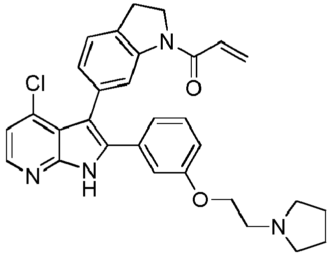 | 1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one |
| **65** | 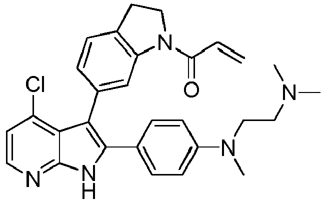 | 1-(6-(4-chloro-2-(4-((2-(dimethylamino)ethyl)(methyl)a mino)phenyl)-1H-pyrrolo[2,3-b]pyridin-3-yl)indolin-1-yl)prop-2-en-1-one |
| **66** | 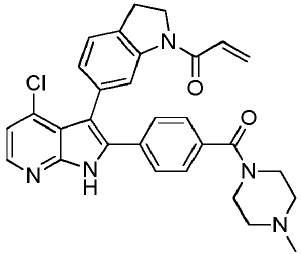 | 1-(6-(4-chloro-2-(4-(4-methylpiperazine-1-carbonyl)phenyl)-1H-pyrrolo[2,3-b]pyridin-3-yl)indolin-1-yl)prop-2-en-1-one |
| **67** | 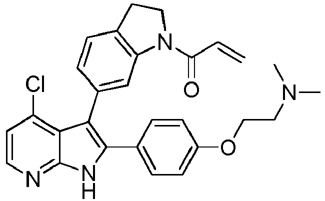 | 1-(6-(4-chloro-2-(4-(2-(dimethylamino)ethoxy)phenyl) -1H-pyrrolo[2,3-b]pyridin-3-yl)indolin-1-yl)prop-2-en-1-one |
| **68** | 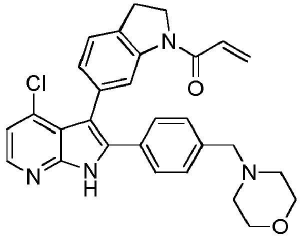 | 1-(6-(4-chloro-2-(4-(morpholinomethyl)phenyl)-1H-pyrrolo[2,3-b]pyridin-3-yl)indolin-1-yl)prop-2-en-1-one |
| **69** | 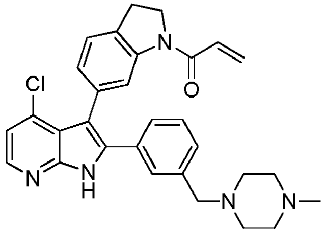 | 1-(6-(4-chloro-2-(3-((4-methylpiperazin-1-yl)methyl)phenyl)-1H-pyrrolo[2,3-b]pyridin-3-yl)indolin-1-yl)prop-2-en-1-one |
| **70** | 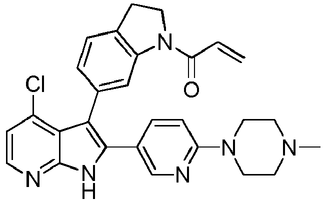 | 1-(6-(4-chloro-2-(6-(4-methylpiperazin-1-yl)pyridin-3-yl)-1H-pyrrolo[2,3-b]pyridin-3-yl)indolin-1-yl)prop-2-en-1-one |
| **71** | 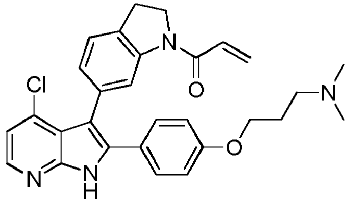 | 1-(6-(4-chloro-2-(4-(3-(dimethylamino)propoxy)phenyl)-1H-pyrrolo[2,3-b]pyridin-3-yl)indolin-1-yl)prop-2-en-1-one |
| **72** | 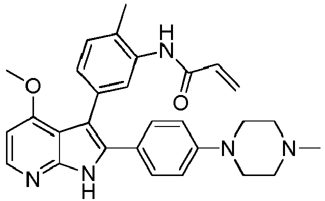 | N-(5-(4-methoxy-2-(4-(4-methylpiperazin-1-yl)phenyl)-1H-pyrrolo[2,3-b]pyridin-3-yl)-2-methylphenyl)acrylamide |
| **73** | 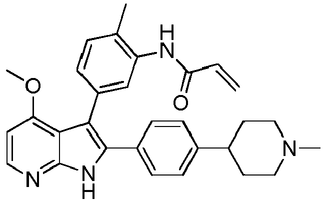 | N-(5-(4-methoxy-2-(4-(1-methylpiperidin-4-yl)phenyl)-1H-pyrrolo[2,3-b]pyridin-3-yl)-2-methylphenyl)acrylamide |
| **74** | 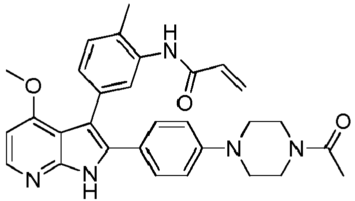 | N-(5-(2-(4-(4-acetylpiperazin-1-yl)phenyl)-4-methoxy-1H-pyrrolo[2,3-b]pyridin-3-yl)-2-methylphenyl)acrylamide |
| **75** | 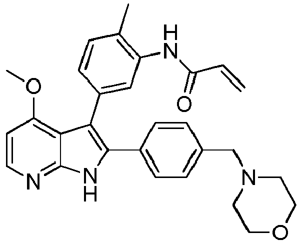 | N-(5-(4-methoxy-2-(4-(morpholinomethyl)phenyl)-1H-pyrrolo[2,3-b]pyridin-3-yl)-2-methylphenyl)acrylamide |
| **76** | 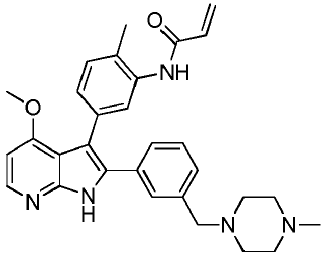 | N-(5-(4-methoxy-2-(3-((4-methylpiperazin-1-yl)methyl)phenyl)-1H-pyrrolo[2,3-b]pyridin-3-yl)-2-methylphenyl)acrylamide |
| **77** | 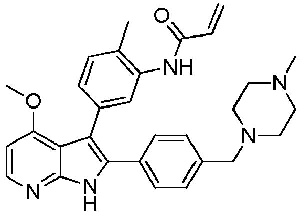 | N-(5-(4-methoxy-2-(4-((4-methylpiperazin1-yl)methyl)phenyl)-1 H-pyrrolo[2,3-b]pyridin-3-yl)-2-methylphenyl)acrylamide |
| **78** | 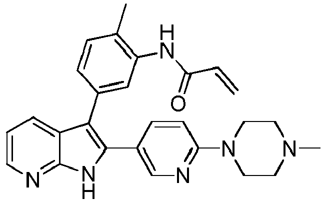 | N-(2-methyl-5-(2-(6-(4-methylpiperazin-1-yl)pyridin-3-yl)-1H-pyrrolo[2,3-b]pyridin-3-yl)phenyl)acrylamide |
| **79** | 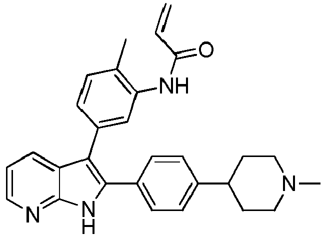 | N-(2-methyl-5-(2-(4-(1-methylpiperidin-4-yl)phenyl)-1H-pyrrolo[2,3-b]pyridin-3-yl)phenyl)acrylamide |
| **80** | 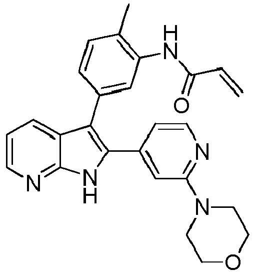 | N-(2-methyl-5-(2-(2-morpholinopyridin-4-yl)-1H-pyrrolo[2,3-b]pyridin-3-yl)phenyl)acrylamide |
| **81** | 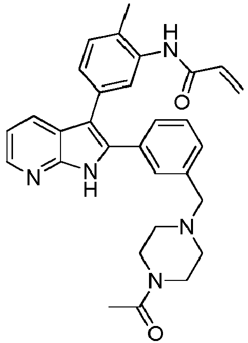 | N-(5-(2-(3-((4-acetylpiperazin-1-yl)methyl)phenyl)-1H-pyrrolo[2,3-b]pyridin-3-yl)-2-methylphenyl)acrylamide |
| **82** | 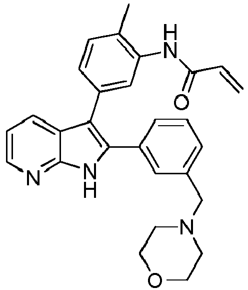 | N-(2-methyl-5-(2-(3-(morpholinomethyl)phenyl)-1H-pyrrolo[2,3-b]pyridin-3-yl)phenyl)acrylamide |
| **83** | 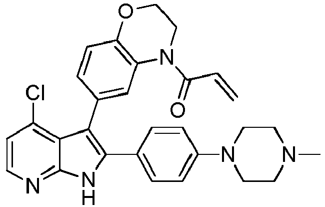 | 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one |
| **84** | 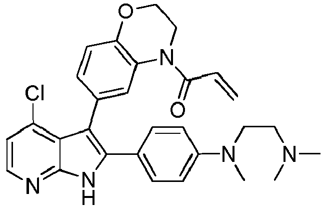 | 1-(6-(4-chloro-2-(4-((2-(dimethylamino)ethyl)(methyl)a mino)phenyl)-1H-pyrrolo[2,3-b]pyridin-3-yl)-2H-benzo[b][1,4]oxazin-4(3H)-yl)prop-2-en-1-one |
| **85** | 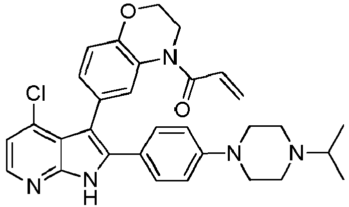 | 1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one |
| **86** | 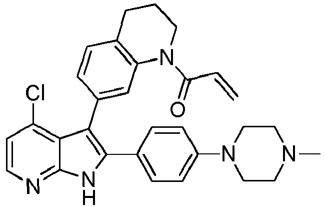 | 1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one |
| **87** | 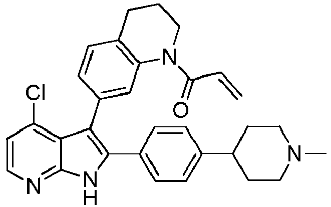 | 1-(7-(4-chloro-2-(4-(1-methylpiperidin-4-yl)phenyl)-1H-pyrrolo[2,3-b]pyridin-3-yl)-3,4-dihydroquinolin-1(2H)-yl)prop-2-en-1-one |
| **88** | 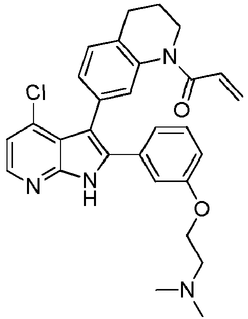 | 1-(7-(4-chloro-2-(3-(2-(dimethylamino)ethoxy)phenyl) -1H-pyrrolo[2,3-b]pyridin-3-yl)-3,4-dihydroquinolin-1(2H)-yl)prop-2-en-1-one |
| **89** | 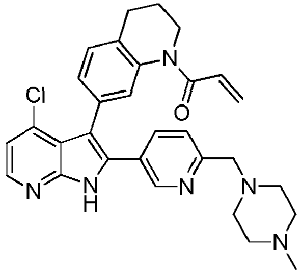 | 1-(7-(4-chloro-2-(6-((4-methylpiperazin-1-yl)methyl)pyridin-3-yl)-1H-pyrrolo[2,3-b]pyridin-3-yl)-3,4-dihydroquinolin-1(2H)-yl)prop-2-en-1-one |
| **90** | 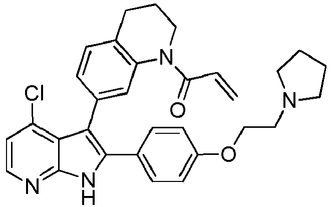 | 1-(7-(4-chloro-2-(4-(2-(pyrrolidin-1-yl)ethoxy)phenyl)-1H-pyrrolo[2,3-b]pyridin-3-yl)-3,4-dihydroquinolin-1(2H)-yl)prop-2-en-1-one |
| **91** | 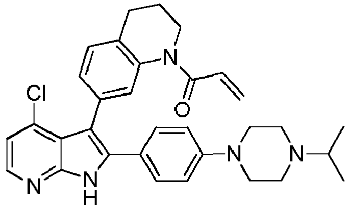 | 1-(7-(4-chloro-2-(4-(4-isopropylpiperazin-1-yl)phenyl)-1H-pyrrolo[2,3-b]pyridin-3-yl)-3,4-dihydroquinolin-1(2H)-yl)prop-2-en-1-one |
| **92** | 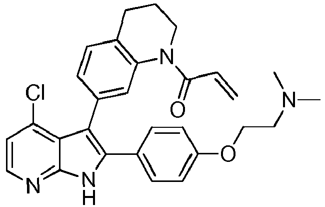 | 1-(7-(4-chloro-2-(4-(2-(dimethylamino)ethoxy)phenyl)-1H-pyrrolo[2,3-b]pyridin-3-yl)-3,4-dihydroquinolin-1(2H)-yl)prop-2-en-1-one |
| **93** | 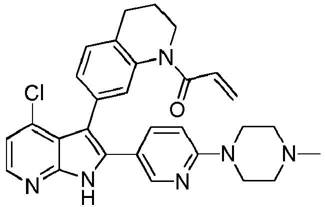 | 1-(7-(4-chloro-2-(6-(4-methylpiperazin-1-yl)pyridin-3-yl)-1H-pyrrolo[2,3-b]pyridin-3-yl)-3,4-dihydroquinolin-1(2H)-yl)prop-2-en-1-one |
| **94** | 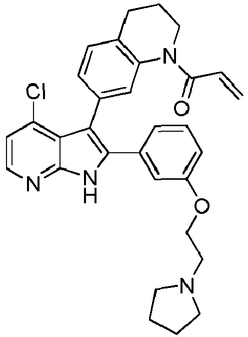 | 1-(7-(4-chloro-2-(3-(2-(pyrrolidin-1-yl)ethoxy)phenyl)-1H-pyrrolo[2,3-b]pyridin-3-yl)-3,4-dihydroquinolin-1(2H)-yl)prop-2-en-1-one |
| **95** | 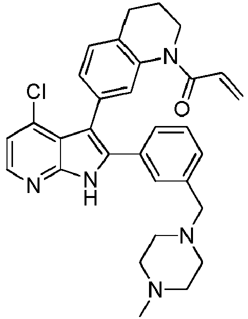 | 1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one |
| **96** | 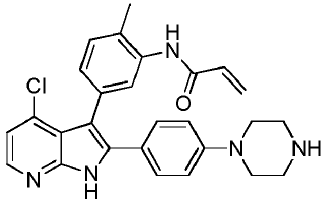 | N-(5-(4-chloro-2-(4-(piperazin-1-yl)phenyl)-1H-pyrrolo[2,3-b]pyridin-3-yl)-2-methylphenyl)acrylamide |
| **97** | 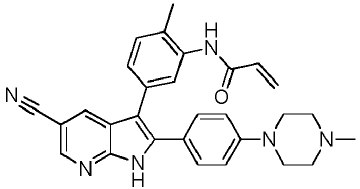 | N-(5-(5-cyano-2-(4-(4-methylpiperazin-1-yl)phenyl)-1H-pyrrolo[2,3-b]pyridin-3-yl)-2-methylphenyl)acrylamide |
| **98** | 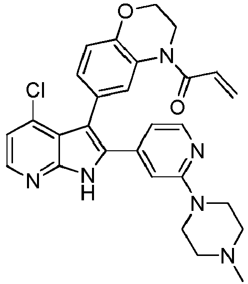 | 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one |
| **99** | 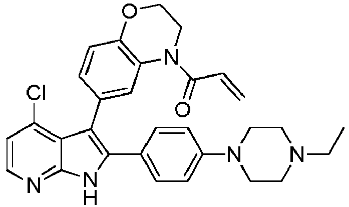 | 1-(6-(4-chloro-2-(4-(4-ethylpiperazin-1-yl)phenyl)-1H-pyrrolo[2,3-b]pyridin-3-yl)-2H-benzo[b][1,4]oxazin-4(3H)-yl)prop-2-en-1-one |
| **100** | 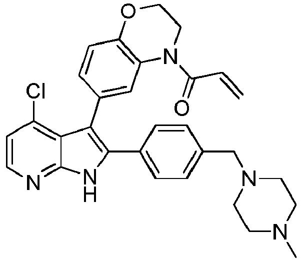 | 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one |
| **101** | 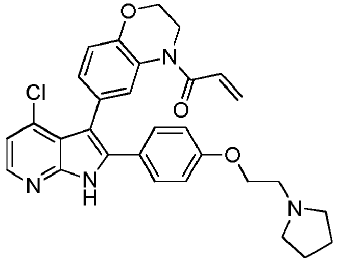 | 1-(6-(4-chloro-2-(4-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one |
| **102** | 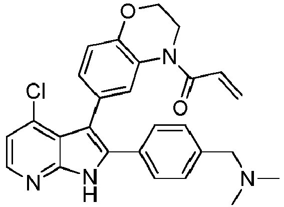 | 1-(6-(4-chloro-2-(4-((dimethylamino)methyl)phenyl)-1H-pyrrolo[2,3-b]pyridin-3-yl)-2H-benzo[b][1,4]oxazin-4(3H)-yl)prop-2-en-1-one |
| **103** | 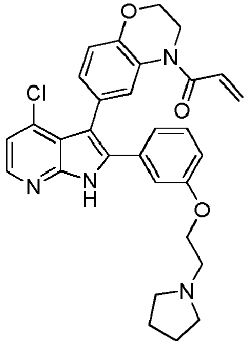 | 1-(6-(4-chloro-2-(3-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one |
| **104** | 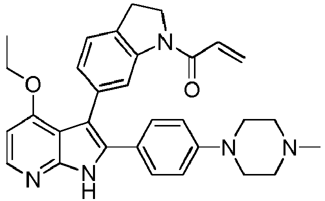 | 1-(6-(4-ethoxy-2-(4-(4-methylpiperazin-1-yl)phenyl)-1H-pyrrolo[2,3-b]pyridin-3-yl)indolin-1-yl)prop-2-en-1-one |
| **105** | 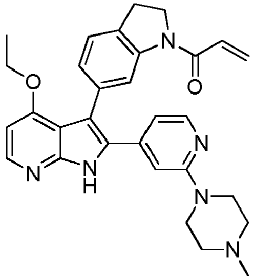 | 1-(6-(4-ethoxy-2-(4-(4-methylpiperazin-1-yl)phenyl)-1H-pyrrolo[2,3-b]pyridin-3-yl)indolin-1-yl)prop-2-en-1-one |
| **106** | 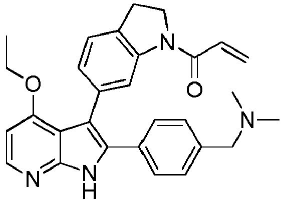 | 1-(6-(2-(4-((dimethylamino)methyl)phenyl)-4-ethoxy-1H-pyrrolo[2,3-b]pyridin-3-yl)indolin-1-yl)prop-2-en-1-one |
| **107** | 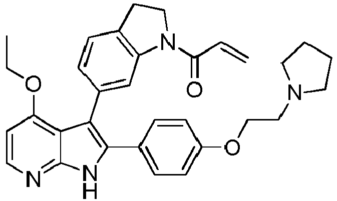 | 1-(6-(4-ethoxy-2-(4-(2-(pyrrolidin-1-yl)ethoxy)phenyl)-1H-pyrrolo[2,3-b]pyridin-3-yl)indolin-1-yl)prop-2-en-1-one |
| **108** | 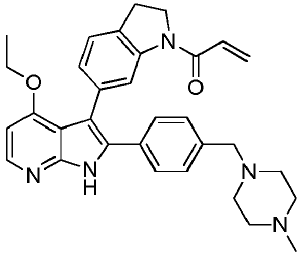 | 1-(6-(4-ethoxy-2-(4-((4-methylpiperazin-1-yl)methyl)phenyl)-1H-pyrrolo[2,3-b]pyridin-3-yl)indolin-1-yl)prop-2-en-1-one |
| **109** | 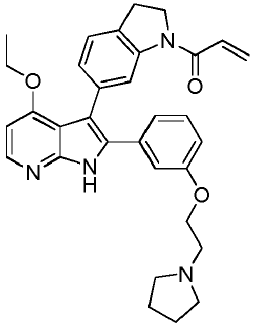 | 1-(6-(4-ethoxy-2-(3-(2-(pyrrolidin-1-yl)ethoxy)phenyl)-1H-pyrrolo[2,3-b]pyridin-3-yl)indolin-1-yl)prop-2-en-1-one |
| **110** | 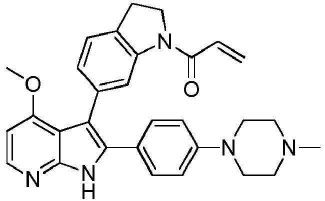 | 1-(6-(4-methoxy-2-(4-(4-methylpiperazin-1-yl)phenyl)-1H-pyrrolo[2,3-b]pyridin-3-yl)indolin-1-yl)prop-2-en-1-one |
| **111** | 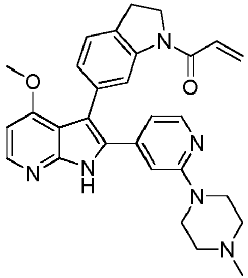 | 1-(6-(4-methoxy-2-(2-(4-methylpiperazin-1-yl)pyridin-4-yl)-1H-pyrrolo[2,3-b]pyridin-3-yl)indolin-1-yl)prop-2-en-1-one |
| **112** | 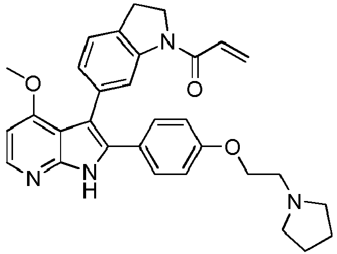 | 1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1-one |
| **113** | 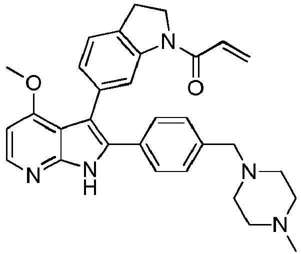 | 1-(6-(4-methoxy-2-(4-((4-methylpiperazin-1-yl)methyl)phenyl)-1H-pyrrolo[2,3-b]pyridin-3-yl)indolin-1-yl)prop-2-en-1-one |
| **114** | 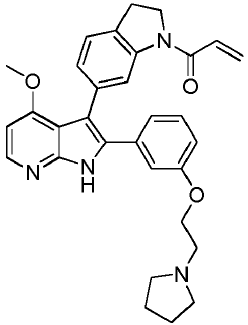 | 1-(6-(4-methoxy-2-(3-(2-(pyrrolidin-1-yl)ethoxy)phenyl)-1H-pyrrolo[2,3-b]pyridin-3-yl)indolin-1-yl)prop-2-en-1-one |
| **115** | 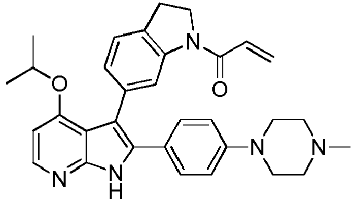 | 1-(6-(4-isopropoxy-2-(4-(4-methylpiperazin-1-yl)phenyl)-1H-pyrrolo[2,3-b]pyridin-3-yl)indolin-1-yl)prop-2-en-1-one |
| **116** | 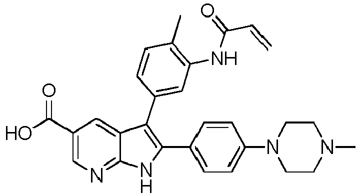 | 3-(3-acrylamido-4-methylphenyl)-2-(4-(4-methylpiperazin-1-yl)phenyl)-1H-pyrrolo[2,3-b]pyridine-5-carboxylic acid |
| **117** | 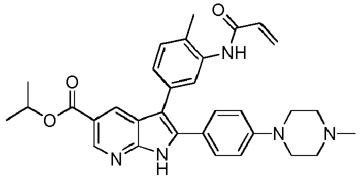 | isopropyl 3-(3-acrylamido-4-methylphenyl)-2-(4-(4-methylpiperazin-1-yl)phenyl)-1H-pyrrolo[2,3-b]pyridine-5-carboxylate |
| **118** | 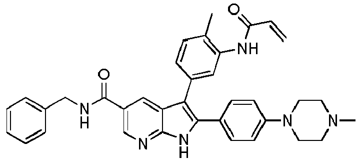 | 3-(3-acrylamido-4-methylphenyl)-N-benzyl-2-(4-(4-methylpiperazin-1-yl)phenyl)-1H-pyrrolo[2,3-b]pyridine-5-carboxamide |
| **119** | 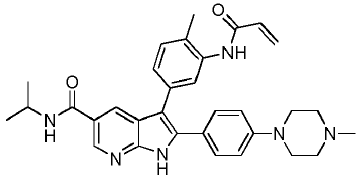 | 3-(3-acrylamido-4-methylphenyl)-N-isopropyl-2-(4-(4-methylpiperazin-1-yl)phenyl)-1H-pyrrolo[2,3-b]pyridine-5-carboxamide |
| **120** | 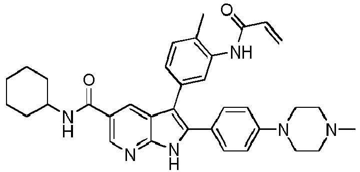 | 3-(3-acrylamido-4-methylphenyl)-N-cyclohexyl-2-(4-(4-methylpiperazin-1-yl)phenyl)-1H-pyrrolo[2,3-b]pyridine-5-carboxamide |
| **121** | 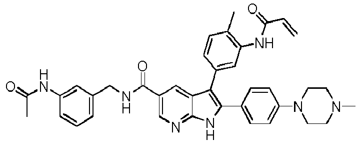 | N-(3-acetamidobenzyl)-3-(3-acrylamido-4-methylphenyl)-2-(4-(4-methylpiperazin-1-yl)phenyl)-1H-pyrrolo[2,3-b]pyridine-5-carboxamide |
| **122** | 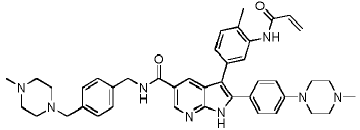 | 3-(3-acrylamido-4-methylphenyl)-N-(4-((4-methylpiperazin-1-yl)methyl)benzyl)-2-(4-(4-methylpiperazin-1-yl)phenyl)-1H-pyrrolo[2,3-b]pyridine-5-carboxamide |
| **123** | 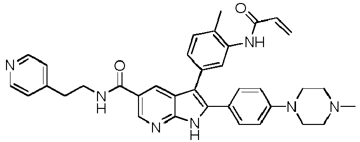 | 3-(3-acrylamido-4-methylphenyl)-2-(4-(4-methylpiperazin-1-yl)phenyl)-N-(2-(pyridin-4-yl)ethyl)-1H-pyrrolo[2,3-b]pyridine-5-carboxamide |
| **124** | 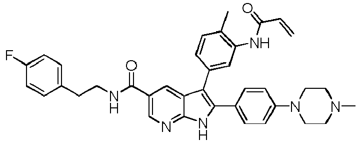 | 3-(3-acrylamido-4-methylphenyl)-N-(4-fluorophenethyl)-2-(4-(4-methylpiperazin-1-yl)phenyl)-1H-pyrrolo[2,3-b]pyridine-5-carboxamide |
| **125** | 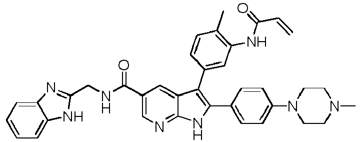 | N-((1H-benzo[d]imidazol-2-yl)methyl)-3-(3-acrylamido-4-methylphenyl)-2-(4-(4-methylpiperazin-1-yl)phenyl)-1H-pyrrolo[2,3-b]pyridine-5-carboxamide |
| **126** | 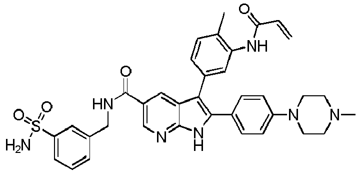 | 3-(3-acrylamido-4-methylphenyl)-2-(4-(4-methylpiperazin-1-yl)phenyl)-N-(3-sulfamoylbenzyl)-1H-pyrrolo[2,3-b]pyridine-5-carboxamide |
| **127** | 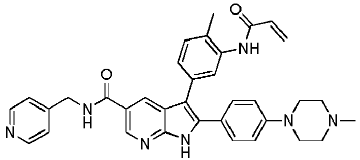 | 3-(3-acrylamido-4-methylphenyl)-2-(4-(4-methylpiperazin-1-yl)phenyl)-N-(pyridin-4-ylmethyl)-1H-pyrrolo[2,3-b]pyridine-5-carboxamide |
| **128** | 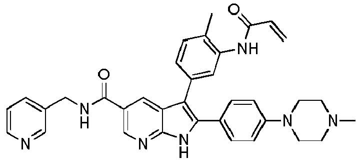 | 3-(3-acrylamido-4-methylphenyl)-2-(4-(4-methylpiperazin-1-yl)phenyl)-N-(pyridin-3-ylmethyl)-1H-pyrrolo[2,3-b]pyridine-5-carboxamide |
| **129** | 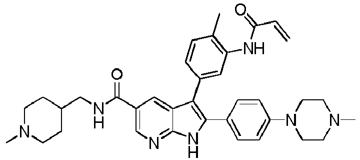 | 3-(3-acrylamido-4-methylphenyl)-2-(4-(4-methylpiperazin-1-yl)phenyl)-N-((1-methylpiperidin-4-yl)methyl)-1H-pyrrolo[2,3-b]pyridine-5-carboxamide |
| **130** | 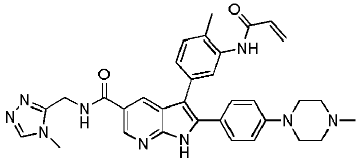 | 3-(3-acrylamido-4-methylphenyl)-N-((4-methyl-4H-1,2,4-triazol-3-yl)methyl)-2-(4-(4-methylpiperazin-1-yl)phenyl)-1H-pyrrolo[2,3-b]pyridine-5-carboxamide |
| **131** | 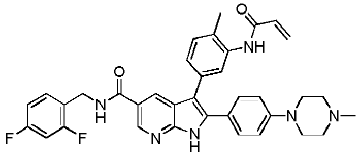 | 3-(3-acrylamido-4-methylphenyl)-N-(2,4-difluorobenzyl)-2-(4-(4-methylpiperazin-1-yl)phenyl)-1H-pyrrolo[2,3-b]pyridine-5-carboxamide |
| **132** | 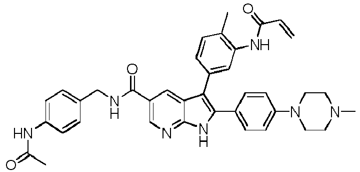 | N-(4-acetamidobenzyl)-3-(3-acrylamido-4-methylphenyl)-2-(4-(4-methylpiperazin-1-yl)phenyl)-1H-pyrrolo[2,3-b]pyridine-5-carboxamide |
| **133** | 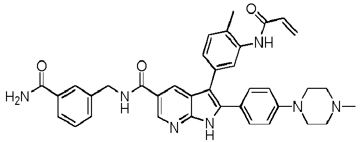 | 3-(3-acrylamido-4-methylphenyl)-N-(3-carbamoylbenzyl)-2-(4-(4-methylpiperazin-1-yl)phenyl)-1H-pyrrolo[2,3-b]pyridine-5-carboxamide |
| **134** | 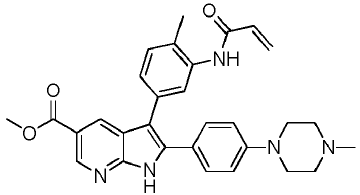 | methyl 3-(3-acrylamido-4-methylphenyl)-2-(4-(4-methylpiperazin-1-yl)phenyl)-1H-pyrrolo[2,3-b]pyridine-5-carboxylate |
| **135** | 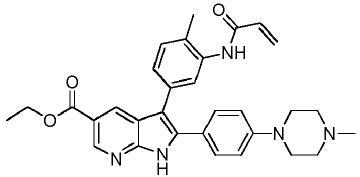 | ethyl 3-(3-acrylamido-4-methylphenyl)-2-(4-(4-methylpiperazin-1-yl)phenyl)-1H-pyrrolo[2,3-b]pyridine-5-carboxylate |
| **136** | 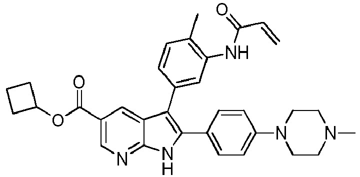 | cyclobutyl 3-(3-acrylamido-4-methylphenyl)-2-(4-(4-methylpiperazin-1-yl)phenyl)-1H-pyrrolo[2,3-b]pyridine-5-carboxylate |
| **137** | 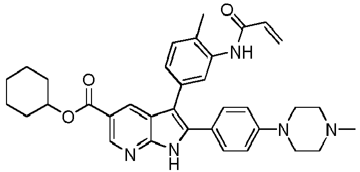 | cyclohexyl 3-(3-acrylamido-4-methylphenyl)-2-(4-(4-methylpiperazin-1-yl)phenyl)-1H-pyrrolo[2,3-b]pyridine-5-carboxylate |
| **138** | 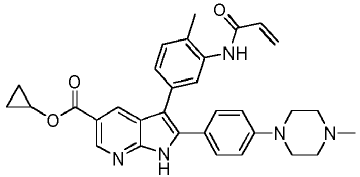 | cyclopropyl 3-(3-acrylamido-4-methylphenyl)-2-(4-(4-methylpiperazin-1-yl)phenyl)-1H-pyrrolo[2,3-b]pyridine-5-carboxylate |
| **139** | 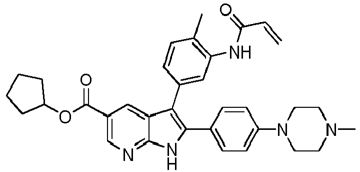 | cyclopentyl 3-(3-acrylamido-4-methylphenyl)-2-(4-(4-methylpiperazin-1-yl)phenyl)-1H-pyrrolo[2,3-b]pyridine-5-carboxylate |
| **140** | 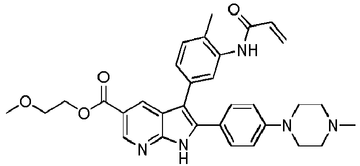 | 2-methoxyethyl 3-(3-acrylamido-4-methylphenyl)-2-(4-(4-methylpiperazin-1-yl)phenyl)-1H-pyrrolo[2,3-b]pyridine-5-carboxylate |
| **141** | 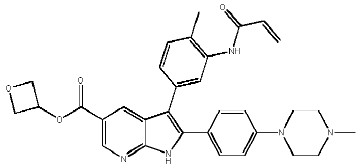 | oxetan-3-yl 3-(3-acrylamido-4-methylphenyl)-2-(4-(4-methylpiperazin-1-yl)phenyl)-1H-pyrrolo[2,3-b]pyridine-5-carboxylate |
| **142** | 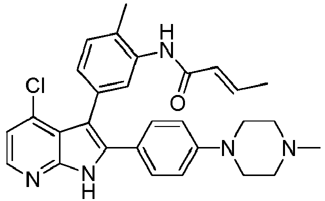 | (E)-N-(5-(4-chloro-2-(4-(4-methylpiperazin-1-yl)phenyl)-1H-pyrrolo[2,3-b]pyridin-3-yl)-2-methylphenyl)but-2-enamide |
| **143** | 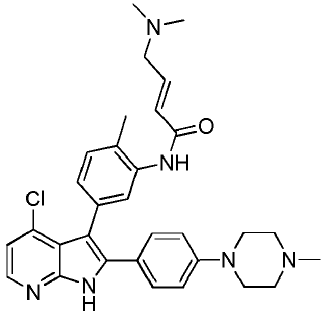 | (E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide |
| **144** | 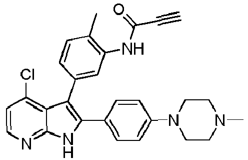 | N-(5-(4-chloro-2-(4-(4-methylpiperazin-1-yl)phenyl)-1H-pyrrolo[2,3-b]pyridin-3-yl)-2-methylphenyl)propiolamide |
| **145** | 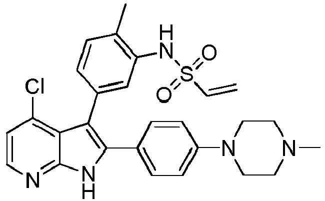 | N-(5-(4-chloro-2-(4-(4-methylpiperazin-1-yl)phenyl)-1H-pyrrolo[2,3-b]pyridin-3-yl)-2-methylphenyl)-ethenesulfonamide |
| **146** | 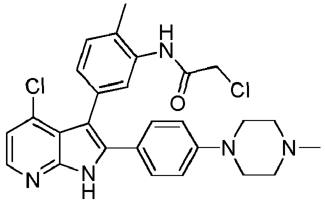 | 2-chloro-N-(5-(4-chloro-2-(4-(4-methylpiperazin-1-yl)phenyl)-1H-pyrrolo[2,3-b]pyridin-3-yl)-2-methylphenyl)acetamide |
| **147** | 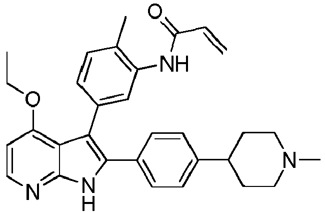 | N-(5-(4-ethoxy-2-(4-(1-methyl piperid in -4-yl)phenyl)-1H-pyrrolo[2,3-b]pyridin-3-yl)-2-methylphenyl)acrylamide |
| **148** | 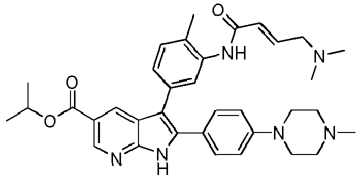 | (E)-isopropyl 3-(3-(4-(dimethylamino)but-2-enamido)-4-methylphenyl)-2-(4-(4-methylpiperazin-1-yl)phenyl)-1H-pyrrolo[2,3-b]pyridine-5-carboxylate |

or an enantiomer, a mixture of enantiomers, a diastereomer, a mixture of diastereomers, a tautomer, a prodrug, a hydrate, a solvate of the above mentioned compounds, or pharmaceutically acceptable salts thereof.

The expression prodrug is defined as a substance, which is applied in an inactive or significantly less active form. Once applied and incorporated, the prodrug is metabolized in the body *in vivo* into the active compound. Conventional procedures for the selection and preparation of suitable prodrug derivatives are described, for example in "Design of Prodrugs", ed. H. B. Bundgaard, Elsevier, 1985.

The present invention also includes within its scope N-oxides of the compounds of formula (I) above. In general, such N-oxides may be formed by conventional means, such as reacting the compound of formula (I) with oxone in the presence of wet alumina.

The expression tautomer is defined as an organic compound that is interconvertible by a chemical reaction called tautomerization. Tautomerization can be catalyzed preferably by bases or acids or other suitable compounds.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

The inventive compounds may exist in a number of different polymorphic forms.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

The inventors found that in order to obtain inhibitors which exhibit metabolic stability against liver microsomes the 2-position at the phenyl ring next to the warhead must be substituted. The warhead can be a but-2-enamide, 4-(dimethylamino)but-2-enamide, propiolamide, ethenesulfonamide, acrylamide, or 2-chloro-acetamide residue. The position 2 on the phenyl ring next to the warhead must be different from hydrogen and is preferably an alkyl or alkylenyl residue such as a methyl group (like in compounds No. 1 - 54, 72 - 82, 116 - 148) or can be part of a ring system preferably containing the nitrogen atom of the warhead (like in compounds No. 55 - 71, 83 - 95, 98 - 115). Without this substituent and especially without this -CH₃ or -CH₂- substituent, no metabolic stability against liver microsomes can be achieved and consequently no pharmaceutical activity can be obtained in vivo.

### Syntheses of compounds

The compound of formula (I) is prepared by reference to the methods illustrated in the following schemes 1-4. The compound of formula (I) is produced by Schemes 1-3 by the suitable selection of reagents with appropriate substitution. Solvents, temperatures, pressures, and other reaction conditions may readily be selected by one of ordinary skill in the art. Starting materials are commercially available or readily prepared by one of ordinary skill in the art.

As shown in Scheme 1, the present invention is directed to a method for producing the compound of formula (**lI**-**1**) comprising:
**Step A1:** perfoming a first cross coupling reaction of pyridine compound 1* with alkyne comopund **2a*** to obtain a compound **3*** in the presence of a first palladium catalyst, and a first base;
**Step B1:** converting a trimethylsilyl group of the compound **3*** to a halide like an iodide to obtain a compound **4*;**
**Step C1:** perfoming a second cross coupling reaction of **4*** with a compound **5*** in the presence of a second palladium catalyst, and a second base to obtain a compound **6*;**
**Step D1:** reducing nitro (NO₂) group of the compound **6*** to a primary amine (NH₂) group to obtain a compound **7*;** and
**Step E1:** perfoming a coupling reaction of the compound **7*** with a compound HO-**R₅** or AG-**R₅** to obtain a product compound of the formula (**Ia-1**).

The compound 1* has following formula 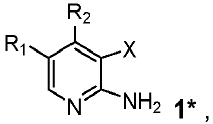
wherein R₁, R₂ have the same meanings as definced in the formula (I);
X is a leaving group and represents Cl, Br, I, or OTf.

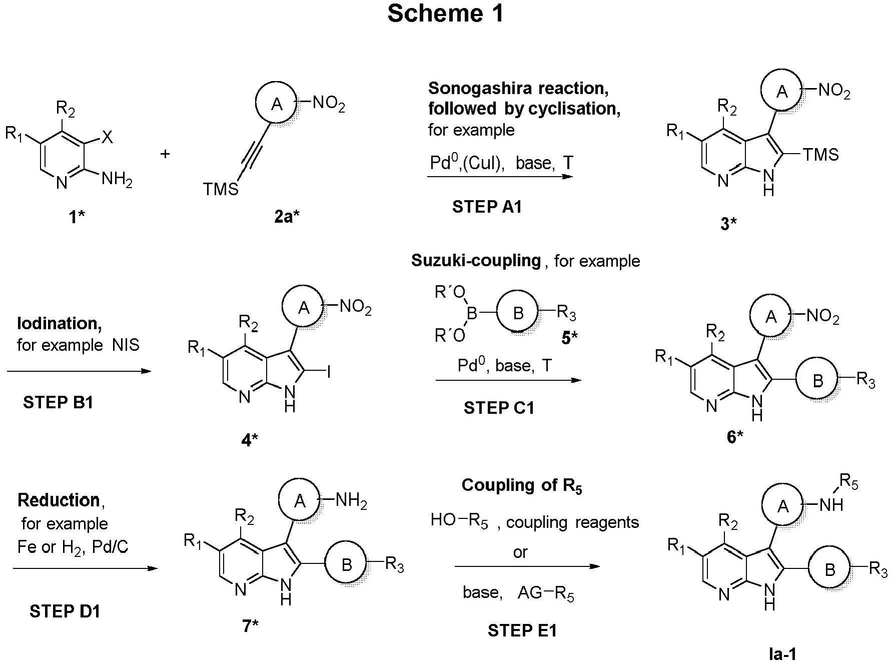

Alternatively, the sequence of the second cross coupling reaction, reduction of nitro group and further coupling reaction may be changed and therefore the product compound la-1 is obtained by the following method.

A method for producing a compound of the formula (**Ia-1**) comprising:
**Step A1**: perfoming a first cross coupling reaction of pyridine compound 1* with alkyne comopund **2a*** to obtain a compound **3*** in the presence of a first palladium catalyst, and a first base;
**Step D2**: reducing nitro (NO₂) group of the compound **3*** to a primary amine (NH₂) group to obtain a compound **10*;**
**Step E2**: perfoming a coupling reaction of the compound **10*** with a compound HO-**R₅** or AG-**R₅** to obtain a compound **11*.**
**Step B2:** converting a trimethylsilyl group of the compound **11*** to a halide like an iodide to obtain a compound **12*;** and
**Step C2:** perfoming a second cross coupling reaction of the compound **12*** with a compound **5*** in the presence of a second palladium catalyst, and a second base to obtain a product compound of the formula (**Ia-1**).

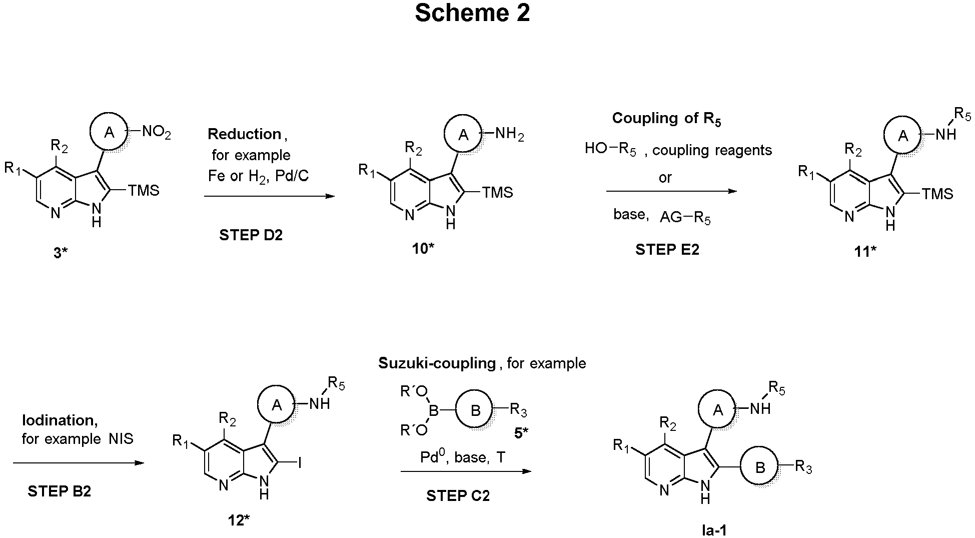

Another aspect of the present invention is a method for producing the compound of formula (Ib) is described in Scheme 3.

A method for producing the compound of formula (Ib) comprising:
**Step A3**:
   i) perfoming a first cross coupling reaction of pyridine compound **1*** with alkyne comopund **2b*** in the presence of a first palladium catalyst, and a first base; and
   ii) removing a protecting group PG of a resulting comopund after the step i) to obtain a compound **3b*;**
**Step E3**: perfoming a coupling reaction of the compound **3b*** with a compound HO-**R₅** or AG-**R₅** to obtain a compound **11b*;**
**Step B3:** converting a trimethylsilyl group of the compound **11b*** to a halide like an iodide to obtain a compound **12b*;** and
**Step C3:** perfoming a second cross coupling reaction of the compound **12b*** with a compound **5*** in the presence of a second palladium catalyst, and a second base to obtain a product compound of the formula (**Ib**).

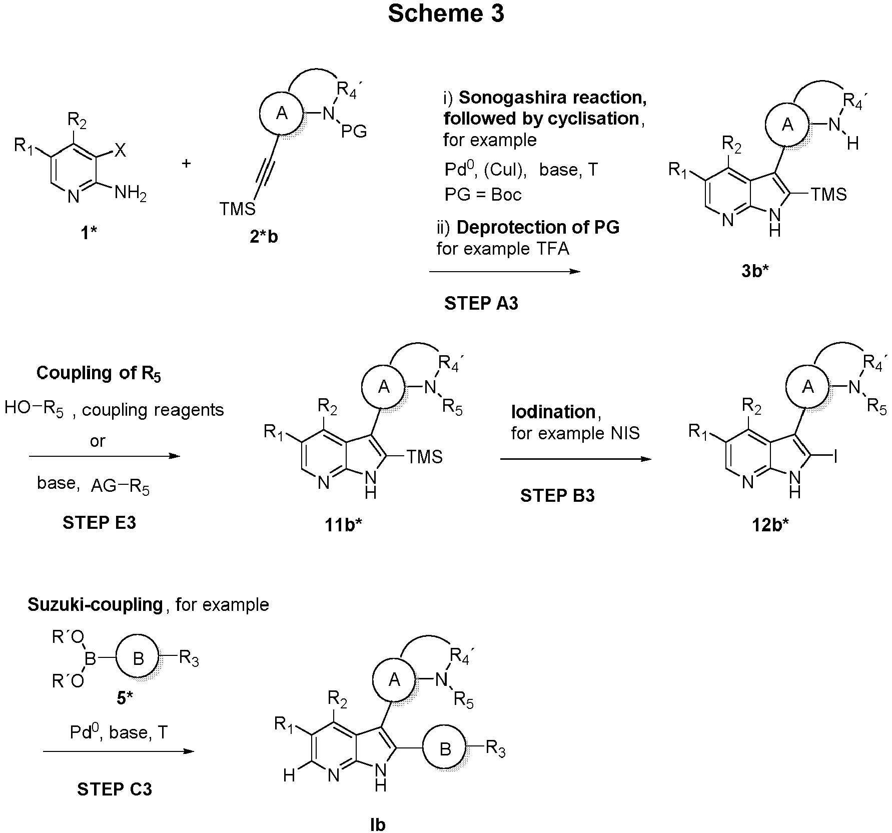

A further method for producing the compound of formula (la-1) is described in Scheme 4.

A further method for producing the compound of formula (la-1) compring:
**Step A4:** perfoming a first cross coupling reaction of pyridine compound **1*** with alkyne comopund **2b*** to obtain a compound **13*** in the presence of a first palladium catalyst, and a first base; and
**Step B4:** converting a trimethylsilyl group of the compound **13*** to a halide like an iodide to obtain a compound **14*;**
**Step C4:** perfoming a second cross coupling reaction of the compound **14*** with a compound **5*** in the presence of a second palladium catalyst, and a second base to obtain a compound **15*;** and
**Step E4:**
   i) removing a protecting group PG of the compound **15*;**
   ii) perfoming a coupling reaction of the resulting compound after step i) with a compound HO-**R₅** or AG-**R₅** to obtain product compound of the formula (**Ib**).

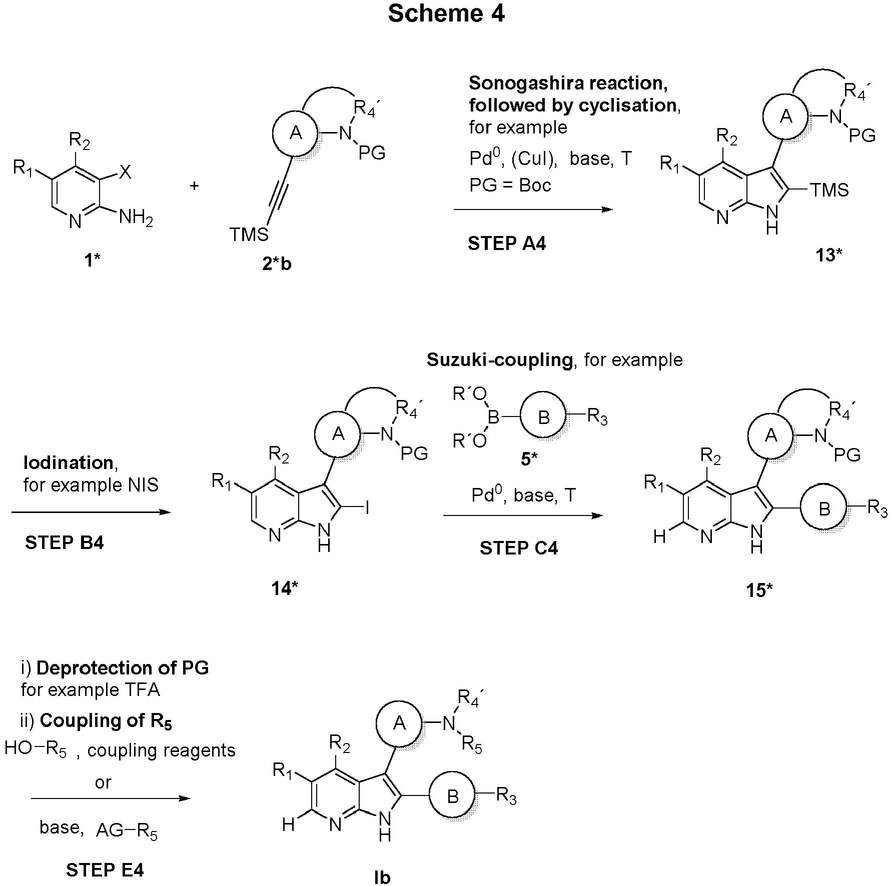

In each of the steps **A1, A3,** and **A4,** Sonogashia coupling reaction of compound **1*** and domino cyclization of the resulting coupling compound is performed in the present of the first palladium catalyst, and the base. Optionally, copper catalyst may be used as cocatalyst and is preferred Cu(I) and more preferred CuI.

The first palladium catalyst for C-C coupling reaction is Pd(0) or Pd(II) catalyst, preferred PdCl₂, Pd(Ph₃)₂, Pd(acac)₂, PdCl₂(CH₃CN)₂, PdCl₂(PCy₃)₂, PdCl₂(Ph₃)₂, Pd(Ph₃)Cl₂, [(π-ally)PdCl]₂, (SlPr)PdCl₂(TEA). Optionally, further phosphine ligand may be used together with the first palladium catalyst.

A ratio of the palladium catalyst to the starting meterial is in the range of 0.01 to 20 mol-%, preferred 0.01-10 mol-%, more preferred 0.01-5 mol-%, most preferred 0.01-1 mol-%.
The first base may be an organic base or inorganic bases. The organic base may be tertiary amine such as Et₃N, and DIPEA, DABCO, DBU, pyrrolidine or piperidine. The inorganic base may be K₂CO₃, CS₂CO₃ or K₃PO₄. A ratio of the first base to the starting meterial is in the range of 1.0 to 5.0 equivalents, preferred 1.0 to 3.0 equivalents, more preferred 1.0 to 3.0 equivalents, most preferred 1.0 to 1.5 equivalents.
Preferred, this reaction is performed in a polar aprotic solvent such as DMF or DMSO under N₂ atmosphere at a temperature in a range of 80 to 200°C, preferred 100 to 180°C, more preferred 100 to 150°C, most preferred 120 to 150°C.

In each of the steps **B1, B2, B3** and **B4,** conversion of trimethylsilyl (TMS) group to iodide group is performed by treating with N-iodosuccinimide (NIS) as an idonation reagent for 15 h at a temperature in a range of 10 to 35°C, preferred, 15 to 30 °C, more preferred 20 to 30 °C. A polar aprotic solvent such as dichlorormethane or chlororform is used.

In each of the steps **C1, C2, C3,** and **C4,** Suzuki coupling reaction of iodinated compound **4*, 12*, 12b*,** or **14*** with a compound **5*** as a boronic acid derivative is performed in the present in the second palladium catalyst and the second base. 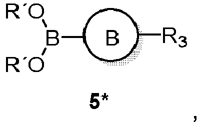 wherein **R'** is H or an alkyl chain with 1-10 carbon atoms or a cycloalkyl chain with 3 to 12 carbon atoms or both residues R' represent together a residue derived from pinacol.
Preferred, the boronic acid derivative may be a boronic acid (R' = -H) or an ester of the boronic acid, e.g. its isopropyl ester (R' = -CH(CH₃)₂), an ester derived from pinacol (R'-R' = -C(CH₃)₂-C(CH₃)₂-).

The second palladium catalyst is Pd(0) or Pd(II) catalyst. The Pd(0) catalyst may be tetrakis(triphenylphosphine)palladium(0) [Pd(PPh₃)₄], tris(dibenzylideneacetone)di-palladium(0) [Pd₂(dba)₃]. Pd(II) catalyst may be dichlorobis(triphenylphosphine)-palladium(II) [Pd(PPh₃)₂Cl₂], palladium(II) acetate and triphenylphosphine or more preferred [1,1'-bis(diphenylphosphino)ferrocene]palladium dichloride. The reaction is preferably carried out in a mixture of a solvent like dioxane, DMF, DME, THF, or isopropanol with water and in the presence of the second base like aqueous sodium bicarbonate or K₃PO₄.

In each of the Steps **D1** and **D2,** a nitro (NO₂) group is reduced to a primary amine (NH₂) group with the treatment of a reducing agent. Preferred, Fe, or Pd/H₂ is used as a reducing agent.

In each of the Steps **E1, E2, E3** and **E4, R**₅ group is introduced by a coupling reaction with HO-**R₅** or AG-**R₅**.
For perfoming the coupling reaction, firstly carboxylic acid or sulfonic acid group of HO-R₅ is activated *in situ* to promote the coupling reaction with amino group of intermediate compound. If HO-R₅ is a carboxylic acid, the activating group (AG) of carboxylic acid may be introduced *in situ* reaction. Preferably, the activating group (AG) may be selected from the group consisting of or comprising: halides such as -F, -Br, -Cl, -I, anhydride group such as - OCOCH₃, N-oxy-benzotriazol group and N-oxy-succinimide. Preferably,

### HO-R₅ represents

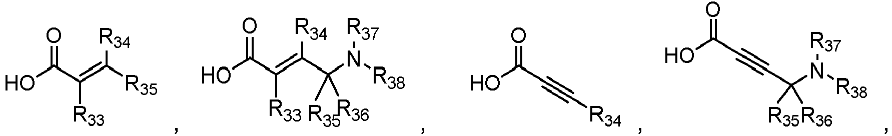

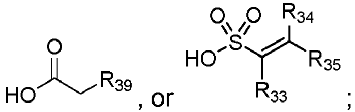

Further, a carboxylic acid of HO-**R₅** is coupled with the amine group of intermediate compound by a well-known amide coupling reaction. Any of the following coupling reagent can be used for amide coupling reaction: BOP, PyBOP, AOP, PyAOP, TBTU, EEDQ, Polyphosphoric Acid (PPA), DPPA, HATU, HOBt, HOAt, DCC, EDCI, BOP-Cl, TFFH, Brop, PyBrop, and CIP.

For perfoming the coupling reaction, also an activated compound AG-R₅ having activated carboxyl sulfony group can be used.

### AG-R₅ represents

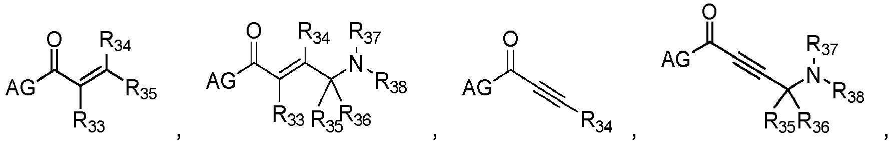

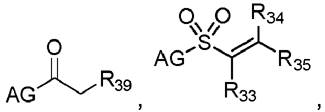

As above described, the activating group (AG) may be selected from the group consisting of or comprising: halides such as -F, -Br, -Cl, -I, anhydride group such as -OCOCH₃, N-oxy-benzotriazol group and N-oxy-succinimide, preferred AG is Cl.

### Preferred, AG-R₅ is

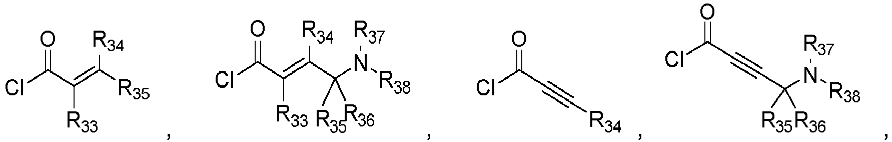

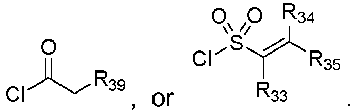

In the Step **A3** and **E4,** a protecting group (PG) is an amino protecting group. The amine protecting group may be t-butyloxycarbonyl (Boc) or benzyloxycarbonyl (Cbz) and removed under acidic condition, for example, treating with HCI, or TFA.

In an embodiment, the present invention is directed to an intermediate compound selected from the group consisting of the compounds **3*, 3b*, 4*, 6*, 7*, 10*, 11*, 11b*, 12*, 12b*, 13*, 14*,** and **15*:** 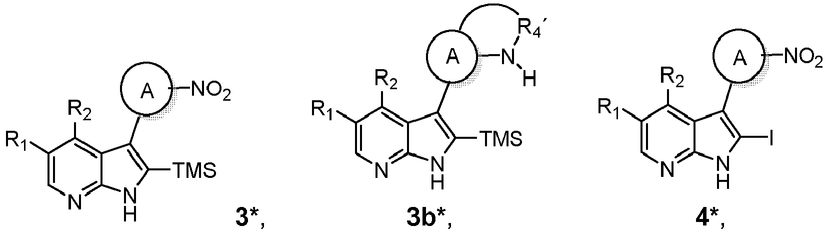 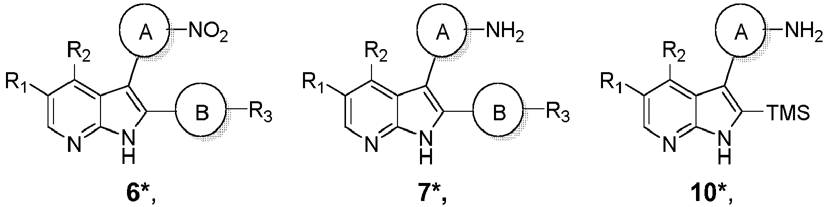 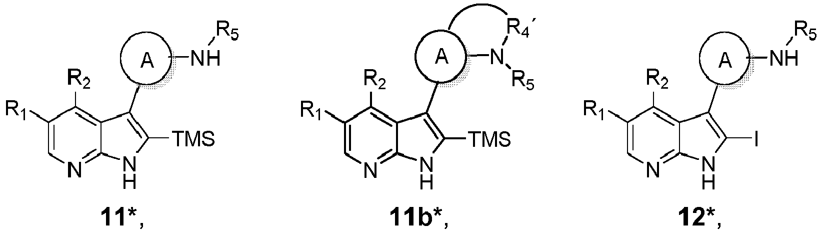 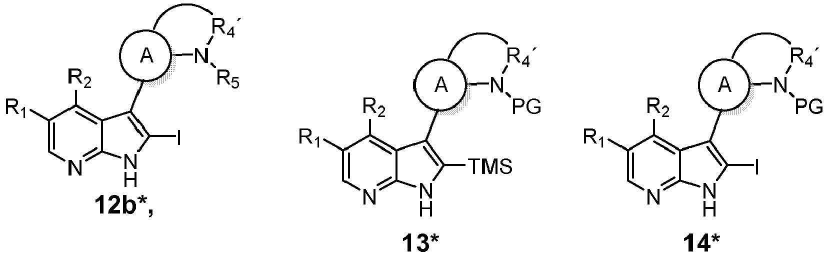 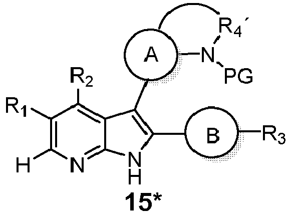 wherein A, B, R₁, R₂, R₃, R₄, R_{4'}, R₅, PG and 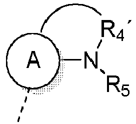 have the same meanings as defined above; and
TMS is trimethyl silyl group.

The structure activity relationship (SAR) of the compounds of the present invention as represented by the following formula (IV) shows covalent inhibitors as cellular potent mutant-selective ErbB inhibitors. The covalent binding mode, obtained for example through the introduction of an acrylic moiety, is crucial for cellular activity. The direct comparison of examples from this invention having a acryl moiety for the covalent binding with corresponding molecules having a propionic moiety are showing the improved cellular activities for the covalent binder.

Table 6 shows comparison of covalent inhibitors (Example 6 and 21) with the corresponding reversible analogues (Refernces 1 and 2). In all cases the covalent inhibitor shows (Example 6 and 21) improved cellular activities compared to the reversible analogues (Refernces 2 and 3).

It is found that the compound of the present invention is useful for the prophylaxis and/or the treatment of cancer having activating mutation of a receptor belonging to the ErbB family of receptor, preferred, the mutation is an insertion within exon 20 of EGFR or within exon 20 of HER2.
The compound of the present invention is a selective inhibitor of mutants of EGFR and Her2, preferred Exon 20 mutations as shown in Table 3.
Thus, the compound of the present invention is useful as a medicament. The compound of the present invention is useful for the prophylaxis and/or the treatment of cell proliferative disease, especially cancer.

Said cancer is selected from breast cancer, colon cancer, prostate cancer, lung cancer, gastric cancer, ovarian cancer, endometrial cancer, renal cancer, hepatocellular cancer, thyroid cancer, uterine cancer, esophagus cancer, squamous cell cancer, leukemia, lymphoma, osteosarcoma, mamma carcinoma, melanoma, glioblastoma and neuroblastoma.

In specific embodiment, the compound is useful for in the the prophylaxis and/or the treatment of non small cell lung cancer (NSCLC) or mamma carcinoma.

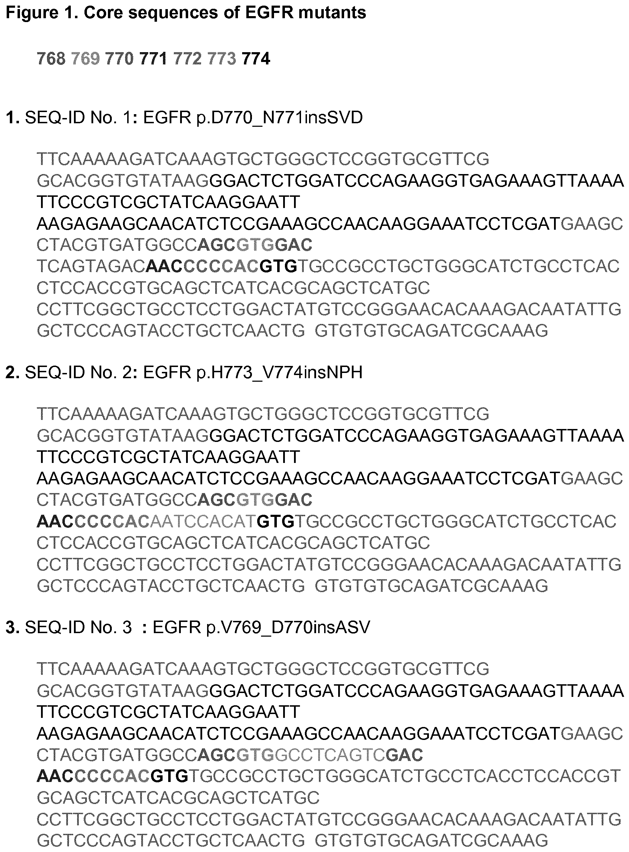

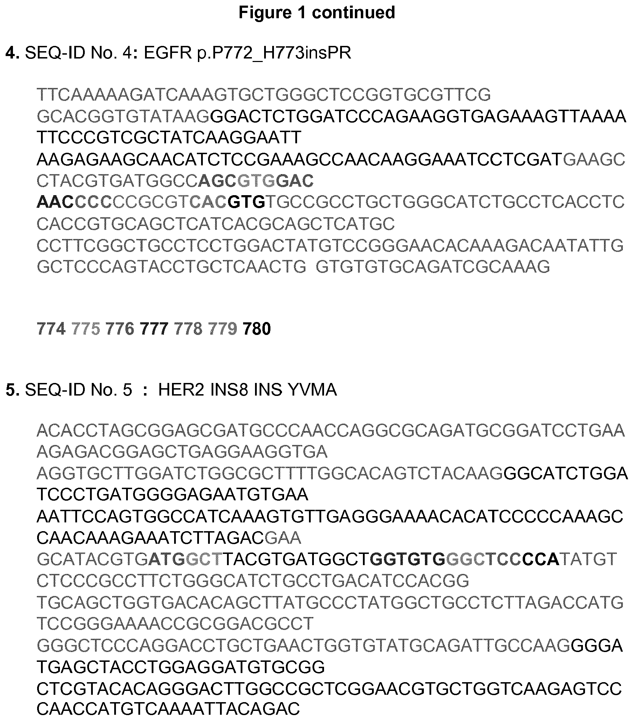

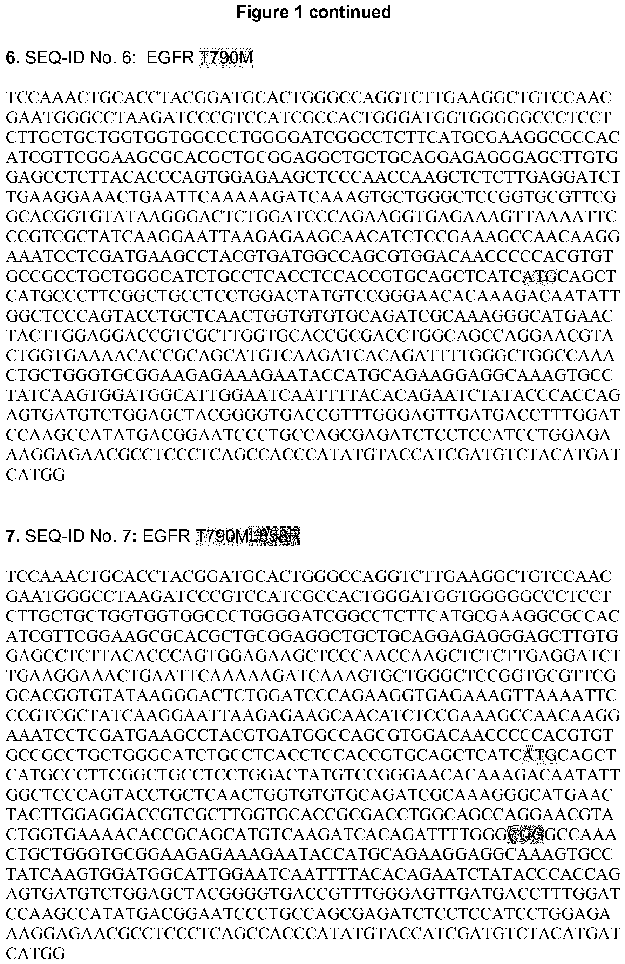

Preferred, the mutation is an insertion within exon 20 of EGFR or within exon 20 of HER2. One aspect herein are the compounds of the present invention for use in the prophylaxis and/or the treatment of cancer caused by or associated with mutations associated with EGFR TKI resistance and in particular caused by or associated with in-frame insertions and/or duplications of 3 to 21 base pairs (bp) between codons 763 and 775 of the Her2 gene or the EGFR (Her1) gene. More preferred, the mutation is selected from the group consisting of Her2 INS8 INS YVMA, EGFR D770_N771 insSVD, EGFR H773 _V774insNPH, EGFR V769_D770insASV, EGFR P772_H773insPR, EGFR T790M and EGFR T790ML858R. The important sequences are shown in SEQ-ID No. 1 to SEQ:NO 7 in Figure 1
The compounds of the present invention have improved stability towards hydrolysis of the acrylamide moiety in liver microsomes as summairzed in Table 5.

### Preferably, the following compounds have improved stability:

N-(5-(4-chloro-2-(4-((4-methylpiperazin-1-yl)methyl)phenyl)-1 H-pyrrolo[2,3-b]pyridin-3-yl)-2-methylphenyl)acrylamide, N-(5-(4-chloro-2-(4-(4-methylpiperazin-1-yl)phenyl)-1H-pyrrolo[2,3-b]pyridin-3-yl)-2-methylphenyl)acrylamide, N-(5-(4-chloro-2-(4-(1-methylpiperidin-4-yl)phenyl)-1H-pyrrolo[2,3-b]pyridin-3-yl)-2-methylphenyl)acrylamide, N-(5-(4-chloro-2-(4-(2-(dimethylamino)ethyl)phenyl)-1 H-pyrrolo[2,3-b]pyridin-3-yl)-2-methylphenyl)acrylamide, N-(5-(4-chloro-2-(4-(morpholinomethyl)phenyl)-1H-pyrrolo[2,3-b]pyridin-3-yl)-2-methylphenyl)acrylamide, N-(5-(4-chloro-2-(3-morpholinophenyl)-1H-pyrrolo[2,3-b]pyridin-3-yl)-2-methylphenyl)acrylamide, N-(5-(2-(4-(4-acetylpiperazin-1-yl)phenyl)-4-chloro-1H-pyrrolo[2,3-b]pyridin-3-yl)-2-methylphenyl)acrylamide, N-(5-(4-chloro-2-(4-(4-methylpiperazine-1-carbonyl)phenyl)-1H-pyrrolo[2,3-b]pyridin-3-yl)-2-methylphenyl)acrylamide, N-(5-(4-chloro-2-(4-((dimethylamino)methyl)phenyl)-1H-pyrrolo[2,3-b]pyridin-3-yl)-2-methylphenyl)acrylamide, N-(5-(4-chloro-2-(4-(morpholine-4-carbonyl)phenyl)-1H-pyrrolo[2,3-b]pyridin-3-yl)-2-methylphenyl)acrylamide, N-(5-(4-chloro-2-(4-(3-(dimethylamino)propoxy)phenyl)-1H-pyrrolo[2,3-b]pyridin-3-yl)-2-methylphenyl)acrylamide, N-(5-(4-chloro-2-(4-(4-(ethylsulfonyl)piperazin-1-yl)phenyl)-1H-pyrrolo[2,3-b]pyridin-3-yl)-2-methylphenyl)acrylamide, N-(5-(4-chloro-2-(3-((4-methylpiperazin-1-yl)methyl)phenyl)-1 H-pyrrolo[2,3-b]pyridin-3-yl)-2-methylphenyl)acrylamide, N-(5-(4-chloro-2-(3-(4-methylpiperazin-1-yl)phenyl)-1H-pyrrolo[2,3-b]pyridin-3-yl)-2-methylphenyl)acrylamide, N-(5-(4-chloro-2-(4-(2-(dimethylamino)ethoxy)phenyl)-1 H-pyrrolo[2,3-b]pyridin-3-yl)-2-methylphenyl)acrylamide, N-(5-(4-chloro-2-(6-(4-methylpiperazin-1-yl)pyridin-3-yl)-1H-pyrrolo[2,3-b]pyridin-3-yl)-2-methylphenyl)acrylamide, N-(5-(4-chloro-2-(3-(3-(dimethylamino)propoxy)phenyl)-1H-pyrrolo[2,3-b]pyridin-3-yl)-2-methylphenyl)acrylamide, N-(5-(4-chloro-2-(2-methoxy-4-(4-methylpiperazin-1-yl)phenyl)-1H-pyrrolo[2,3-b]pyridin-3-yl)-2-methylphenyl)acrylamide, N-(5-(2-(3-((4-acetylpiperazin-1-yl)methyl)phenyl)-4-chloro-1H-pyrrolo[2,3-b]pyridin-3-yl)-2-methylphenyl)acrylamide, N-(5-(4-chloro-2-(4-(3-(4-ethylpiperazin-1-yl)propoxy)phenyl)-1H-pyrrolo[2,3-b]pyridin-3-yl)-2-methylphenyl)acrylamide, N-(5-(4-chloro-2-(pyrimidin-5-yl)-1H-pyrrolo[2,3-b]pyridin-3-yl)-2-methylphenyl)acrylamide, N-(5-(4-chloro-2-(3-(3-(4-ethylpiperazin-1-yl)propoxy)phenyl)-1H-pyrrolo[2,3-b]pyridin-3-yl)-2-methylphenyl)acrylamide, N-(5-(4-chloro-2-(2-(4-methylpiperazin-1-yl)pyridin-4-yl)-1 H-pyrrolo[2,3-b]pyridin-3-yl)-2-methylphenyl)acrylamide, N-(5-(4-chloro-2-(3-(morpholine-4-carbonyl)phenyl)-1H-pyrrolo[2,3-b]pyridin-3-yl)-2-methylphenyl)acrylamide, N-(5-(4-chloro-2-(3-(2-(dimethylamino)ethoxy)phenyl)-1H-pyrrolo[2,3-b]pyridin-3-yl)-2-methylphenyl)acrylamide, N-(5-(4-chloro-2-(4-(piperidin-4-yl)phenyl)-1H-pyrrolo[2,3-b]pyridin-3-yl)-2-methylphenyl)acrylamide, N-(5-(4-chloro-2-(6-morpholinopyridin-3-yl)-1H-pyrrolo[2,3-b]pyridin-3-yl)-2-methylphenyl)acrylamide, N-(5-(2-(6-(4-acetylpiperazin-1-yl)pyridin-3-yl)-4-chloro-1H-pyrrolo[2,3-b]pyridin-3-yl)-2-methylphenyl)acrylamide, N-(5-(4-chloro-2-(2-morpholinopyridin-4-yl)-1H-pyrrolo[2,3-b]pyridin-3-yl)-2-methylphenyl)acrylamide, N-(5-(4-chloro-2-(2-morpholinopyrimidin-5-yl)-1H-pyrrolo[2,3-b]pyridin-3-yl)-2-methylphenyl)acrylamide, N-(5-(4-chloro-2-(3-(2-(pyrrolidin-1-yl)ethoxy)phenyl)-1H-pyrrolo[2,3-b]pyridin-3-yl)-2-methylphenyl)acrylamide, N-(5-(4-chloro-2-(4-(2-(pyrrolidin-1-yl)ethoxy)phenyl)-1H-pyrrolo[2,3-b]pyridin-3-yl)-2-methylphenyl)acrylamide, N-(5-(4-chloro-2-(2-(4-methylpiperazin-1-yl)pyrimidin-5-yl)-1 H-pyrrolo[2,3-b]pyridin-3-yl)-2-methylphenyl)acrylamide, N-(5-(4-chloro-2-(4-(4-ethylpiperazin-1-yl)phenyl)-1H-pyrrolo[2,3-b]pyridin-3-yl)-2-methylphenyl)acrylamide, N-(5-(4-chloro-2-(4-(4-isopropylpiperazin-1-yl)phenyl)-1H-pyrrolo[2,3-b]pyridin-3-yl)-2-methylphenyl)acrylamide, N-(5-(4-chloro-2-(3-fluoro-4-(4-methylpiperazin-1-yl)phenyl)-1H-pyrrolo[2,3-b]pyridin-3-yl)-2-methylphenyl)acrylamide, N-(5-(4-chloro-2-(4-((2-(dimethylamino)ethyl)(methyl)amino)phenyl)-1H-pyrrolo[2,3-b]pyridin-3-yl)-2-methylphenyl)acrylamide, 1-(6-(4-chloro-2-(4-(1-methylpiperidin-4-yl)phenyl)-1H-pyrrolo[2,3-b]pyridin-3-yl)indolin-1-yl)prop-2-en-1-one, 1-(6-(4-chloro-2-(4-(4-methylpiperazin-1-yl)phenyl)-1H-pyrrolo[2,3-b]pyridin-3-yl)indolin-1-yl)prop-2-en-1-one, 1-(6-(4-chloro-2-(3-fluoro-4-(4-methylpiperazin-1-yl)phenyl)-1H-pyrrolo[2,3-b]pyridin-3-yl)indolin-1-yl)prop-2-en-1-one, 1-(6-(2-(4-(4-acetylpiperazin-1-yl)phenyl)-4-chloro-1H-pyrrolo[2,3-b]pyridin-3-yl)indolin-1-yl)prop-2-en-1-one, 1-(6-(4-chloro-2-(2-(4-methylpiperazin-1-yl)pyridin-4-yl)-1H-pyrrolo[2,3-b]pyridin-3-yl)indolin-1-yl)prop-2-en-1-one, 1-(6-(4-chloro-2-(4-((4-methylpiperazin-1-yl)methyl)phenyl)-1H-pyrrolo[2,3-b]pyridin-3-yl)indolin-1-yl)prop-2-en-1-one, 1-(6-(4-chloro-2-(4-((dimethylamino)methyl)phenyl)-1H-pyrrolo[2,3-b]pyridin-3-yl)indolin-1-yl)prop-2-en-1-one, 1-(6-(4-chloro-2-(4-(4-isopropylpiperazin-1-yl)phenyl)-1H-pyrrolo[2,3-b]pyridin-3-yl)indolin-1-yl)prop-2-en-1-one, 1-(6-(4-chloro-2-(4-(4-ethylpiperazin-1-yl)phenyl)-1H-pyrrolo[2,3-b]pyridin-3-yl)indolin-1-yl)prop-2-en-1-one, 1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one, 1-(6-(4-chloro-2-(4-((2-(dimethylamino)ethyl)(methyl)amino)phenyl)-1 H-pyrrolo[2,3-b]pyridin-3-yl)indolin-1-yl)prop-2-en-1-one, 1-(6-(4-chloro-2-(4-(4-methylpiperazine-1-carbonyl)phenyl)-1H-pyrrolo[2,3-b]pyridin-3-yl)indolin-1-yl)prop-2-en-1-one, 1-(6-(4-chloro-2-(4-(2-(dimethylamino)ethoxy)phenyl)-1 H-pyrrolo[2,3-b]pyridin-3-yl)indolin-1-yl)prop-2-en-1-one, 1-(6-(4-chloro-2-(4-(morpholinomethyl)phenyl)-1H-pyrrolo[2,3-b]pyridin-3-yl)indolin-1-yl)prop-2-en-1-one, 1-(6-(4-chloro-2-(3-((4-methylpiperazin-1-yl)methyl)phenyl)-1H-pyrrolo[2,3-b]pyridin-3-yl)indolin-1-yl)prop-2-en-1-one, 1-(6-(4-chloro-2-(6-(4-methylpiperazin-1-yl)pyridin-3-yl)-1H-pyrrolo[2,3-b]pyridin-3-yl)indolin-1-yl)prop-2-en-1-one, 1-(6-(4-chloro-2-(4-(3-(dimethylamino)propoxy)phenyl)-1H-pyrrolo[2,3-b]pyridin-3-yl)indolin-1-yl)prop-2-en-1-one, N-(5-(4-methoxy-2-(4-(4-methylpiperazin-1-yl)phenyl)-1H-pyrrolo[2,3-b]pyridin-3-yl)-2-methylphenyl)acrylamide, N-(5-(4-methoxy-2-(4-(1-methylpiperidin-4-yl)phenyl)-1H-pyrrolo[2,3-b]pyridin-3-yl)-2-methylphenyl)acrylamide, N-(5-(2-(4-(4-acetylpiperazin-1-yl)phenyl)-4-methoxy-1H-pyrrolo[2,3-b]pyridin-3-yl)-2-methylphenyl)acrylamide, N-(5-(4-methoxy-2-(4-(morpholinomethyl)phenyl)-1H-pyrrolo[2,3-b]pyridin-3-yl)-2-methylphenyl)acrylamide, N-(5-(4-methoxy-2-(3-((4-methylpiperazin-1-yl)methyl)phenyl)-1H-pyrrolo[2,3-b]pyridin-3-yl)-2-methylphenyl)acrylamide, N-(5-(4-methoxy-2-(4-((4-methylpiperazin-1-yl)methyl)phenyl)-1 H-pyrrolo[2,3-b]pyridin-3-yl)-2-methylphenyl)acrylamide, N-(2-methyl-5-(2-(6-(4-methylpiperazin-1-yl)pyridin-3-yl)-1H-pyrrolo[2,3-b]pyridin-3-yl)phenyl)acrylamide, N-(2-methyl-5-(2-(4-(1-methylpiperidin-4-yl)phenyl)-1H-pyrrolo[2,3-b]pyridin-3-yl)phenyl)acrylamide, N-(2-methyl-5-(2-(2-morpholinopyridin-4-yl)-1H-pyrrolo[2,3-b]pyridin-3-yl)phenyl)acrylamide, N-(5-(2-(3-((4-acetylpiperazin-1-yl)methyl)phenyl)-1H-pyrrolo[2,3-b]pyridin-3-yl)-2-methylphenyl)acrylamide, N-(2-methyl-5-(2-(3-(morpholinomethyl)phenyl)-1H-pyrrolo[2,3-b]pyridin-3-yl)phenyl)acrylamide, 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-((2-(dimethylamino)ethyl)(methyl)amino)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one, 1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one, 1-(7-(4-chloro-2-(3-(2-(dimethylamino)ethoxy)phenyl)-1H-pyrrolo[2,3-b]pyridin-3-yl)-3,4-dihydroquinolin-1(2H)-yl)prop-2-en-1-one, 1-(7-(4-chloro-2-(4-(4-isopropylpiperazin-1-yl)phenyl)-1H-pyrrolo[2,3-b]pyridin-3-yl)-3,4-dihydroquinolin-1(2H)-yl)prop-2-en-1-one, 1-(7-(4-chloro-2-(4-(2-(dimethylamino)ethoxy)phenyl)-1H-pyrrolo[2,3-b]pyridin-3-yl)-3,4-dihydroquinolin-1 (2H)-yl)prop-2-en-1-one, 1-(7-(4-chloro-2-(6-(4-methylpiperazin-1-yl)pyridin-3-yl)-1H-pyrrolo[2,3-b]pyridin-3-yl)-3,4-dihydroquinolin-1 (2H)-yl)prop-2-en-1-one, 1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one, N-(5-(4-chloro-2-(4-(piperazin-1-yl)phenyl)-1H-pyrrolo[2,3-b]pyridin-3-yl)-2-methylphenyl)acrylamide, N-(5-(5-cyano-2-(4-(4-methylpiperazin-1-yl)phenyl)-1H-pyrrolo[2,3-b]pyridin-3-yl)-2-methylphenyl)acrylamide, 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one, 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one, 1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one, 1-(6-(4-ethoxy-2-(4-(4-methylpiperazin-1-yl)phenyl)-1H-pyrrolo[2,3-b]pyridin-3-yl)indolin-1-yl)prop-2-en-1-one, 1-(6-(4-ethoxy-2-(4-((4-methylpiperazin-1-yl)methyl)phenyl)-1H-pyrrolo[2,3-b]pyridin-3-yl)indolin-1-yl)prop-2-en-1-one, 1-(6-(4-ethoxy-2-(3-(2-(pyrrolidin-1-yl)ethoxy)phenyl)-1H-pyrrolo[2,3-b]pyridin-3-yl)indolin-1-yl)prop-2-en-1-one, 1-(6-(4-methoxy-2-(4-(4-methylpiperazin-1-yl)phenyl)-1H-pyrrolo[2,3-b]pyridin-3-yl)indolin-1-yl)prop-2-en-1-one, 1-(6-(4-methoxy-2-(2-(4-methylpiperazin-1-yl)pyridin-4-yl)-1H-pyrrolo[2,3-b]pyridin-3-yl)indolin-1-yl)prop-2-en-1-one, 1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1-one, 1-(6-(4-methoxy-2-(4-((4-methylpiperazin-1-yl)methyl)phenyl)-1H-pyrrolo[2,3-b]pyridin-3-yl)indolin-1-yl)prop-2-en-1-one, 1-(6-(4-isopropoxy-2-(4-(4-methylpiperazin-1-yl)phenyl)-1H-pyrrolo[2,3-b]pyridin-3-yl)indolin-1-yl)prop-2-en-1-one, 3-(3-acrylamido-4-methylphenyl)-2-(4-(4-methylpiperazin-1-yl)phenyl)-1H-pyrrolo[2,3-b]pyridine-5-carboxylic acid, isopropyl 3-(3-acrylamido-4-methylphenyl)-2-(4-(4-methylpiperazin-1-yl)phenyl)-1H-pyrrolo[2,3-b]pyridine-5-carboxylate, 3-(3-acrylamido-4-methylphenyl)-N-isopropyl-2-(4-(4-methylpiperazin-1-yl)phenyl)-1H-pyrrolo[2,3-b]pyridine-5-carboxamide, 3-(3-acrylamido-4-methylphenyl)-N-cyclohexyl-2-(4-(4-methylpiperazin-1-yl)phenyl)-1 H-pyrrolo[2,3-b]pyridine-5-carboxamide, methyl 3-(3-acrylamido-4-methylphenyl)-2-(4-(4-methylpiperazin-1-yl)phenyl)-1H-pyrrolo[2,3-b]pyridine-5-carboxylate, ethyl 3-(3-acrylamido-4-methylphenyl)-2-(4-(4-methylpiperazin-1-yl)phenyl)-1H-pyrrolo[2,3-b]pyridine-5-carboxylate, cyclopropyl 3-(3-acrylamido-4-methylphenyl)-2-(4-(4-methylpiperazin-1-yl)phenyl)-1H-pyrrolo[2,3-b]pyridine-5-carboxylate, N-(5-(4-ethoxy-2-(4-(1-methylpiperidin-4-yl)phenyl)-1H-pyrrolo[2,3-b]pyridin-3-yl)-2-methylphenyl)acrylamide.

In an embodiment, the present invention is directed to the compound of in combination with at least one anticancer drug for use in treatment of cancer. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway.

The anticancer drug inhibting EGFR/HER2 tyrosine kinases is selected from the group consisting of A928605, ABT414, ABT806, AC480, Adgef (gefitinib), AEE788, AF802 (alectinib hydrochloride), Afatinib, Afinitor (everolimus), AFM21, AG1478, AGT2000, AL6802, ALL3, AMG595, Anti-Cripto-1 monoclonal antibodies PRIMA BIOMED, AP23464, AP26113, ARQ197 (tivantinib), ARRY380, ARRY543 (varlitinib tosylate), ASP7487 (linsitinib), ASP8273, AV203, AVL291, AZD4769, AZD9291, B-Peptimetics ANTYRA, BGB324, BIBW2948BS, Bispecific Anti-Her2 Zybodies ZYNGENIA (trastuzumab), Bispecific Anti-Her3 Zybodies ZYNGENIA (cetuximab), Bispecific Antibody Drug Conjugate Program AVIDBIOLOGICS, BL7030, BMS536924, BMS582664 (brivanib alaninate), BMS690514, BMSATI2, BT2111 (trastuzumab), CAB051, Canertinib, Caprelsa (vandetanib), CCX140, CDX110, CetuGEX, Cetuximab AMGEN, Cetuximab BIOXPRESS THERAPEUTICS, Cetuximab HARVEST MOON, Cetuximab ONCOBIOLOGICS, Cetuximab PANACEA BIOTECH, Cetuximab PLANTFORM, Cetuximab ZENOTECH, CGP59326A, Chemofit (gefitinib), CI1033 (canertinib dihydrochloride), CIMAher (nimotuzumab), Citoprot-P, CMAB009 (cetuximab), cMet-EGFR Dual Inhibitor CRYSTALGENOMICS, CO-1686, Cometriq (cabozantinib), Conmana (icotinib hydrochloride), CTP15 (cetuximab), CTX023, CUDC101, CYC202 (seliciclib), DC101, DXL1218, EDP13, EGF816, EGFR Antibody Drug Conjugate AVIDBIOLOGICS, EGFR BiTE antibody MICROMET, EGFR Monoclonal Antibody REVO, EGFR Probody Drug Conjugate CYTOMX, EGFR-Antibody-Targeted Endostatin Payload, EGFR501, EKB569 (pelitinib), EM1-mAb GENMAB, EMD121974 (cilengitide), EMD72000 (matuzumab), Emfib (gefitinib), Epidermal Growth Factor Receptor (EGFR) humanized antibody CHINA PHARMAHUB, Erbitux (cetuximab), Erlobenz (erlotinib), Erlocip (erlotinib), Erlonat NATCO (erlotinib), Erlonat RADIANCE (erlotinib), Erlons (erlotinib hydrochloride), Erlostar (erlotinib), Erloswift (erlotinib), Erlotad (erlotinib), Erlotaz (erlotinib), Erlotinib CYNO PHARMACEUTICALS (erlotinib hydrochloride), Erlotinib hydrochloride HETERO, Erlotinib Hydrochloride MYLAN, Erlotinib hydrochloride TEVA, Erlotinib LABORATORIOS INDUQUIMICA, Erlotinib NAPROD, Erlotinib P2D BIOSCEINCE, Erlotinib SALIUS, Erlotinib SRS PHARMA, Erlotinib UNITED BIOTECH, Etk/Bmx Kinase Inhibitor ASTEX, EZN3920, Fderceptin SHANGHAI FUDAN-ZHANGJIANG, FV225, Geffy (gefitinib), Geficad (gefitinib), Gefitinib CELOGEN, Gefitinib CYNO PHARMACEUTICALS, Gefitinib HETERO, Gefitinib LABORATORIOS INDUQUIMICA, Gefitinib MARKSANS, Gefitinib MISSION VIVACARE, Gefitinib NAPROD, Gefitinib SALIUS, Gefitinib SAVA, Gefitinib SRS PHARMA, Gefitinib UNITED BIOTECH, Gefitoz (gefitinib), GefiTrust (gefitinib), Gefnib (gefitinib), Geftex (gefitinib), Geftib (gefitinib), Gefticip (gefitinib), Geftifos (gefitinib), Geftiget (gefitinib), Geftin (gefitinib), Geftinat NATCO (gefitinib), Geftinat RADIANCE (gefitinib), Geftistar (gefitinib), Genasense (oblimersen sodium), GI3000, Gilotrif (aftinib), GSK2136773, HC15, HD201 (trastuzumab), HE39, HER-1 therapeutic cancer vaccine BIOVEN, HER1 Cancer Vaccine, HER2 BiTE Antibody AMGEN, Herzuma (trastuzumab), HKI357, HM60390, HM61713, HM78136B (poziotinib), HMPL309 (theliatinib), HMPL504 (volitinib), HMPL813 (epitinib), HuMax-EGFr (zalutumumab), HyERB (cetuximab), ICS283, Imatinib Ecker hydrochloride, Imbruvica (ibrutinib), IMC11F8 (necitumumab), IMCA12 (cixutumumab), IMGN289, INC280, INCB7839 (aderbasib), Inlyta (axitinib), Iressa (gefitinib), ISU101 (cetuximab), JNJ26483327, JNJ28871063, JX929, Kabigef (gefitinib), KD019, KD020, KHK2866, KRN633, KRN951 (tivozanib), KSB102, KT6587, Lapatinib AQVIDA, Lapatinib ditosylate, Lapatinib SRS PHARMA, Lefibenz (gefitinib), Lortinib (erlotinib), LY2875358 (emibetuzumab), MabionEGFR (cetuximab), MDP01, MDX214 (CD89 monoclonal antibody), MDX447, Meftinib (gefitinib), MEHD7945A, Menadione TALON, MGCD265, mir-7 mimetic SILENCE, MM121, MM151, MP412, MP470 (amuvatinib), MT062, Novel Tyrosine kinase Inhibitor MEBIOPHARM, NT004, NT113, ORIL003, ORIL007, OSI632, Panitumumab BIOXPRESS THERAPEUTICS (panitumumab), Panitumumab PANPHARMACEUTICALS, PB272 (neratinib), PBI1737, PBI4050, Perjeta (pertuzumab), PF00299804 (dacomitinib), PKI166, PM92102 (kahalalide F), RadioTheraCIM, RAF and HER inhibitors DECIPHERA, RBLX200, RC3095, Reglycosylated Cetuximab FOUNTAIN BIOPHARM, RG3638 (onartuzumab), RG7160 (imgatuzumab), RX1792, S222611, SAB-Y1 SYNTAB, Sai Pu Ting, SAI-EGFR-ECD MICROMET, SAR103168, SC100, Selatinib Ditosilate QILU, Selective EGFR Inhibitors VICHEM, SL175, SL176, SL433, SL461, SL634, SRXMs MAXOCARE, STIA020X, STIA050X, Stivarga (regorafenib), STP523, STP801, Stridessa (gefitinib), Surrobody Drug Conjugate SEA LANE, Sym004, Sym013, TaiXinSheng (nimotuzumab), TAK285, Tarceva (erlotinib hydrochloride), Targretin (bexarotene), TAS2913, TG03 (apricoxib), TGF therapeutic cancer vaccine BIOVEN, TGF-alpha Cancer Vaccine MICROMET, Trastuzumab ZENOTECH, Trisilensa, Trispecific Anti-Her1/Her3 Zybodies ZYNGENIA (cetuximab), Tykerb (lapatinib ditosylate), Tyrogef, Tyrokinin 100 (erlotinib hydrochloride), U31287 (partitumab), Ultragef (gefitinib), VCC395122, VCC868449, Vectibix (panitumumab), VI14442, Xefta (gefitinib), YMB1005, Zefotib (gefitinib), and Zufinib (gefitinib).

The anticancer drug targeting the RAS/RAF/MEK/ERK signal pathway is selected from the group consisting of: Dabrafenib, GSK2118436, LGX818, Vemurafenib, RAF265, RO5126766, Sorafenib, XL281 (Raf inhibitor); ARRY-300, , AZD8330, E6201, PD-0325901, RO4987655, Bimetinib (ARRY-162/ MEK162), Cobimetinib (GDC-0973/XL518), Refametinib (BAY86-9766/RDEA119), Pisasertib (AS703026), Selumetinib (AZD6244/ARRY-142886), TAK-733, Trametinib (GSK1120212) (MEK inhibitor), BVD-523, and MK8553 (ERK inhibitor) .

The anticancer drug targeting PI3K/AKT/mTOR signal pathway is selected from the group consisting of : BGT226, BEZ235, GDC-0980, GSK2126458, PF-04691502, PF-05212384/PKI-587, SF1126, XL765/SAR245409, BKM120, BYL719, CAL-101, GDC-0032 , GDC-0941, GSK2636771, IPI-145, NVP-BEZ235, PX866, XL147 (PI3K inhibitor); Erucylphosphocholine, GSK2141795, GSK690693, Miltefosine, MK2206, PBI-05204 , Perifosine, RX-0201, SR13668, and XL-418 (AKT inhibitor); AZD2014, AZD8055, CC-223, Everolimus(RAD001), Ridaforolimus (MK-8669), OSI-027, Sirolimus (Rapamycin), Temsirolmus (Torisel®, CCI-779) (mTOR inhibitor).

The anticancer drug targeting JAK/STAT signal pathway is JAK kinase inhibitor or STAT inhibitor. JAK kinase inhibitor has inhibitory activity against JAK1, JAK2, and/or JAK3 and is selected from the group consisting of ABT-494, AT9283, atiprimod dihydrochloride, AZD1480, Baricitinib, BMS-911543, CP 690550, Cucurbitacin I, Decernotinib, , Filgotinib, Gandotinib, GSK2586184, Itacitinib (INCB039110), INCB018424, INCB047986, INCB052793, Lestaurtinib, Momelotinib (CYT387), NS-018, NSC 33994, Pacritinib, Peficitinib, Ruxolitinib (Jakafi), PF-04965842, SD 1008, Tofacitinib, Upadacitinib, XL019, and WP1066. STAT inhibitor has inhibitory activity against STATs 1, 2, 3, 4, 5a, 5b, and 6, in particular STAT3 and STAT5b and is selected from the group consisting of AZD9150, Capsaicin, CPA-1, CPA-7, FLLL11, FLLL12, FLLL32, FLLL62, IS3295, JQ1, OPB-111077, OPB-31121, OPB-51602 and pimozide.

### Pharmaceutical Composition

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically made adjuvant at suitable dosage level in a known way. The preferred preparations are adapted for oral application. These administration forms include, for example, pills, tablets, film tablets, coated tablets, capsules, powders and deposits.

Optionally, the pharmaceutical compositions according to the present invention comprise further at least one anticancer drug. The at least one anticancer is anticancer drug inhibting EGFR/HER2 tyrosine kinases, anticancer drug targeting the RAS/RAF/MEK/ERK signal pathway, anticancer drug targeting PI3K/AKT/mTOR signal pathway, and/or anticancer drug targeting JAK/STAT signal pathway as defined above.

Furthermore, the present invention also includes pharmaceutical preparations for parenteral application, including dermal, intradermal, intragastral, intracutaneous, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the derivatives according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. anti-cancer activity or activity against cancer metastases and the like. Suitable dosage forms for sustained release include tablets having layers of varying disintegration rates or controlled release, polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The compounds of the present invention are suitable for use in medicine, particulary in human medicine, but also in veterinary medicine. The dosage of the compounds may be determined by a skilled practitioner according to the type and severity of the disorder to be treated.

The compounds of the present invention may be admistered as a monotherapy or together with further active agents, particulary chemotherapeutic agents or antitumor antibodies. Furthermore they may be used in combination with surgery and/or irradiation.

### Description of Figures

**Figure 1****:** core sequences of EGFR mutants EGFR D770_N771insSVD, EGFR H773_V774insNPH, EGFR V769_D770insASV, EGFR P772_H773insPR, EGFR T790M and EGFR T790ML858R and HER mutant Her2 INS8 INS YVMA.
**Figure 2** shows the hydrolysis of the acrylamide moiety of reference 1 in the presence of liver microsomes after 50 minutes.

### Preparation of compounds:

### General Information:

All reactions involving air- or moisture-sensitive reagents or intermediates were carried out in flame-dried glassware under an argon atmosphere. Dry solvents (THF, toluene, MeOH, DMF, DCM) were used as commercially available. ¹H-NMR and ¹³C-NMR were recorded on a Bruker DRX400 (400 MHz). Multiplicities are indicated as: br s (broadened singlet), s (singlet), d (doublet), t (triplet), q (quartet), quin (quintet), m (multiplet); and coupling constants (J) are given in Hertz (Hz). HPLC - electrospray mass spectra (HPLC ES-MS) were obtained using Waters Acquity Performance Liquid Chromatography (UPLC) equipped SQ 3100 Mass detector spectrometer. Column: Acquity UPLC BEH C18 1.7um, 2.1x50mm. Flow: 0.5ml/min. Eluents: A: H₂O with 0.05% formic acid and B: ACN with 0.05% TFA. All chemicals and solvents were purchased from commercial sources like Sigma-Aldrich, Fluka, TCI, Acros Organics, ABCR, Alfa Aesar, Enamine, VWR, Combi-Blocks, Apollo Scientific, Aquilla Pharmatech, Ark Pharm, D-L Chiral Chemicals, ChemBridge, Renno Tech, Accela, KeyOrganics, Pharmablock and Chem Impex. Unless otherwise noted, all commercially available compounds were used as received without further purifications.

### Abbreviations used in the description of the chemistry and in the Examples that follow are:

ACN (acetonitrile); br (broad); CDCl₃ (deuterated chloroform); cHex (cyclohexane); DCM (dichloromethane); DIPEA (di-iso-propylethylamine); DMF (dimethylformamide); DMSO (dimethyl sulfoxide); eq. (equivalent); ES (electrospray); EtOAc (ethyl acetate); EtOH (ethanol); HATU (O-(7-azabenzotriazol-1-yl)-*N*,*N*,*N'*,*N*'-tetramethyluronium hexafluorophosphate); HCl (hydrochloric acid); HOAc (acetic acid); H₂O (water); K₂CO₃ (potassium carbonate); KOH (potassium hydroxide); MeOH (methanol); MS (mass spectrometry); Mwt (molecular weight); NaHCO₃ (sodium hydrogencarbonate); NH₃ (ammonia); NH₄Cl (ammonium chloride); NIS (*N*-Iodosuccinimide); NMP (N-methyl-2-pyrrolidon); NMR (nuclear magnetic resonance); Pd(dppf)Cl₂ ([1,1'-bis(diphenylphosphino)ferrocene]dichloro palladium(II) complex with dichloromethane); iPrOH (iso-propanol); PyBroP (Bromotripyrrolidinophosphonium hexafluorophosphate); RP (reversed-phase); RT (room temperature); sat. aq. (saturated aqueous); SiO₂ (silica gel); TFA (trifluoroacetic acid); THF (tetrahydrofurane).

### Preparative Examples

### Example 1:

### N-(5-(4-chloro-2-(4-((4-methylpiperazin-1-yl)methyl)phenyl)-1H-pyrrolo[2,3-b]pyridin-3-yl)-2-methylphenyl)acrylamide 2,2,2-trifluoroacetate (A1)

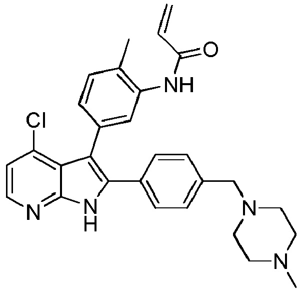

### Step 1: 4-Chloro-3-(4-methyl-3-nitrophenyl)-2-(trimethylsilyl)-1H-pyrrolo[2,3-b]pyridine A1)

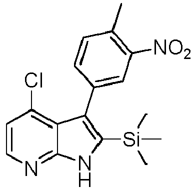

4-Chloro-3-iodopyridine-2-amine (5.0g, 19.0mmol, 1.0eq.), trimethyl((4-methyl-3-nitrophenyl)ethynyl)silane (5.7g, 25.0mmol, 1.25eq.), 1,4-diazabicyclo[2.2.2]octane (3.6g, 32.3mmol, 1.7eq.) and dichlorobis(triphenylphosphine)palladium(II) (1.4g, 2.0mmol, 0.1eq.) in dry DMF (40mL) under N₂ atmosphere was splitted in three microwave vials. Each vial was heated in the microwave at 145°C for 2 h. EtOAc (250mL) was added and the organic phase was washed three times with aq. sat. NaHCO₃-solution. The organic phase was dried over MgSO₄ and solvents were removed in vacuo. The crude product was purified by flash chromatography on silica gel (cHex/EtOAc = 100:0 to 1:1) to yield the desired product **A1** (3.9g, 10.8mmol, 57%) as a beige solid. ¹H NMR (400MHz, d₆-DMSO, 300K) δ 0.11 (s, 9H), 2.58 (s, 3H), 7.12 (d, J = 5.0 Hz, 1H), 7.52 (d, J = 7.9 Hz, 1H), 7.63 (d, J = 7.9 Hz, 1H), 7.91 (s, 1H), 8.21 (d, J = 5.0 Hz, 1H), 12.04 (s, 1H). MS (ES) C₁₇H₁₈ClN₃O₂Si requires: 359, found: 360 (M+H)⁺.

### Step 2: 5-(4-Chloro-2-(trimethylsilyl)-1H-pyrrolo[2,3-b]pyridin-3-yl)-2-methylaniline (A2)

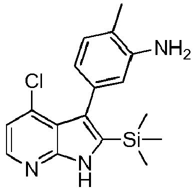

A solution of 4-chloro-3-(4-methyl-3-nitrophenyl)-2-(trimethylsilyl)-1H-pyrrolo[2,3-b]pyridine **A1** (5.6g, 15.56mmol, 1.0eq.) and iron (4.3g, 77.8mmol, 5 eq.) in EtOH (100mL) and aq. sat. NH₄Cl-solution (10mL) was stirred for 5h at 80°C. The solution was filtered through a pad of Celite®. Solvents were removed in vacuo. The crude was solved in EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 0:100) to yield the desired product **A2** (5.0g, 15.2mmol, 97%) as a beige solid. ¹H NMR (400MHz, d₆-DMSO, 300K) δ 0.10 (s, 9H), 2.10 (s, 3H), 4.76 (br s, 2H), 6.44 (d, J = 7.4 Hz, 1H), 6.60 (s, 1H), 6.89 (d, J = 7.4 Hz, 1H), 7.04 (d, J = 5.1 Hz, 1H), 8.15 (d, J = 5.1 Hz, 1H), 11.71 (s, 1H). MS (ES) C₁₇H₂₀ClN₃Si requires: 329, found: 330 (M+H)⁺.

### Step 3: N-(5-(4-chloro-2-(trimethylsilyl)-1H-pyrrolo[2,3-b]pyridin-3-yl)-2-methylphenyl)acrylamide (A3)

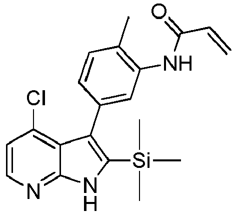

**A3** was prepared either by Procedure A or Procedure B:

### Procedure A:

To a solution of **A2** (2760mg, 8.4mmol, 1.0eq.) and DIPEA (14.6mL, 84.0mmol, 10.0eq.) in dry DCM (100mL) at 0°C was added slowly acrylolyl chloride (757mg, 8.4mmol, 1.0eq.) in dry DCM (5mL). The mixture was stirred for 5min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product **A3** (3070mg, 8.0mmol, 95%) as a white solid. ¹H NMR (400MHz, d₆-DMSO, 300K) δ 0.11 (s, 9H), 2.28 (s, 3H), 5.72 (dd, J = 2.1 Hz, J = 10.2 Hz, 1H), 6.22 (dd, J = 2.1 Hz, J = 16.9 Hz, 1H), 6.55 (dd, J = 10.2 Hz, J = 16.9 Hz, 1H), 7.07 (m, 2H), 7.22 (d, J = 7.7 Hz, 1H), 7.56 (s, 1H), 8.17 (d, J = 5.0 Hz, 1H), 9.42 (s, 1H), 11.83 (s, 1H). MS (ES) C₂₀H₂₂ClN₃OSi requires: 383, found: 384 (M+H)⁺.

### Procedure B:

To a solution of HATU (3460mg, 9.1mmol, 1.5eq.) in DMF (10mL) at 0°C was added acrylic acid (655mg, 9.1mmol, 1.5eq.) and DIPEA (1548mg, 9.1mmol, 2.0eq.). The mixture was stirred for 15min. Then **A2** (2000mg, 6.0mmol, 1.0eq.) was added and the mixture was stirred for 4h at 0°C. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product **A3** (890mg, 2.3mmol, 39%) as a white solid. MS (ES) C₂₀H₂₂ClN₃OSi requires: 383, found: 384 (M+H)⁺.

### Step 4: N-(5-(4-chloro-2-iodo-1H-pyrrolo[2,3-b]pyridin-3-yl)-2-methylphenyl)acrylamide (A4)

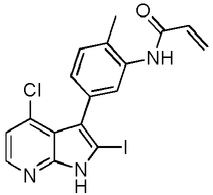

N-(5-(4-chloro-2-(trimethylsilyl)-1H-pyrrolo[2,3-b]pyridin-3-yl)-2-methylphenyl)acrylamide **A3** (890mg, 2.32mmol, 1.0eq.) and N-iodosuccinimide (937mg, 4.18mmol, 1.8eq.) were solved in dry dichloromethane (300mL) and stirred for 15h at RT. The organic phase was washed once with aq. sat. Na₂S₂O₃-sol. and three times with aq. sat. NaHCO₃-solution. The organic phase was dried over Na₂SO₄ and solvents were removed in vacuo yielding the desired product **A4** (970mg, 2.21mmol, 95%) as a yellow solid. The crude was used without purification in the next step. MS (ES) C₁₇H₁₃ClIN₃O requires: 437, found: 438 (M+H)⁺.

### Step 5: N-(5-(4-chloro-2-(4-((4-methylpiperazin-1-yl)methyl)phenyl)-1H-pyrrolo[2,3-b]pyridin-3-yl)-2-methylphenyl)acrylamide 2,2,2-trifluoroacetate (A5)

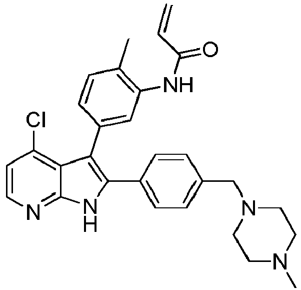

A mixture of N-(5-(4-chloro-2-iodo-1H-pyrrolo[2,3-b]pyridin-3-yl)-2-methylphenyl)acrylamide **A4** (40mg, 0.09mmol, 1.0eq.), {4-[(4-methylpiperazin-1-yl)methyl]phenyl}boronic acid dihydrochloride (32mg, 0.14mmol, 1.5eq.), and K₃PO₄ (38mg, 0.18mmol, 2.0 eq) in dioxane/H₂O (3mL/0.6mL) was degassed with a stream of N₂ for 5min. [1,1'-Bis(diphenylphosphino)ferrocene]palladium dichloride dichloromethane adduct (8mg, 0.01 mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **A5** (5mg, 0.01 mmol, 8%) as a yellow powder. ¹H NMR (400MHz, CDCl₃, 300K) δ 12.45 (s, 1H), 9.44 (s, 1H), 8.16 (dd, J = 1.3 Hz, J = 5.2 Hz, 1H), 7.52 (s, 1H), 7.44 (d, J = 7.9 Hz, 2H), 7.25 (d, J = 7.9 Hz, 2H), 7.20 (d, J = 7.8 Hz, 1H), 7.10 (dd, J = 1.3 Hz, J = 5.2 Hz, 1H), 7.04 (d, J = 7.8 Hz, 1H), 6.51 (dd, J = 10.1 Hz, J = 17.1 Hz, 1H), 6.17 (d, J = 17.1 Hz, 1H), 5.70 (d, J = 10.1 Hz, 1H), 3.58 (s, 2H), 3.01 - 2.89 (m, 4H), 2.74 (s, 3H), 2.65 (m, 2H), 2.31 (m, 2H), 2.25 (s, 3H). MS (ES) C₂₉H₃₀ClN₅O requires: 500, found: 501 (M+H)⁺.

The Examples in the following table were prepared according to the procedure described for **A5** (Example 1).

| **Example** | **Name** | **Mwt** | **[M+H]⁺** |
|---|---|---|---|
| 2 | 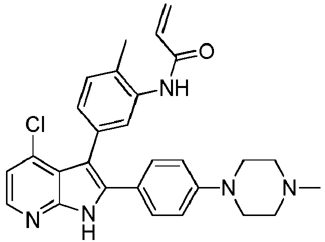 | 485 | 486 |
| 3 | 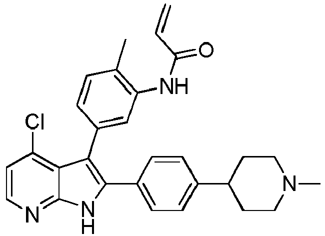 | 484 | 485 |
| 4 | 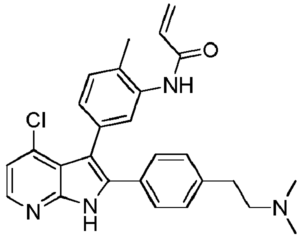 | 458 | 459 |
| 5 | 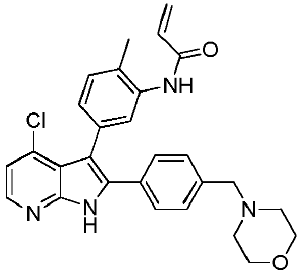 | 486 | 487 |
| 6 | 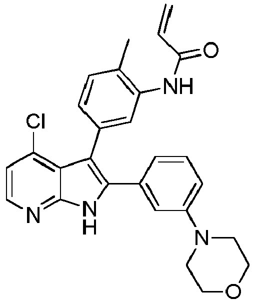 | 472 | 473 |
| 7 | 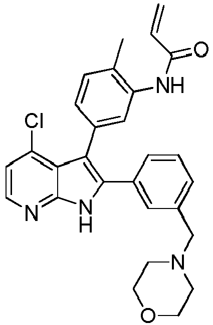 | 486 | 487 |
| 8 | 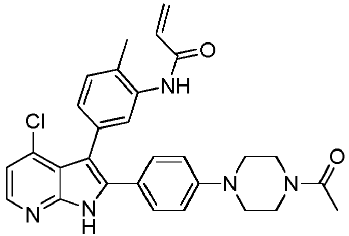 | 513 | 514 |
| 9 | 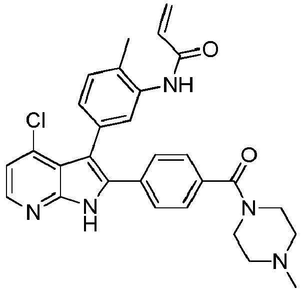 | 513 | 514 |
| 10 | 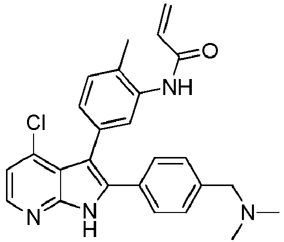 | 444 | 445 |
| 11 | 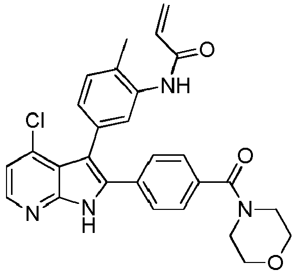 | 500 | 501 |
| 12 | 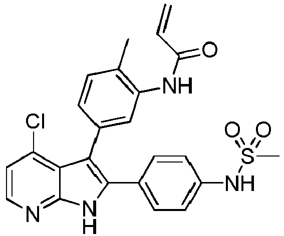 | 480 | 481 |
| 13 | 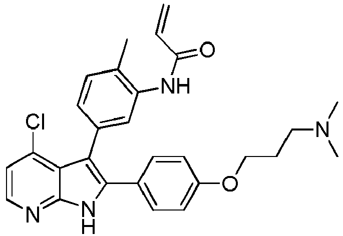 | 488 | 489 |
| 14 | 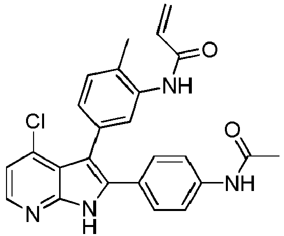 | 444 | 445 |
| 15 | 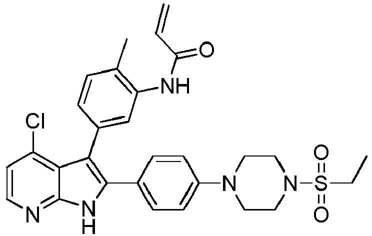 | 563 | 564 |
| 16 | 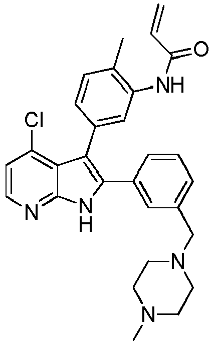 | 499 | 500 |
| 17 | 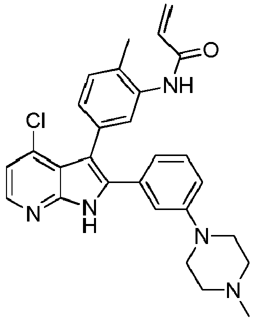 | 485 | 486 |
| 18 | 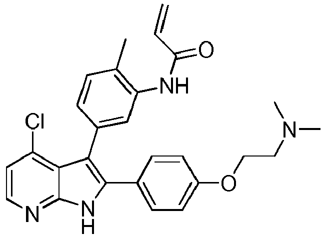 | 474 | 475 |
| 19 | 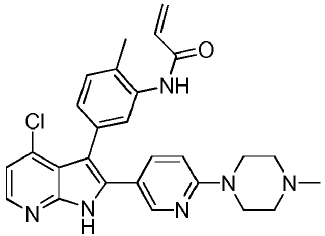 | 486 | 487 |
| 20 | 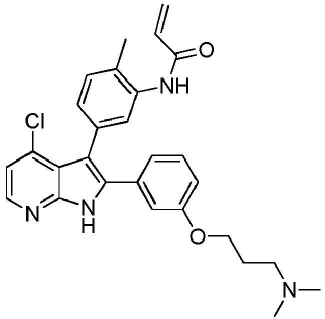 | 488 | 489 |
| 21 | 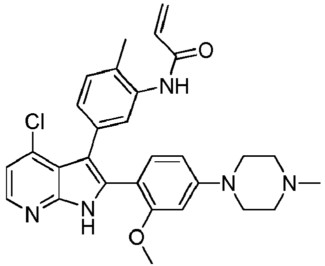 | 515 | 516 |
| 22 | 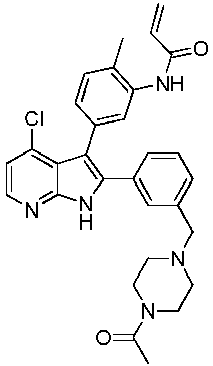 | 527 | 528 |
| 23 | 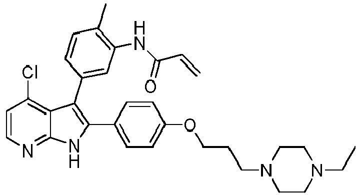 | 557 | 558 |
| 24 | 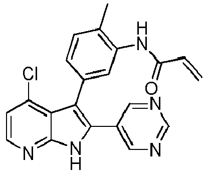 | 389 | 390 |
| 25 | 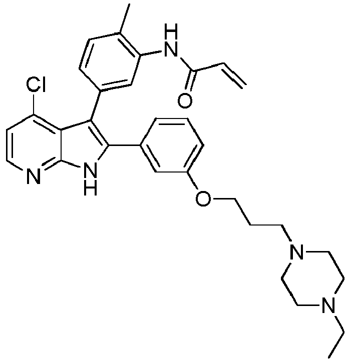 | 557 | 558 |
| 26 | 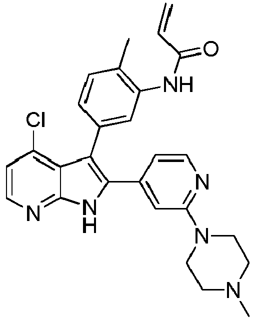 | 486 | 487 |
| 27 | 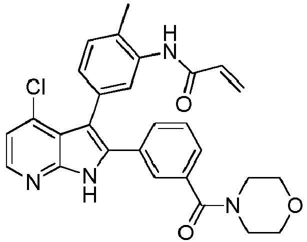 | 500 | 501 |
| 28 | 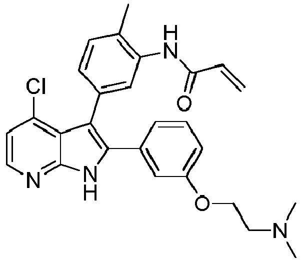 | 474 | 475 |
| 29 | 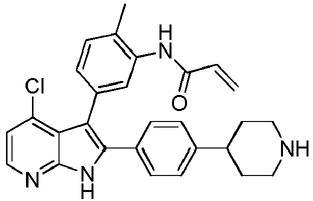 | 470 | 471 |
| 30 | 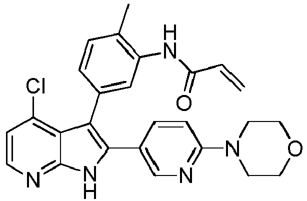 | 473 | 474 |
| 31 | 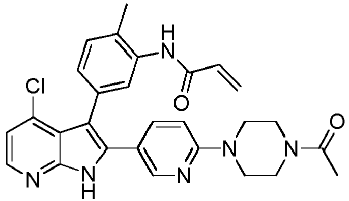 | 514 | 515 |
| 32 | 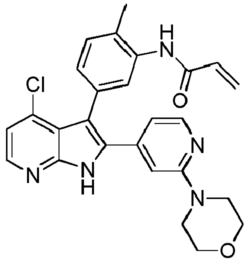 | 473 | 474 |
| 33 | 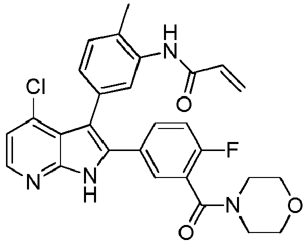 | 518 | 519 |
| 34 | 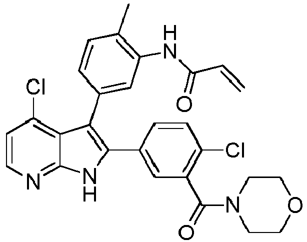 | 534 | 535 |
| 35 | 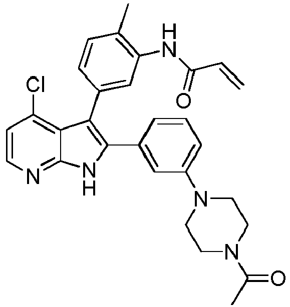 | 513 | 514 |
| 36 | 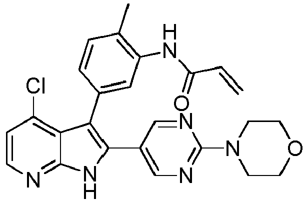 | 474 | 475 |
| 37 | 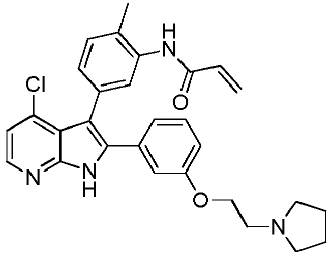 | 500 | 501 |
| 38 | 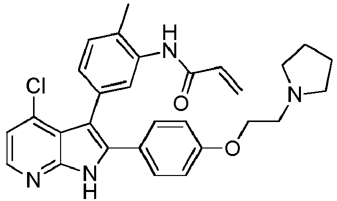 | 500 | 501 |
| 38 | 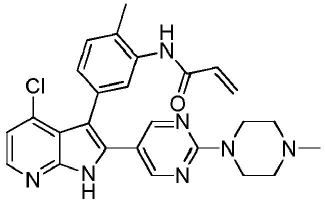 | 487 | 488 |
| 40 | 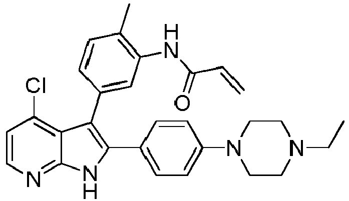 | 499 | 500 |
| 41 | 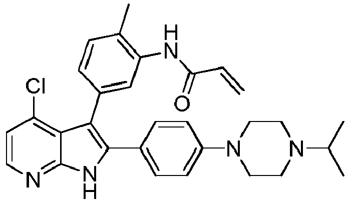 | 513 | 514 |
| 42 | 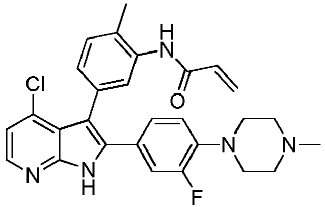 | 503 | 504 |
| 43 | 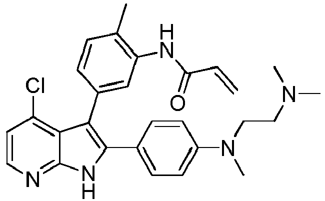 | 487 | 488 |

### Example 44:

### N-(5-(4-chloro-2-(3-(4-methylpiperazine-1-carbonyl)phenyl)-1H-pyrrolo[2,3-b]pyridin-3-yl)-2-methylphenyl)acrylamide 2,2,2-trifluoroacetate (B2)

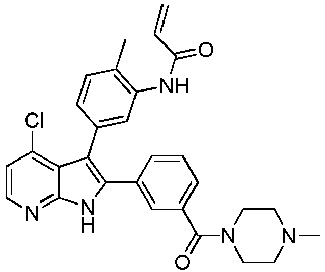

### Step 1: 3-(3-(3-acrylamido-4-methylphenyl)-4-chloro-1H-pyrrolo[2,3-b]pyridin-2-yl)benzoic acid (B1)

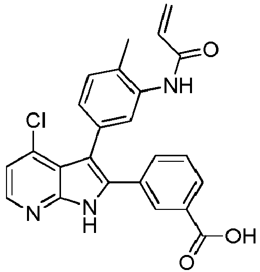

A mixture of N-(5-(4-chloro-2-iodo-1H-pyrrolo[2,3-b]pyridin-3-yl)-2-methylphenyl)acrylamide **A4** (500mg, 1.14mmol, 1.0eq.), 3-carboxyphenylboronic acid (284mg, 1.71mmol, 1.5eq.), and K₃PO₄ (483mg, 2.28mmol, 2.0 eq) in dioxane/H₂O (15mL/3mL) was degassed with a stream of N₂ for 5min. [1,1'-Bis(diphenylphosphino)ferrocene]palladium dichloride dichloromethane adduct (93mg, 0.11 mmol, 0.1 eq) was added and the reaction mixture heated to 130°C for 2 h in the microwave oven. Solvents were removed and the crude was purified by flash chromatography on silica gel (DCM/MeOH = 100:0 to 0:100) to yield the desired product **B1** (200mg, 0.46mmol, 41%) as a beige solid. MS (ES) C₂₄H₁₈ClN₃O₃ requires: 431, found: 432 (M+H)⁺.

### Step 2: N-(5-(4-chloro-2-(3-(4-methylpiperazine-1-carbonyl)phenyl)-1H-pyrrolo[2,3-b]pyridin-3-yl)-2-methylphenyl)acrylamide 2,2,2-trifluoroacetate (B2)

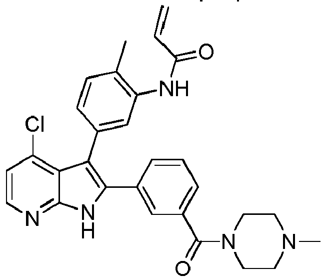

To a solution of **B1** (10mg, 0.02mmol, 1.0eq.) and HATU (18mg, 0.05mmol, 2.0eq.) in DMF (2mL) at 0°C was added 1-methylpiperazine (4mg, 0.03mmol, 1.5 eq.) and DIPEA (20uL, 0.11mmol, 5.0eq.). The mixture was stirred for 1h. The reaction mixture was diluted with EtOAc, washed three times with aq. sat. NaHCO₃-solution. The organic phase was dried over MgSO₄ and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **B2** (3mg, 0.003mmol, 14%) as a yellow powder. ¹H NMR (400MHz, MeOD, 300K) δ 8.17 (d, J = 5.3 Hz, 1H), 7.77 (d, J = 7.9 Hz, 1H), 7.57 (t, J = 7.7 Hz, 1H), 7.46 (d, J = 7.7 Hz, 1H), 7.40 (m, 2H), 7.25 (d, J = 7.7 Hz, 1H), 7.12 (m, 2H), 6.50 (dd, J = 10.2 Hz, J = 17.0 Hz, 1H), 6.35 (d, J = 17.0 Hz, 1H), 5.80 (d, J = 10.2 Hz, 1H), 3.54 - 2.80 (m, 8H), 2.95 (s, 3H), 2.32 (s, 3H). MS (ES) C₂₉H₂₈ClN₅O₂ requires: 513, found: 514 (M+H)⁺.

The Examples in the following table were prepared according to the procedure described for **B2** (Example 44).

| **Example** | **Name** | **Mwt** | **[M+H]⁺** |
|---|---|---|---|
| 45 | 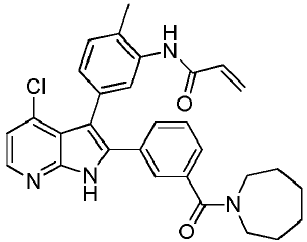 | 512 | 513 |
| 46 | 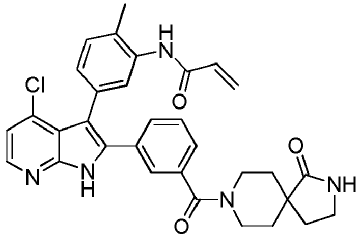 | 567 | 568 |
| 47 | 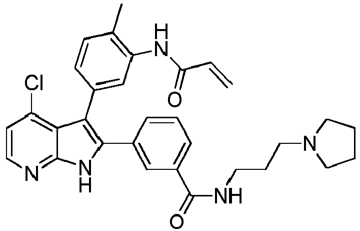 | 541 | 542 |
| 48 | 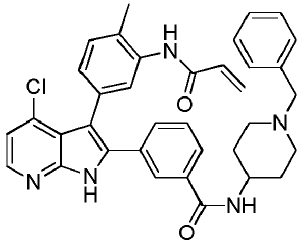 | 603 | 604 |
| 49 | 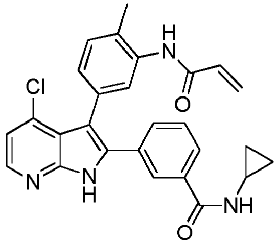 | 470 | 471 |
| 50 | 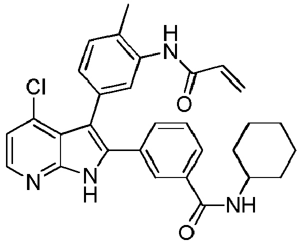 | 512 | 513 |
| 51 | 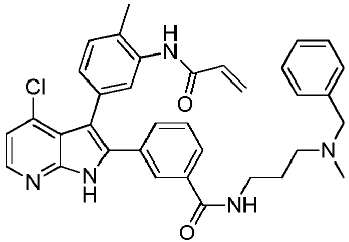 | 591 | 592 |
| 52 | 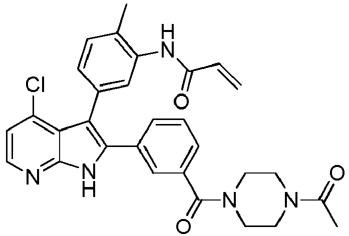 | 541 | 542 |
| 53 | 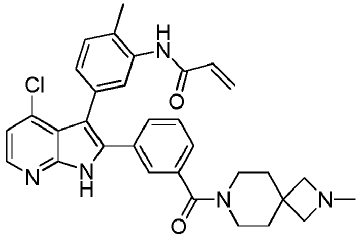 | 553 | 554 |
| 54 | 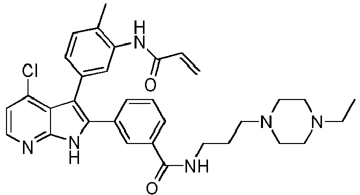 | 584 | 585 |

### Example 55:

### 1-(6-(4-Chloro-2-(4-(1-methylpiperidin-4-yl)phenyl)-1H-pyrrolo[2,3-b]pyridin-3-yl)indolin-1-yl)prop-2-en-1-one 2,2,2-trifluoroacetate (C5)

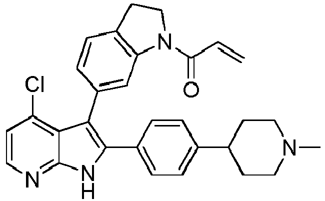

### Step 1: Tert-butvl 6-(4-chloro-2-(trimethylsilyl)-1H-pyrrolo[2,3-b]pyridin-3-yl)indoline-1-carboxylate (C1)

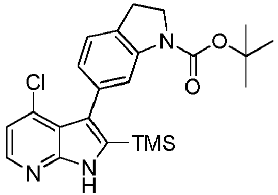

**C1** (1.0g, 2.26mmol, 76%, beige solid) was prepared from 4-chloro-3-iodopyridine-2-amine and tert-butyl 6-((trimethylsilyl)ethynyl)indoline-1-carboxylate following the general procedure reported in Preparative Example 1 Step 1. MS (ES) C₂₃H₂₈ClN₃O₂Si requires: 441, found: 442 (M+H)⁺.

### Step 2: 4-Chloro-3-(indolin-6-yl)-2-(trimethylsilyl)-1H-pyrrolo[2,3-b]pyridine (C2)

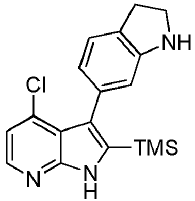

Tert-butyl 6-(4-chloro-2-(trimethylsilyl)-1H-pyrrolo[2,3-b]pyridin-3-yl)indoline-1-carboxylate **C1** (1.0g, 2.26mmol) in 30% TFA in DCM (60mL) at RT was stirred for 2h. Evaporation of the solvents yielded the desired product **C2** as beige solid, which was used in the next step without purification. MS (ES) C₁₈H₂₀ClN₃Si requires: 341, found: 342 (M+H)⁺.

### Step 3: 1-(6-(4-Chloro-2-(trimethylsilyl)-1H-pyrrolo[2,3-b]pyridin-3-yl)indolin-1-yl)prop-2-en-1-one (C3)

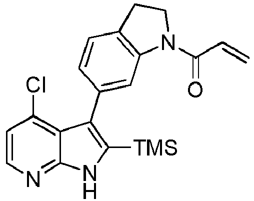

**C3** (50mg, 0.13mmol, 6%, beige solid) was prepared from **C2** following the general procedure reported in Preparative Example 1 Step 3 Procedure A. MS (ES) C₂₁H₂₂ClN₃OSi requires: 395, found: 396 (M+H)⁺.

### Step 4: 1-(6-(4-Chloro-2-iodo-1H-pyrrolo[2,3-b]pyridin-3-yl)indolin-1-yl)prop-2-en-1-one (C4)

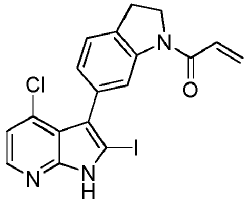

**C4** (51mg, 0.11mmol, 84%, yellow solid) was prepared from **C3** following the general procedure reported in Preparative Example 1 Step 4. MS (ES) C₁₈H₁₃ClIN₃O requires: 448, found: 449 (M+H)⁺.

### Step 5: 1-(6-(4-chloro-2-(4-(1-methylpiperidin-4-yl)phenyl)-1H-pyrrolo[2,3-b]pyridin-3-yl)indolin-1-yl)prop-2-en-1-one 2,2,2-trifluoroacetate (C5)

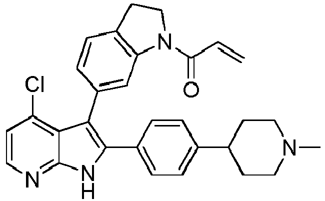

**C5** was prepared from **C4** following the general procedure reported in Preparative Example 1 Step 5. The crude was purified by reverse phase RP-HPLC (column: C18), using H₂O (0.1%TFA) and ACN (0.1%TFA) as eluents. The desired fractions were lyophilized to yield the title compound **C5** (10mg, 0.01 mmol, 12%) as a yellow solid. ¹H NMR (400MHz, MeOD, 300K) δ 1.92 (m, 3H), 2.10 (d, J = 13.6 Hz, 2H), 2.85 (m, 1H), 2.91 (s, 3H), 3.15 (m, 3H), 3.59 (d, J = 10.3 Hz, 2H), 4.31 (t, J = 8.5 Hz, 2H), 5.82 (d, J = 10.6 Hz, 1H), 6.33 (d, J = 16.8 Hz, 1H), 6.76 (dd, J = 10.6 Hz, J = 16.8 Hz, 1H), 7.05 (d, J = 7.7 Hz, 1H), 7.10 (d, J = 5.2 Hz, 1H), 7.30 - 7.19 (m, 3H), 7.46 (d, J = 8.1 Hz, 2H), 7.71 (d, J = 8.1 Hz, 1H), 8.12 (d, J = 5.2 Hz, 1H), 8.25 (s, 1H). MS (ES) C₃₀H₂₉ClN₄O requires: 496, found: 497 (M+H)⁺.

The Examples in the following table were prepared according to the procedure described for **C5** (Example 55).

| **Example** | **Name** | **Mwt** | **[M+H]⁺** |
|---|---|---|---|
| 56 | 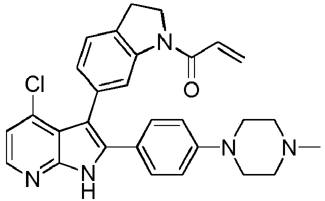 | 497 | 498 |
| 57 | 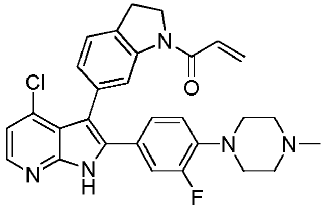 | 515 | 516 |
| 58 | 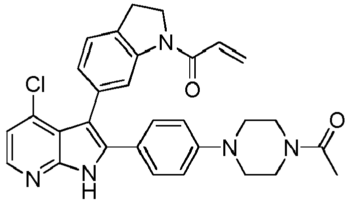 | 525 | 526 |
| 59 | 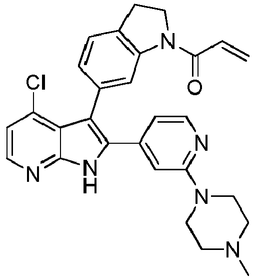 | 498 | 499 |
| 60 | 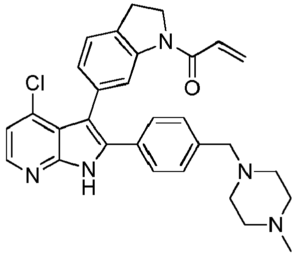 | 511 | 512 |
| 61 | 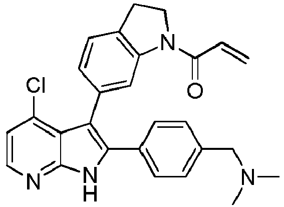 | 456 | 457 |
| 62 | 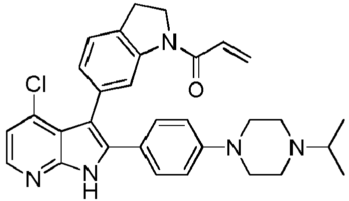 | 525 | 526 |
| 63 | 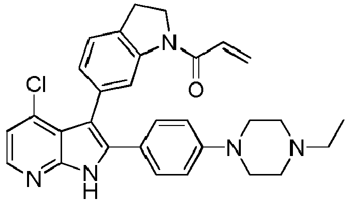 | 511 | 512 |
| 64 | 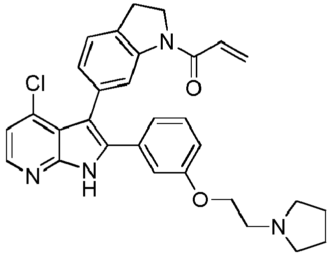 | 512 | 513 |
| 65 | 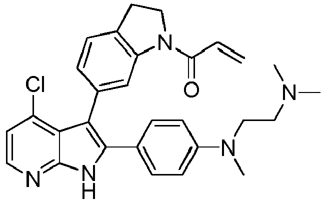 | 499 | 500 |
| 66 | 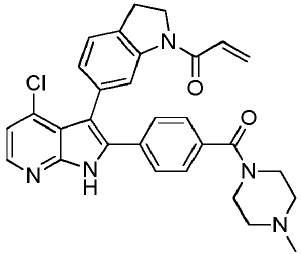 | 525 | 526 |
| 67 | 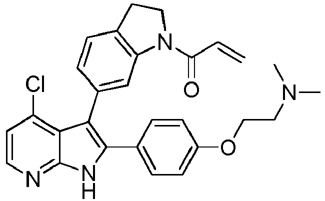 | 486 | 487 |
| 68 | 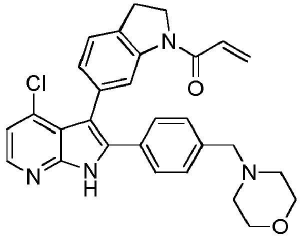 | 498 | 499 |
| 69 | 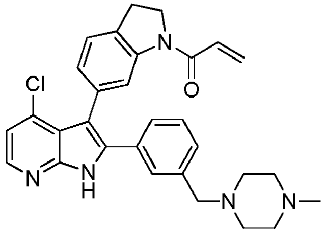 | 511 | 512 |
| 70 | 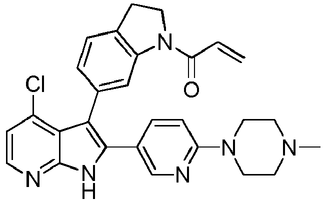 | 498 | 499 |
| 71 | 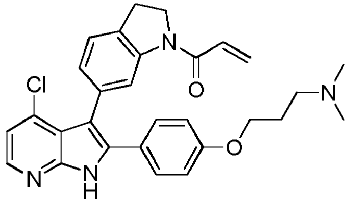 | 500 | 501 |

### Example 72

### N-(5-(4-methoxy-2-(4-(4-methylpiperazin-1-yl)phenyl)-1H-pyrrolo[2,3-b]pyridin-3-yl)-2-methylphenyl)acrylamide 2,2,2-trifluoroacetate (D5)

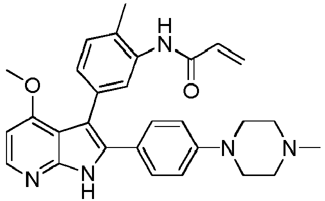

### Step 1: 4-Methoxy-3-(4-methyl-3-nitrophenyl)-2-(trimethylsilyl)-1H-pyrrolo[2,3-b]pyridine (D1)

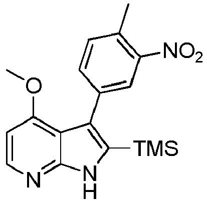

**D1** (3.50g, 9.86mmol, 38%, beige solid) was prepared from 3-iodo-4-methoxypyridin-2-amine and trimethyl((4-methyl-3-nitrophenyl)ethynyl)silane following the general procedure reported in Preparative Example 1 Step 1. MS (ES) C₁₈H₂₁N₃O₃Si requires: 355, found: 356 (M+H)⁺.

### Step 2: 5-(4-Methoxy-2-(trimethylsilyl)-1H-pyrrolo[2,3-b]pyridin-3-yl)-2-methylaniline (D2)

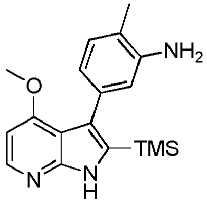

A solution of **D1** (2.5g, 7mmol, 1eq.) and iron (1.9g, 35mmol, 5eq.) in EtOH (100mL) and aq. sat. NH₄Cl-solution (10mL) was stirred for 5h at 80°C. The solution was filtered through a pad of Celite®. Solvents were removed in vacuo. The crude was solved in EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 0:100) to yield the desired product **D2** (1.0g, 3.1mmol, 44%) as a beige solid. MS (ES) C₁₈H₂₃N₃OSi requires: 325, found: 326 (M+H)⁺.

### Step 3: N-(5-(4-methoxy-2-(trimethylsilyl)-1H-pyrrolo[2,3-b]pyridin-3-yl)-2-methylphenyl)acrylamide (D3)

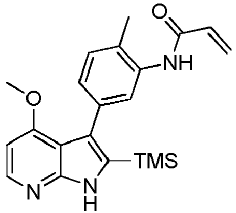

**D3** (712mg, 1.87mmol, 40%, white solid) was prepared from **D2** following the general procedure reported in Preparative Example 1 Step 3 Procedure A. MS (ES) C₂₁H₂₅N₃O₂Si requires: 379, found: 380 (M+H)⁺.

### Step 4: N-(5-(2-iodo-4-methoxy-1H-pyrrolo[2,3-b]pyridin-3-yl)-2-methylphenyl)acrylamide (D4)

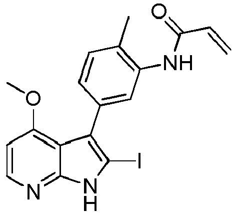

**D4** (512mg, 1.18mmol, 66%, yellow solid) was prepared from **D3** following the general procedure reported in Preparative Example 1 Step 4. MS (ES) C₁₈H₁₆IN₃O₂ requires: 433, found: 434 (M+H)⁺.

### Step 5: N-(5-(4-methoxy-2-(4-(4-methylpiperazin-1-yl)phenyl)-1H-pyrrolo[2,3-b]pyridin-3-yl)-2-methylphenyl)acrylamide 2,2,2-trifluoroacetate (D5)

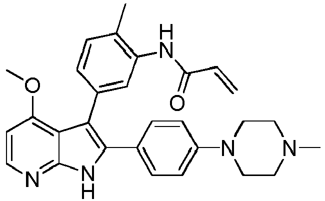

**D5** was prepared from **D4** following the general procedure reported in Preparative Example 1 Step 5. The crude was purified by reverse phase RP-HPLC (column: C18), using H₂O (0.1%TFA) and ACN (0.1%TFA) as eluents. The desired fractions were lyophilized to yield the title compound **D5** (2mg, 0.01 mmol, 3%) as a yellow solid. ¹H NMR (400MHz, MeOD, 300K) δ 2.30 (s, 3H), 2.96 (s, 3H), 3.08 (m, 2H), 3.26 (m, 2H), 3.57 (m, 2H), 3.91 (m, 2H), 3.98 (s, 3H), 5.78 (dd, J = 10.2 Hz, J = 1.5 Hz, 1H), 6.34 (dd, J = 17.0 Hz, J = 1.5 Hz, 1H), 6.51 (dd, J = 10.2 Hz, J = 17.0 Hz, 1H), 6.99 (d, J = 8.8 Hz, 2H), 7.03 (d, J = 6.7 Hz, 1H), 7.08 (d, J = 8.1 Hz, 1H), 7.19 (d, J = 7.8 Hz, 1H), 7.38 (d, J = 8.8 Hz, 2H), 7.46 (s, 1H), 8.22 (d, J = 7.6 Hz, 1H). MS (ES) C₂₉H₃₁N₅O₂ requires: 481, found: 482 (M+H)⁺.

The Examples in the following table were prepared according to the procedure described for **D5** (Example 72).

| **Example** | **Name** | **Mwt** | **[M+H]⁺** |
|---|---|---|---|
| 73 | 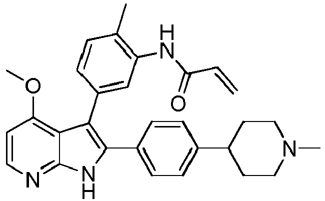 | 480 | 481 |
| 74 | 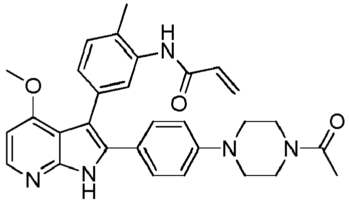 | 509 | 510 |
| 75 | 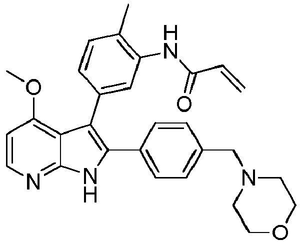 | 482 | 483 |
| 76 | 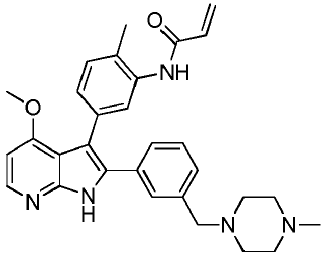 | 495 | 496 |
| 77 | 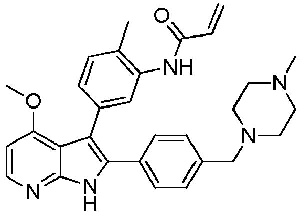 | 495 | 496 |

### Example 78:

### N-(2-methyl-5-(2-(6-(4-methylpiperazin-1-yl)pyridin-3-yl)-1H-pyrrolo[2,3-b]pyridin-3-yl)phenyl)acrylamide 2,2,2-trifluoroacetate (E1)

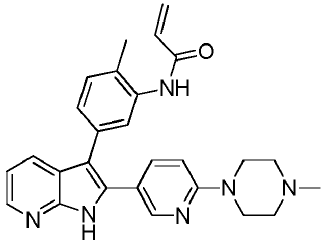

### Step 1: 3-(4-methyl-3-nitrophenyl)-2-(trimethylsilyl)-1H-pyrrolo[2,3-b]pyridine (E1)

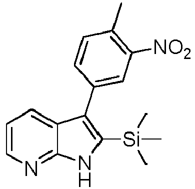

2-Amino-3-iodopyridine (5.65g, 25.71mmol, 1.2eq.), trimethyl((4-methyl-3-nitrophenyl)ethynyl)silane (5.00g, 21.43mmol, 1.0eq.), 1,4-diazabicyclo[2.2.2]octane (4.08g, 32.3mmol, 1.7eq.) and dichlorobis(triphenylphosphine)palladium(II) (1.51g, 2.14mmol, 0.1eq.) in dry DMF (40mL) under N₂ atmosphere was splitted in three microwave vials. Each vial was heated in the microwave at 145°C for 2 h. EtOAc (250mL) was added and the organic phase was washed three times with aq. sat. NaHCO₃-solution. The organic phase was dried over MgSO₄ and solvents were removed in vacuo. The crude product was purified by flash chromatography on silica gel (DCM/MeOH = 100:0 to 10:1) to yield the desired product **E1** (4.96g, 10.25mmol, 71%) as an orange solid. MS (ES) C₁₇H₁₉N₃O₂Si requires: 325, found: 326 (M+H)⁺.

### Step 2: 2-Methyl-5-(2-(trimethylsilyl)-1H-pyrrolo[2,3-b]pyridin-3-yl)aniline (E2)

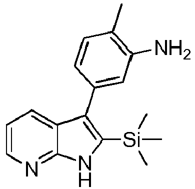

A solution of 3-(4-methyl-3-nitrophenyl)-2-(trimethylsilyl)-1H-pyrrolo[2,3-b]pyridine **E1** (4.96g, 15.24mmol, 1.0eq.) and iron (8.51g, 152.4mmol, 10 eq.) in EtOH (100mL) and aq. sat. NH₄Cl-solution (20mL) was stirred for 15h at 80°C. The solution was filtered through a pad of Celite®. Solvents were removed in vacuo. The crude was solved in EtOAc and washed twice with aq. sat. NaHCO₃-solution. The organic phase was dried over MgSO₄ and solvent was removed in vacuo. The crude product (3.04g, 10.3mmol, 68%, orange solid) was used without further purification in the next step. MS (ES) C₁₇H₂₁N₃Si requires: 295, found: 296 (M+H)⁺.

### Step 3: N-(2-methyl-5-(2-(trimethylsilyl)-1H-pyrrolo[2,3-b]pyridin-3-yl)phenyl)acrylamide (E3)

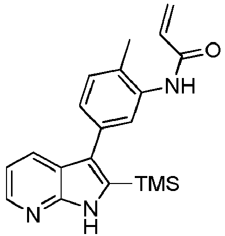

To a solution of **E2** (1000mg, 3.38mmol, 1.0eq.) and DIPEA (2.96mL, 16.92mmol, 5.0eq.) in dry DCM (100mL) at 0°C was added slowly acrylolyl chloride (306mg, 3.38mmol, 1.0eq.) in dry DCM (5mL). The mixture was stirred for 30min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:2) to yield the desired product **E3** (498mg, 1.43mmol, 42%) as a white solid. MS (ES) C₂₀H₂₃N₃OSi requires: 349, found: 350 (M+H)⁺.

### Step 4: N-(5-(2-iodo-1H-pyrrolo[2,3-b]pyridin-3-yl)-2-methylphenyl)acrylamide (E4)

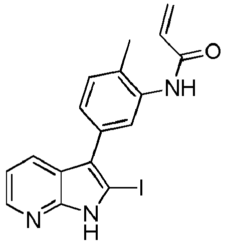

N-(2-methyl-5-(2-(trimethylsilyl)-1H-pyrrolo[2,3-b]pyridin-3-yl)phenyl)acrylamide **E3** (497mg, 1.42mmol, 1.0eq.) and N-iodosuccinimide (576mg, 2.56mmol, 1.8eq.) were solved in dry dichloromethane (100mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **E4** (337mg, 0.84mmol, 59%) as a yellow solid. The crude was used without purification in the next step. MS (ES) C₁₇H₁₄IN₃O requires: 403, found: 404 (M+H)⁺.

### Step 5: N-(2-methyl-5-(2-(6-(4-methylpiperazin-1-yl)pyridin-3-yl)-1H-pyrrolo[2,3-b]pyridin-3-yl)phenyl)acrylamide 2,2,2-trifluoroacetate (E5)

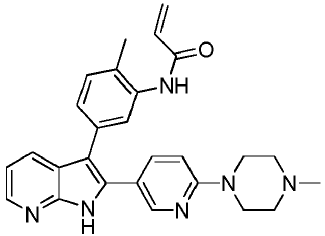

A mixture of N-(5-(2-iodo-1H-pyrrolo[2,3-b]pyridin-3-yl)-2-methylphenyl)acrylamide **E4** (50mg, 0.12mmol, 1.0eq.), 2-(4-methylpiperazino)pyridine-5-boronic acid pinacol ester (49mg, 0.16mmol, 1.3eq.), and K₃PO₄ (53mg, 0.24mmol, 2.0 eq) in dioxane/H₂O (3mL/0.6mL) was degassed with a stream of N₂ for 5min. [1,1'-Bis(diphenylphosphino)ferrocene]palladium dichloride dichloromethane adduct (10mg, 0.01 mmol, 0.1 eq) was added and the reaction mixture heated to 110°C for 1 h in the microwave oven. The crude solution was directly purified by reverse phase RP-HPLC (column: C18), using H₂O (0.1%TFA) and ACN (0.1%TFA) as eluents. The desired fractions were lyophilized to yield the title compound **E5** (22mg, 0.03mmol, 23%) as a yellow powder. ¹H NMR (400MHz, d₆-DMSO, 300K) δ 2.27 (s, 3H), 2.85 (s, 3H), 3.12 (m, 4H), 3.51 (d, J = 11.3 Hz, 2H), 4.46 (d, J = 12.3 Hz, 2H), 5.76 (d, J = 10.3 Hz, 1H), 6.24 (d, J = 17.0 Hz, 1H), 6.57 (dd, J = 10.3 Hz, J = 17.0 Hz, 1H), 6.99 (m, 2H), 7.12 (dd, J = 4.7 Hz, J = 7.9 Hz, 1H), 7.24 (d, J = 7.9 Hz, 1H), 7.67 (s, 1H), 7.72 (dd, J = 2.4 Hz, J = 8.7 Hz, 1H), 7.89 (d, J = 7.9 Hz, 1H), 8.26 (d, J = 4.7 Hz, 1H), 8.33 (d, J = 2.4 Hz, 1H), 9.51 (s, 1H), 12.14 (s, 1H). MS (ES) C₂₇H₂₈N₆O requires: 452, found: 453 (M+H)⁺.

The Examples in the following table were prepared according to the procedure described for **E5** (Example 78).

| **Example** | **Name** | **Mwt** | **[M+H]⁺** |
|---|---|---|---|
| 79 | 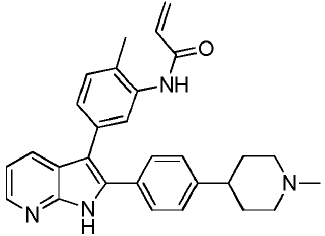 | 450 | 451 |
| 80 | 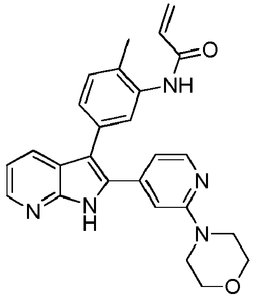 | 439 | 440 |
| 81 | 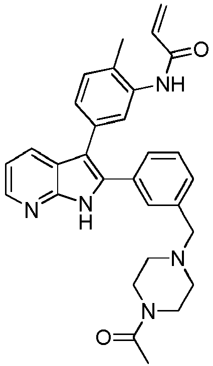 | 493 | 494 |
| 82 | 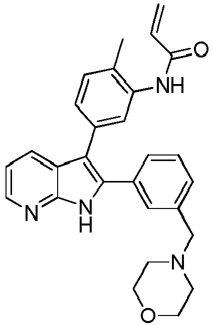 | 452 | 453 |

### Example 83:

### 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one 2,2,2-trifluoroacetate (F4)

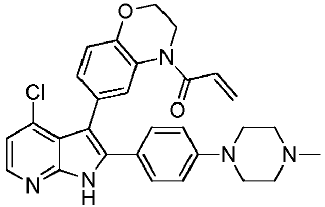

### Step 1: tert-butvl 6-(4-chloro-2-(trimethylsilyl)-1H-pyrrolo[2,3-b]pyridin-3-yl)-2H-benzo[b][1,4]oxazine-4(3H)-carboxylate (F1)

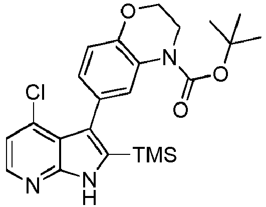

**F1** (632mg, 1.37mmol, 40%, beige solid) was prepared from 4-chloro-3-iodopyridine-2-amine (867mg, 3.41mmol, 1.0 eq.) and tert-butyl 6-((trimethylsilyl)ethynyl)-2H-benzo[b][1,4]oxazine-4(3H)-carboxylate (1.131mg, 3.41mmol, 1.0eq.) following the general procedure reported in Preparative Example 1 Step 1. MS (ES) C₂₃H₂₈ClN₃O₃Si requires: 458, found: 459 (M+H)⁺.

### Step 2: tert-butvl 6-(4-chloro-2-iodo-1H-pyrrolo[2,3-b]pyridin-3-yl)-2H-benzo[b][1,4]oxazine-4(3H)-carboxylate (F2)

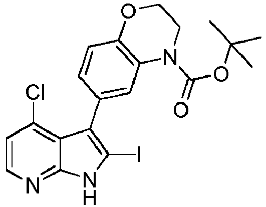

Tert-butyl 6-(4-chloro-2-(trimethylsilyl)-1H-pyrrolo[2,3-b]pyridin-3-yl)-2H-benzo[b][1,4]oxazine-4(3H)-carboxylate **F1** (300mg, 0.65mmol) and N-iodosuccinimide (264mg, 1.17mmol, 1.8eq.) were solved in dry dichloromethane (100mL) and stirred for 5h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **F2** (341mg, 0.65mmol, 100%) as a brown solid. The crude was used without purification in the next step. MS (ES) C₂₀H₁₉ClIN₃O₃ requires: 511, found: 512 (M+H)⁺.

### Step 3: 6-(4-chloro-2-(4-(4-methylpiperazin-1-yl)phenyl)-1H-pyrrolo[2,3-b]pyridin-3-yl)-3,4-dihydro-2H-benzo[b][1,4]oxazine (F3)

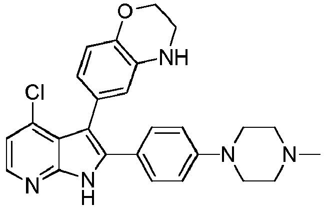

A mixture of tert-butyl 6-(4-chloro-2-iodo-1H-pyrrolo[2,3-b]pyridin-3-yl)-2H-benzo[b][1,4]oxazine-4(3H)-carboxylate **F2** (341mg, 0.65mmol, 1.0eq.), 4-(4-methylpiperazin-1-yl)phenylboronic acid (186mg, 0.85mmol, 1.3eq.), and K₃PO₄ (275mg, 1.30mmol, 2.0 eq) in dioxane/H₂O (10mL/1mL) was degassed with a stream of N₂ for 5min. [1,1'-Bis(diphenylphosphino)ferrocene]palladium dichloride dichloromethane adduct (53mg, 0.06mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude was solved in 30% TFA in DCM (60mL) and stirred for 2h at RT. Solvents were removed in vacuo. The crude product was purified by flash chromatography on silica gel (DCM/MeOH with 0.1% NEt₃ = 100:0 to 5:1) to yield the desired product **F3** (145mg, 0.31mmol, 49%) as a white solid. MS (ES) C₂₆H₂₆ClN₅O requires: 459, found: 460 (M+H)⁺.

### Step 4: 1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one 2,2,2-trifluoroacetate (F4)

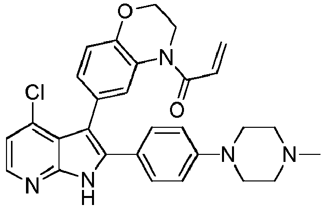

To a solution of **F3** (29mg, 0.063mmol, 1.0eq.) and DIPEA (110uL, 0.63mmol, 10.0eq.) in dry DCM (4mL) at 0°C was added slowly acrylolyl chloride (5.1mg, 0.057mmol, 0.9eq.) in dry DCM (5mL). The mixture was stirred for 5min. The solution was diluted with EtOAc and washed twice with aq. sat. NaHCO₃-solution. The organic phase was dried over MgSO₄ and solvent was removed in vacuo. The crude solution was purified by reverse phase RP-HPLC (column: C18), using H₂O (0.1%TFA) and ACN (0.1%TFA) as eluents. The desired fractions were lyophilized to yield the title compound **F4** (24mg, 0.03mmol, 51%) as a yellow powder. ¹H NMR (400MHz, d₄-MeOH, 300K) δ 2.96 (s, 3H), 3.06 (m, 2H), 3.25 (m, 4H), 3.59 (m, 2H), 3.92 (m, 2H), 4.34 (m, 2H), 5.38 (d, J = 10.4 Hz, 1H), 6.16 (dd, J = 2.0 Hz, J = 16.8 Hz, 1H), 6.36 (m, 1H), 6.98 (d, J = 8.3 Hz, 1H), 7.02 (d, J = 8.9 Hz, 2H), 7.12 (d, J = 5.4 Hz, 1H), 7.22 (d, J = 8.3 Hz, 1H), 7.37 (d, J = 8.9 Hz, 2H), 8.10 (d, J = 5.4 Hz, 1H). MS (ES) C₂₃H₂₈ClN₃O₃Si requires: 513, found: 514 (M+H)⁺.

The Examples in the following table were prepared according to the procedure described for **F4** (Example 83).

| **Example** | **Name** | **Mwt** | **[M+H]⁺** |
|---|---|---|---|
| 84 | 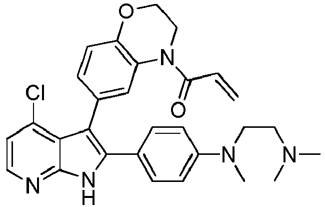 | 515 | 516 |
| 85 | 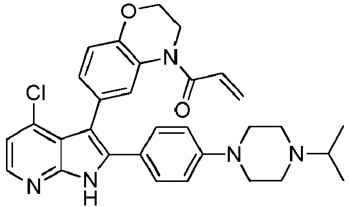 | 541 | 542 |

### Example 86:

### 1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one 2,2,2-trifluoroacetate (G5)

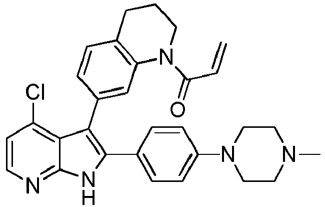

### Step 1: tert-butvl 7-(4-chloro-2-(trimethylsilyl)-1H-pyrrolo[2,3-b]pyridin-3-yl)-3,4-dihydroquinoline-1(2H)-carboxylate (G1)

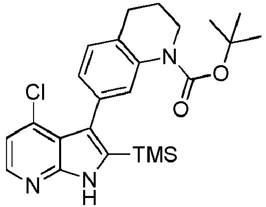

4-Chloro-3-iodopyridine-2-amine (1175mg, 4.6mmol, 1.0 eq.), tert-butyl 7-((trimethylsilyl)ethynyl)-3,4-dihydroquinoline-1(2H)-carboxylate (1522mg, 4.6mmol, 1.0 eq.), 1,4-diazabicyclo[2.2.2]octane (876mg, 7.8mmol, 1.7eq.) and dichlorobis(triphenylphosphine)palladium(II) (351mg, 0.4mmol, 0.1eq.) in dry DMF (30mL) under N2 atmosphere was splitted in three microwave vials. Each vial was heated in the microwave at 145°C for 2 h. The solvent was removed in vacuo. The crude product was purified by flash chromatography on silica gel (cHex/EtOAc = 100:0 to 1:1) to yield the desired product **G1** (1010mg, 2.2mmol, 48%) as a beige solid. ¹H NMR (400MHz, d₆-DMSO, 300K) δ 0.09 (s, 9H), 1.39 (s, 9H), 1.87 (m, 2H), 2.79 (7, J = 6.5 Hz, 2H), 3.65 (m, 2H), 6.94 (dd, J = 1.7 Hz, J = 7.7 Hz, 1H), 7.06 (d, J = 5.1 Hz, 1H), 7.09 (d, J = 7.7 Hz, 1H), 7.61 (d, J = 1.7 Hz, 1H), 8.17 (d, J = 5.1 Hz, 1H), 11.82 (s, 1H). MS (ES) C₂₄H₃₀ClN₃O₂Si requires: 455, found: 456 (M+H)⁺.

### Step 2: 7-(4-chloro-2-(trimethylsilyl)-1H-pyrrolo[2,3-b]pyridin-3-yl)-1,2,3,4-tetrahydroquinoline (G2)

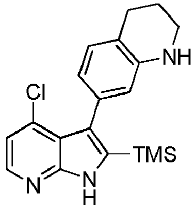

Tert-butyl 7-(4-chloro-2-(trimethylsilyl)-1H-pyrrolo[2,3-b]pyridin-3-yl)-3,4-dihydroquinoline-1(2H)-carboxylate **G1** (1.0g, 2.19mmol) in 50% TFA in DCM (20mL) at RT was stirred for 2h. Evaporation of the solvents yielded the desired product **G2** as brown solid, which was used in the next step without purification. MS (ES) C₁₉H₂₂ClN₃Si requires: 355, found: 356 (M+H)⁺.

### Step 3: 1-(7-(4-chloro-2-(trimethylsilyl)-1H-pyrrolo[2,3-b]pyridin-3-yl)-3,4-dihydroquinolin-1(2H)-yl)prop-2-en-1-one (G3)

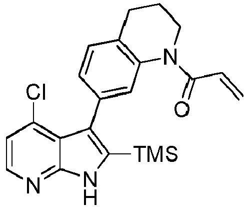

To a solution of **G2** (2.19mmol, 1.0eq.) and DIPEA (2.5mL, 14.5mmol, 5.0eq.) in dry DCM (40mL) at 0°C was added slowly acrylolyl chloride (235mg, 2.6mmol, 1.0eq.) in dry DCM (5mL). The mixture was stirred for 10min. The solution was diluted with EtOAc and washed twice with aq. sat. NaHCO₃-solution. The organic phase was dried over MgSO₄ and solvent was removed in vacuo. The crude product was purified by flash chromatography on silica gel (cHex/EtOAc = 100:0 to 1:1) to yield the desired product **G3** (581mg, 1.42mmol, 65%) as a white solid. MS (ES) C₂₂H₂₄ClN₃OSi requires: 409, found: 410 (M+H)⁺.

### Step 4: 1-(7-(4-chloro-2-iodo-1H-pyrrolo[2,3-b]pyridin-3-yl)-3,4-dihydroquinolin-1(2H)-yl)prop-2-en-1-one (G4)

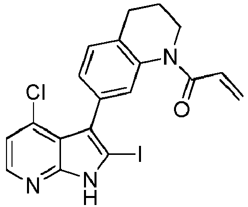

1-(7-(4-chloro-2-(trimethylsilyl)-1H-pyrrolo[2,3-b]pyridin-3-yl)-3,4-dihydroquinolin-1(2H)-yl)prop-2-en-1-one **G3** (371mg, 0.91mmol, 1.0eq.) and N-iodosuccinimide (366mg, 1.63mmol, 1.8eq.) were solved in dry dichloromethane (300mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **G4** as a yellow solid. The crude was used without purification in the next step. MS (ES) C₁₉H₁₅ClIN₃O requires: 462, found: 463 (M+H)⁺.

### Step 5: 1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one 2,2,2-trifluoroacetate (G5)

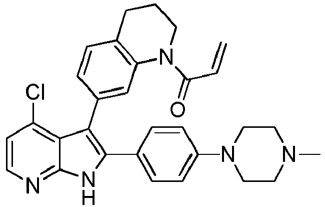

A mixture of 1-(7-(4-chloro-2-iodo-1H-pyrrolo[2,3-b]pyridin-3-yl)-3,4-dihydroquinolin-1(2H)-yl)prop-2-en-1-one **G4** (50mg, 0.10mmol, 1.0eq.), 4-(4-methylpiperazin-1-yl)phenylboronic acid (31mg, 0.14mmol, 1.5eq.), and K₃PO₄ (38mg, 0.18mmol, 2.0 eq) in dioxane/H₂O (3mL/0.6mL) was degassed with a stream of N₂ for 5min. [1,1'-Bis(diphenylphosphino)ferrocene]palladium dichloride dichloromethane adduct (8mg, 0.01 mmol, 0.1 eq) was added and the reaction mixture heated to 130°C for 2 h in the microwave oven. The crude reaction mixture was purified by reverse phase RP-HPLC (column: C18), using H₂O (0.1%TFA) and ACN (0.1%TFA) as eluents. The desired fractions were lyophilized to yield the title compound **G5** (22mg, 0.03mmol, 30%) as a yellow powder. ¹H NMR (400MHz, d₆-DMSO, 300K) δ 1.92 (q, J = 6.5 Hz, 2H), 2.52 (m, 2H), 2.79 (t, J = 6.5 Hz, 2H), 2.85 (s, 3H), 2.96 (t, J = 12.2 Hz, 2 H), 3.12 (m, 2H), 3.51 (d, J = 12.2 Hz, 2H), 3.75 (m, 2H), 5.22 (dd, J = 2.4 Hz, J = 10.2 Hz, 1H), 6.03 (dd, J = 2.4 Hz, J = 16.7 Hz, 1H), 6.25 (dd, J = 10.2 Hz, J = 16.7 Hz, 1H), 6.88 (s, 1H), 6.99 (d, J = 9.0 Hz, 2H), 7.12 (d, J = 5.2 Hz, 1H), 7.21 (dd, J = 1.6 Hz, J = 7.6 Hz, 1H), 7.28 (d, J = 7.6 Hz, 1H), 7.31 (d, J = 9.0 Hz, 2H), 8.15 (d, J = 5.2 Hz, 1H), 12.40 (s, 1H). MS (ES) C₃₀H₃₀ClN₅O requires: 511, found: 512 (M+H)⁺.

The Examples in the following table were prepared according to the procedure described for **G5** (Example 86).

| **Example** | **Name** | **Mwt** | **[M+H]⁺** |
|---|---|---|---|
| 87 | 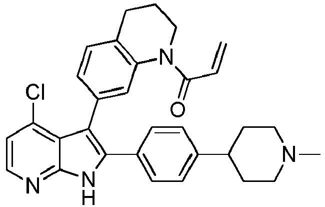 | 510 | 511 |
| 88 | 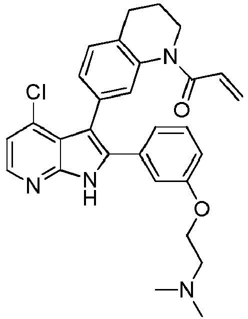 | 500 | 501 |
| 89 | 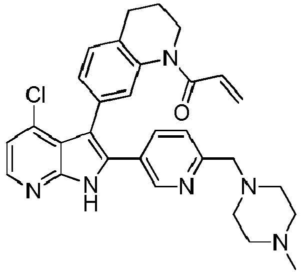 | 526 | 527 |
| 90 | 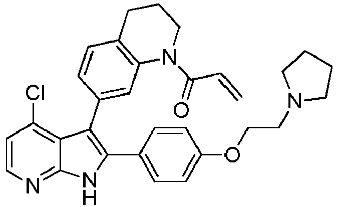 | 526 | 527 |
| 91 | 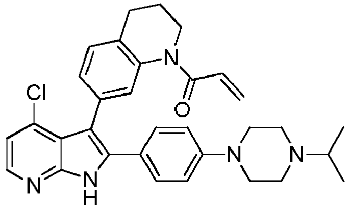 | 539 | 540 |
| 92 | 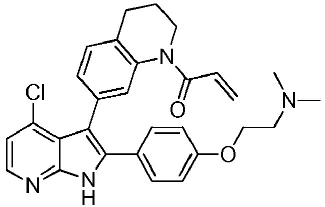 | 500 | 501 |
| 93 | 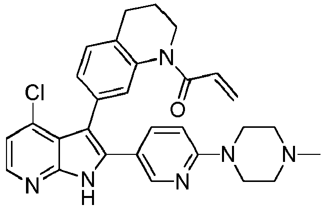 | 512 | 513 |
| 94 | 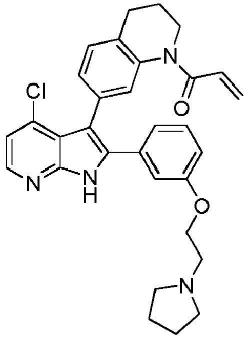 | 526 | 527 |
| 95 | 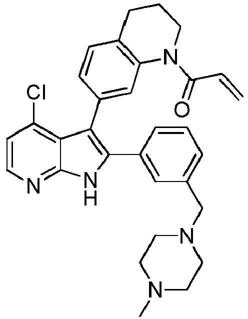 | 525 | 526 |

### Example 96:

### N-(5-(4-chloro-2-(4-(piperazin-1-yl)phenyl)-1H-pyrrolo[2,3-b]pyridin-3-yl)-2-methylphenyl)acrylamide 2,2,2-trifluoroacetate (H2)

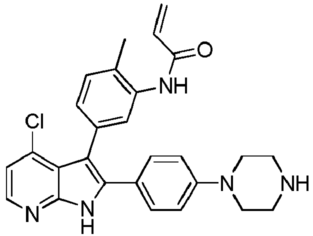

### Step 1: tert-butvl 4-(4-(3-(3-acrylamido-4-methylphenyl)-4-chloro-1H-pyrrolo[2,3-b]pyridin-2-yl)phenyl)piperazine-1-carboxylate (H1)

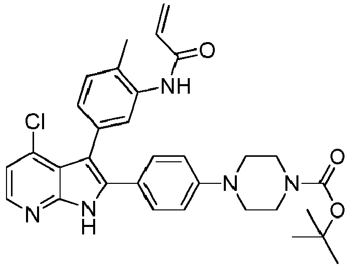

A mixture of N-(5-(4-chloro-2-iodo-1H-pyrrolo[2,3-b]pyridin-3-yl)-2-methylphenyl)acrylamide **A4** (100mg, 0.22mmol, 1.0eq.), 4-(4-boc-piperazino)phenylboronic acid (105mg, 0.34mmol, 1.5eq.), and K₃PO₄ (97mg, 0.44mmol, 2.0 eq) in dioxane/H₂O (3mL/0.6mL) was degassed with a stream of N₂ for 5min. [1,1'-bis(diphenylphosphino)ferrocene]palladium dichloride dichloromethane adduct (19mg, 0.02mmol, 0.1 eq) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude product was purified by flash chromatography on silica gel (cHex/EtOAc = 100:0 to 0:100) to yield the desired product **H1** (41mg, 0.07mmol, 32%) as a beige solid. MS (ES) C₃₂H₃₄ClN₅O₃ requires: 571, found: 572 (M+H)⁺.

### Step 2: N-(5-(4-chloro-2-(4-(piperazin-1-yl)phenyl)-1H-pyrrolo[2,3-b]pyridin-3-yl)-2-methylphenyl)acrylamide 2,2,2-trifluoroacetate (H2)

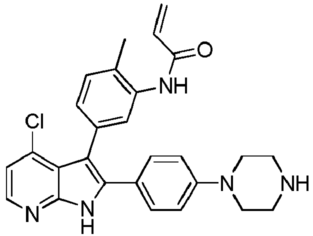

Tert-butyl 4-(4-(3-(3-acrylamido-4-methylphenyl)-4-chloro-1H-pyrrolo[2,3-b]pyridin-2-yl)phenyl)piperazine-1-carboxylate **H1** (41mg, 0.07mmol) in 30% TFA in DCM (5mL) at RT was stirred for 2h. After evaporation of the solvents, the crude was purified by reverse phase RP-HPLC (column: C18), using H₂O (0.1%TFA) and ACN (0.1%TFA) as eluents. The desired fractions were lyophilized to yield the title compound **H2** (18mg, 0.02mmol, 37%) as a yellow powder. ¹H NMR (400MHz, d₄-MeOH, 300K) δ 2.11 (s, 3H), 3.14 (m, 4H), 3.22 (m, 4H), 5.57 (dd, J = 1.3 Hz, J = 10.2 Hz, 1H), 6.13 (dd, J = 1.3 Hz, J = 16.9 Hz, 1H), 6.30 (dd, J = 10.2 Hz, J = 16.9 Hz, 1H), 6.75 (d, J = 8.8 Hz, 2H), 6.90 (m, 2H), 7.03 (d, J = 7.8 Hz, 1H), 7.23 (m, 3H), 7.89 (d, J = 5.4 Hz, 1H). MS (ES) C₂₇H₂₆ClN₅O requires: 471, found: 472 (M+H)⁺.

### Example 97:

### N-(5-(5-cyano-2-(4-(4-methylpiperazin-1-yl)phenyl)-1H-pyrrolo[2,3-b]pyridin-3-yl)-2-methylphenyl)acrylamide 2,2,2-trifluoroacetate (15)

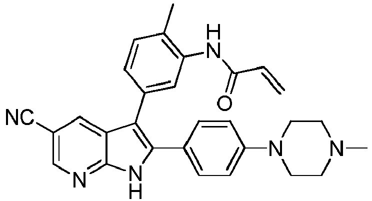

### Step 1: 3-(4-methyl-3-nitrophenyl)-2-(trimethylsilyl)-1H-pyrrolo[2,3 b]pyridine-5-carbonitrile (I1)

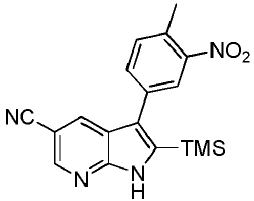

**I1** (2.6g, 7.43mmol, 40%, yellow solid) was prepared from 6-amino-5-bromonicotinonitrile (3.6g, 18.2mmol, 1.00eq.) and trimethyl((4-methyl-3-nitrophenyl)ethynyl)silane (4.5g, 19.0mmol, 1.05eq.) following the general procedure reported in Preparative Example 1 Step 1. MS (ES) C₁₈H₁₈N₄O₂Si requires: 350, found: 351 (M+H)⁺.

### Step 2: 3-(4-methyl-3-nitrophenyl)-2-(trimethylsilyl)-1H-pyrrolo[2,3-b]pyridine-5-carbonitrile (I2)

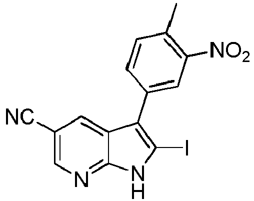

3-(4-Methyl-3-nitrophenyl)-2-(trimethylsilyl)-1H-pyrrolo[2,3-b]pyridine-5-carbonitrile **I1** (270mg, 0.8mmol, 1.0eq.) and N-iodosuccinimide (208mg, 0.96mmol, 1.2eq.) were solved in dry dichloromethane (40mL) and stirred for 15h at RT. The organic phase was washed once with aq. sat. Na₂S₂O₃-solution and three times with aq. sat. NaHCO₃-solution. The organic phase was dried over Na₂SO₄ and solvents were removed in vacuo yielding the desired product **I2** (123mg, 0.30mmol, 38%) as a brown solid. The crude was used without purification in the next step. MS (ES) C₁₅H₉IN₄O₂ requires: 403, found: 404 (M+H)⁺.

### Step 3: 3-(4-methyl-3-nitrophenyl)-2-(4-(4-methylpiperazin-1-yl)phenyl)-1H-pyrrolo[2,3-b]pyridine-5-carbonitrile (I3)

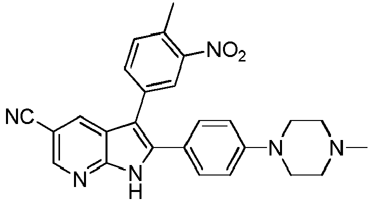

A mixture of 3-(4-methyl-3-nitrophenyl)-2-(trimethylsilyl)-1H-pyrrolo[2,3-b]pyridine-5-carbonitrile **I2** (1.41g, 3.5mmol, 1.0eq.), 4-(4-methylpiperazin-1-yl)phenylboronic acid (1.14mg, 5.2mmol, 1.5eq.), and K₃PO₄ (1.47g, 7.0mmol, 2.0 eq) in dioxane/H₂O (40mL/4mL) was degassed with a stream of N₂ for 5min. [1,1'-Bis(diphenylphosphino)ferrocene]palladium dichloride dichloromethane adduct (284mg, 0.35mmol, 0.1eq.) was added and the reaction mixture heated to 130°C for 2 h in the microwave oven. The reaction mixture was diluted with EtOAc, washed three times with aq. sat. NaHCO₃-solution. The organic phase was dried over MgSO₄ and solvents were removed in vacuo. The crude product was purified by flash chromatography on silica gel (DCM/MeOH with 0.1% NEt₃ = 100:0 to 1:1) to yield the desired product **I3** (500mg, 1.10mmol, 32%) as a yellow solid. MS (ES) C₂₆H₂₄N₆O₂ requires: 452, found: 453 (M+H)⁺.

### Step 4: 3-(3-amino-4-methylphenyl)-2-(4-(4-methylpiperazin-1-yl)phenyl)-1H-pyrrolo[2,3-b]pyridine-5-carbonitrile (I4)

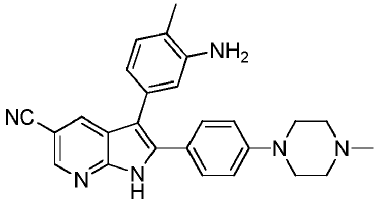

**I4** (93mg, 0.22mmol) was prepared from **I3** following the general procedure reported in Preparative Example 1 Step 2. MS (ES) C₁₈H₁₈N₄O₂Si requires: 350, found: 351 (M+H)⁺.

### Step 5: N-(5-(5-cyano-2-(4-(4-methylpiperazin-1-yl)phenyl)-1H-pyrrolo[2,3-b]pyridin-3-yl)-2-methylphenyl)acrylamide 2,2,2-trifluoroacetate (I5)

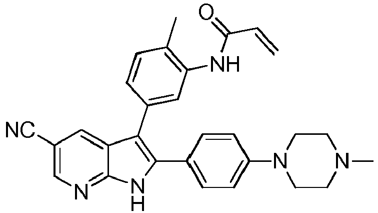

To a solution of **I4** (30mg, 0.071mmol, 1.0eq.) and DIPEA (92mg, 0.71mmol, 10.0eq.) in dry DCM (2mL) at 0°C was added slowly acrylolyl chloride (32mg, 0.355mmol, 5.0eq.) in dry DCM (1mL). The mixture was stirred for 5min. The crude solution was directly purified by reverse phase RP-HPLC (column: C18), using H₂O (0.1%TFA) and ACN (0.1%TFA) as eluents. The desired fractions were lyophilized to yield the title compound **I6** (3mg, 0.004mmol, 5%) as a yellow powder. ¹H NMR (400MHz, d₄-MeOH, 300K) δ 2.31 (s, 3H), 2.95 (s, 3H), 3.30 - 3.48 (m, 8H), 5.79 (dd, J = 1.6 Hz, J = 10.2 Hz, 1H), 6.36 (dd, J = 1.6 Hz, J = 17.0 Hz, 1H), 6.52 (dd, J = 10.2 Hz, J = 17.0 Hz, 1H), 7.02 (d, J = 9.0 Hz, 2H), 7.09 (d, J = 8.1 Hz, 1H), 7.27 (d, J = 7.8 Hz, 1H), 7.50 (m, 3H), 8.23 (d, J = 1.9 Hz, 1H), 8.51 (d, J = 1.9 Hz, 1H). MS (ES) C₂₉H₂₈N₆O requires: 476, found: 477 (M+H)⁺.

### Example 98:

### 1-(6-(4-chloro-2-(2-(4-methylpiperazin-1-yl)pyridin-4-yl)-1H-pyrrolor2,3-b]pyridin-3-yl)-2H-benzo[b][1,4]oxazin-4(3H)-yl)prop-2-en-1-one 2,2,2-trifluoroacetate (J4)

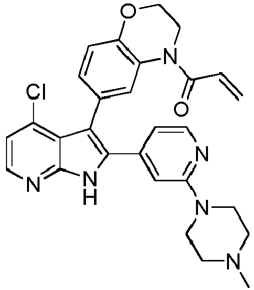

### Step 1: 6-(4-chloro-2-(trimethylsilyl)-1H-pyrrolo[2,3-b]pyridin-3-yl)-3,4-dihydro-2H-benzo[b][1,4]oxazine (J1)

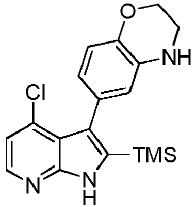

Tert-butyl 6-(4-chloro-2-(trimethylsilyl)-1H-pyrrolo[2,3-b]pyridin-3-yl)-2H-benzo[b][1,4]oxazine-4(3H)-carboxylate **F1** (3489mg, 7.62mmol) in 30% TFA in DCM (20mL) at RT was stirred for 2h. Evaporation of the solvents yielded a brown solid, which was purified by flash chromatography on silica gel (DCM/MeOH = 100:0 to 5:1) to yield the desired product **J1** (2726mg, 7.62mmol, 100%) as a yellow solid. MS (ES) C₁₈H₂₀ClN₃OSi requires: 357, found: 358 (M+H)⁺.

### Step 2: 1-(6-(4-chloro-2-(trimethylsilyl)-1H-pyrrolo[2,3-b]pyridin-3-yl)-2H-benzo[b][1,4]oxazin-4(3H)-yl)prop-2-en-1-one (J2)

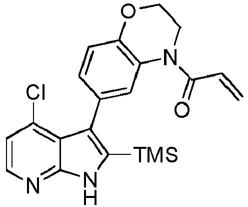

**J2** (635mg, 1.54mmol, 55%, white solid) was prepared from **J1** following the general procedure reported in Preparative Example 1 Step 3 Procedure A. MS (ES) C₂₁H₂₂ClN₃O₂Si requires: 411, found: 412 (M+H)⁺.

### Step 3: 1-(6-(4-chloro-2-iodo-1H-pyrrolo[2,3-b]pyridin-3-yl)-2H-benzo[b][1,4]oxazin-4(3H)-yl)prop-2-en-1-one (J3)

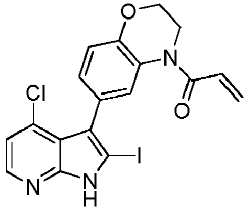

**J3** (465mg, 1.00mmol, 100%, yellow solid) was prepared from **J2** following the general procedure reported in Preparative Example 1 Step 4. MS (ES) C₁₈H₁₃ClN₃O₂ requires: 465, found: 466 (M+H)⁺.

### Step 4: 1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one 2,2,2-trifluoroacetate (J4)

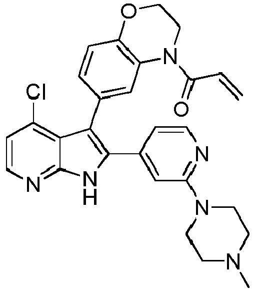

**J4** (7.4mg, 0.01 mmol, 9%, yellow solid) was prepared from **J3** and 2-(4-methylpiperazino)pyridine-4-boronic acid pinacol ester following the general procedure reported in Preparative Example 1 Step 5. ¹H NMR (400MHz, d₆-DMSO, 300K) δ 2.86 (s, 3H), 3.06 (m, 4H), 3.50 (d, J = 10.3 Hz, 2H), 4.27 - 4.40 (m, 6H), 5.52 (d, J = 10.6 Hz, 1H), 6.15 (dd, J = 1.9 Hz, J = 16.9 Hz, 1H), 6.59 (m, 1H), 6.66 (d, J = 5.3 Hz, 1H), 7.00 (d, J = 8.3 Hz, 1H), 7.03 (s, 1H), 7.12 (d, J = 7.6 Hz, 1H), 7.20 (d, J = 5.1 Hz, 1H), 8.10 (d, J = 5.3 Hz, 1H), 8.26 ( d, J = 5.1 Hz, 1H), 9.73 (s, 1H), 12.66 (s, 1H). MS (ES) C₂₈H₂₇ClN₆O₂ requires: 514, found: 515 (M+H)⁺.

The Examples in the following table were prepared according to the procedure described for **J4** (Example 98).

| **Example** | **Name** | **Mwt** | **[M+H]⁺** |
|---|---|---|---|
| 99 | 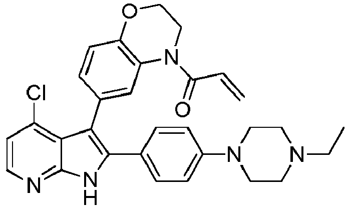 | 527 | 528 |
| 100 | 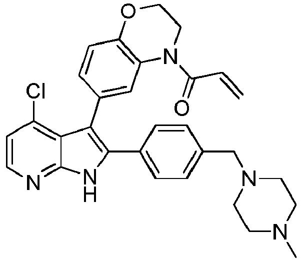 | 527 | 528 |
| 101 | 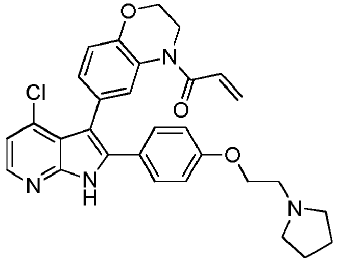 | 528 | 529 |
| 102 | 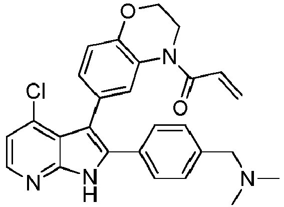 | 472 | 473 |
| 103 | 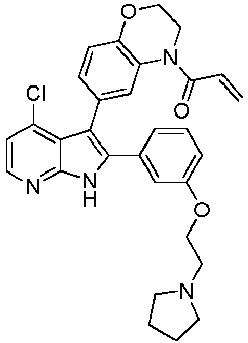 | 528 | 529 |

### Example 104:

### 1-(6-(4-ethoxy-2-(4-(4-methylpiperazin-1-yl)phenyl)-1H-pyrrolo[2,3-b]pyridin-3-yl)indolin-1-yl)prop-2-en-1-one 2,2,2-trifluoroacetate (K5)

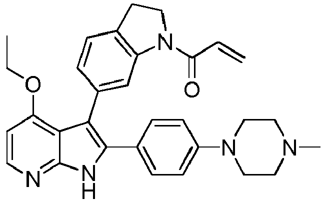

### Step 1: tert-butvl 6-(4-ethoxy-2-(trimethylsilyl)-1H-pyrrolo[2,3-b]pyridin-3-yl)indoline-1-carboxylate (K1)

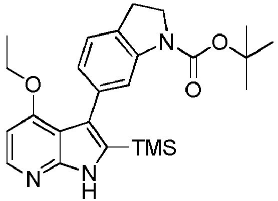

**K1** (3.74g, 8.29mmol, 73%, yellow solid) was prepared from 4-ethoxy-3-iodopyridin-2-amine and tert-butyl 6-((trimethylsilyl)ethynyl)indoline-1-carboxylate following the general procedure reported in Preparative Example 1 Step 1. MS (ES) C₂₅H₃₃N₃O₃Si requires: 451, found: 452 (M+H)⁺.

### Step 2: 4-ethoxy-3-(indolin-6-yl)-2-(trimethylsilyl)-1H-pyrrolo[2,3-b]pyridine (K2)

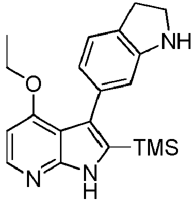

Tert-butyl 6-(4-ethoxy-2-(trimethylsilyl)-1H-pyrrolo[2,3-b]pyridin-3-yl)indoline-1-carboxylate **K1** (1.5g, 3.3mmol) in 30% TFA in DCM (60mL) at RT was stirred for 2h. Evaporation of the solvents yielded a brown solid. To the crude was added aq. sat. NaHCO₃-solution and the mixture were extracted three times with DCM. The organic phase was dried over MgSO₄ and solvent was removed in vacuo. The desired product **K2** which has an impurity of 4-ethoxy-3-(indolin-6-yl)-1H-pyrrolo[2,3-b]pyridine was used without further purification in the next step. MS (ES) C₂₀H₂₅N₃OSi requires: 351, found: 352 (M+H)⁺.

### Step 3: 1-(6-(4-ethoxy-2-(trimethylsilyl)-1H-pyrrolo[2,3-b]pyridin-3-yl)indolin-1-yl)prop-2-en-1-one (K3)

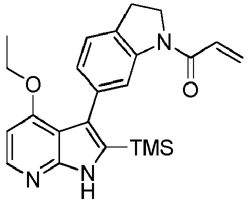

**K3** (371mg, 0.91mmol, 32%, white solid) was prepared from **K2** following the general procedure reported in Preparative Example 1 Step 3 Procedure A. MS (ES) C₂₃H₂N₃O₂Si requires: 405 found: 406 (M+H)⁺.

### Step 4: 1-(6-(4-ethoxy-2-iodo-1H-pyrrolo[2,3-b]pyridin-3-yl)indolin-1-yl)prop-2-en-1-one (K4)

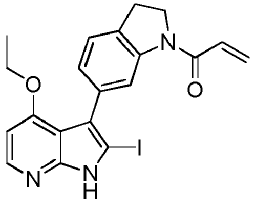

**K4** (380mg, 0.83mmol, yellow solid) was prepared from **K3** following the general procedure reported in Preparative Example 1 Step 4. MS (ES) C₂₀H₁₈IN₃O₂ requires: 459, found: 460 (M+H)⁺.

### Step 5: 1-(6-(4-ethoxy-2-(4-(4-methylpiperazin-1-yl)phenyl)-1H-pyrrolo[2,3-b]pyridin-3-yl)indolin-1-yl)prop-2-en-1-one 2,2,2-trifluoroacetate (K5)

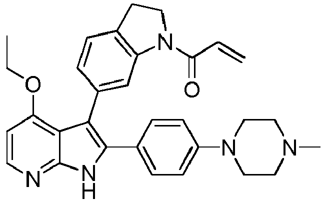

**K5** (14mg, 0.019mmol, 17%, yellow solid) was prepared from **K4** and 4-(4-methylpiperazin-1-yl)phenylboronic acid following the general procedure reported in Preparative Example 1 Step 5. ¹H NMR (400MHz, d₆-DMSO, 300K) δ 1.09 (t, J = 6.9 Hz, 3H), 2.85 (s, 3H), 2.97 (t, J = 12.6 Hz, 2H), 3.12 (m, 2H), 3.21 (t, J = 8.5 Hz, 2H), 3.51 (d, J = 11.9 Hz, 2H), 3.91 (d, J = 13.3 Hz, 2H), 4.11 (quart., J = 6.9 Hz, 2H), 4.26 (t, J = 8.5 Hz, 2H), 5.80 (d, J = 10.3 Hz, 1H), 6.25 (d, J = 16.5 Hz, 1H), 6.76 (m, 2H), 6.91 (d, J = 7.6 Hz, 1H), 6.96 (d, J = 8.5 Hz, 2H), 7.17 (d, J = 7.6 Hz, 1H), 7.34 (d, J = 8.5 Hz, 2H), 8.19 (d, J = 6.0 Hz, 1H), 8.25 (s, 1H), 9.79 (s, 1H), 12.39 (s, 1H). MS (ES) C₃₁H₃₃N₅O₂ requires: 507, found: 508 (M+H)⁺.

The Examples in the following table were prepared according to the procedure described for **K5** (Example 104).

| **Example** | **Name** | **Mwt** | **[M+H]⁺** |
|---|---|---|---|
| 105 | 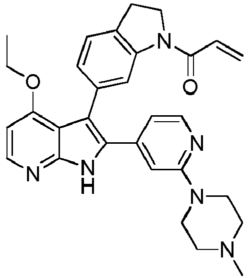 | 508 | 509 |
| 106 | 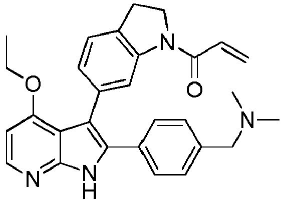 | 466 | 467 |
| 107 | 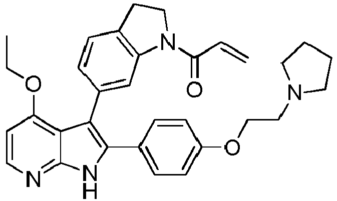 | 522 | 523 |
| 108 | 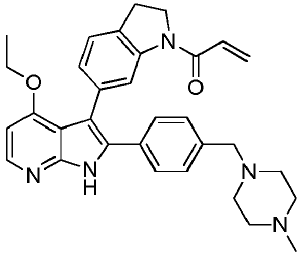 | 521 | 522 |
| 109 | 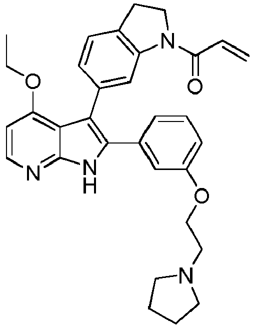 | 522 | 523 |

The Examples in the following table were prepared according to the procedure described for **K5** (Example 104) using 4-methoxy-3-iodopyridin-2-amine as a starting material in the step 1.

| **Example** | **Name** | **Mwt** | **[M+H]⁺** |
|---|---|---|---|
| 110 | 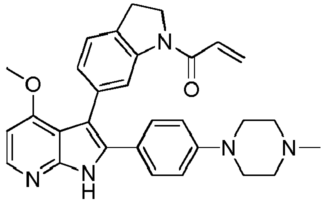 | 493 | 494 |
| 111 | 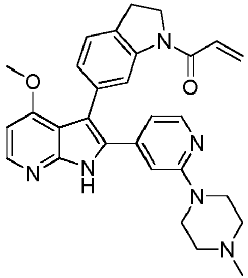 | 494 | 495 |
| 112 | 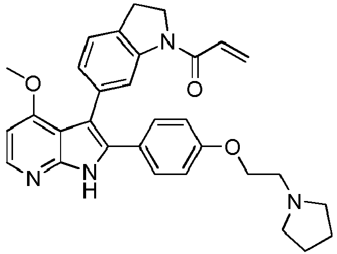 | 508 | 509 |
| 113 | 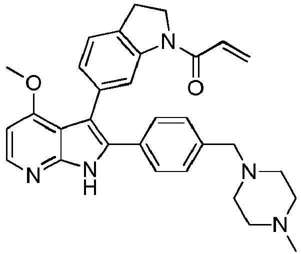 | 507 | 508 |
| 114 | 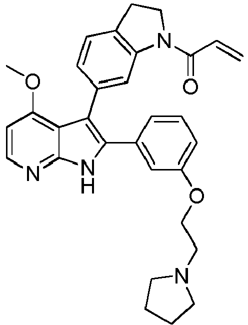 | 508 | 509 |

### Example 115:

### 1-(6-(4-isopropoxy-2-(4-(4-methylpiperazin-1-yl)phenyl)-1H-pyrrolo[2,3-b]pyridin-3-yl)indolin-1-yl)prop-2-en-1-one 2,2,2-trifluoroacetate (L1)

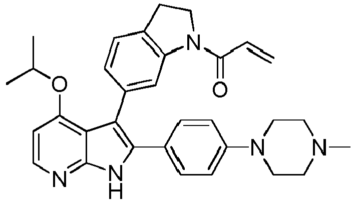

**L1** (15mg, 0.2mmol, yellow solid) was prepared according to the procedure described for **K5** (Example 104) using 3-iodo-4-isopropoxypyridin-2-amine as a starting material in the step 1. ¹H NMR (400MHz, d₆-DMSO, 300K) δ 1.13 (d, J = 6.0 Hz, 6H), 2.85 (s, 3H), 3.00 (m, 2H), 3.12 (m, 2H), 3.22 (t, J = 8.3 Hz, 2H), 3.50 (m, 2H), 3.92 (m, 2H), 4.27 (t, J = 8.3 Hz, 2H), 4.85 (quint., J = 6.0 Hz, 1H), 5.80 (d, J = 10.3 Hz, 1H), 6.25 (d, J = 16.6 Hz, 1H), 6.77 (d, J = 10.3 Hz, J = 16.6 Hz, 1H), 6.90 (d, J = 7.6 Hz, 1H), 6.94 (d, J = 6.5 Hz, 1H), 6.98 (d, J = 8.5 Hz, 2H), 7.18 (d, J = 7.6 Hz, 1H), 7.35 (d, J = 8.5 Hz, 2H), 8.27 (m, 2H), 10.02 (s, 1H), 12.81 (s, 1H). MS (ES) C₃₂H₃₅N₅O₂ requires: 521, found: 522 (M+H)⁺.

### Example 116:

### 3-(3-acrylamido-4-methylphenyl)-2-(4-(4-methylpiperazin-1-yl)phenyl)-1H-pyrrolo[2,3-b]pyridine-5-carboxylic acid 2,2,2-trifluoroacetate (M3)

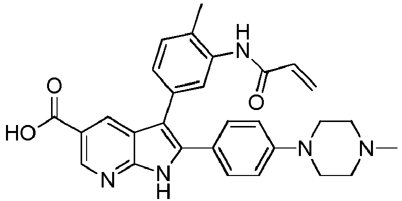

### Step 1: 3-(4-methyl-3-nitrophenyl)-2-(4-(4-methylpiperazin-1-yl)phenyl)-1H-pyrrolo[2,3-b]pyridine-5-carboxylic acid hydrochloric acid (M1)

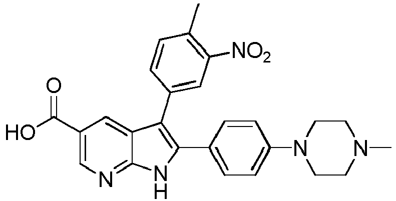

A mixture of 3-(4-methyl-3-nitrophenyl)-2-(4-(4-methylpiperazin-1-yl)phenyl)-1 H-pyrrolo[2,3-b]pyridine-5-carbonitrile **I3** (3.0g, 6.6mmol) in conc. aq. HCl-solution (50mL) was heated for 3 h at 100°C. Solvent was removed in vacuo yielding the desired product **M1** as a brown solid (3.1g, 6.6mmol). MS (ES) C₂₆H₂₅N₅O₄ requires: 471, found: 472 (M+H)⁺.

### Step 2: 3-(3-amino-4-methylphenyl)-2-(4-(4-methylpiperazin-1-yl)phenyl)-1H-pyrrolo[2,3-b]pyridine-5-carboxylic acid (M2)

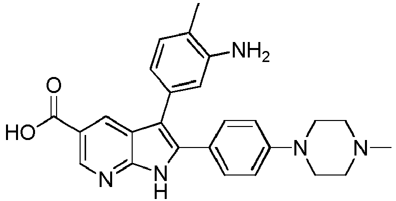

A mixture of 3-(4-methyl-3-nitrophenyl)-2-(4-(4-methylpiperazin-1-yl)phenyl)-1H-pyrrolo[2,3-b]pyridine-5-carboxylic acid **M1** (3.0g, 6.6mmol, 1.0eq.) and iron (1.8g, 31.8mmol, 5eq.) in EtOH (100mL) and sat. aq. NH₄Cl-solution (20mL) was heated for 15h at 80°C. Solvents were removed in vacuo and the crude was purified by reverse phase RP-HPLC (column: C18), using H₂O (0.1%TFA) and ACN (0.1%TFA) as eluents. The desired fractions were lyophilized to yield the title compound **M2** (1.7g, 4.0mmol, 60%) as a yellow powder. MS (ES) C₂₆H₂₇N₅O₂ requires: 441, found: 442 (M+H)⁺.

### Step 3: 3-(3-acrylamido-4-methylphenyl)-2-(4-(4-methylpiperazin-1-yl)phenyl)-1H-pyrrolo[2,3-b]pyridine-5-carboxylic acid 2,2,2-trifluoroacetate (M3)

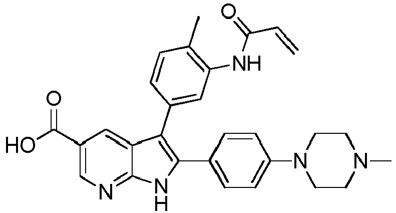

To a solution of 3-(3-amino-4-methylphenyl)-2-(4-(4-methylpiperazin-1-yl)phenyl)-1H-pyrrolo[2,3-b]pyridine-5-carboxylic acid **M2** (580mg, 1.31mmol, 1.0eq.) and DIPEA (2.3mL, 13.1mmol, 10eq.) in dry THF (10ml) at 0°C was added slowly acryloyl chloride (0.11mL, 1.31mmol, 1.0eq.) in dry THF (1mL). After 10min a drop of water was added and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **M3** (170mg, 0.34mmol, 26%) as a yellow powder. ¹H NMR (400MHz, d₆-DMSO, 300K) δ 2.27 (s, 3H), 2.85 (s, 3H), 3.01 (t, J = 12.5 Hz, 2H), 3.13 (m, 2H), 3.51 (d, J = 12.0 Hz, 2H), 3.93 (d, J = 13.3 Hz, 2H), 5.74 (dd, J = 10.1 Hz, J = 2.0 Hz, 1H), 6.22 (dd, J = 17.1 Hz, J = 2.0 Hz, 1H), 6.56 (dd, J = 17.1 Hz, J = 10.1 Hz, 1H), 7.03 (m, 3H), 7.27 (d, J = 7.9 Hz, 1H), 7.47 (d, J = 6.4 Hz, 2H), 7.60 (s, 1H), 8.26 (d, J = 2.0 Hz, 1H), 8.80 (d, J = 2.0 Hz, 1H), 9.55 (s, 1H), 9.72 (br s, 1H), 12.41 (s, 1H), 12.86 (br s, 1H). MS (ES) C₂₉H₂₉N₅O₃ requires: 495, found: 496 (M+H)⁺.

### Example 117:

### isopropyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (N3)

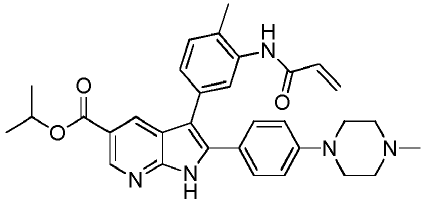

### Step 1: isopropyl 3-(4-methyl-3-nitrophenyl)-2-(4-(4-methylpiperazin-1-yl)phenyl)-1H-pyrrolo[2,3-b]pyridine-5-carboxylate (N1)

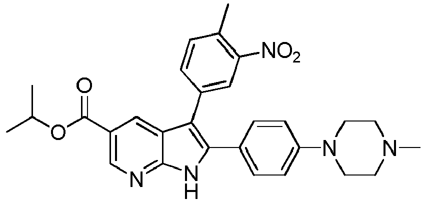

A mixture of 3-(4-methyl-3-nitrophenyl)-2-(4-(4-methylpiperazin-1-yl)phenyl)-1 H-pyrrolo[2,3-b]pyridine-5-carbonitrile **I3** (100mg, 0.2mmol) and conc. H₂SO₄ (1mL) in isopropyl alcohol (2mL) was heated for 48h at 80°C. After cooling to RT, EtOAc and sat. aq. NaHCO₃-solution was added. The aq. phase was extracted with EtOAc (3x) and the combined organic phase was dried over MgSO₄. Evaporation of solvents yielded the crude product of **N1** (28mg, 0.05mmol, 27%) as a yellow solid. The crude was used in the next step without further purification. MS (ES) C₂₉H₃₁N₅O₄ requires: 513, found: 514 (M+H)⁺.

### Step 2: isopropyl 3-(3-amino-4-methylphenyl)-2-(4-(4-methylpiperazin-1-yl)phenyl)-1H-pyrrolo[2,3-b]pyridine-5-carboxylate (N2)

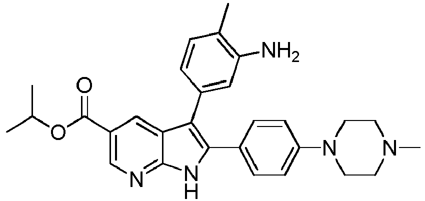

Isopropyl 3-(4-methyl-3-nitrophenyl)-2-(4-(4-methylpiperazin-1-yl)phenyl)-1H-pyrrolo[2,3-b]pyridine-5-carboxylate **N1** (28mg, 0.05mmol, 1.0eq.) and iron (15mg, 0.3mmol, 5eq.) in EtOH (5mL) and sat. aq. NH₄Cl-solution (1mL) was heated for 3h at 80°C. Sat. aq. NaHCO₃-solution was added and the aq. phase was extracted with DCM (3x). The organic phase was dried over MgSO₄ and solvents were removed in vacuo. The crude product was purified by flash chromatography on silica gel (DCM/MeOH with 0.1% NEt₃ = 100:0 to 1:1) to yield the desired product **N2** (25mg, 0.05mmol, 94%) as a brown solid. MS (ES) C₂₉H₃₃N₅O₂ requires: 483, found: 484 (M+H)⁺.

### Step 3: isopropyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (N3)

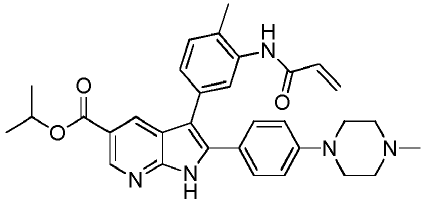

**N3** (2mg, 0.003mmol, 4%, yellow solid) was prepared from **N2** following the general procedure reported in Preparative Example 98 Step 3. The crude product was purified reverse phase RP-HPLC (column: C18), using H₂O (0.1%TFA) and ACN (0.1%TFA) as eluents. The desired fractions were lyophilized to yield the title compound **N3** as a yellow solid. ¹H NMR (400MHz, d₆-DMSO, 300K) δ 1.31 (d, J = 6.2 Hz, 6H), 2.26 (s, 3H), 2.85 (s, 3H), 2.98 (t, J = 13.0 Hz, 2H), 3.10 (m, 2H), 3.50 (d, J = 12.0 Hz, 2H), 3.92 (d, J = 13.0 Hz, 2H), 5.14 (quint., J = 6.2 Hz, 1H), 5.72 (dd, J = 10.2 Hz, J = 2.0 Hz, 1H), 6.21 (dd, J = 17.1 Hz, J = 2.0 Hz, 1H), 6.55 (dd, J = 10.2 Hz, J = 17.1 Hz, 1H), 6.99 (d, J = 8.6 Hz, 2H), 7.25 (d, J = 7.9 Hz, 1H), 7.45 (d, J = 8.6 Hz, 2H), 7.64 (s, 1H), 8.31 (d, J = 2.0 Hz, 1H), 8.79 (d, J = 2.0 Hz, 1H), 9.50 (s, 1H), 9.58 (br. s, 1H), 12.43 (s, 1H). MS (ES) C₃₂H₃₅N₅O₃ requires: 537 found: 538 (M+H)⁺.

### Example 118:

### 3-(3-acrylamido-4-methylphenyl)-N-benzyl-2-(4-(4-methylpiperazin-1-yl)phenyl)-1H-pyrrolo[2,3-b]pyridine-5-carboxamide 2,2,2-trifluoroacetate (O1)

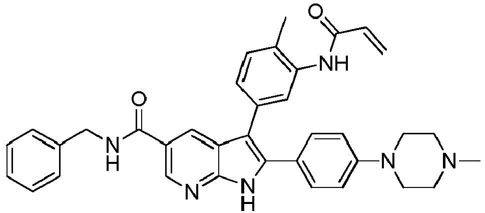

A solution of 3-(3-acrylamido-4-methylphenyl)-2-(4-(4-methylpiperazin-1-yl)phenyl)-1H-pyrrolo[2,3-b]pyridine-5-carboxylic acid **M3** (30mg, 0.06mmol, 1.0eq.), DIPEA (0.1mL, 0.60mmol, 10eq.) and HATU (46mg, 0.12mmol, 2.0eq.) in dry DMF (1mL) was stirred for 5min at 0°C. Then benzyl amine (13mg, 0.12mmol, 2.0eq.) was added and the mixture was stirred for 30min at 0°C. Sat. aq. NaHCO₃-solution was added and the mixture was extracted with EtOAc. The combined organic phase was dried over MgSO₄ and and solvents were removed in vacuo. The crude was purified by reverse phase RP-HPLC (column: C18), using H₂O (0.1%TFA) and ACN (0.1%TFA) as eluents. The desired fractions were lyophilized to yield the title compound **O1** (10mg, 0.01mmol, 21%) as a yellow powder. ¹H NMR (400MHz, d₄-MeOH, 300K) δ 2.30 (s, 3H), 2.97 (s, 3H), 3.07 (t, J = 12.4 Hz, 2H), 3.26 (m, 2H), 3.61 (d, J = 12.4 Hz, 2H), 3.93 (d, J = 13.7 Hz, 2H), 4.59 (s, 2H), 5.77 (dd, J = 10.2 Hz, J = 1.7 Hz, 1H), 6.32 (dd, J = 17.0 Hz, J = 1.7 Hz, 1H), 6.50 (dd, J = 10.2 Hz, J = 17.0 Hz, 1H), 7.01 (d, J = 8.9 Hz, 2H), 7.12 (d, J = 8.1 Hz, 1H), 7.20-7.37 (m, 6H), 7.50 (d, J = 8.9 Hz, 2H), 7.54 (s, 1H), 8.48 ( d, J = 2.1 Hz, 1H), 8.75 (d, J = 2.1 Hz, 1H). MS (ES) C₃₆H₃₆N₆O₂ requires: 584 found: 585 (M+H)⁺.

The Examples in the following table were prepared according to the procedure described for **O1** (Example 118).

| **Example** | **Name** | **Mwt** | **[M+H]⁺** |
|---|---|---|---|
| 119 | 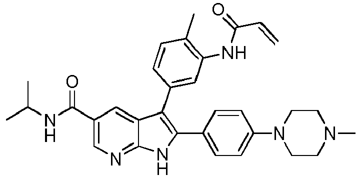 | 536 | 537 |
| 120 | 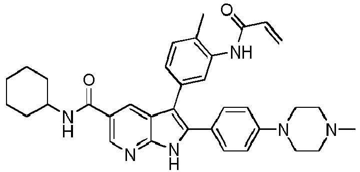 | 576 | 577 |
| 121 | 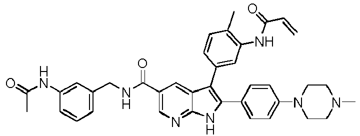 | 641 | 642 |
| 122 | 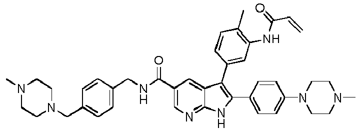 | 696 | 670 |
| 123 | 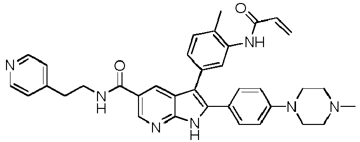 | 599 | 600 |
| 124 | 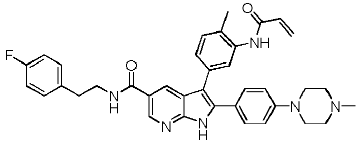 | 616 | 617 |
| 125 | 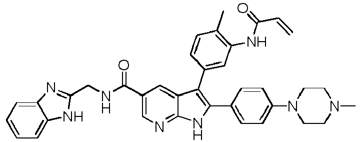 | 624 | 625 |
| 126 | 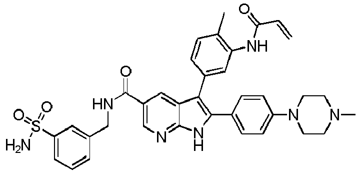 | 663 | 664 |
| 127 | 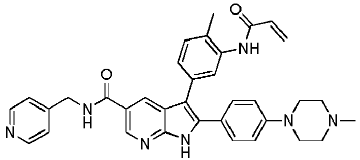 | 585 | 586 |
| 128 | 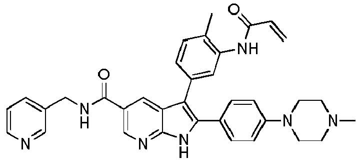 | 585 | 586 |
| 129 | 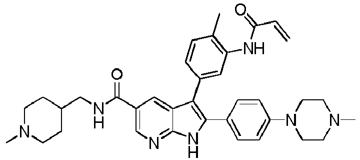 | 605 | 606 |
| 130 | 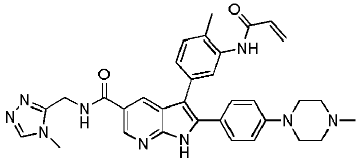 | 589 | 590 |
| 131 | 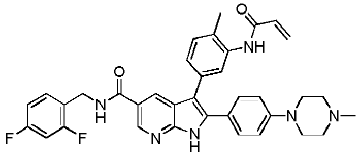 | 620 | 621 |
| 132 | 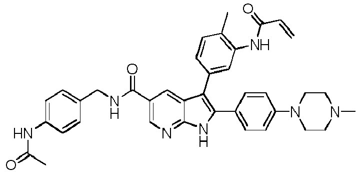 | 641 | 642 |
| 133 | 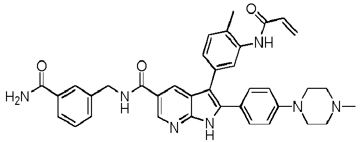 | 627 | 628 |

### Example 134:

### Methyl 3-(3-acrylamido-4-methylphenyl)-2-(4-(4-methylpiperazin-1-yl)phenyl)-1H-pyrrolo[2,3-b]pyridine-5-carboxylate 2,2,2-trifluoroacetate (P1)

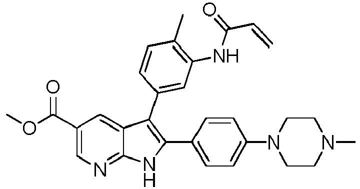

A solution of 3-(3-acrylamido-4-methylphenyl)-2-(4-(4-methylpiperazin-1-yl)phenyl)-1H-pyrrolo[2,3-b]pyridine-5-carboxylic acid **M3** (40mg, 0.08mmol, 1.0eq.), DMAP (5mg, 0.04mmol, 0.5eq.), *N*-(3-dimethylaminopropyl)-*N'-*ethylcarbodiimide hydrochloride (17mg, 0.09mmol, 1.1eq.) and dry methanol (7mg, 0.2mmol, 2.5eq.) in DCM (2mL) was stirred for 15h at RT. The crude solution was directly purified by reverse phase RP-HPLC (column: C18), using H₂O (0.1%TFA) and ACN (0.1%TFA) as eluents. The desired fractions were lyophilized to yield the title compound **P1** (20mg, 0.03mmol, 34%) as a yellow powder. ¹H NMR (400MHz, d₆-DMSO, 300K) δ 2.26 (s, 3H), 2.84 (s, 3H), 2.98 (t, J = 12.0 Hz, 2H), 3.11 (m, 2H), 3.50 (t, J = 12.0 Hz, 2H), 3.84 (s, 3H), 3.92 (d, J = 13.3 Hz, 2H), 5.72 (dd, J = 10.2 Hz, J = 2.0 Hz, 1H), 6.21 (dd, J = 17.0 Hz, J = 2.0 Hz, 1H), 6.54 (dd, J = 17.0 Hz, J = 10.2 Hz, 1H), 6.99 (d, J = 9.0 Hz, 2H), 7.02 (m, 1H), 7.26 (d, J = 7.9 Hz, 1H), 7.44 (d, J = 9.0 Hz, 2H), 7.58 (s, 1H), 8.26 (d, J = 2.1 Hz, 1H), 8.80 (d, J = 2.1 Hz, 1H), 9.52 (s, 1H), 9.72 (s, 1H), 12.45 (s, 1H). MS (ES) C₃₀H₃₁N₅O₃ requires: 509 found: 510 (M+H)⁺.

The Examples in the following table were prepared according to the procedure described for **P1** (Example 134).

| **Example** | **Name** | **Mwt** | **[M+H]⁺** |
|---|---|---|---|
| 135 | 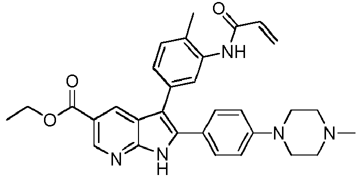 | 523 | 524 |
| 136 | 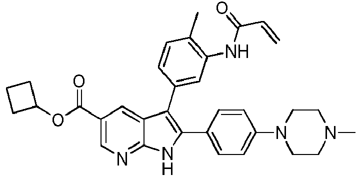 | 549 | 550 |
| 137 | 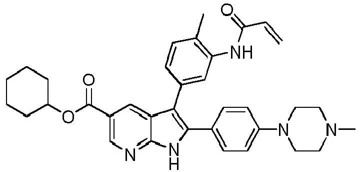 | 577 | 578 |
| 138 | 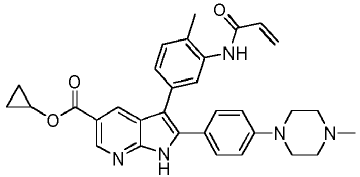 | 535 | 536 |
| 139 | 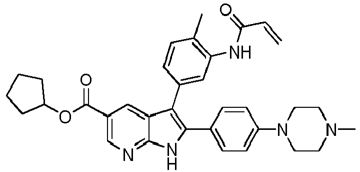 | 563 | 564 |
| 140 | 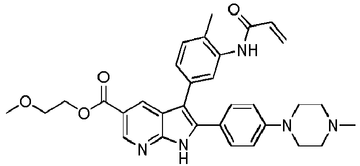 | 553 | 554 |
| 141 | 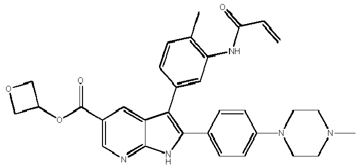 | 551 | 552 |

### Example 142:

### (E)-N-(5-(4-chloro-2-(4-(4-methylpiperazin-1-yl)phenyl)-1H-pyrrolo[2,3-b]pyridin-3-yl)-2-methylphenyl)but-2-enamide 2,2,2-trifluoroacetate (Q2)

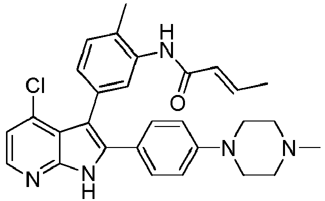

### Step 1: 5-(4-chloro-2-(4-(4-methylpiperazin-1-yl)phenyl)-1H-pyrrolo[2,3 b]pyridin-3-yl)-2-methylaniline (Q1)

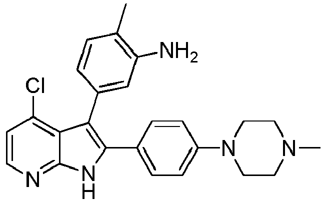

**Q1** was prepared from 4-chloro-3-(4-methyl-3-nitrophenyl)-2-(trimethylsilyl)-1H-pyrrolo[2,3-b]pyridine **A1** following the procedure reported in Preparative Example 97 Step 2 - 4. MS (ES) C₂₅H₂₆ClN₅ requires: 431 found: 432 (M+H)⁺.

### Step 2: (E)-N-(5-(4-chloro-2-(4-(4-methylpiperazin-1-yl)phenyl)-1H-pyrrolo[2,3-b]pyridin-3-yl)-2-methylphenyl)but-2-enamide 2,2,2-trifluoroacetate (Q2)

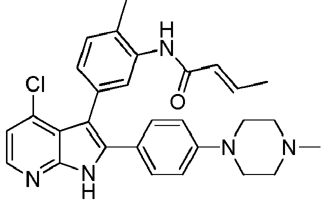

To a solution of **Q1** (20mg, 0.05mmol, 1.0eq.) and DIPEA (60mg, 0.46mmol, 10.0eq.) in dry THF (2mL) at 0°C was added slowly crotonoyl chloride (5mg, 0.05mmol, 1.1eq.) in dry THF (0.5mL). The mixture was stirred for 10min. The solution was diluted with EtOAc and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **Q2** (13mg, 0.02mmol, 36%) as a yellow powder. ¹H NMR (400MHz, d₆-DMSO, 300K) δ 1.82 (dd, J = 7.0 Hz, J = 1.7 Hz, 3H), 2.23 (s, 3H), 2.82 (s, 3H), 2.95 (t, J = 12.4 Hz, 2H), 3.07 (m, 2H), 3.43 (m, 2H), 3.90 (d, J = 13.3 Hz, 2H), 6.21 (d, J = 15.2 Hz, 1H), 6.70 (dq, J = 7.0 Hz, J = 15.2 Hz, 1H), 6.92 (d, J = 9.0 Hz, 2H), 7.00 (dd, J = 7.5 Hz, J = 1.8 Hz, 1H), 7.06 (d, J = 5.2 Hz, 1H), 7.18 (d, J = 7.9 Hz, 1H), 7.35 (d, J = 9.0 Hz, 2H), 7.52 (s, 1H), 8.12 (d, J = 5.2 Hz, 1H), 9.21 (s, 1H), 9.69 (s, 1H), 12.30 (s, 1H). MS (ES) C₂₉H₃₀ClN₅O requires: 500, found: 501 (M+H)⁺.

### Example 143:

### (E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide 2,2,2-trifluoroacetate (R1)

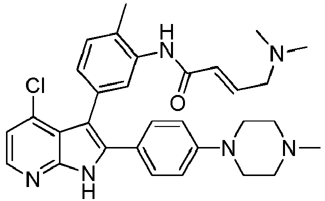

To a solution of trans-4-dimethylaminocrotonic acid hydrochlorid (46mg, 0.28mmol, 3eq.) and a drop of dry DMF in dry DCM (1mL) at 0°C was added slowly oxalyl chloride (20uL, 0.23mmol, 2.5eq.) in dry DCM (0.2mL). After 1h this mixture was added to a solution of **Q1** (40mg, 0.09mmol, 1.0eq.) in dry DCM (1mL) and dry NMP (1mL). The mixture was stirred for 10min. The solution was diluted with DCM and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **R1** (26mg, 0.03mmol, 33%) as a yellow powder. ¹H NMR (400MHz, d₆-DMSO, 300K) δ 2.28 (s, 3H), 2.80 (s, 6H), 2.84 (s, 3H), 2.97 (t, J = 12.6 Hz, 2H), 3.09 (m, 2H), 3.48 (m, 2H), 3.93 (m, 2H), 6.57(d, J = 15.4 Hz, 1H), 6.68 (dt, J = 7.0 Hz, J = 15.4 Hz, 1H), 6.94 (d, J = 9.0 Hz, 2H), 7.05 (d, J = 7.7 Hz, 1H), 7.08 (d, J = 5.2 Hz, 1H), 7.23 (d, J = 7.7 Hz, 1H), 7.36 (d, J = 9.0 Hz, 2H), 7.56 (s, 1H), 8.14 (d, J = 5.2 Hz, 1H), 9.65 (s, 1H), 9.74 (s, 1H), 12.34 (s, 1H). MS (ES) C₃₁H₃₅ClN₆O requires: 543, found: 544 (M+H)⁺.

### Example 144:

### N-(5-(4-chloro-2-(4-(4-methylpiperazin-1-yl)phenyl)-1H-pyrrolo[2,3-b]pyridin-3-yl)-2-methylphenyl)propiolamide 2,2,2-trifluoroacetate (S1)

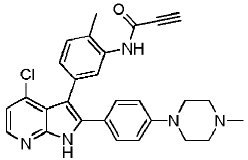

To a solution of **Q1** (70mg, 0.16mmol, 1.0eq.) and DIPEA (0.28mL, 1.62mmol, 10.0eq.) in dry THF (2mL) at 0°C was added slowly 3-(trimethylsilyl)propioloyl chloride (29mg, 0.18mmol, 1.1eq.) in dry THF (0.5mL). The mixture was stirred for 10min. Then K₂CO₃ (223mg, 1.62mmol, 10eq.) was added and the solution was stirred for 30min at at 0°C. The mixture was directly purified by reverse phase RP-HPLC (column: C18), using H₂O (0.1 %TFA) and ACN (0.1 %TFA) as eluents. The desired fractions were lyophilized to yield the title compound **S1** (30mg, 0.04mmol, 26%) as a yellow powder. ¹H NMR (400MHz, d₆-DMSO, 300K) δ 2.25 (s, 3H), 2.85 (s, 3H), 2.98 (t, J = 12.4 Hz, 2H), 3.10 (m, 2H), 3.48 (d, J = 12.0 Hz, 2H), 3.92 (d, J = 13.3 Hz, 2H), 4.32 (s, 1H), 6.93 (d, J = 8.8 Hz, 2H), 7.08 (m, 2H), 7.23 (d, J = 7.8 Hz, 1H), 7.31 (s, 1H), 7.35 (d, J = 8.8 Hz, 2H), 8.14 (d, J = 5.2 Hz, 1H), 9.78 (s, 1H), 10.23 (s, 1H), 12.35 (s, 1H). MS (ES) C₂₈H₂₆ClN₅O requires: 483, found: 484 (M+H)⁺.

### Example 145:

### N-(5-(4-chloro-2-(4-(4-methylpiperazin-1-yl)phenyl)-1H-pyrrolo[2,3-b]pyridin-3-yl)-2-methylphenyl)ethenesulfonamide 2,2,2-trifluoroacetate (T1)

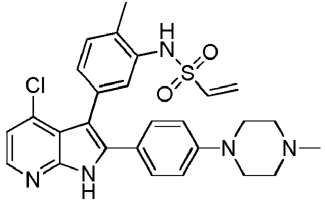

To a solution of **Q1** (25mg, 0.06mmol, 1.0eq.) and triethylamine (0.10mL, 0.58mmol, 10.0eq.) in dry THF (2mL) at -10°C was added slowly 2-chloroethanesulfonyl chloride (10mg, 0.06mmol, 1.1eq.) in dry THF (0.5mL). After 2h stirring at -10°C, the mixture was directly purified by reverse phase RP-HPLC (column: C18), using H₂O (0.1%TFA) and ACN (0.1%TFA) as eluents. The desired fractions were lyophilized to yield the title compound **T1** (18mg, 0.02mmol, 40%) as a yellow powder. ¹H NMR (400MHz, d₆-DMSO, 300K) δ 2.32 (s, 3H), 2.82 (s, 3H), 2.95 (m, 2H), 3.08 (m, 2H), 3.42 (m, 2H), 3.90 (m, 2H), 5.79 (d, J = 9.8 Hz, 1H), 5.80 (d, J = 16.4 Hz, 1H), 6.64 (dd, J = 9.8 Hz, J = 16.4 Hz, 1H), 6.91 (d, J = 9.0 Hz, 2H), 7.07 (d, J = 5.2 Hz, 1H), 7.08 (d, J = 1.8 Hz, 1H), 7.13 (d, J = 1.8 Hz, 1H), 7.21 (d, J = 7.7 Hz, 1H), 7.27 (d, J = 9.0 Hz, 2H), 8.12 (d, J = 5.2 Hz, 1H), 9.22 (s,1H), 9.85 (s, 1H), 12.33 (s, 1H). MS (ES) C₂₇H₂₈ClN₅O₂S requires: 522, found: 523 (M+H)⁺.

### Example146:

### 2-chloro-N-(5-(4-chloro-2-(4-(4-methylpiperazin-1-yl)phenyl)-1H-pyrrolo[2,3-b]pyridin-3-yl)-2-methylphenyl)acetamide 2,2,2-trifluoroacetate (U1)

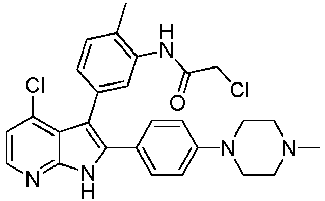

To a solution of **Q1** (30mg, 0.07mmol, 1.0eq.) and DIPEA (0.12mL, 0.69mmol, 10.0eq.) in dry THF (2mL) at 0°C was added slowly chloroacetyl chloride (39mg, 0.35mmol, 5.0eq.) in dry THF (0.5mL). The mixture was stirred for 10min. Then sat. aq. NaHCO₃-solution was added and the aq. phase was extracted with DCM. The combined organic phase was dried over MgSO₄ and solvents were removed in vacuo. The crude product was purified by reverse phase RP-HPLC (column: C18), using H₂O (0.1 %TFA) and ACN (0.1 %TFA) as eluents. The desired fractions were lyophilized to yield the title compound **U1** (13mg, 0.02mmol, 25%) as a yellow powder. ¹H NMR (400MHz, d₆-DMSO, 300K) δ 2.25 (s, 3H), 2.81 (s, 3H), 2.96 (t, J = 12.6 Hz, 2H), 3.08 (m, 2H), 3.46 (d, J = 12.0 Hz, 2H), 3.90 (d, J = 13.2 Hz, 2H), 4.26 (s, 2H), 6.91 (d, J = 8.9 Hz, 2H), 7.04 (d, J = 7.6 Hz, 1H), 7.07 (d, J = 5.2 Hz, 1H), 7.20 (d, J = 7.6 Hz, 1H), 7.34 (d, J = 8.9 Hz, 2H), 7.43 (s, 1H), 8.12 (d, J = 5.2 Hz, 1H), 9.62 (s, 1H), 9.73 (s, 1H), 12.32 (s, 1H). MS (ES) C₂₇H₂₇Cl₂N₅O requires: 507, found: 508 (M+H)⁺.

### Example 147:

### N-(5-(4-ethoxy-2-(4-(4-methylpiperazin-1-yl)phenyl)-1H-pyrrolo[2,3-b]pyridin-3-yl)-2-methylphenyl)acrylamide 2,2,2-trifluoroacetate (V1)

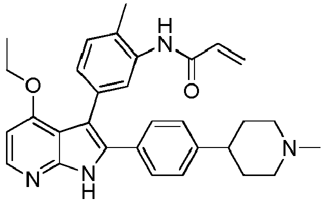

**V1** was prepared from 3-iodo-4-ethoxypyridin-2-amine, trimethyl((4-methyl-3-nitrophenyl)ethynyl)silane and 4-(1-boc-4-piperidyl)phenylboronic acid pinacol ester following the general procedure reported in Preparative Example 72 Step 1-5. MS (ES) C₃₁H₃₄N₄O₂ requires: 494, found: 495 (M+H)⁺.

### Example 148:

### (E)-isopropyl 3-(3-(4-(dimethylamino)but-2-enamido)-4-methylphenyl)-2-(4-(4-methylpiperazin-1-yl)phenyl)-1H-pyrrolo[2,3-b]pyridine-5-carboxylate 2,2,2-trifluoroacetate (W1)

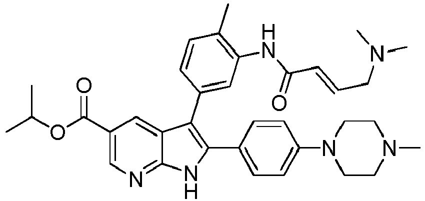

To a solution of trans-4-dimethylaminocrotonic acid hydrochlorid (30mg, 0.18mmol, 3.5eq.) and a drop of dry DMF in dry THF (1mL) at 0°C was added slowly oxalyl chloride (14uL, 0.16mmol, 3.0eq.) in dry THF (0.2mL). After 90min the mixture was added to a solution of **N1** (25mg, 0.05mmol, 1.0eq.) in dry NMP (1mL). The mixture was stirred for 10min. The solution was diluted with DCM and washed twice with aq. sat. NaHCO₃-solution. The organic phase was dried over MgSO₄ and solvent was removed in vacuo. The crude product was purified by reverse phase RP-HPLC (column: C18), using H₂O (0.1%TFA) and ACN (0.1%TFA) as eluents. The desired fractions were lyophilized to yield the title compound **W1** (13mg, 0.01 mmol, 28%) as a yellow powder. ¹H NMR (400MHz, d₆-DMSO, 300K) δ 1.34 (d, J = 6.3 Hz, 6H), 2.29 (s, 3H), 2.81 (s, 6H), 2.86 (s, 3H), 3.04 (m, 2H), 3.13 (m, 2H), 3.43 (m, 2H), 3.90 (m, 2H), 3.94 (d, J = 7.0 Hz, 2H), 5.16 (sept., J = 6.3 Hz, 1H), 6.62 (d, J = 15.4 Hz, 1H), 6.73 (dt, J = 15.4 Hz, J = 7.0 Hz, 1H), 7.02 (m, 3H), 7.27 (d, J = 7.9 Hz, 1H), 7.47 (d, J = 8.9 Hz, 2H), 7.67 (s, 1H), 8.33 (d, J = 2.0 Hz, 1H), 8.82 (d, J = 2.0 Hz, 1H), 9.75 (s, 1H), 10.02 (s, 1H), 12.47 (s, 1H). MS (ES) C₃₅H₄₂N₆O₃ requires: 594, found: 595 (M+H)⁺.

### Biological Assays

The exemplified compounds described herein were tested for activity and were found to have an IC₅₀ value less than 10uM, particularly less than 500nM, in one of the following assays:

### 1. Measurement of HER2 INS YVMA kinase activity

This protocol describes how the Lance Kinase Activity Assay was performed to determine IC₅₀ values of compounds of general formula (I) against HER2 INS YVMA. The principle behind this enzymatic assay is based upon the phosphorylation of the Ulight-peptide substrate. It is detected by using a specific EU-labeled anti-phospho peptide antibody. The binding of the Eu labeled anti-phospho peptide antibody to the phosphorylated ULight labeled peptide gives rise to a FRET-signal.

Binding of an inhibitor to the kinase prevents phosphorylation of the Ulight-substrate, resulting in a loss of FRET. In table 2 is summarized the relevant information for the LANCE assay.

**Table 2: Reagents, stock concentrations and final assay concentrations for Her2 INS YVMA.**

| Reagents | Stock concentration | Working concentration | Final assay concentration | Supplier |
|---|---|---|---|---|
| ULight™-Poly GT substrate | 10 µM | 250 nM | 100 nM | PerkinElmer |
| Eu-Anti-PT66 Antibody (AB) | 3125 nM | 4nM | 2 nM | PerkinElmer |
| Her2 ins YVMA | 41,83 µM | 2,5 nM | 1 nM | DPF |
| ATP | 100 mM | 8 µM | 1,6 µM | Sigma |

The compounds of general formula (I) summarized in Table 3 were serial diluted from a 10 mM DMSO stock solution 1:3 over 8 steps in a total volume of 20 µl.
For every sample, 8 µl of kinase-substrate mix was transferred into a suitable assay plate (e.g. Corning #3673). Compound was added via pintool transfer (10nl/well) using a Biomek FX robot (BeckmanCoulter). Reaction was started by addition of 2µl ATP working solution and mixed using variomag teleshaker (Thermo Fischer Scientific). After 1 h incubation at room temperature the reaction was stopped with 10µl detection mix containing the Eu-labeled phosphospecific antibody and 10mM EDTA. After a second incubation period of 1 h at room temperature the FRET signal was measured at 340 nm excitation, 665 nm and 615 nm emission (for the ULight-substrate and Eu-AB, respectively) with an Envision spectrophotometer (Perkin Elmer, Waltham, MA, USA) with 50 µs delay and 300 µs integration time. IC₅₀ values were determined from the sigmoidal dose response curves with the software Quattro Workflow (Quattro GmbH, Munich, Germany).

### 2. Measurement of cellular activity

The CellTiter-Glo Luminescent Cell Viability Assay (Promega) is a homogeneous method of determining the number of viable cells in culture. It is based on quantification of ATP, indicating the presence of metabolically active cells. Cells are seeded on day 1 at cell numbers that assure assay linearity and optimal signal intensity. After incubation for 24h in humidified chambers at 37°C and 5% CO₂, compounds in DMSO are added at different concentrations. Cells are further incubated for 72 h at 37°C and 5% CO₂. Cells treated with the compound vehicle DMSO are used as positive controls and cells treated with 10 µM Staurosporine serve as negative controls. At day 5 the CellTiter Glo Reagent is prepared according to the instructions of the kit (Promega Inc.): Reagent is mixed 1:1 with cell culture medium. Thereon, mixture and assay plates are equilibrated at room temperature for 20 min. Equal volumes of the reagent-medium-mixture is added to the volume of culture medium present in each well. The plates are mixed at ∼200 rpm for 2 minutes on an orbital shaker. The microplates are then incubated at room temperature for 10 minutes for stabilization of the luminescent signal. Following incubation the luminescence is recorded on a Victor microplate reader (Perkin Elmer) using a 200 ms integration time. The data is then analyzed with Excel using the XLFIT Plugin (dose response Fit 205) for IC₅₀-determination. As quality control the Z'-factor is calculated from 16 positive and negative control values. Only assay results showing a Z'-factor ≥ 0.5 are used for further analysis.

Table 3 shows activity data in the biochemical Her2 Exon 20 INSYVMA Lance assay and cellular Her2 Exon 20 INSYVMA Ba/F3 CellTiter-Glo and EGFR Exon 20 INSNPH Ba/F3 CellTiter-Glo assays. Inhibition is indicated as IC₅₀ [nM] ("-" = not measured). Compounds having an activity designated as "A" provided an IC₅₀ ≤ 50nM; compounds having an activity designated as "B" provided an 50nM < IC₅₀ ≤ 100nM; compounds having an activity designated as "C" provided an 100nM < IC₅₀ ≤ 500nM; compounds having an activity designated as "D" provided an 500nM < IC₅₀ ≤ 1000nM; compounds having an activity designated as "E" provided an 1000nM < IC₅₀ ≤ 10000nM; and compounds having an activity designated as "F" provided an IC₅₀ > 10000nM.

**Table 3:**

| **Example** | **Her2 Exon 20 INSYVMA Lance IC₅₀ [nM]** | **Her2 Exon 20 INSYVMA Ba/F3 CTG IC₅₀ [nM]** | **EGFR Exon 20 INSNPH Ba/F3 CTG IC₅₀ [nM]** |
|---|---|---|---|
| **1** | A | C | B |
| **2** | A | B | A |
| **3** | A | B | A |
| **4** | A | C | C |
| **5** | B | C | C |
| **6** | D | D | D |
| **7** | D | D | E |
| **8** | B | C | C |
| **9** | A | C | C |
| **10** | A | B | B |
| **11** | A | C | C |
| **12** | C | C | C |
| **13** | A | C | C |
| **14** | C | D | D |
| **15** | C | C | C |
| **16** | A | C | C |
| **17** | A | C | C |
| **18** | A | C | B |
| **19** | C | D | D |
| **20** | A | D | D |
| **21** | C | D | D |
| **22** | C | D | E |
| **23** | A | C | C |
| **24** | C | D | D |
| **25** | A | C | C |
| **26** | A | mC | C |
| **27** | D | E | E |
| **28** | A | C | C |
| **29** | B | D | D |
| **30** | C | E | D |
| **31** | C | E | E |
| **32** | C | D | C |
| **33** | E | E | E |
| **34** | E | E | E |
| **35** | C | D | D |
| **36** | D | E | E |
| **37** | A | C | C |
| **38** | A | B | B |
| **39** | C | D | E |
| **40** | A | B | B |
| **41** | A | B | A |
| **42** | A | C | C |
| **43** | A | B | B |
| **44** | C | E | E |
| **45** | F | E | E |
| **46** | E | E | E |
| **47** | A | E | E |
| **48** | B | C | C |
| **49** | D | D | E |
| **50** | F | E | E |
| **51** | C | D | D |
| **52** | C | E | E |
| **53** | B | E | E |
| **54** | C | E | E |
| **55** | A | D | D |
| **56** | A | C | C |
| **57** | A | C | D |
| **58** | B | E | E |
| **59** | A | C | C |
| **60** | A | C | C |
| **61** | A | C | C |
| **62** | A | C | C |
| **63** | A | C | C |
| **64** | A | C | C |
| **65** | A | C | D |
| **66** | A | C | D |
| **67** | A | C | C |
| **68** | A | D | E |
| **69** | A | C | C |
| **70** | A | C | D |
| **71** | A | C | D |
| **72** | A | B | B |
| **73** | A | B | B |
| **74** | B | D | C |
| **75** | A | C | B |
| **76** | A | C | C |
| **77** | A | C | C |
| **78** | C | C | D |
| **79** | A | C | C |
| **80** | D | E | E |
| **81** | E | E | E |
| **82** | E | E | E |
| **83** | A | C | C |
| **84** | A | C | C |
| **85** | A | C | C |
| **86** | B | D | D |
| **87** | A | D | D |
| **88** | A | E | E |
| **89** | A | C | C |
| **90** | A | D | E |
| **91** | B | D | D |
| **92** | A | D | E |
| **93** | C | D | E |
| **94** | B | E | E |
| **95** | B | E | E |
| **96** | A | C | C |
| **97** | A | C | B |
| **98** | A | C | C |
| **99** | A | C | C |
| **100** | A | C | D |
| **101** | A | C | C |
| **102** | A | C | C |
| **103** | A | C | C |
| **104** | A | C | C |
| **105** | A | C | C |
| **106** | A | C | C |
| **107** | A | C | C |
| **108** | A | C | C |
| **109** | A | C | C |
| **110** | A | C | C |
| **111** | A | A | A |
| **112** | A | D | D |
| **113** | A | B | B |
| **114** | - | - | - |
| **115** | A | C | C |
| **116** | A | - | - |
| **117** | A | A | A |
| **118** | A | C | C |
| **119** | A | C | C |
| **120** | A | C | C |
| **121** | B | F | F |
| **122** | A | E | E |
| **123** | C | E | E |
| **124** | B | C | D |
| **125** | D | E | E |
| **126** | B | F | F |
| **127** | A | - | - |
| **128** | B | - | - |
| **129** | D | - | - |
| **130** | C | - | - |
| **131** | C | - | - |
| **132** | D | - | - |
| **133** | C | - | - |
| **134** | A | - | - |
| **135** | A | - | - |
| **136** | B | - | - |
| **137** | C | - | - |
| **138** | A | - | - |
| **139** | C | - | - |
| **140** | A | - | - |
| **141** | - | - | - |
| **142** | D | - | - |
| **143** | A | - | - |
| **144** | - | - | - |
| **145** | B | - | - |
| **146** | A | - | - |
| **147** | A | D | D |
| **148** | - | - | - |

Table 4 shows activity data in the biochemical EGFR T790ML858R Lance and EGFR wt Lance assays and the cellular H1975 CellTiter-Glo, H1781 CellTiter-Glo and A431 CellTiter-Glo assays. Inhibition is indicated as IC₅₀ [nM] ("-" = not measured). Compounds having an activity designated as "A" provided an IC₅₀ ≤ 50nM; compounds having an activity designated as "B" provided an 50nM < IC₅₀ ≤ 100nM; compounds having an activity designated as "C" provided an 100nM < IC₅₀ ≤ 500nM; compounds having an activity designated as "D" provided an 500nM < IC₅₀ ≤ 1000nM; compounds having an activity designated as "E" provided an 1000nM < IC₅₀ ≤ 10000nM; and compounds having an activity designated as "F" provided an IC₅₀ > 10000nM.

**Table 4:**

| **Example** | **EGFR T790M L858R Lance IC₅₀ [nM]** | **EGFR wt Lance IC₅₀ [nM]** | **H1975 CTG IC₅₀ [nM]** | **H1781 CTG IC₅₀ [nM]** | **A431 CTG IC₅₀ [nM]** |
|---|---|---|---|---|---|
| **1** | A | A | A | C | C |
| **2** | A | A | A | C | C |
| **3** | A | A | A | C | C |
| **4** | A | A | A | C | C |
| **5** | A | A | B | F | F |
| **6** | A | B | C | - | - |
| **7** | A | C | C | - | - |
| **8** | A | A | A | C | C |
| **9** | A | A | A | - | - |
| **10** | A | A | B | C | B |
| **11** | A | A | A | D | C |
| **12** | A | A | B | - | - |
| **13** | A | A | A | C | C |
| **14** | A | A | C | - | - |
| **15** | A | A | A | D | C |
| **16** | A | A | B | D | D |
| **17** | A | A | A | C | C |
| **18** | A | A | A | C | C |
| **19** | A | A | C | - | E |
| **20** | A | A | A | - | - |
| **21** | A | A | A | - | - |
| **22** | A | C | C | - | - |
| **23** | A | A | A | C | C |
| **24** | B | C | C | - | - |
| **25** | A | A | A | D | C |
| **26** | A | A | A | D | C |
| **27** | B | C | C | - | - |
| **28** | A | A | A | C | C |
| **29** | A | A | A | - | - |
| **30** | A | A | C | - | - |
| **31** | A | D | C | - | - |
| **32** | A | A | B | - | - |
| **33** | C | D | C | - | - |
| **34** | C | E | D | - | - |
| **35** | A | A | B | - | - |
| **36** | B | B | C | - | E |
| **37** | A | A | A | - | C |
| **38** | A | A | A | - | C |
| **39** | B | A | C | - | E |
| **40** | A | A | A | - | C |
| **41** | A | A | A | - | C |
| **42** | A | A | A | - | C |
| **43** | A | A | A | - | C |
| **44** | A | C | C | - | - |
| **45** | C | E | E | - | - |
| **46** | B | D | E | - | - |
| **47** | A | A | A | - | D |
| **48** | A | A | A | C | C |
| **49** | A | C | C | - | - |
| **50** | C | E | D | - | - |
| **51** | A | A | B | - | - |
| **52** | A | C | C | - | - |
| **53** | A | A | C | - | - |
| **54** | A | A | C | - | E |
| **55** | A | A | B | E | E |
| **56** | A | A | B | D | D |
| **57** | A | A | C | E | E |
| **58** | A | A | C | E | E |
| **59** | A | A | B | C | C |
| **60** | A | A | C | D | D |
| **61** | A | A | B | D | C |
| **62** | A | A | B | - | D |
| **63** | A | A | B | - | D |
| **64** | A | A | B | - | D |
| **65** | A | A | B | - | D |
| **66** | A | A | C | - | E |
| **67** | A | A | C | - | E |
| **68** | A | A | C | - | E |
| **69** | A | A | B | - | D |
| **70** | A | A | C | - | D |
| **71** | A | A | C | - | D |
| **72** | A | A | A | C | C |
| **73** | A | A | A | C | C |
| **74** | A | A | A | - | D |
| **75** | A | A | A | C | C |
| **76** | A | A | A | D | D |
| **77** | A | A | A | C | C |
| **78** | A | A | A | D | C |
| **79** | A | A | A | D | C |
| **80** | A | C | C | - | E |
| **81** | B | C | E | - | E |
| **82** | B | C | D | - | E |
| **83** | A | A | C | E | E |
| **84** | A | A | C | - | D |
| **85** | A | A | C | - | D |
| **86** | A | A | C | E | E |
| **87** | A | A | C | - | E |
| **88** | A | A | C | - | E |
| **89** | A | A | C | - | D |
| **90** | A | A | C | - | E |
| **91** | A | A | C | - | E |
| **92** | A | A | D | - | E |
| **93** | A | A | D | - | E |
| **94** | A | A | C | - | E |
| **95** | A | B | D | - | E |
| **96** | A | A | A | C | C |
| **97** | A | A | A | - | C |
| **98** | A | A | C | - | D |
| **99** | A | A | C | - | E |
| **100** | A | A | C | - | E |
| **101** | A | A | C | - | E |
| **102** | A | A | C | - | E |
| **103** | A | A | C | - | D |
| **104** | A | A | A | - | D |
| **105** | A | A | A | - | C |
| **106** | A | A | A | - | D |
| **107** | A | A | A | - | D |
| **108** | A | A | A | - | D |
| **109** | A | A | A | - | D |
| **110** | A | A | B | - | D |
| **111** | A | A | A | - | C |
| **112** | A | A | C | - | E |
| **113** | A | A | A | - | C |
| **114** | - | - | - | - | - |
| **115** | A | A | A | - | D |
| **116** | A | A | - | - | - |
| **117** | A | A | A | - | C |
| **118** | B | A | D | - | D |
| **119** | A | A | D | - | D |
| **120** | A | A | C | - | C |
| **121** | C | A | F | - | F |
| **122** | A | A | E | - | E |
| **123** | B | A | F | - | E |
| **124** | B | A | D | - | D |
| **125** | C | A | C | - | E |
| **126** | A | A | F | - | F |
| **127** | A | A | - | - | - |
| **128** | B | A | - | - | - |
| **129** | C | C | - | - | - |
| **130** | C | B | - | - | - |
| **131** | D | A | - | - | - |
| **132** | C | B | - | - | - |
| **133** | C | B | - | - | - |
| **134** | A | A | - | - | - |
| **135** | A | A | - | - | - |
| **136** | A | A | - | - | - |
| **137** | A | A | - | - | - |
| **138** | A | A | - | - | - |
| **139** | A | A | - | - | - |
| **140** | A | A | - | - | - |
| **141** | - | - | - | - | - |
| **142** | A | B | - | - | - |
| **143** | A | A | - | - | - |
| **144** | - | - | - | - | - |
| **145** | A | A | - | - | - |
| **146** | A | A | - | - | - |
| **147** | A | A | A | - | - |
| **148** | - | - | - | - | - |

### 3. Metabolic stability

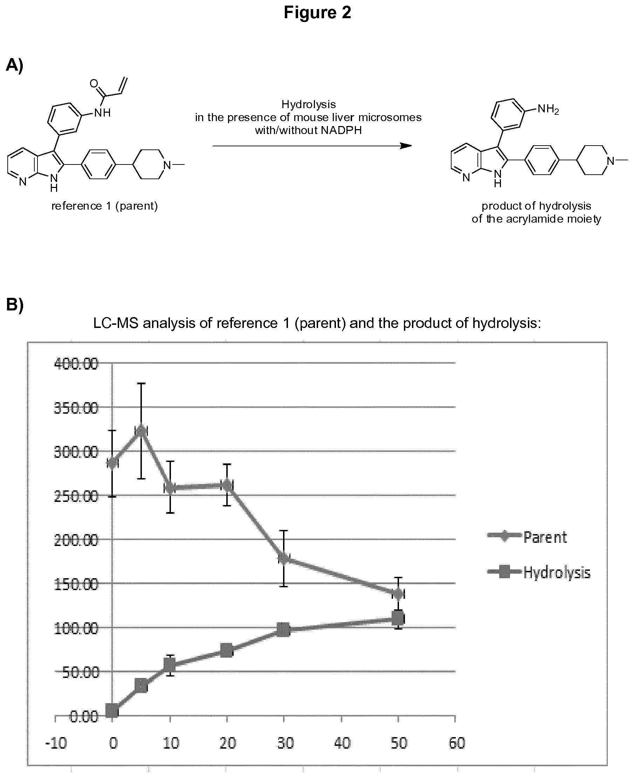

There are two major groups of enzyme reactions catalyzed by drug metabolizing enzymes, the so called Phase I and Phase II reactions. The basic processes in phase I reactions are oxidation, reduction and/or hydrolysis mostly catalyzed by the cytochrome P450 (CYP) family of enzymes. Exploring the metabolic stability of reference 1 we have observed as a major metabolite the hydrolysis of the acrylamide moiety in the presence of mouse liver microsomes with and without the cofactor NADPH. **Figure 2** shows the hydrolysis of the acrylamide moiety of reference 1 in the presence of mouse liver microsomes after 50 minutes.

The following procedures describe the determination of the in vitro Phase I metabolic stability of test compounds using liver microsomes by measuring compound depletion over time. Test compound at the concentration of 3 µM was incubated with the liver microsomes from the mouse species and the experiment was performed without NADPH. The depletion of the test compounds was measured over time up to 50 minutes at 37 °C by LC-MS/MS. Different compounds were tested under these conditions for microsomal stability (see Table 5). Prevention of hydrolysis of the acrylamide moiety was achieved either by the introduction of substituents like a methyl group (as in Example 1) next to the acrylamide moiety or by modifying the secondary acrylamide of reference 1 to a tertiary acrylamide (like Example 55).

**Table 5: Stability with and without cofactor NADPH in mouse liver microsomes**

| **Example** | **Structure** | **Stability in presence of mouse liver microsomes without cofactor NADPH [% remaining after 50min]** |
|---|---|---|
| **Reference 1** | 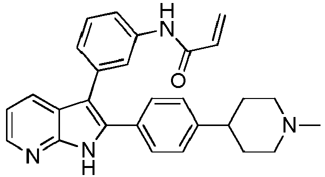 | 33 |
| **Reference 2** | 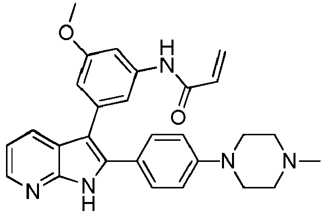 | 48 |
| **1** | 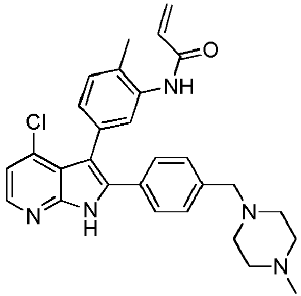 | >99 |
| **2** | 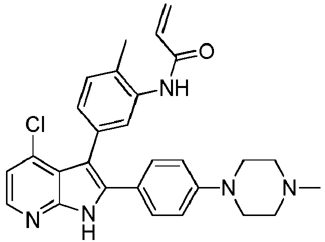 | >99 |
| **3** | 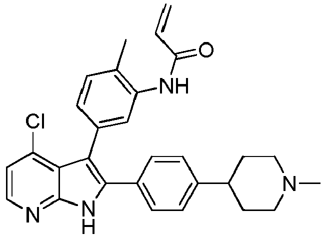 | >99 |
| **4** | 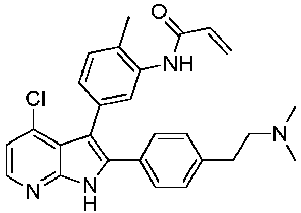 | >99 |
| **5** | 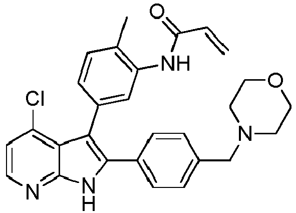 | >99 |
| **6** | 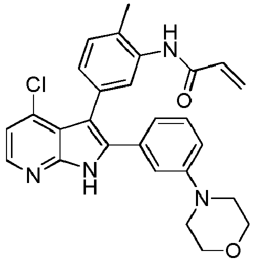 | >99 |
| **7** | 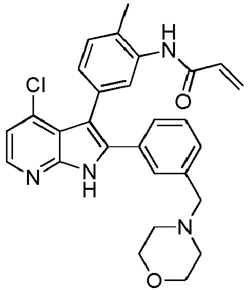 | >99 |
| **8** | 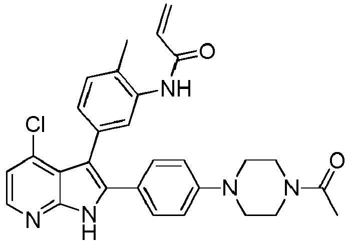 | >99 |
| **9** | 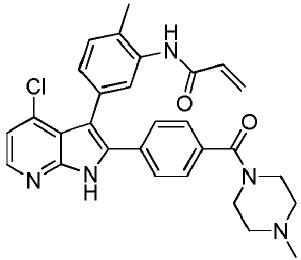 | >99 |
| **10** | 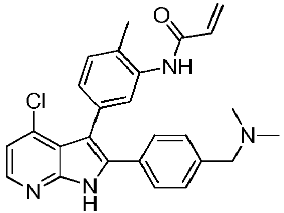 | >99 |
| **11** | 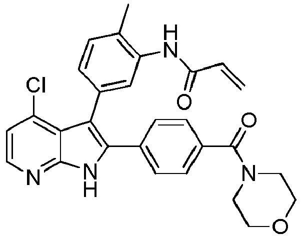 | >99 |
| **12** | 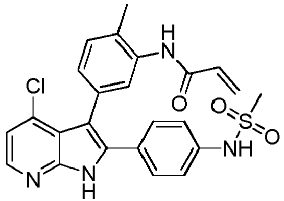 | >99 |
| **13** | 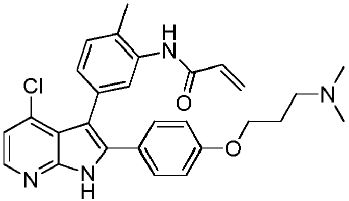 | 95 |
| **14** | 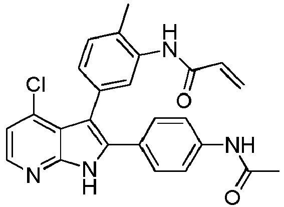 | 68 |
| **15** | 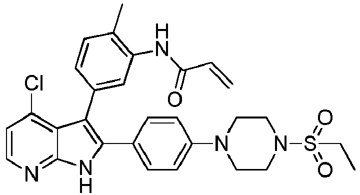 | >99 |
| **16** | 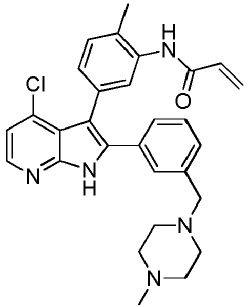 | >99 |
| **17** | 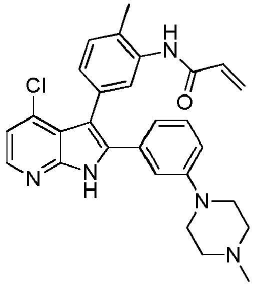 | 90 |
| **18** | 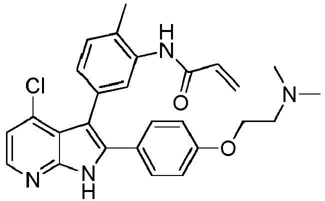 | 90 |
| **19** | 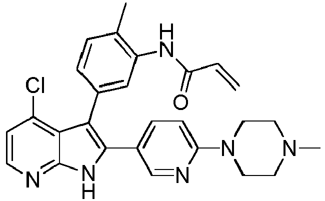 | >99 |
| **20** | 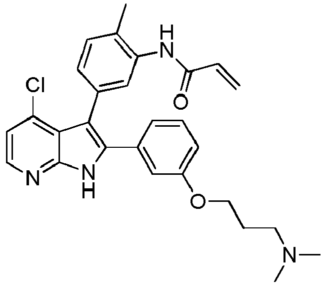 | >99 |
| **21** | 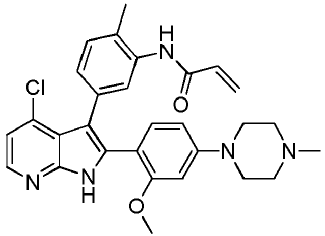 | 93 |
| **22** | 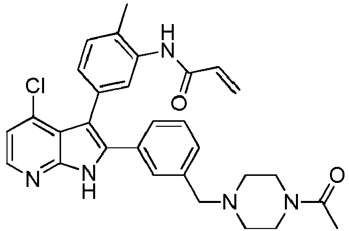 | >99 |
| **23** | 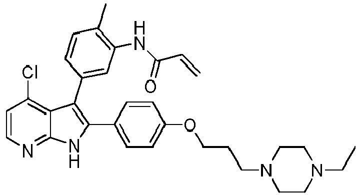 | >99 |
| **24** | 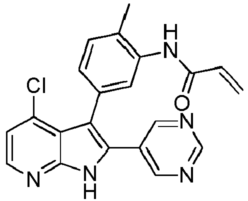 | >99 |
| **25** | 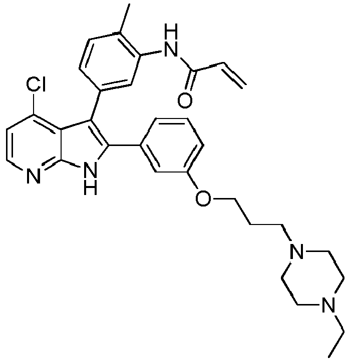 | >99 |
| **26** | 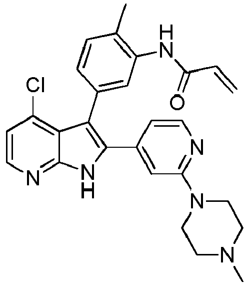 | >99 |
| **27** | 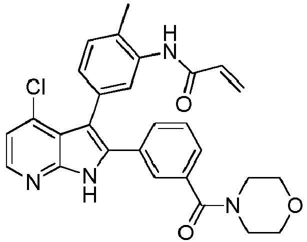 | >99 |
| **28** | 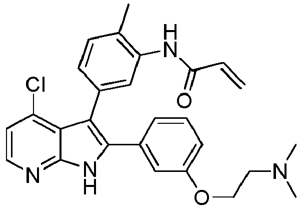 | >99 |
| **29** | 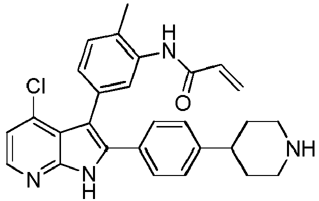 | >99 |
| **30** | 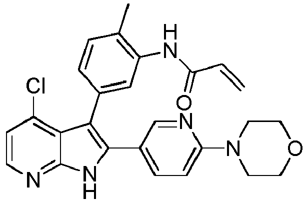 | >99 |
| **31** | 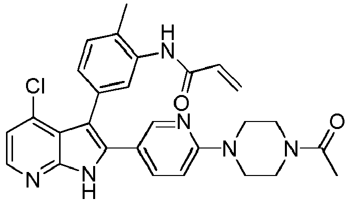 | >99 |
| **32** | 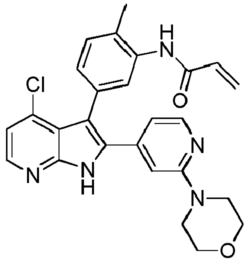 | >99 |
| **36** | 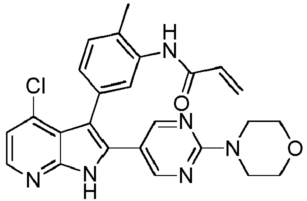 | >99 |
| **37** | 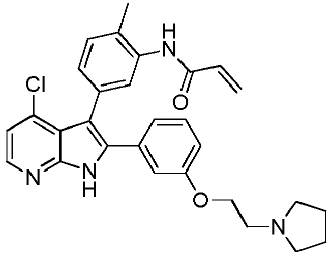 | >99 |
| **38** | 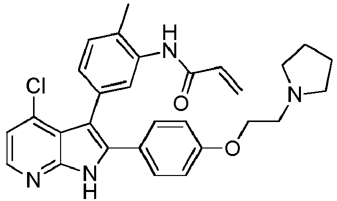 | >99 |
| **39** | 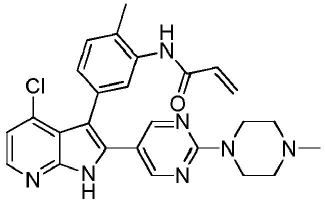 | >99 |
| **40** | 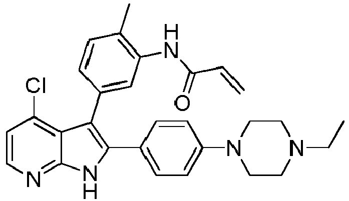 | >99 |
| **41** | 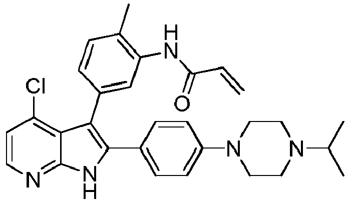 | >99 |
| **42** | 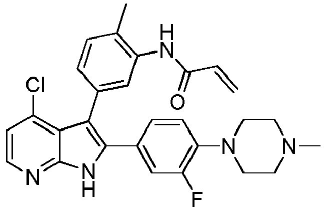 | >99 |
| **43** | 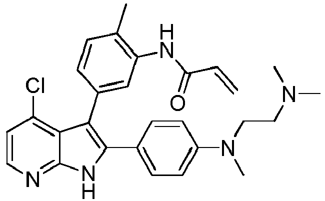 | >99 |
| **55** | 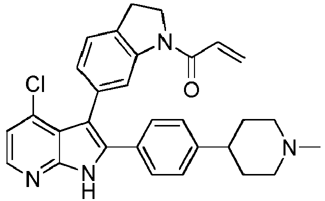 | >99 |
| **56** | 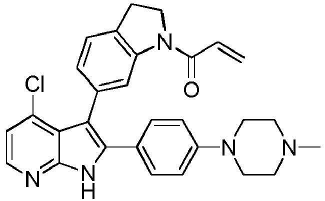 | >99 |
| **57** | 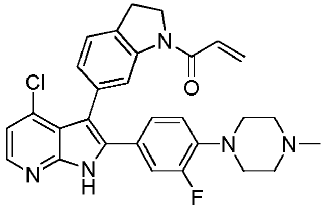 | >99 |
| **58** | 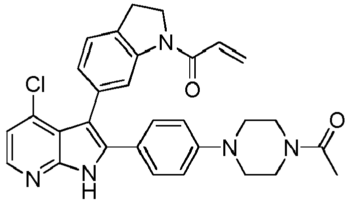 | >99 |
| **59** | 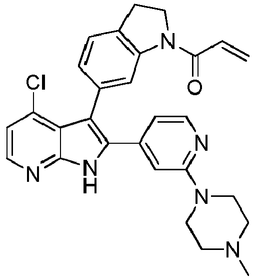 | >99 |
| **60** | 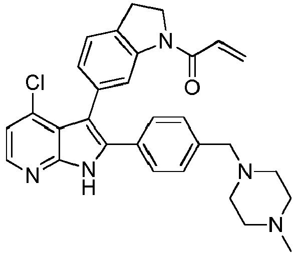 | >99 |
| **61** | 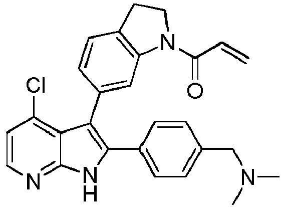 | >99 |
| **62** | 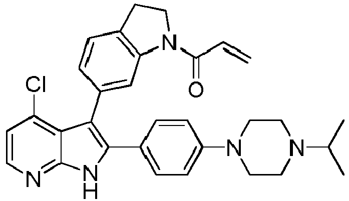 | >99 |
| **63** | 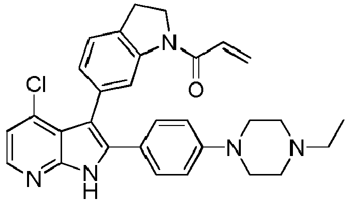 | >99 |
| **64** | 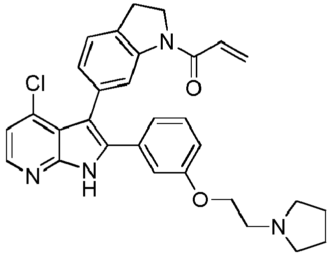 | >99 |
| **65** | 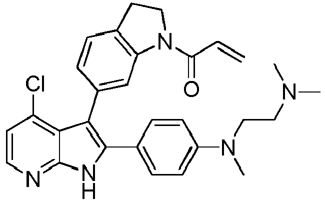 | >99 |
| **66** | 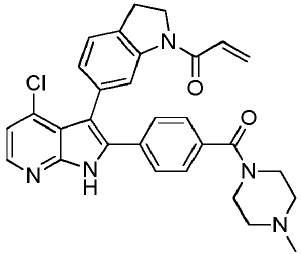 | >99 |
| **67** | 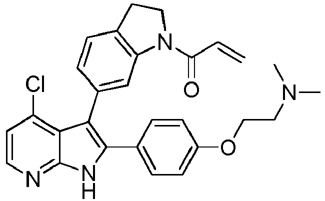 | >99 |
| **68** | 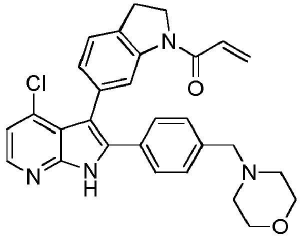 | >99 |
| **69** | 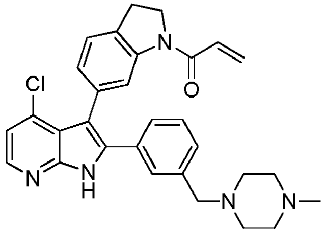 | 98 |
| **70** | 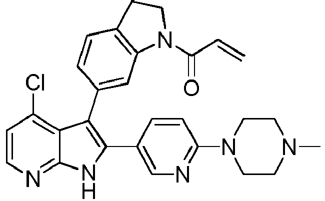 | >99 |
| **71** | 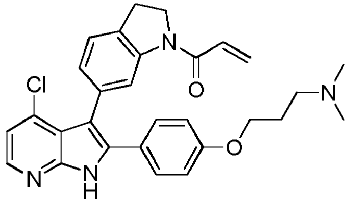 | >99 |
| **72** | 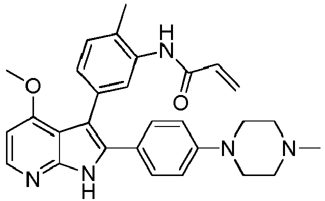 | >99 |
| **73** | 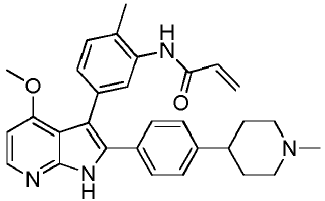 | 99 |
| **74** | 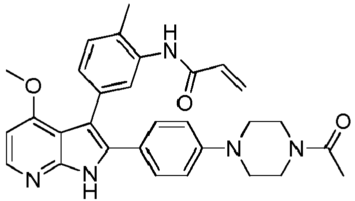 | 90 |
| **75** | 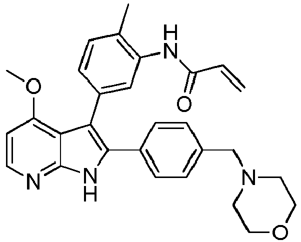 | >99 |
| **76** | 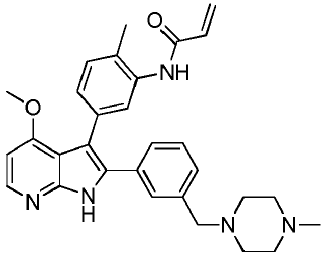 | >99 |
| **77** | 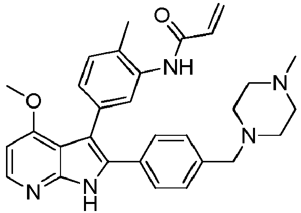 | >99 |
| **78** | 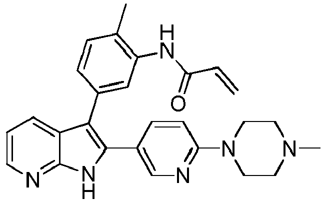 | >99 |
| **79** | 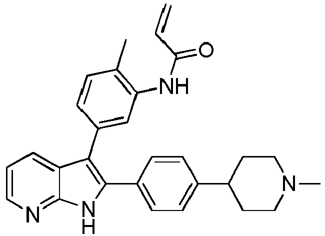 | >99 |
| **80** | 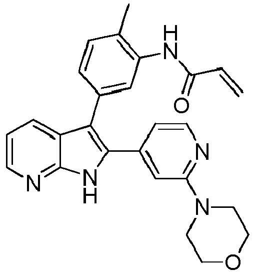 | >99 |
| **81** | 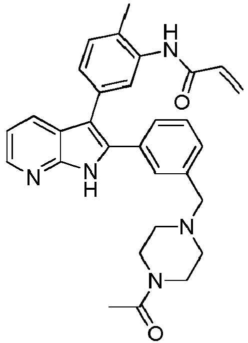 | 80 |
| **82** | 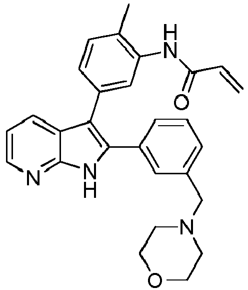 | 97 |
| **83** | 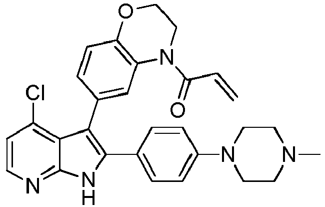 | >99 |
| **84** | 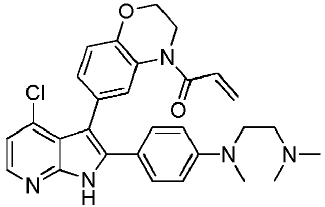 | 94 |
| **85** | 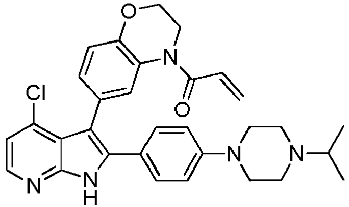 | >99 |
| **86** | 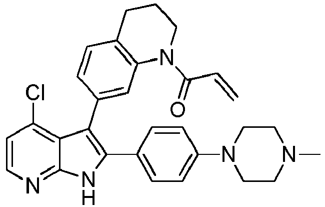 | >99 |
| **88** | 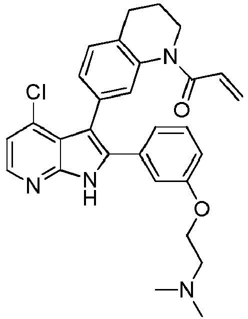 | >99 |
| **91** | 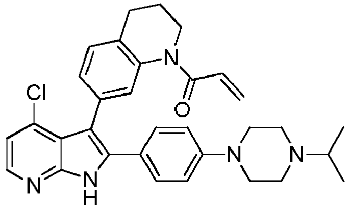 | >99 |
| **92** | 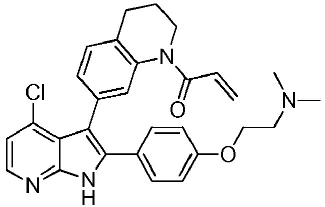 | >99 |
| **93** | 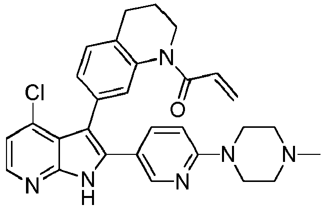 | >99 |
| **95** | 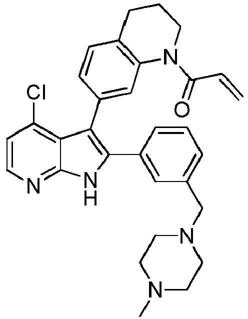 | >99 |
| **96** | 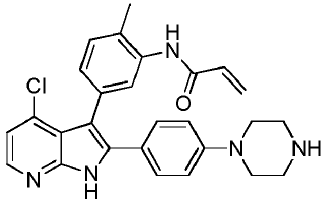 | 92 |
| **97** | 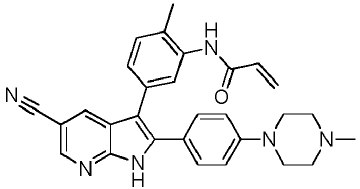 | >99 |
| **98** | 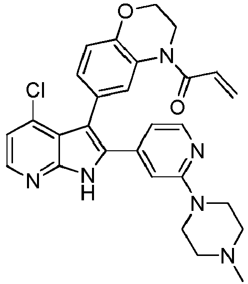 | >99 |
| **99** | 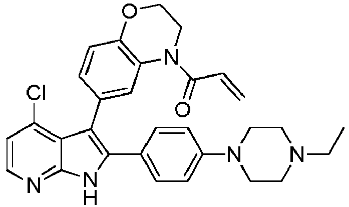 | >99 |
| **100** | 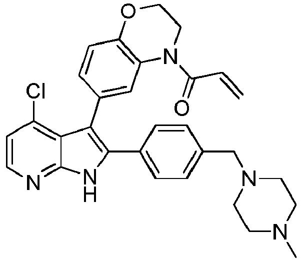 | >99 |
| **104** | 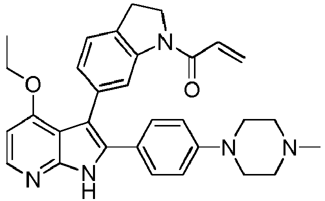 | 89 |
| **108** | 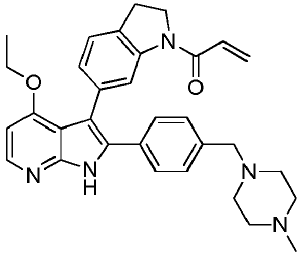 | 86 |
| **109** | 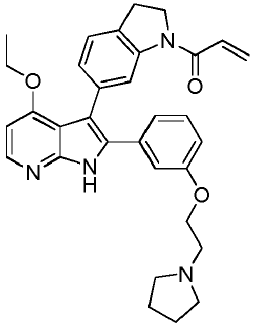 | 77 |
| **110** | 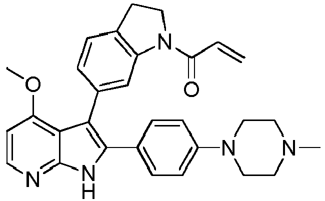 | 93 |
| **111** | 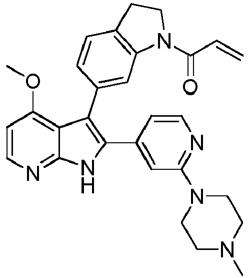 | 90 |
| **112** | 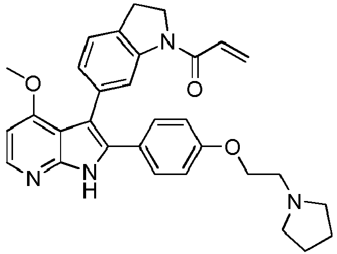 | 79 |
| **113** | 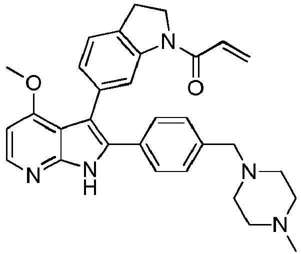 | 96 |
| **115** | 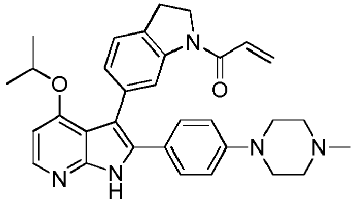 | >99 |
| **116** | 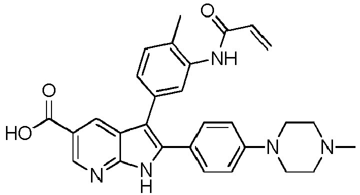 | 92 |
| **117** | 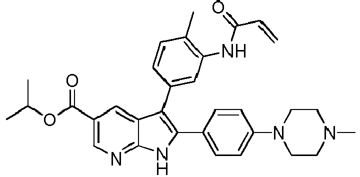 | >100 |
| **119** | 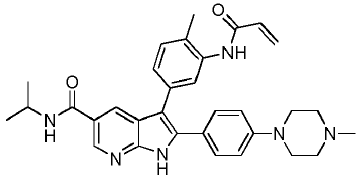 | >99 |
| **120** | 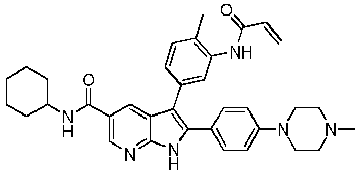 | >99 |
| **134** | 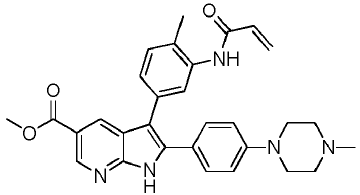 | >99 |
| **135** | 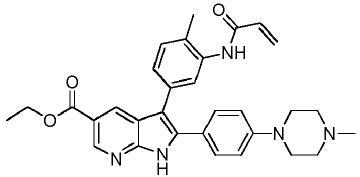 | 95 |
| **138** | 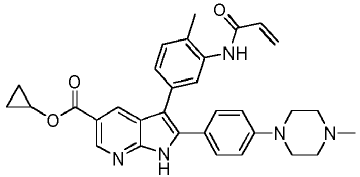 | >99 |
| **147** | 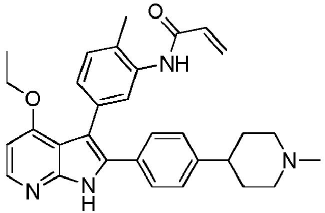 | 94 |

### 4. SAR

The structure activity relationship (SAR) of the compounds of the present invention as represented by the Formula (Ia and Ib) shows covalent inhibitor as cellular potent mutant-selective ErbB inhibitors. The covalent binding mode, obtained for example through the introduction of an acrylic moiety, is crucial for improved biochemical and cellular activity. The direct comparison of examples from this invention having a acryl moiety for the covalent binding with corresponding molecules having a propionic moiety are showing the improved cellular activities for the covalent binder.

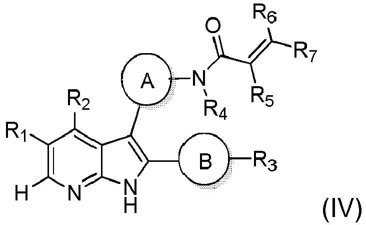

Table 6 shows comparison of covalent inhibitors with the corresponding reversible analogues. In all cases the covalent inhibitor shows improved cellular activities compared to the reversible analogues.

**Table 6: Comparison covalent inhibitors with corresponding reversible one.**

| **Binding Mode** | **Structure** | **Her2 Exon 20 INSYVMA Lance IC₅₀ [nM]** | **Her2 Exon 20 INSYVMA Ba/F3 CTG IC₅₀ [nM]** | **EGFR Exon 20 INSNPH Ba/F3 CTG IC₅₀ [nM]** |
|---|---|---|---|---|
| Covalent | 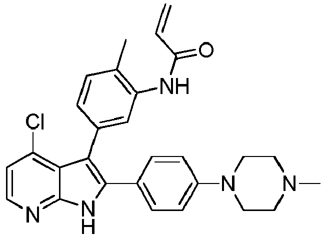 | 5 | 64 | 35 |
| | Example 2 | | | |
| Reversibel | 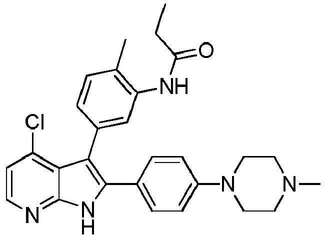 | 916 | | |

### SEQUENCE LIST

SEQ-ID No. 1: EGFR p.D770_N771insSVD 
ttcaaaaaga tcaaagtgct gggctccggt gcgttcggca cggtgtataa
gggactctgg atcccagaag gtgagaaagt taaaattccc gtcgctatca
aggaattaag agaagcaaca tctccgaaag ccaacaagga aatcctcgat
gaagcctacg tgatggccag cgtggactca gtagacaacc cccacgtgtg
ccgcctgctg ggcatctgcc tcacctccac cgtgcagctc atcacgcagc
tcatgccctt cggctgcctc ctggactatg tccgggaaca caaagacaat
attggctccc agtacctgct caactggtgt gtgcagatcg caaag
SEQ-ID No. 2: EGFR p.H773_V774insNPH 
ttcaaaaaga tcaaagtgct gggctccggt gcgttcggca cggtgtataa
gggactctgg atcccagaag gtgagaaagt taaaattccc gtcgctatca
aggaattaag agaagcaaca tctccgaaag ccaacaagga aatcctcgat
gaagcctacg tgatggccag cgtggacaac ccccacaatc cacatgtgtg
ccgcctgctg ggcatctgcc tcacctccac cgtgcagctc atcacgcagc
tcatgccctt cggctgcctc ctggactatg tccgggaaca caaagacaat
attggctccc agtacctgct caactggtgt gtgcagatcg caaag
SEQ-ID No. 3: EGFR p.V769_D770insASV 
ttcaaaaaga tcaaagtgct gggctccggt gcgttcggca cggtgtataa
gggactctgg atcccagaag gtgagaaagt taaaattccc gtcgctatca
aggaattaag agaagcaaca tctccgaaag ccaacaagga aatcctcgat
gaagcctacg tgatggccag cgtggcctca gtcgacaacc cccacgtgtg
ccgcctgctg ggcatctgcc tcacctccac cgtgcagctc atcacgcagc
tcatgccctt cggctgcctc ctggactatg tccgggaaca caaagacaat
attggctccc agtacctgct caactggtgt gtgcagatcg caaag
SEQ-ID No. 4: EGFR p.P772_H773insPR 
ttcaaaaaga tcaaagtgct gggctccggt gcgttcggca cggtgtataa
gggactctgg atcccagaag gtgagaaagt taaaattccc gtcgctatca
aggaattaag agaagcaaca tctccgaaag ccaacaagga aatcctcgat
gaagcctacg tgatggccag cgtggacaac cccccgcgtc acgtgtgccg
cctgctgggc atctgcctca cctccaccgt gcagctcatc acgcagctca
tgcccttcgg ctgcctcctg gactatgtcc gggaacacaa agacaatatt
ggctcccagt acctgctcaa ctggtgtgtg cagatcgcaa ag
SEQ-ID No. 5: HER2 INS8 INS YVMA 
acacctagcg gagcgatgcc caaccaggcg cagatgcgga tcctgaaaga
gacggagctg aggaaggtga aggtgcttgg atctggcgct tttggcacag
tctacaaggg catctggatc cctgatgggg agaatgtgaa aattccagtg
gccatcaaag tgttgaggga aaacacatcc cccaaagcca acaaagaaat
cttagacgaa gcatacgtga tggcttacgt gatggctggt gtgggctccc
catatgtctc ccgccttctg ggcatctgcc tgacatccac ggtgcagctg
gtgacacagc ttatgcccta tggctgcctc ttagaccatg tccgggaaaa
ccgcggacgc ctgggctccc aggacctgct gaactggtgt atgcagattg
ccaaggggat gagctacctg gaggatgtgc ggctcgtaca cagggacttg
gccgctcgga acgtgctggt caagagtccc aaccatgtca aaattacaga
c
6. SEQ-ID No. 6: EGFR T790M 
tccaaactgc acctacggat gcactgggcc aggtcttgaa ggctgtccaa
cgaatgggcc taagatcccg tccatcgcca ctgggatggt gggggccctc
ctcttgctgc tggtggtggc cctggggatc ggcctcttca tgcgaaggcg
ccacatcgtt cggaagcgca cgctgcggag gctgctgcag gagagggagc
ttgtggagcc tcttacaccc agtggagaag ctcccaacca agctctcttg
aggatcttga aggaaactga attcaaaaag atcaaagtgc tgggctccgg
tgcgttcggc acggtgtata agggactctg gatcccagaa ggtgagaaag
ttaaaattcc cgtcgctatc aaggaattaa gagaagcaac atctccgaaa
gccaacaagg aaatcctcga tgaagcctac gtgatggcca gcgtggacaa
cccccacgtg tgccgcctgc tgggcatctg cctcacctcc accgtgcagc
tcatcatgca gctcatgccc ttcggctgcc tcctggacta tgtccgggaa
cacaaagaca atattggctc ccagtacctg ctcaactggt gtgtgcagat
cgcaaagggc atgaactact tggaggaccg tcgcttggtg caccgcgacc
tggcagccag gaacgtactg gtgaaaacac cgcagcatgt caagatcaca
gattttgggc tggccaaact gctgggtgcg gaagagaaag aataccatgc
agaaggaggc aaagtgccta tcaagtggat ggcattggaa tcaattttac
acagaatcta tacccaccag agtgatgtct ggagctacgg ggtgaccgtt
tgggagttga tgacctttgg atccaagcca tatgacggaa tccctgccag
 
cgagatctcc tccatcctgg agaaaggaga acgcctccct cagccaccca
tatgtaccat cgatgtctac atgatcatgg
7. SEQ-ID No. 7: EGFR T790ML858R

tccaaactgc acctacggat gcactgggcc aggtcttgaa ggctgtccaa
cgaatgggcc taagatcccg tccatcgcca ctgggatggt gggggccctc
ctcttgctgc tggtggtggc cctggggatc ggcctcttca tgcgaaggcg
ccacatcgtt cggaagcgca cgctgcggag gctgctgcag gagagggagc
ttgtggagcc tcttacaccc agtggagaag ctcccaacca agctctcttg
aggatcttga aggaaactga attcaaaaag atcaaagtgc tgggctccgg
tgcgttcggc acggtgtata agggactctg gatcccagaa ggtgagaaag
ttaaaattcc cgtcgctatc aaggaattaa gagaagcaac atctccgaaa
gccaacaagg aaatcctcga tgaagcctac gtgatggcca gcgtggacaa
cccccacgtg tgccgcctgc tgggcatctg cctcacctcc accgtgcagc
tcatcatgca gctcatgccc ttcggctgcc tcctggacta tgtccgggaa
cacaaagaca atattggctc ccagtacctg ctcaactggt gtgtgcagat
cgcaaagggc atgaactact tggaggaccg tcgcttggtg caccgcgacc
tggcagccag gaacgtactg gtgaaaacac cgcagcatgt caagatcaca
gattttgggc gggccaaact gctgggtgcg gaagagaaag aataccatgc
agaaggaggc aaagtgccta tcaagtggat ggcattggaa tcaattttac
acagaatcta tacccaccag agtgatgtct ggagctacgg ggtgaccgtt
tgggagttga tgacctttgg atccaagcca tatgacggaa tccctgccag
cgagatctcc tccatcctgg agaaaggaga acgcctccct cagccaccca
tatgtaccat cgatgtctac atgatcatgg

## Claims

1. A compound of the formula (I) 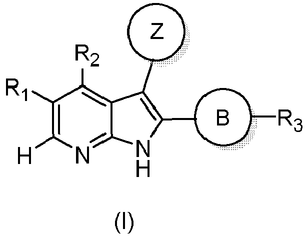 wherein
Z represents 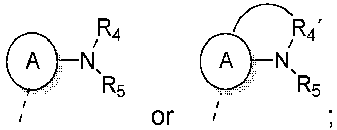
A represents 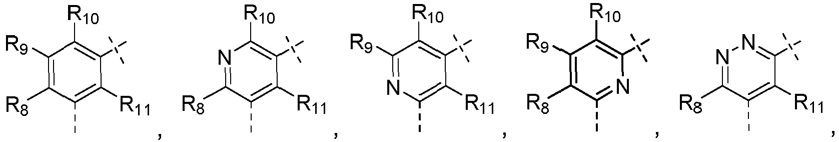 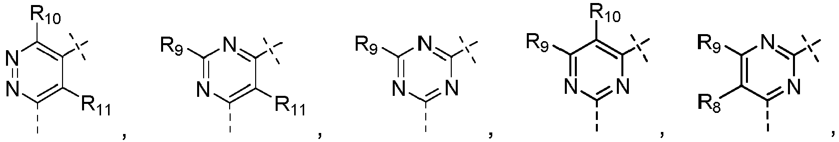 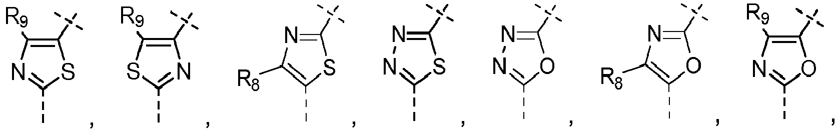 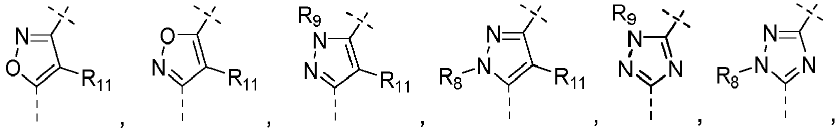 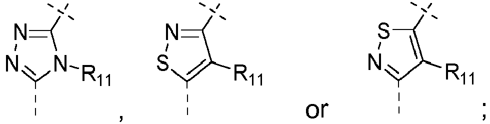 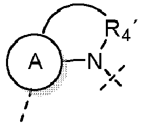 represents 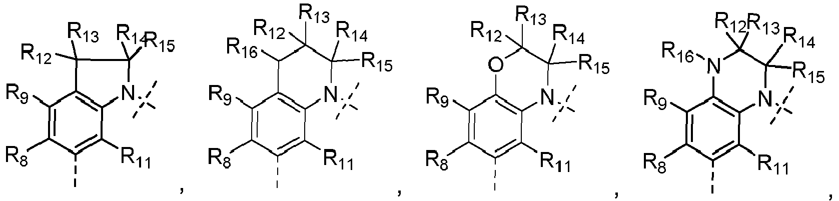 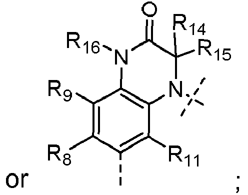
B represents 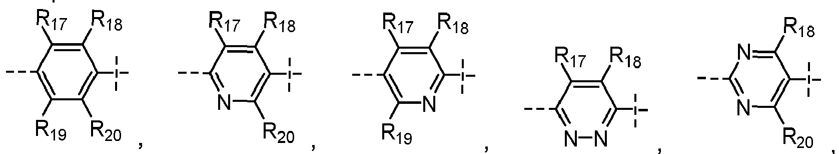 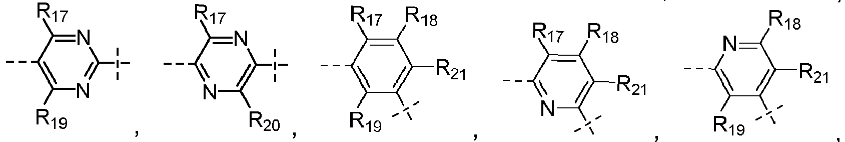 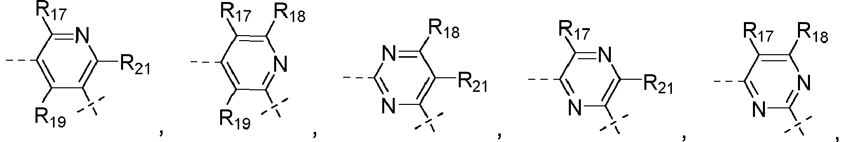 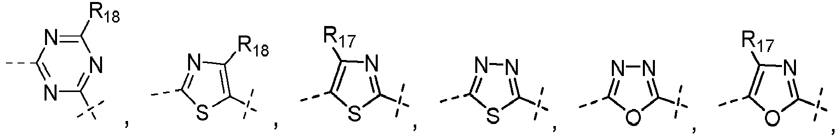 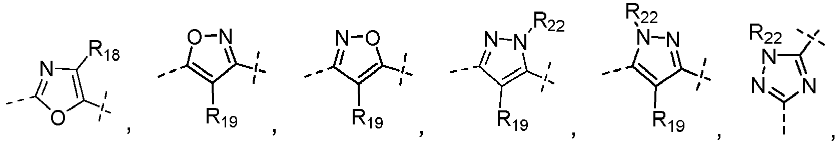 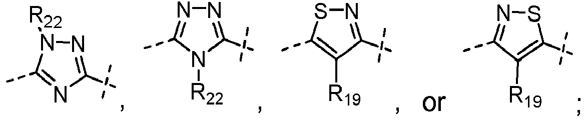
**R₁** represents -H, **-R*,** -CH₂-**R***, -CHR^{a}-**R***, -CR^{a}R^{b}-**R***, -CH₂CH₂-**R***, -CR^{a}R^{b}-CR^{c}R^{d}-**R***, -CH=CH-**R***, -CR^{a}=CR^{b}-**R***, -CH₂CH₂CH₂-**R*** or -CR^{a}R^{b}-CR^{c}R^{d}-CR^{e}R^{f}-**R***;
**R**^{a} - **R**^{f} represent independently of each other -H, -F, -CI, -CH₃, -OCH₃, -CH₂CH₃, -CH₂CH₂CH₃, -CH(CH₃)₂, -CH₂CH(CH₃)₂, or -cyclo-C₃H₅;
**R*** represents -H, -F, -Cl, -Br, -I, -OH, -CN, -NH₂, -NHCH₃, -NHC₂H₅, -NHC₃H₇, -NHCH(CH₃)₂, -NHC(CH₃)₃, -N(CH₃)₂, -N(C₂H₅)₂, -N(C₃H₇)₂, -N[CH(CH₃)₂]₂, -N[C(CH₃)₃]₂, -NO₂, -OCH₃, -OC₂H₅, OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅, -CHO, -COCH₃, -COC₂H₅, -COC₃H₇, -COCH(CH₃)₂, -COC(CH₃)₃, -OOC-CH₃, -OOC-C₂H₅, -OOC-C₃H₇, -OOC-CH(CH₃)₂, -OOC-cyclo-C₃H₅, -OOC-cyclo-C₃H₅, -OCF₃, -OC₂F₅, -NHSO₂CH₃, -NHSO₂C₂H₅, -NHSO₂C₃H₇, -NHS0₂CH(CH₃)₂, -NHSO₂₋cyclo-C₃H₅, -SOCH₃, -SO₂CH₃, -SO₂CF₃, -SO₂C₂H₅, -SO₂CH₂CF₃, -SO₂C₃H₇, -SO₂CH(CH₃)₂, -SO₂-cyclo-C₃H₅, -SO(NH)CH₃, -SO(NH)C₂H₅, -SO(NH)C₃H₇, -SO(NH)CH(CH₃)₂, -SO(NH)-cyclco-C₃H₅, -SO(NCH₃)CH₃, -SO(NCH₃)C₂H₅, -SO(NCH₃)C₃H₇, -SO(NCH₃)CH(CH₃)₂, -SO(NCH₃)-cyclo-C₃H₅, 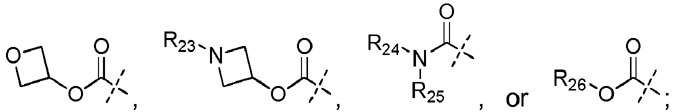
**R₂** represents -H, -F, -CI, -Br, -I, -OH, -CN, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅, or -O-C₂H₄-cyclo-C₃H₅;
**R₃** represents -H, -CH₃, -NR₂₇R₂₈, -CH₂NR₂₇R₂₈, -CH₂CH₂NR₂₇R₂₈, -CH₂CH₂CH₂NR₂₇R₂₈, -NHCOR₂₇, -NHSO₂R₂₇, -OCH₂NR₂₇R₂₈, -OCH₂CH₂NR₂₇R₂₈, -OCH₂CH₂CH₂NR₂₇R₂₈, -NR₂₉CH₂NR₂₇R₂₈, -NR₂₉CH₂CH₂NR₂₇R₂₈, -NR₂₉CH₂CH₂CH₂NR₂₇R₂₈, -CONR₂₇R₂₈, -CONHCH₂NR₂₇R₂₈, -CONHCH₂CH₂NR₂₇R₂₈, -CONH CH₂CH₂CH₂NR₂₇R₂₈, 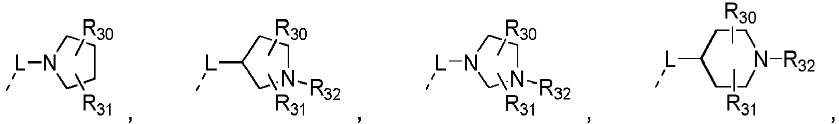 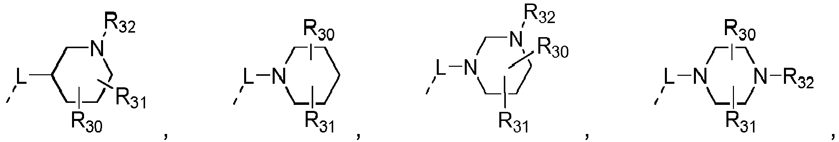 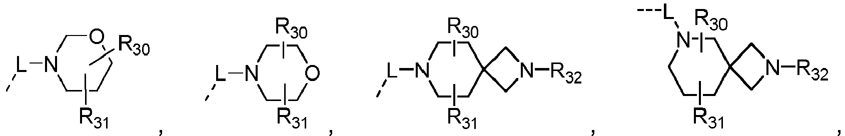 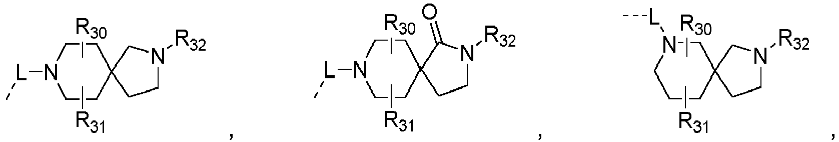 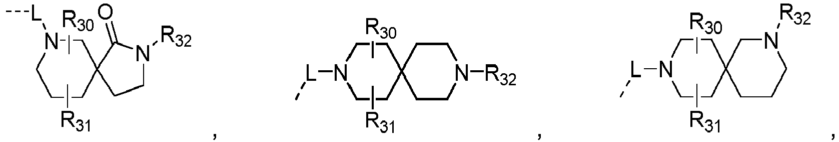 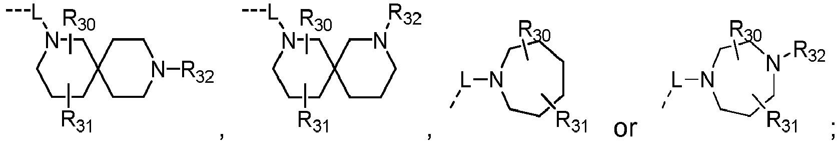
L is a bond, -CH₂-, -CH(CH₃)-, -CH₂CH₂-, -CH₂CH₂CH₂-, -CF₂-, -CF₂CH₂-, -OCH₂-, -OCH₂CH₂-, -OCH₂CH₂CH₂-, -NHCH₂-, -NHCH₂CH₂-, -NHCH₂CH₂CH₂-, -N(CH₃)CH₂-, -N(CH₃)CH₂CH₂-, -N(CH₃)CH₂CH₂CH₂-, -NHCO-, -NHCOCH₂-, -NHCOCH₂CH₂-, -CO-, -CH₂CO-, -CH₂CH₂CO-, -COCH₂-, -COCH₂CH₂-, -COCH₂CO-, -COCH₂CH₂CO-, -CONH-, -CONHCH₂-, -CONHCH₂CH₂-, -CONHCH₂CH₂CH₂-, -OCO-, -CH₂OCO- or -CH₂CH₂OCO-;
**R₄** represents -H, -CH₃, or -C₂H₅;
**R₅** represents 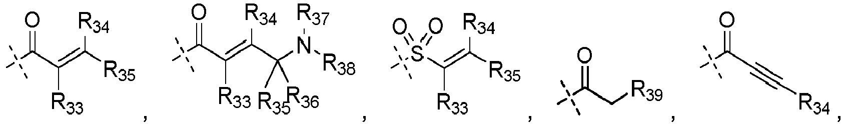 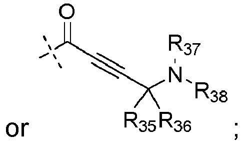
**R₆** and **R₇** represent independently of each other -H, -F, -CH₃, -C₂H₅, -CN, -CF₃, -NO₂, -CHO, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, -COOC(CH₃)₃, -CH₂NHCH₃, -CH₂NHC₂H₅, -CH₂NHC₃H₇, -CH₂NHCH(CH₃)₂, -CH₂N(CH₃)₂, -CH₂N(C₂H₅)₂, -CH₂N(C₃H₇)₂, -CH₂CH₂NHCH₃, -CH₂CH₂NHC₂H₅, -CH₂CH₂NHC₃H₇, -CH₂CH₂NHCH(CH₃)₂, -CH₂CH₂N(CH₃)₂, - CH₂CH₂N(C₂H₅)₂, or -CH₂CH₂N(C₃H₇)₂;
**R₈** - **R₁₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇,
or **R₈** and **R₉** or **R₉** and **R₁₀** form together -CH₂-, -CH₂CH₂-, -CH₂CH₂CH₂-, -CH₂CH₂CH₂CH₂-, -CH₂OCH₂-, -CH₂OCH₂CH₂-, -OCH₂O-, or -OCH₂CH₂O-;
**R₁₂** - **R₁₆** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₁₇** - **R₂₁** represent independently of each other -H, -F, -CI, -CN, -OCH₃, -OC₂H₅, -OC₃H₇, -OCH(CH₃)₂, -OC(CH₃)₃, -OC₄H₉, -OCH₂CH(CH₃)₂, -OC₂H₄OCH₃, -CH₂OCH₃, -CH₃, -CF₃, -C₂H₅, or -C₃H₇;
**R₂₂** represents -H, -CH₃, -CF₃, -C₂H₅, -C₃H₇, -COCH₃, -COC₂H₅, -SO₂CH₃, or -SO₂C₂H₅;
**R₂₃** represents -H, -CH₃, -CF₃, -C₂H₅, -C₃H₇, -CH₂CH₂OCH₃, or -C₄H₉;
**R₂₄** and **R₂₅** represent independently of each other -H, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, 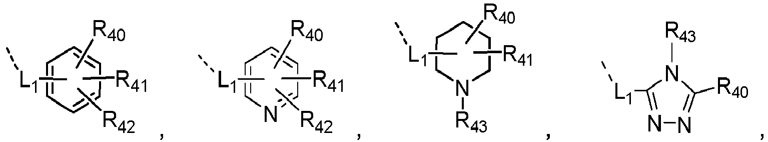 or 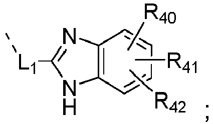
or **R²⁴** and **R²⁵** form together -CH₂-, -CH₂CH₂-, -CH₂CH₂CH₂-, -CH₂CH₂CH₂CH₂-, -CH₂OCH₂-, or -CH₂OCH₂CH₂-;
**L₁** represents a bond, -CH₂-, -CH₂CH₂-, or -CH₂CH₂CH₂-;
**R₂₆** represents -H, -CH₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, -cyclo-C₆H₁₁, -C(CH₃)₃, -Ph, or -CH₂Ph;
**R₂₇** - **R₃₁** represent independently of each other -H, -F, -CI, -OH, -CN, -NH₂, -OCH₃, -OC₂H₅, -OC₃H₇, -OCH(CH₃)₂, -CH₃, -CF₃, -C₂H₅, -C₃H₇, -CH₂CH₂OCH₃, -CH(CH₃)₂, -C₄H₉, -cyclo-C₃H₅, -cyclo-C₄H₇, -cyclo-C₅H₉, or -cyclo-C₆H₁₁;
**R₃₂, R₃₇, R₃₈,** and **R₄₃** represent independently of each other -H, -CH₃, -CF₃, -C₂H₅, -C₃H₇, -CH(CH₃)₂, -Ph, -CH₂Ph, -COCH₃, -COCF₃, -COC₂H₅, -COCH(CH₃)₂, -COC(CH₃)₃, -COPh, -CO₂CH₃, -CO₂C₂H₅, -CO₂CH(CH₃)₂, -CO₂C(CH₃)₃, -CO₂Ph, -CO₂CH₂Ph, -SO₂CH₃, -SO₂C₂H₅, -SO₂CF₃, or -SO₂Ph;
**R₃₃, R₃₄, R₃₅,** and **R₃₆** represent independently of each other -H, -CH₃, -CF₃, -C₂H₅, -C₃H₇, -CH(CH₃)₂, -CN, -NO₂, -COCH₃, -COC₂H₅, -COC₃H₇, -COCH(CH₃)₂, -COC(CH₃)₃, -COOH, -COOCH₃, -COOC₂H₅, -COOC₃H₇, -COOCH(CH₃)₂, or -COOC(CH₃)₃;
**R₃₉** represents -F, -Br, -CI, or -I;
**R₄₀, R₄₁,** and **R₄₂** represent independently of each other -H, -F, -CI, -OH, -CN, -NH₂, -CH₃, -CF₃, -C₂H₅, -C₃H₇, -CH(CH₃)₂, -OCH₃, -OC₂H₅, -OC₃H₇, -OCH(CH₃)₂, -COCH₃, -COCF₃, -COC₂H₅, -COCH(CH₃)₂, -NHCH₃, -NHC₂H₅, -N(CH₃)₂, -N(C₂H₅)₂, -NHCOCH₃, -NHCOCF₃, -NHCOC₂H₅, -NHCOCH(CH₃)₂, -CO₂H, -CO₂CH₃, -CO₂C₂H₅, -CO₂CH(CH₃)₂, -CO₂Ph, -CO₂CH₂Ph, -CONH₂, -CONHCH₃, -CONHC₂H₅, -CONHCH(CH₃)₂, -CON(CH₃)₂, -SO₂CH₃, -SO₂C₂H₅, -SO₂CF₃, -SO₂Ph, -SO₂NH₂, -SO₂NHCH₃, 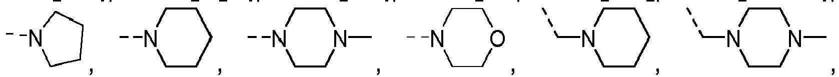 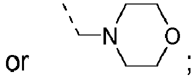
or an enantiomer, a mixture of enantiomers, a diastereomer, a mixture of diastereomers, a tautomer, a hydrate, a solvate, or pharmaceutically acceptable salts thereof.

2. The compound according to Claim 1, wherein
A represents 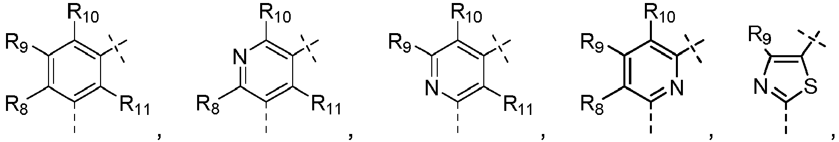 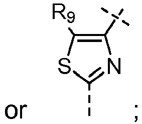 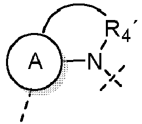 represents 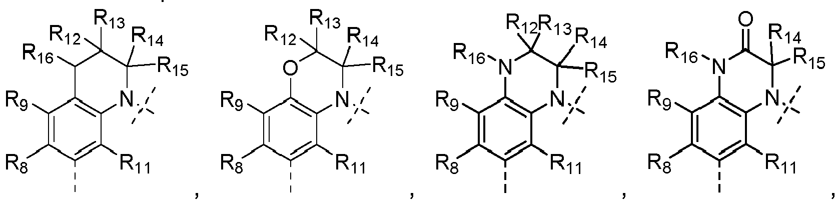 or 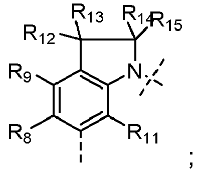
B represents 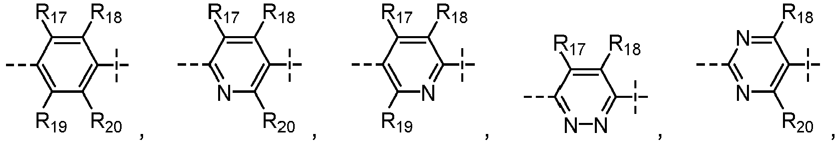 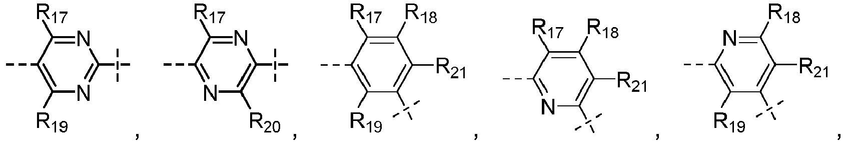 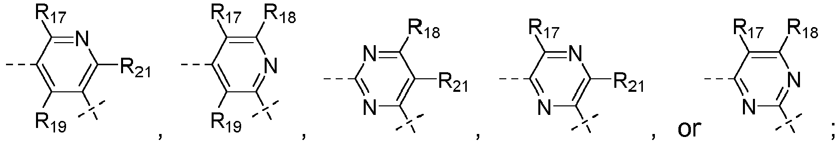
and **R₈** - **R₂₁** have the same meanings as defined in claim 1.

3. The compound according to Claim 1 or 2, wherein
**A** represents 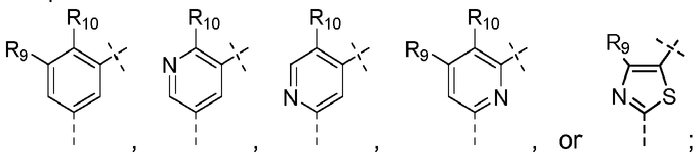 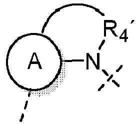 represents 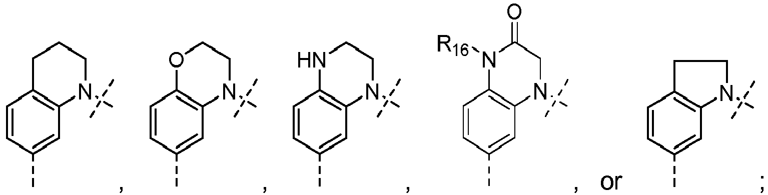
**B** represents 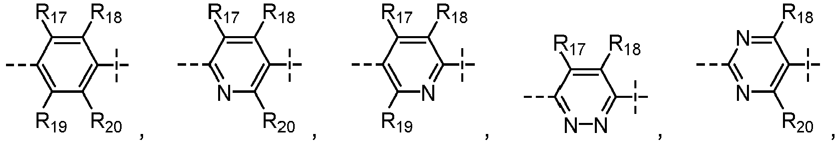 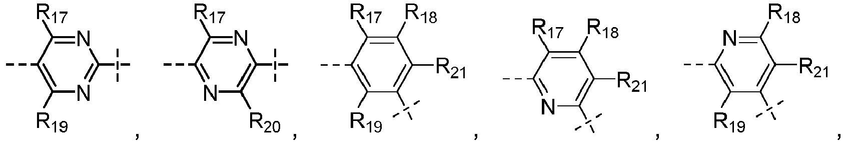 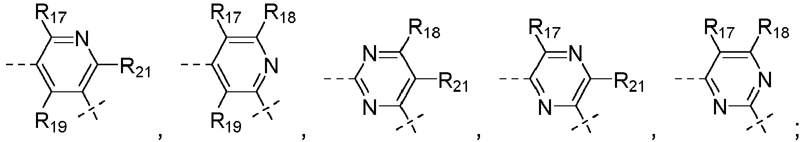
and **R₉ - R₁₀,** and **R₁₆ - R₂₁** have the meanings as defined in Claim 1.

4. The compound according to Claim 1, wherein the compound has the formula **(Ia)** or **(Ib):** 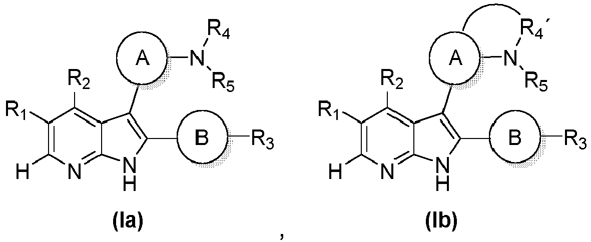 wherein
A represents 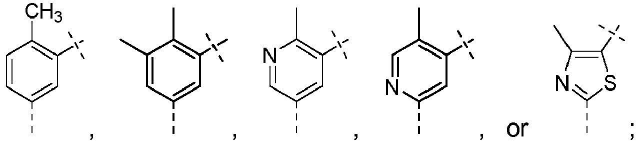 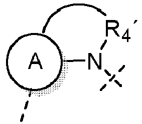 represents 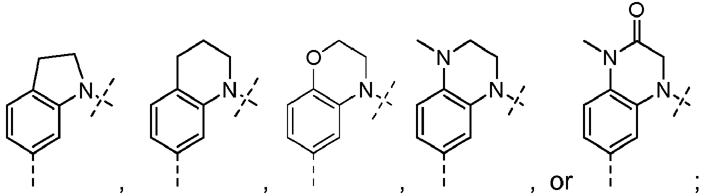
B represents 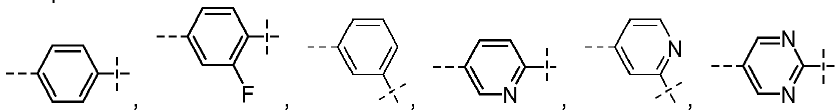 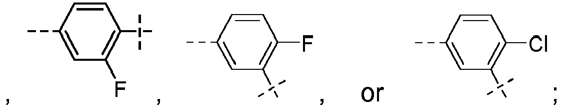
and **R₁, R₂, R₃, R₄,** and **R₅** have the same meanings as defined in claim 1.

5. The compound according to any one of claim 1 - 4, wherein
Z represents 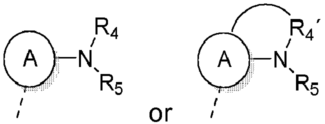 and is selected from 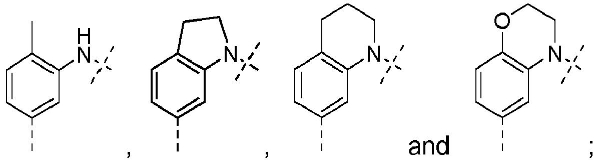
**R₁** represents -H, -CN, -COOH, -COOCH₃, -COOCH₂CH₃, -COOCH(CH₃)₂, -COOCH₂CH₂OCH₃, -COO-cyclo-C₃H₅, -COO-cyclo-C₄H₇, -COO-cyclo-C₅H₉, -COO-cyclo-C₆H₁₁, -CONHCH(CH₃)₂, -CONH-cyclo-C₆H₁₁, 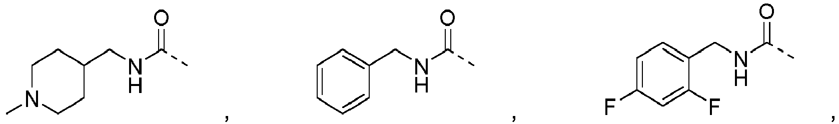 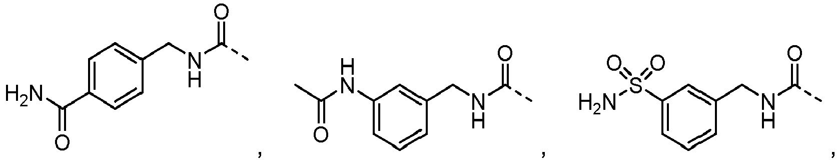 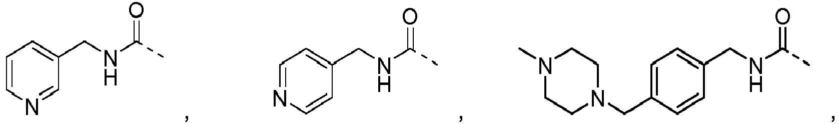 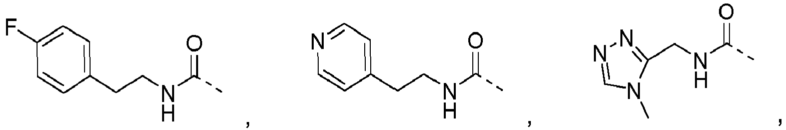 or 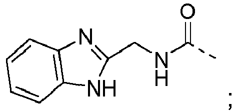
**R₂** represents -H, -CI, -OCH₃, -OC₂H₅, or -OCH(CH₃)₂;
B represents 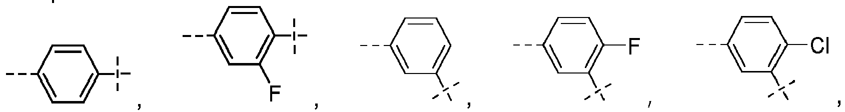 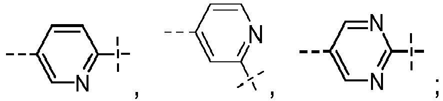
**R₃** represents -H, -CH₂N(CH₃)₂, -CH₂CH₂N(CH₃)₂, -OCH₂CH₂N(CH₃)₂, -OCH₂CH₂CH₂N(CH₃)₂, -CH₂N(CH₃)₂, -NHCOCH₃, -NHSO₂CH₃, -N(CH₃)CH₂CH₂N(CH₃)₂, -CONH-cyclo-C₃H₅, -CONH-cyclo-C₆H₁₁, -CONHCH₂CH₂N(CH₃)CH₂Ph, 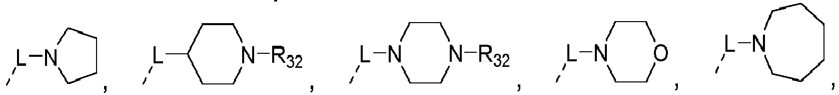 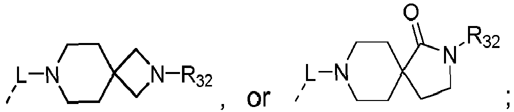
**R₅** represents 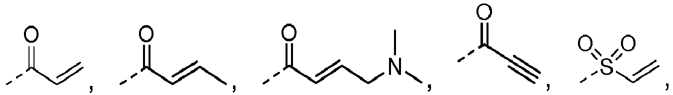 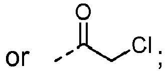
L is a bond, -CH₂-, -OCH₂CH₂-, -CO-, -CONH-, or -CONHCH₂CH₂CH₂-;
and
**R₃₂** represents -H, -CH₃, -C₂H₅, -CH(CH₃)₂, -CH₂Ph, -COCH₃, or -SO₂C₂H₅.

6. The compound according to Claim 1 selected from the group consisting of:
N-(2-methyl-5-(2-(4-(1-methylpiperidin-4-yl)phenyl)-1H-pyrrolo[2,3-b]pyridin-3-yl)phenyl)acrylamide;
N-(5-(4-chloro-2-(4-(4-methylpiperazin-1-yl)phenyl)-1 H-pyrrolo[2,3-b]pyridin-3-yl)-2-methylphenyl)acrylamide;
N-(5-(4-chloro-2-(4-(1-methylpiperidin-4-yl)phenyl)-1H-pyrrolo[2,3-b]pyridin-3-yl)-2-methylphenyl)acrylamide;
1-(6-(2-(4-(4-methylpiperazin-1-yl)phenyl)-1H-pyrrolo[2,3-b]pyridin-3-yl)indolin-1-yl)prop-2-en-1-one;
N-(5-(4-chloro-2-(4-(2-(dimethylamino)ethyl)phenyl)-1H-pyrrolo[2,3-b]pyridin-3-yl)-2-methylphenyl)acrylamide;
N-(5-(4-chloro-2-(4-(morpholinomethyl)phenyl)-1 H-pyrrolo[2,3-b]pyridin-3-yl)-2-methylphenyl)acrylamide;
N-(5-(4-chloro-2-(3-morpholinophenyl)-1H-pyrrolo[2,3-b]pyridin-3-yl)-2-methylphenyl)acrylamide;
N-(5-(4-chloro-2-(3-(morpholinomethyl)phenyl)-1 H-pyrrolo[2,3-b]pyridin-3-yl)-2-methylphenyl)acrylamide;
N-(5-(2-(4-(4-acetylpiperazin-1-yl)phenyl)-4-chloro-1H-pyrrolo[2,3-b]pyridin-3-yl)-2-methylphenyl)acrylamide;
N-(5-(4-chloro-2-(4-((4-methylpiperazin-1-yl)methyl)phenyl)-1 H-pyrrolo[2,3-b]pyridin-3-yl)-2-methylphenyl)acrylamide;
N-(5-(4-chloro-2-(4-(4-methylpiperazine-1-carbonyl)phenyl)-1 H-pyrrolo[2,3-b]pyridin-3-yl)-2-methylphenyl)acrylamide;
N-(5-(4-chloro-2-(4-((dimethylamino)methyl)phenyl)-1H-pyrrolo[2,3-b]pyridin-3-yl)-2-methylphenyl)acrylamide;
N-(5-(4-chloro-2-(4-(morpholine-4-carbonyl)phenyl)-1H-pyrrolo[2,3-b]pyridin-3-yl)-2-methylphenyl)acrylamide;
N-(5-(4-chloro-2-(4-(methylsulfonamido)phenyl)-1 H-pyrrolo[2,3-b]pyridin-3-yl)-2-methylphenyl)acrylamide;
N-(5-(4-chloro-2-(4-(3-(dimethylamino)propoxy)phenyl)-1H-pyrrolo[2,3-b]pyridin-3-yl)-2-methylphenyl)acrylamide;
N-(5-(2-(4-acetamidophenyl)-4-chloro-1H-pyrrolo[2,3-b]pyridin-3-yl)-2-methylphenyl)acrylamide;
N-(5-(4-chloro-2-(4-(4-(ethylsulfonyl)piperazin-1-yl)phenyl)-1 H-pyrrolo[2,3-b]pyridin-3-yl)-2-methylphenyl)acrylamide;
N-(5-(4-chloro-2-(3-((4-methylpiperazin-1-yl)methyl)phenyl)-1 H-pyrrolo[2,3-b]pyridin-3-yl)-2-methylphenyl)acrylamide;
N-(5-(4-chloro-2-(3-(4-methylpiperazin-1-yl)phenyl)-1 H-pyrrolo[2,3-b]pyridin-3-yl)-2-methylphenyl)acrylamide;
N-(5-(4-chloro-2-(4-(2-(dimethylamino)ethoxy)phenyl)-1H-pyrrolo[2,3-b]pyridin-3-yl)-2-methylphenyl)acrylamide;
N-(5-(4-methoxy-2-(4-(1-methylpiperidin-4-yl)phenyl)-1H-pyrrolo[2,3-b]pyridin-3-yl)-2-methylphenyl)acrylamide;
N-(5-(4-methoxy-2-(4-(4-methylpiperazin-1-yl)phenyl)-1 H-pyrrolo[2,3-b]pyridin-3-yl)-2-methylphenyl)acrylamide;
N-(5-(2-(4-(4-acetylpiperazin-1-yl)phenyl)-4-methoxy-1H-pyrrolo[2,3-b]pyridin-3-yl)-2-methylphenyl)acrylamide;
N-(5-(4-methoxy-2-(4-(morpholinomethyl)phenyl)-1 H-pyrrolo[2,3-b]pyridin-3-yl)-2-methylphenyl)acrylamide;
N-(5-(4-methoxy-2-(3-((4-methylpiperazin-1-yl)methyl)phenyl)-1 H-pyrrolo[2,3-b]pyridin-3-yl)-2-methylphenyl)acrylamide;
N-(5-(4-methoxy-2-(4-((4-methylpiperazin-1-yl)methyl)phenyl)-1 H-pyrrolo[2,3-b]pyridin-3-yl)-2-methylphenyl)acrylamide;
N-(5-(4-chloro-2-(6-(4-methylpiperazin-1-yl)pyridin-3-yl)-1 H-pyrrolo[2,3-b]pyridin-3-yl)-2-methylphenyl)acrylamide;
N-(5-(4-ethoxy-2-(4-(1-methylpiperidin-4-yl)phenyl)-1H-pyrrolo[2,3-b]pyridin-3-yl)-2-methylphenyl)acrylamide;
N-(5-(4-chloro-2-(3-(3-(dimethylamino)propoxy)phenyl)-1H-pyrrolo[2,3-b]pyridin-3-yl)-2-methylphenyl)acrylamide;
N-(5-(4-chloro-2-(2-methoxy-4-(4-methylpiperazin-1-yl)phenyl)-1 H-pyrrolo[2,3-b]pyridin-3-yl)-2-methylphenyl)acrylamide;
N-(5-(2-(3-((4-acetylpiperazin-1-yl)methyl)phenyl)-4-chloro-1H-pyrrolo[2,3-b]pyridin-3-yl)-2-methylphenyl)acrylamide;
N-(5-(4-chloro-2-(4-(3-(4-ethylpiperazin-1-yl)propoxy)phenyl)-1H-pyrrolo[2,3-b]pyridin-3-yl)-2-methylphenyl)acrylamide;
N-(5-(4-chloro-2-(pyrimidin-5-yl)-1H-pyrrolo[2,3-b]pyridin-3-yl)-2-methylphenyl)acrylamide; N-(5-(4-chloro-2-(3-(3-(4-ethylpiperazin-1-yl)propoxy)phenyl)-1H-pyrrolo[2,3-b]pyridin-3-yl)-2-methylphenyl)acrylamide;
N-(5-(4-chloro-2-(2-(4-methylpiperazin-1-yl)pyridin-4-yl)-1 H-pyrrolo[2,3-b]pyridin-3-yl)-2-methylphenyl)acrylamide;
N-(5-(4-chloro-2-(3-(morpholine-4-carbonyl)phenyl)-1H-pyrrolo[2,3-b]pyridin-3-yl)-2-methylphenyl)acrylamide;
N-(5-(4-chloro-2-(3-(2-(dimethylamino)ethoxy)phenyl)-1H-pyrrolo[2,3-b]pyridin-3-yl)-2-methylphenyl)acrylamide;
N-(5-(4-chloro-2-(4-(piperidin-4-yl)phenyl)-1H-pyrrolo[2,3-b]pyridin-3-yl)-2-methylphenyl)acrylamide;
N-(5-(4-chloro-2-(6-morpholinopyridin-3-yl)-1H-pyrrolo[2,3-b]pyridin-3-yl)-2-methylphenyl)acrylamide;
N-(5-(2-(6-(4-acetylpiperazin-1-yl)pyridin-3-yl)-4-chloro-1H-pyrrolo[2,3-b]pyridin-3-yl)-2-methylphenyl)acrylamide;
N-(5-(4-chloro-2-(2-morpholinopyridin-4-yl)-1 H-pyrrolo[2,3-b]pyridin-3-yl)-2-methylphenyl)acrylamide;
1-(6-(4-chloro-2-(4-(1-methylpiperidin-4-yl)phenyl)-1H-pyrrolo[2,3-b]pyridin-3-yl)indolin-1-yl)prop-2-en-1-one;
N-(5-(4-chloro-2-(3-(4-methylpiperazine-1-carbonyl)phenyl)-1 H-pyrrolo[2,3-b]pyridin-3-yl)-2-methylphenyl)acrylamide;
N-(5-(2-(3-(4-acetylpiperazin-1-yl)phenyl)-4-chloro-1H-pyrrolo[2,3-b]pyridin-3-yl)-2-methylphenyl)acrylamide; 3-(3-(3-acrylamido-4-methylphenyl)-4-chloro-1H-pyrrolo[2,3-b]pyridin-2-yl)-N-(3-(pyrrolidin-1-yl)propyl)benzamide;
3-(3-(3-acrylamido-4-methylphenyl)-4-chloro-1H-pyrrolo[2,3-b]pyridin-2-yl)-N-(1-benzylpiperidin-4-yl)benzamide;
3-(3-(3-acrylamido-4-methylphenyl)-4-chloro-1H-pyrrolo[2,3-b]pyridin-2-yl)-N-cyclopropylbenzamide;
3-(3-(3-acrylamido-4-methylphenyl)-4-chloro-1H-pyrrolo[2,3-b]pyridin-2-yl)-N-(3-(benzyl(methyl)amino)propyl)benzamide;
N-(5-(2-(3-(4-acetylpiperazine-1-carbonyl)phenyl)-4-chloro-1H-pyrrolo[2,3-b]pyridin-3-yl)-2-methylphenyl)acrylamide;
N-(5-(4-chloro-2-(3-(2-methyl-2,7-diazaspiro[3.5]nonane-7-carbonyl)phenyl)-1H-pyrrolo[2,3-b]pyridin-3-yl)-2-methylphenyl)acrylamide; 3-(3-(3-acrylamido-4-methylphenyl)-4-chloro-1H-pyrrolo[2,3-b]pyridin-2-yl)-N-(3-(4-ethylpiperazin-1-yl)propyl)benzamide;
1-(6-(4-chloro-2-(4-(4-methylpiperazin-1-yl)phenyl)-1H-pyrrolo[2,3-b]pyridin-3-yl)-2H-benzo[b][1,4]oxazin-4(3H)-yl)prop-2-en-1-one;
1-(7-(4-chloro-2-(4-(4-methylpiperazin-1-yl)phenyl)-1H-pyrrolo[2,3-b]pyridin-3-yl)-3,4-dihydroquinolin-1(2H)-yl)prop-2-en-1-one;
N-(5-(4-chloro-2-(2-morpholinopyrimidin-5-yl)-1 H-pyrrolo[2,3-b]pyridin-3-yl)-2-methylphenyl)acrylamide;
N-(5-(4-chloro-2-(3-(2-(pyrrolidin-1-yl)ethoxy)phenyl)-1H-pyrrolo[2,3-b]pyridin-3-yl)-2-methylphenyl)acrylamide;
N-(5-(4-chloro-2-(4-(2-(pyrrolidin-1-yl)ethoxy)phenyl)-1 H-pyrrolo[2,3-b]pyridin-3-yl)-2-methylphenyl)acrylamide;
1-(7-(4-chloro-2-(4-(1-methylpiperidin-4-yl)phenyl)-1H-pyrrolo[2,3-b]pyridin-3-yl)-3,4-dihydroquinolin-1(2H)-yl)prop-2-en-1-one;
1-(7-(4-chloro-2-(3-(2-(dimethylamino)ethoxy)phenyl)-1H-pyrrolo[2,3-b]pyridin-3-yl)-3,4-dihydroquinolin-1(2H)-yl)prop-2-en-1-one;
1-(7-(4-chloro-2-(6-((4-methylpiperazin-1-yl)methyl)pyridin-3-yl)-1H-pyrrolo[2,3-b]pyridin-3-yl)-3,4-dihydroquinolin-1(2H)-yl)prop-2-en-1-one;
N-(5-(4-chloro-2-(2-(4-methylpiperazin-1-yl)pyrimidin-5-yl)-1 H-pyrrolo[2,3-b]pyridin-3-yl)-2-methylphenyl)acrylamide;
N-(5-(4-chloro-2-(4-(4-ethylpiperazin-1-yl)phenyl)-1 H-pyrrolo[2,3-b]pyridin-3-yl)-2-methylphenyl)acrylamide;
N-(5-(4-chloro-2-(4-(4-isopropylpiperazin-1-yl)phenyl)-1H-pyrrolo[2,3-b]pyridin-3-yl)-2-methylphenyl)acrylamide;
1-(7-(4-chloro-2-(4-(2-(pyrrolidin-1-yl)ethoxy)phenyl)-1H-pyrrolo[2,3-b]pyridin-3-yl)-3,4-dihydroquinolin-1(2H)-yl)prop-2-en-1-one;
1-(7-(4-chloro-2-(4-(4-isopropylpiperazin-1-yl)phenyl)-1H-pyrrolo[2,3-b]pyridin-3-yl)-3,4-dihydroquinolin-1 (2H)-yl)prop-2-en-1-one;
1-(7-(4-chloro-2-(4-(2-(dimethylamino)ethoxy)phenyl)-1H-pyrrolo[2,3-b]pyridin-3-yl)-3,4-dihydroquinolin-1(2H)-yl)prop-2-en-1-one;
1-(7-(4-chloro-2-(6-(4-methylpiperazin-1-yl)pyridin-3-yl)-1H-pyrrolo[2,3-b]pyridin-3-yl)-3,4-dihydroquinolin-1(2H)-yl)prop-2-en-1-one;
1-(7-(4-chloro-2-(3-(2-(pyrrolidin-1-yl)ethoxy)phenyl)-1H-pyrrolo[2,3-b]pyridin-3-yl)-3,4-dihydroquinolin-1(2H)-yl)prop-2-en-1-one;
1-(7-(4-chloro-2-(3-((4-methylpiperazin-1-yl)methyl)phenyl)-1H-pyrrolo[2,3-b]pyridin-3-yl)-3,4-dihydroquinolin-1(2H)-yl)prop-2-en-1-one;
N-(5-(4-chloro-2-(4-(piperazin-1-yl)phenyl)-1H-pyrrolo[2,3-b]pyridin-3-yl)-2-methylphenyl)acrylamide;
1-(6-(4-chloro-2-(4-(4-methylpiperazin-1-yl)phenyl)-1H-pyrrolo[2,3-b]pyridin-3-yl)indolin-1-yl)prop-2-en-1-one;
1-(6-(4-chloro-2-(3-fluoro-4-(4-methylpiperazin-1-yl)phenyl)-1H-pyrrolo[2,3-b]pyridin-3-yl)indolin-1-yl)prop-2-en-1-one;
1-(6-(2-(4-(4-acetylpiperazin-1-yl)phenyl)-4-chloro-1H-pyrrolo[2,3-b]pyridin-3-yl)indolin-1-yl)prop-2-en-1-one;
1-(6-(4-chloro-2-(2-(4-methylpiperazin-1-yl)pyridin-4-yl)-1H-pyrrolo[2,3-b]pyridin-3-yl)indolin-1-yl)prop-2-en-1-one;
1-(6-(4-chloro-2-(4-((4-methylpiperazin-1-yl)methyl)phenyl)-1H-pyrrolo[2,3-b]pyridin-3-yl)indolin-1-yl)prop-2-en-1-one;
1-(6-(4-chloro-2-(4-((dimethylamino)methyl)phenyl)-1H-pyrrolo[2,3-b]pyridin-3-yl)indolin-1-yl)prop-2-en-1-one;
1-(6-(4-chloro-2-(4-(4-isopropylpiperazin-1-yl)phenyl)-1H-pyrrolo[2,3-b]pyridin-3-yl)indolin-1-yl)prop-2-en-1-one;
1-(6-(4-chloro-2-(4-(4-ethylpiperazin-1-yl)phenyl)-1H-pyrrolo[2,3-b]pyridin-3-yl)indolin-1-yl)prop-2-en-1-one;
1-(6-(4-chloro-2-(3-(2-(pyrrolidin-1-yl)ethoxy)phenyl)-1H-pyrrolo[2,3-b]pyridin-3-yl)indolin-1-yl)prop-2-en-1-one;
1-(6-(4-chloro-2-(4-((2-(dimethylamino)ethyl)(methyl)amino)phenyl)-1H-pyrrolo[2,3-b]pyridin-3-yl)indolin-1-yl)prop-2-en-1-one;
1-(6-(4-chloro-2-(4-(4-methylpiperazine-1-carbonyl)phenyl)-1H-pyrrolo[2,3-b]pyridin-3-yl)indolin-1-yl)prop-2-en-1-one;
1-(6-(4-chloro-2-(4-(2-(dimethylamino)ethoxy)phenyl)-1H-pyrrolo[2,3-b]pyridin-3-yl)indolin-1-yl)prop-2-en-1-one;
1-(6-(4-chloro-2-(4-(morpholinomethyl)phenyl)-1H-pyrrolo[2,3-b]pyridin-3-yl)indolin-1-yl)prop-2-en-1-one;
1-(6-(4-chloro-2-(3-((4-methylpiperazin-1-yl)methyl)phenyl)-1H-pyrrolo[2,3-b]pyridin-3-yl)indolin-1-yl)prop-2-en-1-one;
1-(6-(4-chloro-2-(6-(4-methylpiperazin-1-yl)pyridin-3-yl)-1H-pyrrolo[2,3-b]pyridin-3-yl)indolin-1-yl)prop-2-en-1-one;
1-(6-(4-chloro-2-(4-(3-(dimethylamino)propoxy)phenyl)-1H-pyrrolo[2,3-b]pyridin-3-yl)indolin-1-yl)prop-2-en-1-one;
N-(5-(4-chloro-2-(3-fluoro-4-(4-methylpiperazin-1-yl)phenyl)-1H-pyrrolo[2,3-b]pyridin-3-yl)-2-methylphenyl)acrylamide;
1-(6-(4-chloro-2-(4-((2-(dimethylamino)ethyl)(methyl)amino)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-(4-isopropylpiperazin-1-yl)phenyl)-1H-pyrrolo[2,3-b]pyridin-3-yl)-2H-benzo[b][1,4]oxazin-4(3H)-yl)prop-2-en-1-one;
N-(5-(4-chloro-2-(4-((2-(dimethylamino)ethyl)(methyl)amino)phenyl)-1H-pyrrolo[2,3-b]pyridin-3-yl)-2-methylphenyl)acrylamide;
N-(5-(5-cyano-2-(4-(4-methylpiperazin-1-yl)phenyl)-1H-pyrrolo[2,3-b]pyridin-3-yl)-2-methylphenyl)acrylamide;
3-(3-acrylamido-4-methylphenyl)-N-isopropyl-2-(4-(4-methylpiperazin-1-yl)phenyl)-1H-pyrrolo[2,3-b]pyridine-5-carboxamide;
1-(6-(4-chloro-2-(2-(4-methylpiperazin-1-yl)pyridin-4-yl)-1H-pyrrolo[2,3-b]pyridin-3-yl)-2H-benzo[b][1,4]oxazin-4(3H)-yl)prop-2-en-1-one;
1-(6-(4-chloro-2-(4-(4-ethylpiperazin-1-yl)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-((4-methylpiperazin-1-yl)methyl)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one;
1-(6-(4-chloro-2-(4-((dimethylamino)methyl)phenyl)-1 H-pyrrolo[2,3-b]pyridin-3-yl)-2H-benzo[b][1,4]oxazin-4(3H)-yl)prop-2-en-1-one;
1-(6-(4-chloro-2-(3-(2-(pyrrolidin-1-yl)ethoxy)phenyl)-1H-pyrrolo[2,3-b]pyridin-3-yl)-2H-benzo[b][1,4]oxazin-4(3H)-yl)prop-2-en-1-one;
Isopropyl 3-(3-acrylamido-4-methylphenyl)-2-(4-(4-methylpiperazin-1-yl)phenyl)-1H-pyrrolo[2,3-b]pyridine-5-carboxylate;
1-(6-(4-ethoxy-2-(2-(4-methylpiperazin-1-yl)pyridin-4-yl)-1H-pyrrolo[2,3-b]pyridin-3-yl)indolin-1-yl)prop-2-en-1-one;
1-(6-(2-(4-((dimethylamino)methyl)phenyl)-4-ethoxy-1H-pyrrolo[2,3-b]pyridin-3-yl)indolin-1-yl)prop-2-en-1-one;
1-(6-(4-ethoxy-2-(4-(2-(pyrrolidin-1-yl)ethoxy)phenyl)-1H-pyrrolo[2,3-b]pyridin-3-yl)indolin-1-yl)prop-2-en-1-one;
1-(6-(4-ethoxy-2-(4-((4-methylpiperazin-1-yl)methyl)phenyl)-1H-pyrrolo[2,3-b]pyridin-3-yl)indolin-1-yl)prop-2-en-1-one;
1 -(6-(4-ethoxy-2-(4-(4-methylpiperazin-1-yl)phenyl)-1H-pyrrolo[2,3-b]pyridin-3-yl)indolin-1-yl)prop-2-en-1-one;
1-(6-(4-ethoxy-2-(3-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-cyclohexyl-2-(4-(4-methylpiperazin-1-yl)phenyl)-1H-pyrrolo[2,3-b]pyridine-5-carboxamide;
1-(6-(4-methoxy-2-(4-(4-methylpiperazin-1-yl)phenyl)-1H-pyrrolo[2,3-b]pyridin-3-yl)indolin-1-yl)prop-2-en-1-one;
1-(6-(4-methoxy-2-(2-(4-methylpiperazin-1-yl)pyridin-4-yl)-1H-pyrrolo[2,3-b]pyridin-3-yl)indolin-1-yl)prop-2-en-1-one;
1-(6-(4-methoxy-2-(4-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-benzyl-2-(4-(4-methylpiperazin-1-yl)phenyl)-1H-pyrrolo[2,3-b]pyridine-5-carboxamide;
N-(3-acetamidobenzyl)-3-(3-acrylamido-4-methylphenyl)-2-(4-(4-methylpiperazin-1-yl)phenyl)-1H-pyrrolo[2,3-b]pyridine-5-carboxamide; 1-(6-(4-isopropoxy-2-(4-(4-methylpiperazin-1-yl)phenyl)-1H-pyrrolo[2,3-b]pyridin-3-yl)indolin-1-yl)prop-2-en-1-one;
1-(6-(4-methoxy-2-(4-((4-methylpiperazin-1-yl)methyl)phenyl)-1H-pyrrolo[2,3-b]pyridin-3-yl)indolin-1-yl)prop-2-en-1-one;
1-(6-(4-methoxy-2-(3-(2-(pyrrolidin-1-yl)ethoxy)phenyl)-1H-pyrrolo[2,3-b]pyridin-3-yl)indolin-1-yl)prop-2-en-1-one;
3-(3-acrylamido-4-methylphenyl)-N-(4-((4-methylpiperazin-1-yl)methyl)benzyl)-2-(4-(4-methylpiperazin-1-yl)phenyl)-1H-pyrrolo[2,3-b]pyridine-5-carboxamide;
3-(3-acrylamido-4-methylphenyl)-2-(4-(4-methylpiperazin-1-yl)phenyl)-N-(2-(pyridin-4-yl)ethyl)-1H-pyrrolo[2,3-b]pyridine-5-carboxamide;
3-(3-acrylamido-4-methylphenyl)-N-(4-fluorophenethyl)-2-(4-(4-methylpiperazin-1-yl)phenyl)-1H-pyrrolo[2,3-b]pyridine-5-carboxamide;
N-((1H-benzo[d]imidazol-2-yl)methyl)-3-(3-acrylamido-4-methylphenyl)-2-(4-(4-methylpiperazin-1-yl)phenyl)-1H-pyrrolo[2,3-b]pyridine-5-carboxamide;
N-(2-methyl-5-(2-(2-morpholinopyridin-4-yl)-1H-pyrrolo[2,3-b]pyridin-3-yl)phenyl)acrylamide;
N-(2-methyl-5-(2-(6-(4-methylpiperazin-1-yl)pyridin-3-yl)-1H-pyrrolo[2,3-b]pyridin-3-yl)phenyl)acrylamide;
3-(3-acrylamido-4-methylphenyl)-2-(4-(4-methylpiperazin-1-yl)phenyl)-N-(3-sulfamoylbenzyl)-1H-pyrrolo[2,3-b]pyridine-5-carboxamide;
3-(3-acrylamido-4-methylphenyl)-2-(4-(4-methylpiperazin-1-yl)phenyl)-N-(pyridin-4-ylmethyl)-1H-pyrrolo[2,3-b]pyridine-5-carboxamide;
3-(3-acrylamido-4-methylphenyl)-2-(4-(4-methylpiperazin-1-yl)phenyl)-N-(pyridin-3-ylmethyl)-1H-pyrrolo[2,3-b]pyridine-5-carboxamide;
3-(3-acrylamido-4-methylphenyl)-2-(4-(4-methylpiperazin-1-yl)phenyl)-N-((1-methylpiperidin-4-yl)methyl)-1H-pyrrolo[2,3-b]pyridine-5-carboxamide;
3-(3-acrylamido-4-methylphenyl)-N-((4-methyl-4H-1,2,4-triazol-3-yl)methyl)-2-(4-(4-methylpiperazin-1-yl)phenyl)-1H-pyrrolo[2,3-b]pyridine-5-carboxamide;
3-(3-acrylamido-4-methylphenyl)-N-(2,4-difluorobenzyl)-2-(4-(4-methylpiperazin-1-yl)phenyl)-1H-pyrrolo[2,3-b]pyridine-5-carboxamide;
N-(4-acetamidobenzyl)-3-(3-acrylamido-4-methylphenyl)-2-(4-(4-methylpiperazin-1-yl)phenyl)-1H-pyrrolo[2,3-b]pyridine-5-carboxamide;
3-(3-acrylamido-4-methylphenyl)-N-(3-carbamoylbenzyl)-2-(4-(4-methylpiperazin-1-yl)phenyl)-1H-pyrrolo[2,3-b]pyridine-5-carboxamide;
3-(3-acrylamido-4-methylphenyl)-2-(4-(4-methylpiperazin-1-yl)phenyl)-1H-pyrrolo[2,3-b]pyridine-5-carboxylic acid;
2-Chloro-N-(5-(4-chloro-2-(4-(4-methylpiperazin-1-yl)phenyl)-1H-pyrrolo[2,3-b]pyridin-3-yl)-2-methylphenyl)acetamide;
(E)-N-(5-(4-chloro-2-(4-(4-methylpiperazin-1-yl)phenyl)-1H-pyrrolo[2,3-b]pyridin-3-yl)-2-methylphenyl)but-2-enamide;
N-(5-(4-chloro-2-(4-(4-methylpiperazin-1-yl)phenyl)-1H-pyrrolo[2,3-b]pyridin-3-yl)-2-methylphenyl)ethenesulfonamide;
(E)-N-(5-(4-chloro-2-(4-(4-methylpiperazin-1-yl)phenyl)-1H-pyrrolo[2,3-b]pyridin-3-yl)-2-methylphenyl)-4-(dimethylamino)but-2-enamide;
Methyl 3-(3-acrylamido-4-methylphenyl)-2-(4-(4-methylpiperazin-1-yl)phenyl)-1H-pyrrolo[2,3-b]pyridine-5-carboxylate;
Ethyl 3-(3-acrylamido-4-methylphenyl)-2-(4-(4-methylpiperazin-1-yl)phenyl)-1H-pyrrolo[2,3-b]pyridine-5-carboxylate; cyclobutyl 3-(3-acrylamido-4-methylphenyl)-2-(4-(4-methylpiperazin-1-yl)phenyl)-1H-pyrrolo[2,3-b]pyridine-5-carboxylate;
Cyclohexyl 3-(3-acrylamido-4-methylphenyl)-2-(4-(4-methylpiperazin-1-yl)phenyl)-1H-pyrrolo[2,3-b]pyridine-5-carboxylate;
Cyclopropyl 3-(3-acrylamido-4-methylphenyl)-2-(4-(4-methylpiperazin-1-yl)phenyl)-1H-pyrrolo[2,3-b]pyridine-5-carboxylate;
Cyclopentyl 3-(3-acrylamido-4-methylphenyl)-2-(4-(4-methylpiperazin-1-yl)phenyl)-1H-pyrrolo[2,3-b]pyridine-5-carboxylate;
2-methoxyethyl 3-(3-acrylamido-4-methylphenyl)-2-(4-(4-methylpiperazin-1-yl)phenyl)-1H-pyrrolo[2,3-b]pyridine-5-carboxylate; and N-(5-(4-chloro-2-(4-(4-methylpiperazin-1-yl)phenyl)-1H-pyrrolo[2,3-b]pyridin-3-yl)-2-methylphenyl)propiolamide,
or an enantiomer, a mixture of enantiomers, a diastereomer, a mixture of diastereomers, a tautomer, a hydrate, a solvate of the above mentioned compounds, or pharmaceutically acceptable salts thereof.

7. A compound according to any one of claims 1 - 6 as selective inhibitor of Exon 20 mutations of EGFR and Her2.

8. A compound according to any one of claims 1 - 6 for use as a medicament.

9. A compound according to any one of claims 1 - 6 for use in the treatment of cancer or for use in the treatment of cancer, wherein the cancer has an activating mutation of a receptor belonging to an ErbB family of receptors.

10. The compound for use according to claim 9, wherein the activating mutation of the receptor is an insertion within exon 20 of epidermal growth factor receptor (EGFR) or within exon 20 of human epidermal growth factor receptor (HER) or wherein the activating mutation of the receptor is selected from the group consisting of Her2 A775_G776insYVMA, EGFR D770_N771insSVD, EGFR H773_V774insNPH, EGFR V769_D770insASV, EGFR P772_H773insPR, EGFR T790M and EGFR T790ML858R..

11. The compound for use according to claim 9, wherein the cancer is selected from the group consisting of breast cancer, colon cancer, prostate cancer, lung cancer, gastric cancer, ovarian cancer, renal cancer, hepatocellular cancer, thyroid cancer, uterine cancer, esophagus cancer, squamous cell cancer, leukemia, lymphoma, osteosarcoma, melanoma, glioblastoma and neuroblastoma.

12. The compound for use according to any one of claims 9 - 11, wherein the cancer is non-small cell lung cancer or mamma carcinoma.

13. A compound according to claim 1 - 12 in combination with at least one anticancer drug for use in treatment of cancer.

14. A method for producing a compound of the formula **(Ia-1),** comprising:
**Step A1:** perfoming a first cross coupling reaction of pyridine compound 1* with alkyne comopund **2a*** to obtain a compound **3*** in the presence of a first palladium catalyst, and a first base;
**Step B1:** converting a trimethylsilyl group of the compound **3*** to halide like an iodide -to obtain a compound **4*;**
**Step C1:** perfoming a second cross coupling reaction of **4*** with a compound **5*** in the presence of a second palladium catalyst, and a second base to obtain a compound **6*;**
**Step D1:** reducing nitro (NO₂) group of the compound **6*** to a primary amine (NH₂) group to obtain a compound **7*;** and
**Step E1:** perfoming a coupling reaction of the compound **7*** with a compound HO-**R₅** or AG-**R₅** to obtain a product compound of the formula **(Ia-1);**
a method for producing a compound of the formula **(Ia-1),** comprising:
**Step A1:** perfoming a first cross coupling reaction of pyridine compound **1*** with alkyne comopund **2a*** to obtain a compound **3*** in the presence of a first palladium catalyst, and a first base;
**Step D2:** reducing nitro (NO₂) group of the compound **3*** to a primary amine (NH₂) group to obtain a compound **10*;**
**Step E2:** perfoming a coupling reaction of the compound **10*** with a compound HO-**R₅** or AG-**R₅** to obtain a compound **11*.**
**Step B2:** converting a trimethylsilyl group of the compound **11*** to a halide like an iodide to obtain a compound **12*;** and
**Step C2:** perfoming a second cross coupling reaction of the compound **12*** with a compound **5*** in the presence of a second palladium catalyst, and a second base to obtain a product compound of the formula **(Ia-1);**
a method for producing a compound of the formula **(Ib),** comprising:
**Step A3:**
i) perfoming a first cross coupling reaction of pyridine compound **1*** with alkyne comopund **2b*** in the presence of a first palladium catalyst, and a first base; and
ii) removing a protecting group PG of a resulting comopund after the step i) to obtain a compound **3b*;**
**Step E3:** perfoming a coupling reaction of the compound **3b*** with a compound HO-**R₅** or AG-**R₅** to obtain a compound **11b*;**
**Step B3:** converting a trimethylsilyl group of the compound **11b*** to a halide like iodide to obtain a compound **12b*;** and
**Step C3:** perfoming a second cross coupling reaction of the compound **12b*** with a compound **5*** in the presence of a second palladium catalyst, and a second base to obtain a product compound of the formula **(Ib).**

15. An intermediate compound selected from the group consisting of the compounds **3*, 3b*, 4*, 6*, 7*, 10*, 11*, 11b*, 12*** and **12b*:**


SEQUENCE LISTING

<110>  Lead Discovery Center GmbH

<120>  4-Substituted pyrrolo[2,3-b]pyridine as ErbB modulators useful
       for treating cancer

<130>  LDC-P03722EP

<160>  7

<170>  PatentIn version 3.5

<210>  1
<211>  345
<212>  DNA
<213>  Artificial Sequence

<220>
<223>  EGFR p.D770_N771insSVD mutation in BaF3

<400>  1
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggactca     180

gtagacaacc cccacgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                     345


<210>  2
<211>  345
<212>  DNA
<213>  Artificial Sequence

<220>
<223>  EGFR p.H773_V774insNPH mutation in BaF3

<400>  2
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggacaac     180

ccccacaatc cacatgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                     345


<210>  3
<211>  345
<212>  DNA
<213>  Artificial Sequence

<220>

<223>  EGFR p.V769_D770insASV mutation in BaF3

<400>  3
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggcctca     180

gtcgacaacc cccacgtgtg ccgcctgctg ggcatctgcc tcacctccac cgtgcagctc     240

atcacgcagc tcatgccctt cggctgcctc ctggactatg tccgggaaca caaagacaat     300

attggctccc agtacctgct caactggtgt gtgcagatcg caaag                    345


<210>  4
<211>  342
<212>  DNA
<213>  Artificial Sequence

<220>
<223>  EGFR p.P772_H773insPR mutation in BaF3

<400>  4
ttcaaaaaga tcaaagtgct gggctccggt gcgttcggca cggtgtataa gggactctgg      60

atcccagaag gtgagaaagt taaaattccc gtcgctatca aggaattaag agaagcaaca     120

tctccgaaag ccaacaagga aatcctcgat gaagcctacg tgatggccag cgtggacaac     180

cccccgcgtc acgtgtgccg cctgctgggc atctgcctca cctccaccgt gcagctcatc     240

acgcagctca tgcccttcgg ctgcctcctg gactatgtcc gggaacacaa agacaatatt     300

ggctcccagt acctgctcaa ctggtgtgtg cagatcgcaa ag                        342


<210>  5
<211>  501
<212>  DNA
<213>  Artificial Sequence

<220>
<223>  HER2 INS8 INS YVMA mutation BaF3

<400>  5
acacctagcg gagcgatgcc caaccaggcg cagatgcgga tcctgaaaga gacggagctg      60

aggaaggtga aggtgcttgg atctggcgct tttggcacag tctacaaggg catctggatc     120

cctgatgggg agaatgtgaa aattccagtg gccatcaaag tgttgaggga aaacacatcc     180

cccaaagcca acaaagaaat cttagacgaa gcatacgtga tggcttacgt gatggctggt     240

gtgggctccc catatgtctc ccgccttctg ggcatctgcc tgacatccac ggtgcagctg     300

gtgacacagc ttatgcccta tggctgcctc ttagaccatg tccgggaaaa ccgcggacgc     360

ctgggctccc aggacctgct gaactggtgt atgcagattg ccaaggggat gagctacctg     420

gaggatgtgc ggctcgtaca cagggacttg gccgctcgga acgtgctggt caagagtccc     480

aaccatgtca aaattacaga c                                              501

<210>  6
<211>  980
<212>  DNA
<213>  Artificial Sequence

<220>
<223>  EGFR T790M mutation in BaF3

<400>  6
ggtgttccgg accccacaca gattcctaca ggccctcatg atattttaaa acacagcatc      60

ctcaaccttg aggcggaggt cttcataaca aagatactat cagttcccaa actcagagat     120

caggtgactc cgactcctcc tttatccaat gtgctcctca tggccactgt tgcctgggcc     180

tctctgtcat ggggaatccc cagatgcacc caggaggggc cctctcccac tgcatctgtc     240

acttcacagc cctgcgtaaa cgtccctgtg ctaggtcttt tgcaggcaca gcttttcctc     300

catgagtacg tattttgaaa ctcaagatcg cattcatgcg tcttcacctg gaaggggtcc     360

atgtgcccct ccttctggcc accatgcgaa gccacactga cgtgcctctc cctccctcca     420

ggaagcctac gtgatggcca gcgtggacaa cccccacgtg tgccgcctgc tgggcatctg     480

cctcacctcc accgtgcagc tcatcatgca gctcatgccc ttcggctgcc tcctggacta     540

tgtccgggaa cacaaagaca atattggctc ccagtacctg ctcaactggt gtgtgcagat     600

cgcaaaggta atcagggaag ggagatacgg ggaggggaga taaggagcca ggatcctcac     660

atgcggtctg cgctcctggg atagcaagag tttgccatgg ggatatgtgt gtgcgtgcat     720

gcagcacaca cacattcctt tattttggat tcaatcaagt tgatcttctt gtgcacaaat     780

cagtgcctgt cccatctgca tgtggaaact ctcatcaatc agctaccttt gaagaatttt     840

ctctttattg agtgctcagt gtggtctgat gtctctgttc ttatttctct ggaattcttt     900

gtgaatactg tggtgatttg tagtggagaa ggaatattgc ttcccccatt caggacttga     960

taacaaggta agcaagccag                                                 980

<210>  7
<211>  980
<212>  DNA
<213>  Artificial Sequence

<220>
<223>  EGFR T790ML858R mutantion in BaF3

<400>  7
tccaaactgc acctacggat gcactgggcc aggtcttgaa ggctgtccaa cgaatgggcc      60

taagatcccg tccatcgcca ctgggatggt gggggccctc ctcttgctgc tggtggtggc     120

cctggggatc ggcctcttca tgcgaaggcg ccacatcgtt cggaagcgca cgctgcggag     180

gctgctgcag gagagggagc ttgtggagcc tcttacaccc agtggagaag ctcccaacca     240

agctctcttg aggatcttga aggaaactga attcaaaaag atcaaagtgc tgggctccgg 300

tgcgttcggc acggtgtata agggactctg gatcccagaa ggtgagaaag ttaaaattcc 360

cgtcgctatc aaggaattaa gagaagcaac atctccgaaa gccaacaagg aaatcctcga 420

tgaagcctac gtgatggcca gcgtggacaa cccccacgtg tgccgcctgc tgggcatctg 480

cctcacctcc accgtgcagc tcatcatgca gctcatgccc ttcggctgcc tcctggacta 540

tgtccgggaa cacaaagaca atattggctc ccagtacctg ctcaactggt gtgtgcagat 600

cgcaaagggc atgaactact tggaggaccg tcgcttggtg caccgcgacc tggcagccag 660

gaacgtactg gtgaaaacac cgcagcatgt caagatcaca gattttgggc gggccaaact 720

gctgggtgcg gaagagaaag aataccatgc agaaggaggc aaagtgccta tcaagtggat 780

ggcattggaa tcaattttac acagaatcta tacccaccag agtgatgtct ggagctacgg 840

ggtgaccgtt tgggagttga tgacctttgg atccaagcca tatgacggaa tccctgccag 900

cgagatctcc tccatcctgg agaaaggaga acgcctccct cagccaccca tatgtaccat 960

cgatgtctac atgatcatgg 980